# EUROPÄISCHE PATENTSCHRIFT

(11) **EP 0 669 394 B1**
(45) Veröffentlichungstag und Bekanntmachung des Hinweises auf die Patenterteilung: **26.01.2000**
(21) Anmeldenummer: 94108872.6
(22) Anmeldetag: 09.06.1994
(51) Int. Cl.: C12N 9/72, C07K 19/00, C07K 14/81, A61K 38/49, C12N 15/58, C12N 15/62

(54) **Bifunktionelle Urokinasevarianten mit verbesserten fibrinolytischen Eigenschaften und thrombinhemmender Wirkung**
Bifunctional derivatives of Urokinase with improved fibrinolytic activity and Thrombin inhibiting activity
Dérivés bifonctionels de l'urokinase ayant des propriétés améliorées fibrinolytiques et inhibant la thrombine

(30) Priorität: 15.07.1993 DE 4323754
(43) Veröffentlichungstag der Anmeldung: 30.08.1995
(73) Patentinhaber: Grünenthal GmbH, 52078 Aachen (DE)
(72) Erfinder: Steffens, Gerd Josef, Prof. Dr., D-52072 Aachen (DE); Wnendt, Stephan, Dr., D-52066 Aachen (DE); Schneider, Johannes, Dr., D-52223 Stolberg (DE); Heinzel-Wieland, Regina, Dr., D-52078 Aachen (DE); Saunders, Derek John, Dr., D-52072 Aachen (DE)

(56) Entgegenhaltungen:
- WO-A-91/01142
- WO-A-91/09125
- WO-A-92/18139
- BIOCHEMISTRY, Bd. 29, Seite 7095 MARAGANORE, J.M. ET AL. 'Design and characterization of Hirulogs: A novel class of bivalent peptide inhibitors of Thrombin'
- J. BIOL. CHEM., Bd. 268, Seite 8590 STRUBE, K.H. ET AL. 'Isolation, sequence analysis, and cloning of Haemadin'
- NATURE, Bd. 353, Seite 674 VU, T.-K. ET AL. 'Domains specifying thrombin receptor interaction'

SEQUENZPROTOKOLL

(1) ALLGEMEINE ANGABEN:

(i) ANMELDER:
(A) NAME: Gruenenthal GmbH
(B) STRASSE: Zieglerstrasse 6
(C) ORT: Aachen
(E) LAND: Deutschland
(F) POSTLEITZAHL: 52078

(ii) BEZEICHNUNG DER ERFINDUNG: Bifunktionelle Urokinasevarianten mit
verbesserten fibrinolytischen Eigenschaften und
thrombinhemmender Wirkung

(iii) ANZAHL DER SEQUENZEN: 102

(iv) COMPUTER-LESBARE FASSUNG:
(A) DATENTRÄGER: Floppy disk
(B) COMPUTER: IBM PC compatible
(C) BETRIEBSSYSTEM: PC-DOS/MS-DOS
(D) SOFTWARE: PatentIn Release #1.0, Version #1.30 (EPA)

(v) DATEN DER JETZIGER ANMELDUNG:
ANMELDENUMMER: EP 94 108 872.6-1270

(2) ANGABEN ZU SEQ ID NO: 1:

(i) SEQUENZKENNZEICHEN:
(A) LÄNGE: 1182 Basenpaare
(B) ART: Nucleotid
(C) STRANGFORM: Einzelstrang
(D) TOPOLOGIE: linear

(ii) ART DES MOLEKÜLS: Sonstige Nucleinsäure

(iii) HYPOTHETISCH: NEIN

(iv) ANTISENSE: NEIN

(ix) MERKMAL:
(A) NAME/SCHLÜSSEL: CDS
(B) LAGE:1..1182
(D) SONSTIGE ANGABEN:/function= "N-term. Methionin wird
in E.coli proteolytisch abgespalten"
/product= "Synthetisches Gen fuer das Protein M24
(pSJ101)"

(xi) SEQUENZBESCHREIBUNG: SEQ ID NO: 1:

ATG AGC AAA ACT TGC TAC GAA GGT AAC GGT CAC TTC TAC CGT GGT AAG 48
Met Ser Lys Thr Cys Tyr Glu Gly Asn Gly His Phe Tyr Arg Gly Lys
1               5                   10                  15

GCT TCT ACC GAC ACC ATG GGT CGT CCG TGC CTG CCG TGG AAC TCT GCT 96
Ala Ser Thr Asp Thr Met Gly Arg Pro Cys Leu Pro Trp Asn Ser Ala
            20                  25                  30

ACC GTT CTG CAG CAG ACC TAC CAC GCT CAC CGT TCT GAT GCA TTG CAG 144
Thr Val Leu Gln Gln Thr Tyr His Ala His Arg Ser Asp Ala Leu Gln
        35                  40                  45

CTG GGT CTG GGT AAA CAC AAC TAC TGC CGT AAC CCG GAC AAC CGT CGT 192
Leu Gly Leu Gly Lys His Asn Tyr Cys Arg Asn Pro Asp Asn Arg Arg
    50                  55                  60

CGT CCG TGG TGC TAC GTT CAG GTT GGT CTG AAA CCG CTA GTT CAG GAA 240
Arg Pro Trp Cys Tyr Val Gln Val Gly Leu Lys Pro Leu Val Gln Glu
65                  70                  75                  80

TGC ATG GTT CAC GAC TGC GCT GAC GGT AAA AAA CCG TCT TCT CCG CCG 288
Cys Met Val His Asp Cys Ala Asp Gly Lys Lys Pro Ser Ser Pro Pro
                85                  90                  95

GAA GAG CTC AAA TTC CAG TGC GGT CAA AAA ACC CTA CGT CCG CGT TTT 336
Glu Glu Leu Lys Phe Gln Cys Gly Gln Lys Thr Leu Arg Pro Arg Phe
            100                 105                 110

AAA ATC ATC GGT GGT GAG TTC ACC ACC ATC GAA AAC CAG CCG TGG TTC 384
Lys Ile Ile Gly Gly Glu Phe Thr Thr Ile Glu Asn Gln Pro Trp Phe
        115                 120                 125

GCT GCT ATC TAC CGT CGT CAC CGT GGT GGT TCT GTT ACC TAC GTT TGC 432
Ala Ala Ile Tyr Arg Arg His Arg Gly Gly Ser Val Thr Tyr Val Cys
    130                 135                 140

GGT GGT TCT CTG ATC TCT CCG TGC TGG GTT ATC TCT GCT ACC CAC TGC 480
Gly Gly Ser Leu Ile Ser Pro Cys Trp Val Ile Ser Ala Thr His Cys
145                 150                 155                 160

TTC ATC GAC TAC CCG AAA AAA GAA GAC TAC ATC GTT TAC CTC GGC CGT 528
Phe Ile Asp Tyr Pro Lys Lys Glu Asp Tyr Ile Val Tyr Leu Gly Arg
                165                 170                 175

TCT CGT TTA AAC TCT AAC ACC CAG GGT GAA ATG AAA TTC GAA GTT GAA 576
Ser Arg Leu Asn Ser Asn Thr Gln Gly Glu Met Lys Phe Glu Val Glu
            180                 185                 190

AAC CTG ATC CTG CAC AAA GAC TAC TCT GCT GAC ACC CTG GCT CAC CAC 624
Asn Leu Ile Leu His Lys Asp Tyr Ser Ala Asp Thr Leu Ala His His
        195                 200                 205

AAC GAC ATC GCT CTG CTA AAA ATC CGT TCT AAA GAA GGT CGT TGC GCT 672
Asn Asp Ile Ala Leu Leu Lys Ile Arg Ser Lys Glu Gly Arg Cys Ala
    210                 215                 220

CAG CCG TCT CGT ACC ATC CAG ACC ATC TGC CTG CCG TCT ATG TAC AAC 720
Gln Pro Ser Arg Thr Ile Gln Thr Ile Cys Leu Pro Ser Met Tyr Asn
225                 230                 235                 240

GAC CCG CAG TTC GGT ACC TCT TGC GAA ATC ACC GGT TTC GGT AAA GAA 768
Asp Pro Gln Phe Gly Thr Ser Cys Glu Ile Thr Gly Phe Gly Lys Glu
                245                 250                 255

AAC TCT ACC GAC TAC CTG TAC CCG GAA CAG CTG AAA ATG ACC GTT GTT 816
Asn Ser Thr Asp Tyr Leu Tyr Pro Glu Gln Leu Lys Met Thr Val Val
            260                 265                 270

AAA CTG ATC TCT CAC CGT GAA TGC CAG CAG CCG CAC TAC TAC GGT TCT 864
Lys Leu Ile Ser His Arg Glu Cys Gln Gln Pro His Tyr Tyr Gly Ser
        275                 280                 285

GAA GTT ACC ACC AAA ATG CTG TGC GCT GCT GAC CCG CAG TGG AAA ACC 912
Glu Val Thr Thr Lys Met Leu Cys Ala Ala Asp Pro Gln Trp Lys Thr
    290                 295                 300

GAC TCT TGC CAA GGT GAC TCT GGT GGT CCA CTA GTT TGC TCT CTC CAG 960
Asp Ser Cys Gln Gly Asp Ser Gly Gly Pro Leu Val Cys Ser Leu Gln
305                 310                 315                 320

GGT CGT ATG ACC CTG ACC GGT ATT GTT TCT TGG GGT CGT GGT TGC GCT 1008
Gly Arg Met Thr Leu Thr Gly Ile Val Ser Trp Gly Arg Gly Cys Ala
                325                 330                 335

CTG AAA GAC AAA CCG GGT GTT TAC ACC CGT GTT TCT CAC TTC CTG CCG 1056
Leu Lys Asp Lys Pro Gly Val Tyr Thr Arg Val Ser His Phe Leu Pro
            340                 345                 350

TGG ATC CGT TCT CAC ACC AAA GAA GAA AAC GGT CTG GCT CTG AGC CCG 1104
Trp Ile Arg Ser His Thr Lys Glu Glu Asn Gly Leu Ala Leu Ser Pro
        355                 360                 365

GTT AAG GCT TGG CCG CGG CCG CTG GGT GGT GGT GGT AAC GGT GAC TTC 1152
Val Lys Ala Trp Pro Arg Pro Leu Gly Gly Gly Gly Asn Gly Asp Phe
    370                 375                 380

GAA GAA ATC CCG GAA GAA TAC CTG CAA TAA 1182
Glu Glu Ile Pro Glu Glu Tyr Leu Gln *
385                 390

(2) ANGABEN ZU SEQ ID NO: 2:

(i) SEQUENZKENNZEICHEN:
(A) LÄNGE: 394 Aminosäuren
(B) ART: Aminosäure
(D) TOPOLOGIE: linear

(ii) ART DES MOLEKÜLS: Protein
(xi) SEQUENZBESCHREIBUNG: SEQ ID NO: 2:

Met Ser Lys Thr Cys Tyr Glu Gly Asn Gly His Phe Tyr Arg Gly Lys
1               5                   10                  15

Ala Ser Thr Asp Thr Met Gly Arg Pro Cys Leu Pro Trp Asn Ser Ala
            20                  25                  30

Thr Val Leu Gln Gln Thr Tyr His Ala His Arg Ser Asp Ala Leu Gln
        35                  40                  45

Leu Gly Leu Gly Lys His Asn Tyr Cys Arg Asn Pro Asp Asn Arg Arg
    50                  55                  60

Arg Pro Trp Cys Tyr Val Gln Val Gly Leu Lys Pro Leu Val Gln Glu
65                  70                  75                  80

Cys Met Val His Asp Cys Ala Asp Gly Lys Lys Pro Ser Ser Pro Pro
                85                  90                  95

Glu Glu Leu Lys Phe Gln Cys Gly Gln Lys Thr Leu Arg Pro Arg Phe
            100                 105                 110

Lys Ile Ile Gly Gly Glu Phe Thr Thr Ile Glu Asn Gln Pro Trp Phe
        115                 120                 125

Ala Ala Ile Tyr Arg Arg His Arg Gly Gly Ser Val Thr Tyr Val Cys
    130                 135                 140

Gly Gly Ser Leu Ile Ser Pro Cys Trp Val Ile Ser Ala Thr His Cys
145                 150                 155                 160

Phe Ile Asp Tyr Pro Lys Lys Glu Asp Tyr Ile Val Tyr Leu Gly Arg
                165                 170                 175

Ser Arg Leu Asn Ser Asn Thr Gln Gly Glu Met Lys Phe Glu Val Glu
            180                 185                 190

Asn Leu Ile Leu His Lys Asp Tyr Ser Ala Asp Thr Leu Ala His His
        195                 200                 205

Asn Asp Ile Ala Leu Leu Lys Ile Arg Ser Lys Glu Gly Arg Cys Ala
    210                 215                 220

Gln Pro Ser Arg Thr Ile Gln Thr Ile Cys Leu Pro Ser Met Tyr Asn
225                 230                 235                 240

Asp Pro Gln Phe Gly Thr Ser Cys Glu Ile Thr Gly Phe Gly Lys Glu
                245                 250                 255

Asn Ser Thr Asp Tyr Leu Tyr Pro Glu Gln Leu Lys Met Thr Val Val
            260                 265                 270

Lys Leu Ile Ser His Arg Glu Cys Gln Gln Pro His Tyr Tyr Gly Ser
        275                 280                 285

Glu Val Thr Thr Lys Met Leu Cys Ala Ala Asp Pro Gln Trp Lys Thr
    290                 295                 300

Asp Ser Cys Gln Gly Asp Ser Gly Gly Pro Leu Val Cys Ser Leu Gln
305                 310                 315                 320

Gly Arg Met Thr Leu Thr Gly Ile Val Ser Trp Gly Arg Gly Cys Ala
                325                 330                 335

Leu Lys Asp Lys Pro Gly Val Tyr Thr Arg Val Ser His Phe Leu Pro
            340                 345                 350

Trp Ile Arg Ser His Thr Lys Glu Glu Asn Gly Leu Ala Leu Ser Pro
        355                 360                 365

Val Lys Ala Trp Pro Arg Pro Leu Gly Gly Gly Gly Asn Gly Asp Phe
    370                 375                 380

Glu Glu Ile Pro Glu Glu Tyr Leu Gln  *
385                 390

(2) ANGABEN ZU SEQ ID NO: 3:

    (i) SEQUENZKENNZEICHEN:
        (A) LÄNGE: 1182 Basenpaare
        (B) ART: Nucleotid
        (C) STRANGFORM: Einzelstrang
        (D) TOPOLOGIE: linear

    (ii) ART DES MOLEKÜLS: Sonstige Nucleinsäure

    (iii) HYPOTHETISCH: NEIN

    (iv) ANTISENSE: NEIN

(ix) MERKMAL:
(A) NAME/SCHLÜSSEL: CDS
(B) LAGE:1..1182
(D) SONSTIGE ANGABEN:/function= "N-term. Methionin wird
in E.coli proteolytisch abgespalten"
/product= "Synthetisches Gen fuer das Protein M25
(pSJ102)"

(xi) SEQUENZBESCHREIBUNG: SEQ ID NO: 3:

ATG AGC AAA ACT TGC TAC GAA GGT AAC GGT CAC TTC TAC CGT GGT AAG 48
Met Ser Lys Thr Cys Tyr Glu Gly Asn Gly His Phe Tyr Arg Gly Lys
1               5                   10                  15

GCT TCT ACC GAC ACC ATG GGT CGT CCG TGC CTG CCG TGG AAC TCT GCT 96
Ala Ser Thr Asp Thr Met Gly Arg Pro Cys Leu Pro Trp Asn Ser Ala
            20                  25                  30

ACC GTT CTG CAG CAG ACC TAC CAC GCT CAC CGT TCT GAT GCA TTG CAG 144
Thr Val Leu Gln Gln Thr Tyr His Ala His Arg Ser Asp Ala Leu Gln
        35                  40                  45

CTG GGT CTG GGT AAA CAC AAC TAC TGC CGT AAC CCG GAC AAC CGT CGT 192
Leu Gly Leu Gly Lys His Asn Tyr Cys Arg Asn Pro Asp Asn Arg Arg
    50                  55                  60

CGT CCG TGG TGC TAC GTT CAG GTT GGT CTG AAA CCG CTA GTT CAG GAA 240
Arg Pro Trp Cys Tyr Val Gln Val Gly Leu Lys Pro Leu Val Gln Glu
65                  70                  75                  80

TGC ATG GTT CAC GAC TGC GCT GAC GGT AAA AAA CCG TCT TCT CCG CCG 288
Cys Met Val His Asp Cys Ala Asp Gly Lys Lys Pro Ser Ser Pro Pro
                85                  90                  95

GAA GAG CTC AAA TTC CAG TGC GGT CAA AAA ACC CTA CGT CCG CGT TTT 336
Glu Glu Leu Lys Phe Gln Cys Gly Gln Lys Thr Leu Arg Pro Arg Phe
            100                 105                 110

AAA ATC ATC GGT GGT GAG TTC ACC ACC ATC GAA AAC CAG CCG TGG TTC 384
Lys Ile Ile Gly Gly Glu Phe Thr Thr Ile Glu Asn Gln Pro Trp Phe
        115                 120                 125

GCT GCT ATC TAC CGT CGT CAC CGT GGT GGT TCT GTT ACC TAC GTT TGC 432
Ala Ala Ile Tyr Arg Arg His Arg Gly Gly Ser Val Thr Tyr Val Cys
    130                 135                 140

GGT GGT TCT CTG ATC TCT CCG TGC TGG GTT ATC TCT GCT ACC CAC TGC 480
Gly Gly Ser Leu Ile Ser Pro Cys Trp Val Ile Ser Ala Thr His Cys
145                 150                 155                 160

TTC ATC GAC TAC CCG AAA AAA GAA GAC TAC ATC GTT TAC CTC GGC CGT 528
Phe Ile Asp Tyr Pro Lys Lys Glu Asp Tyr Ile Val Tyr Leu Gly Arg
                165                 170                 175

TCT CGT TTA AAC TCT AAC ACC CAG GGT GAA ATG AAA TTC GAA GTT GAA 576
Ser Arg Leu Asn Ser Asn Thr Gln Gly Glu Met Lys Phe Glu Val Glu
            180                 185                 190

AAC CTG ATC CTG CAC AAA GAC TAC TCT GCT GAC ACC CTG GCT CAC CAC 624
Asn Leu Ile Leu His Lys Asp Tyr Ser Ala Asp Thr Leu Ala His His
        195                 200                 205

AAC GAC ATC GCT CTG CTA AAA ATC CGT TCT AAA GAA GGT CGT TGC GCT 672
Asn Asp Ile Ala Leu Leu Lys Ile Arg Ser Lys Glu Gly Arg Cys Ala
    210                 215                 220

CAG CCG TCT CGT ACC ATC CAG ACC ATC TGC CTG CCG TCT ATG TAC AAC 720
Gln Pro Ser Arg Thr Ile Gln Thr Ile Cys Leu Pro Ser Met Tyr Asn
225                 230                 235                 240

GAC CCG CAG TTC GGT ACC TCT TGC GAA ATC ACC GGT TTC GGT AAA GAA 768
Asp Pro Gln Phe Gly Thr Ser Cys Glu Ile Thr Gly Phe Gly Lys Glu
                245                 250                 255

AAC TCT ACC GAC TAC CTG TAC CCG GAA CAG CTG AAA ATG ACC GTT GTT 816
Asn Ser Thr Asp Tyr Leu Tyr Pro Glu Gln Leu Lys Met Thr Val Val
            260                 265                 270

AAA CTG ATC TCT CAC CGT GAA TGC CAG CAG CCG CAC TAC TAC GGT TCT 864
Lys Leu Ile Ser His Arg Glu Cys Gln Gln Pro His Tyr Tyr Gly Ser
        275                 280                 285

GAA GTT ACC ACC AAA ATG CTG TGC GCT GCT GAC CCG CAG TGG AAA ACC 912
Glu Val Thr Thr Lys Met Leu Cys Ala Ala Asp Pro Gln Trp Lys Thr
    290                 295                 300

GAC TCT TGC CAA GGT GAC TCT GGT GGT CCA CTA GTT TGC TCT CTC CAG 960
Asp Ser Cys Gln Gly Asp Ser Gly Gly Pro Leu Val Cys Ser Leu Gln
305                 310                 315                 320

GGT CGT ATG ACC CTG ACC GGT ATT GTT TCT TGG GGT CGT GGT TGC GCT 1008
Gly Arg Met Thr Leu Thr Gly Ile Val Ser Trp Gly Arg Gly Cys Ala
                325                 330                 335

CTG AAA GAC AAA CCG GGT GTT TAC ACC CGT GTT TCT CAC TTC CTG CCG 1056
Leu Lys Asp Lys Pro Gly Val Tyr Thr Arg Val Ser His Phe Leu Pro
            340                 345                 350

TGG ATC CGT TCT CAC ACC AAA GAA GAA AAC GGT CTG GCT CTG AGC CCG 1104
Trp Ile Arg Ser His Thr Lys Glu Glu Asn Gly Leu Ala Leu Ser Pro
        355                 360                 365

GTT AAG GCT TTC CCG CGG CCG CTG GGT GGT GGT GGT AAC GGT GAC TTC 1152
Val Lys Ala Phe Pro Arg Pro Leu Gly Gly Gly Gly Asn Gly Asp Phe
    370                 375                 380

GAA GAA ATC CCG GAA GAA TAC CTG CAA TAA 1182
Glu Glu Ile Pro Glu Glu Tyr Leu Gln *
385                 390

(2) ANGABEN ZU SEQ ID NO: 4:

(i) SEQUENZKENNZEICHEN:
(A) LÄNGE: 394 Aminosäuren
(B) ART: Aminosäure
(D) TOPOLOGIE: linear

(ii) ART DES MOLEKÜLS: Protein
(xi) SEQUENZBESCHREIBUNG: SEQ ID NO: 4:

Met Ser Lys Thr Cys Tyr Glu Gly Asn Gly His Phe Tyr Arg Gly Lys
1               5                   10                  15

Ala Ser Thr Asp Thr Met Gly Arg Pro Cys Leu Pro Trp Asn Ser Ala
            20                  25                  30

Thr Val Leu Gln Gln Thr Tyr His Ala His Arg Ser Asp Ala Leu Gln
        35                  40                  45

Leu Gly Leu Gly Lys His Asn Tyr Cys Arg Asn Pro Asp Asn Arg Arg
    50                  55                  60

Arg Pro Trp Cys Tyr Val Gln Val Gly Leu Lys Pro Leu Val Gln Glu
65                  70                  75                  80

Cys Met Val His Asp Cys Ala Asp Gly Lys Lys Pro Ser Ser Pro Pro
                85                  90                  95

Glu Glu Leu Lys Phe Gln Cys Gly Gln Lys Thr Leu Arg Pro Arg Phe
            100                 105                 110

Lys Ile Ile Gly Gly Glu Phe Thr Thr Ile Glu Asn Gln Pro Trp Phe
        115                 120                 125

Ala Ala Ile Tyr Arg Arg His Arg Gly Gly Ser Val Thr Tyr Val Cys
    130                 135                 140

Gly Gly Ser Leu Ile Ser Pro Cys Trp Val Ile Ser Ala Thr His Cys
145                 150                 155                 160

Phe Ile Asp Tyr Pro Lys Lys Glu Asp Tyr Ile Val Tyr Leu Gly Arg
                165                 170                 175

Ser Arg Leu Asn Ser Asn Thr Gln Gly Glu Met Lys Phe Glu Val Glu
            180                 185                 190

Asn Leu Ile Leu His Lys Asp Tyr Ser Ala Asp Thr Leu Ala His His
        195                 200                 205

Asn Asp Ile Ala Leu Leu Lys Ile Arg Ser Lys Glu Gly Arg Cys Ala
    210                 215                 220

Gln Pro Ser Arg Thr Ile Gln Thr Ile Cys Leu Pro Ser Met Tyr Asn
225                 230                 235                 240

Asp Pro Gln Phe Gly Thr Ser Cys Glu Ile Thr Gly Phe Gly Lys Glu
                245                 250                 255

Asn Ser Thr Asp Tyr Leu Tyr Pro Glu Gln Leu Lys Met Thr Val Val
            260                 265                 270

Lys Leu Ile Ser His Arg Glu Cys Gln Gln Pro His Tyr Tyr Gly Ser
        275                 280                 285

Glu Val Thr Thr Lys Met Leu Cys Ala Ala Asp Pro Gln Trp Lys Thr
    290                 295                 300

Asp Ser Cys Gln Gly Asp Ser Gly Gly Pro Leu Val Cys Ser Leu Gln
305                 310                 315                 320

Gly Arg Met Thr Leu Thr Gly Ile Val Ser Trp Gly Arg Gly Cys Ala
                325                 330                 335

Leu Lys Asp Lys Pro Gly Val Tyr Thr Arg Val Ser His Phe Leu Pro
            340                 345                 350

Trp Ile Arg Ser His Thr Lys Glu Glu Asn Gly Leu Ala Leu Ser Pro
        355                 360                 365

Val Lys Ala Phe Pro Arg Pro Leu Gly Gly Gly Gly Asn Gly Asp Phe
    370                 375                 380

Glu Glu Ile Pro Glu Glu Tyr Leu Gln  *
385                 390

(2) ANGABEN ZU SEQ ID NO: 5:

     (i) SEQUENZKENNZEICHEN:
          (A) LÄNGE: 1182 Basenpaare
          (B) ART: Nucleotid
          (C) STRANGFORM: Einzelstrang
          (D) TOPOLOGIE: linear

    (ii) ART DES MOLEKÜLS: Sonstige Nucleinsäure

   (iii) HYPOTHETISCH: NEIN

    (iv) ANTISENSE: NEIN

    (ix) MERKMAL:
          (A) NAME/SCHLÜSSEL: CDS
          (B) LAGE:1..1182
          (D) SONSTIGE ANGABEN:/function= "N-term. Methionin wird
               in E.coli proteolytisch abgespalten"
               /product= "Synthetisches Gen fuer das Protein M26
               (pSJ103)"

    (xi) SEQUENZBESCHREIBUNG: SEQ ID NO: 5:

ATG AGC AAA ACT TGC TAC GAA GGT AAC GGT CAC TTC TAC CGT GGT AAG        48
Met Ser Lys Thr Cys Tyr Glu Gly Asn Gly His Phe Tyr Arg Gly Lys
  1               5                  10                  15

GCT TCT ACC GAC ACC ATG GGT CGT CCG TGC CTG CCG TGG AAC TCT GCT        96
Ala Ser Thr Asp Thr Met Gly Arg Pro Cys Leu Pro Trp Asn Ser Ala
             20                  25                  30

ACC GTT CTG CAG CAG ACC TAC CAC GCT CAC CGT TCT GAT GCA TTG CAG       144
Thr Val Leu Gln Gln Thr Tyr His Ala His Arg Ser Asp Ala Leu Gln
         35                  40                  45

CTG GGT CTG GGT AAA CAC AAC TAC TGC CGT AAC CCG GAC AAC CGT CGT       192
Leu Gly Leu Gly Lys His Asn Tyr Cys Arg Asn Pro Asp Asn Arg Arg
     50                  55                  60

CGT CCG TGG TGC TAC GTT CAG GTT GGT CTG AAA CCG CTA GTT CAG GAA       240
Arg Pro Trp Cys Tyr Val Gln Val Gly Leu Lys Pro Leu Val Gln Glu
 65                  70                  75                  80

TGC ATG GTT CAC GAC TGC GCT GAC GGT AAA AAA CCG TCT TCT CCG CCG       288
Cys Met Val His Asp Cys Ala Asp Gly Lys Lys Pro Ser Ser Pro Pro
                 85                  90                  95

GAA GAG CTC AAA TTC CAG TGC GGT CAA AAA ACC CTA CGT CCG CGT TTT       336
Glu Glu Leu Lys Phe Gln Cys Gly Gln Lys Thr Leu Arg Pro Arg Phe
            100                 105                 110

AAA ATC ATC GGT GGT GAG TTC ACC ACC ATC GAA AAC CAG CCG TGG TTC       384
Lys Ile Ile Gly Gly Glu Phe Thr Thr Ile Glu Asn Gln Pro Trp Phe
        115                 120                 125

GCT GCT ATC TAC CGT CGT CAC CGT GGT GGT TCT GTT ACC TAC GTT TGC 432
Ala Ala Ile Tyr Arg Arg His Arg Gly Gly Ser Val Thr Tyr Val Cys
    130                 135                 140

GGT GGT TCT CTG ATC TCT CCG TGC TGG GTT ATC TCT GCT ACC CAC TGC 480
Gly Gly Ser Leu Ile Ser Pro Cys Trp Val Ile Ser Ala Thr His Cys
145                 150                 155                 160

TTC ATC GAC TAC CCG AAA AAA GAA GAC TAC ATC GTT TAC CTC GGC CGT 528
Phe Ile Asp Tyr Pro Lys Lys Glu Asp Tyr Ile Val Tyr Leu Gly Arg
                165                 170                 175

TCT CGT TTA AAC TCT AAC ACC CAG GGT GAA ATG AAA TTC GAA GTT GAA 576
Ser Arg Leu Asn Ser Asn Thr Gln Gly Glu Met Lys Phe Glu Val Glu
            180                 185                 190

AAC CTG ATC CTG CAC AAA GAC TAC TCT GCT GAC ACC CTG GCT CAC CAC 624
Asn Leu Ile Leu His Lys Asp Tyr Ser Ala Asp Thr Leu Ala His His
        195                 200                 205

AAC GAC ATC GCT CTG CTA AAA ATC CGT TCT AAA GAA GGT CGT TGC GCT 672
Asn Asp Ile Ala Leu Leu Lys Ile Arg Ser Lys Glu Gly Arg Cys Ala
    210                 215                 220

CAG CCG TCT CGT ACC ATC CAG ACC ATC TGC CTG CCG TCT ATG TAC AAC 720
Gln Pro Ser Arg Thr Ile Gln Thr Ile Cys Leu Pro Ser Met Tyr Asn
225                 230                 235                 240

GAC CCG CAG TTC GGT ACC TCT TGC GAA ATC ACC GGT TTC GGT AAA GAA 768
Asp Pro Gln Phe Gly Thr Ser Cys Glu Ile Thr Gly Phe Gly Lys Glu
                245                 250                 255

AAC TCT ACC GAC TAC CTG TAC CCG GAA CAG CTG AAA ATG ACC GTT GTT 816
Asn Ser Thr Asp Tyr Leu Tyr Pro Glu Gln Leu Lys Met Thr Val Val
            260                 265                 270

AAA CTG ATC TCT CAC CGT GAA TGC CAG CAG CCG CAC TAC TAC GGT TCT 864
Lys Leu Ile Ser His Arg Glu Cys Gln Gln Pro His Tyr Tyr Gly Ser
        275                 280                 285

GAA GTT ACC ACC AAA ATG CTG TGC GCT GCT GAC CCG CAG TGG AAA ACC 912
Glu Val Thr Thr Lys Met Leu Cys Ala Ala Asp Pro Gln Trp Lys Thr
    290                 295                 300

GAC TCT TGC CAA GGT GAC TCT GGT GGT CCA CTA GTT TGC TCT CTC CAG 960
Asp Ser Cys Gln Gly Asp Ser Gly Gly Pro Leu Val Cys Ser Leu Gln
305                 310                 315                 320

GGT CGT ATG ACC CTG ACC GGT ATT GTT TCT TGG GGT CGT GGT TGC GCT 1008
Gly Arg Met Thr Leu Thr Gly Ile Val Ser Trp Gly Arg Gly Cys Ala
                325                 330                 335

CTG AAA GAC AAA CCG GGT GTT TAC ACC CGT GTT TCT CAC TTC CTG CCG 1056
Leu Lys Asp Lys Pro Gly Val Tyr Thr Arg Val Ser His Phe Leu Pro
            340                 345                 350

TGG ATC CGT TCT CAC ACC AAA GAA GAA AAC GGT CTG GCT CTG AGC CCG 1104
Trp Ile Arg Ser His Thr Lys Glu Glu Asn Gly Leu Ala Leu Ser Pro
        355                 360                 365

GTT GTA GTT TTC CCG CGG CCG CTG GGT GGT GGT GGT AAC GGT GAC TTC 1152
Val Val Val Phe Pro Arg Pro Leu Gly Gly Gly Gly Asn Gly Asp Phe
    370                 375                 380

GAA GAA ATC CCG GAA GAA TAC CTG CAA TAA 1182
Glu Glu Ile Pro Glu Glu Tyr Leu Gln *
385                 390

(2) ANGABEN ZU SEQ ID NO: 6:

(i) SEQUENZKENNZEICHEN:
(A) LÄNGE: 394 Aminosäuren
(B) ART: Aminosäure
(D) TOPOLOGIE: linear

(ii) ART DES MOLEKÜLS: Protein
(xi) SEQUENZBESCHREIBUNG: SEQ ID NO: 6:

Met Ser Lys Thr Cys Tyr Glu Gly Asn Gly His Phe Tyr Arg Gly Lys
1               5                   10                  15

Ala Ser Thr Asp Thr Met Gly Arg Pro Cys Leu Pro Trp Asn Ser Ala
            20                  25                  30

Thr Val Leu Gln Gln Thr Tyr His Ala His Arg Ser Asp Ala Leu Gln
        35                  40                  45

Leu Gly Leu Gly Lys His Asn Tyr Cys Arg Asn Pro Asp Asn Arg Arg
    50                  55                  60

Arg Pro Trp Cys Tyr Val Gln Val Gly Leu Lys Pro Leu Val Gln Glu
65                  70                  75                  80

Cys Met Val His Asp Cys Ala Asp Gly Lys Lys Pro Ser Ser Pro Pro
                85                  90                  95

Glu Glu Leu Lys Phe Gln Cys Gly Gln Lys Thr Leu Arg Pro Arg Phe
            100                 105                 110

Lys Ile Ile Gly Gly Glu Phe Thr Thr Ile Glu Asn Gln Pro Trp Phe
        115                 120                 125

Ala Ala Ile Tyr Arg Arg His Arg Gly Gly Ser Val Thr Tyr Val Cys
    130                 135                 140

Gly Gly Ser Leu Ile Ser Pro Cys Trp Val Ile Ser Ala Thr His Cys
145                 150                 155                 160

Phe Ile Asp Tyr Pro Lys Lys Glu Asp Tyr Ile Val Tyr Leu Gly Arg
                165                 170                 175

Ser Arg Leu Asn Ser Asn Thr Gln Gly Glu Met Lys Phe Glu Val Glu
            180                 185                 190

Asn Leu Ile Leu His Lys Asp Tyr Ser Ala Asp Thr Leu Ala His His
        195                 200                 205

Asn Asp Ile Ala Leu Leu Lys Ile Arg Ser Lys Glu Gly Arg Cys Ala
    210                 215                 220

Gln Pro Ser Arg Thr Ile Gln Thr Ile Cys Leu Pro Ser Met Tyr Asn
225                 230                 235                 240

Asp Pro Gln Phe Gly Thr Ser Cys Glu Ile Thr Gly Phe Gly Lys Glu
                245                 250                 255

Asn Ser Thr Asp Tyr Leu Tyr Pro Glu Gln Leu Lys Met Thr Val Val
            260                 265                 270

Lys Leu Ile Ser His Arg Glu Cys Gln Gln Pro His Tyr Tyr Gly Ser
        275                 280                 285

Glu Val Thr Thr Lys Met Leu Cys Ala Ala Asp Pro Gln Trp Lys Thr
    290                 295                 300

Asp Ser Cys Gln Gly Asp Ser Gly Gly Pro Leu Val Cys Ser Leu Gln
305                 310                 315                 320

Gly Arg Met Thr Leu Thr Gly Ile Val Ser Trp Gly Arg Gly Cys Ala
                325                 330                 335

Leu Lys Asp Lys Pro Gly Val Tyr Thr Arg Val Ser His Phe Leu Pro
            340                 345                 350

Trp Ile Arg Ser His Thr Lys Glu Glu Asn Gly Leu Ala Leu Ser Pro
        355                 360                 365

Val Val Val Phe Pro Arg Pro Leu Gly Gly Gly Gly Asn Gly Asp Phe
    370                 375                 380

Glu Glu Ile Pro Glu Glu Tyr Leu Gln *
385                 390

(2) ANGABEN ZU SEQ ID NO: 7:

(i) SEQUENZKENNZEICHEN:
(A) LÄNGE: 1182 Basenpaare
(B) ART: Nucleotid
(C) STRANGFORM: Einzelstrang
(D) TOPOLOGIE: linear

(ii) ART DES MOLEKÜLS: Sonstige Nucleinsäure

(iii) HYPOTHETISCH: NEIN

(iv) ANTISENSE: NEIN

(ix) MERKMAL:
(A) NAME/SCHLÜSSEL: CDS
(B) LAGE:1..1182
(D) SONSTIGE ANGABEN:/function= "N-term. Methionin wird
in E.coli proteolytisch abgespalten"
/product= "Synthetisches Gen fuer das Protein M27
(pSJ104)"

(ix) MERKMAL:
(A) NAME/SCHLÜSSEL: CDS
(B) LAGE:1..1182

(xi) SEQUENZBESCHREIBUNG: SEQ ID NO: 7:

ATG AGC AAA ACT TGC TAC GAA GGT AAC GGT CAC TTC TAC CGT GGT AAG 48
Met Ser Lys Thr Cys Tyr Glu Gly Asn Gly His Phe Tyr Arg Gly Lys
1               5                   10                  15

GCT TCT ACC GAC ACC ATG GGT CGT CCG TGC CTG CCG TGG AAC TCT GCT 96
Ala Ser Thr Asp Thr Met Gly Arg Pro Cys Leu Pro Trp Asn Ser Ala
            20                  25                  30

ACC GTT CTG CAG CAG ACC TAC CAC GCT CAC CGT TCT GAT GCA TTG CAG 144
Thr Val Leu Gln Gln Thr Tyr His Ala His Arg Ser Asp Ala Leu Gln
        35                  40                  45

CTG GGT CTG GGT AAA CAC AAC TAC TGC CGT AAC CCG GAC AAC CGT CGT 192
Leu Gly Leu Gly Lys His Asn Tyr Cys Arg Asn Pro Asp Asn Arg Arg
    50                  55                  60

CGT CCG TGG TGC TAC GTT CAG GTT GGT CTG AAA CCG CTA GTT CAG GAA 240
Arg Pro Trp Cys Tyr Val Gln Val Gly Leu Lys Pro Leu Val Gln Glu
65                  70                  75                  80

TGC ATG GTT CAC GAC TGC GCT GAC GGT AAA AAA CCG TCT TCT CCG CCG 288
Cys Met Val His Asp Cys Ala Asp Gly Lys Lys Pro Ser Ser Pro Pro
                85                  90                  95

GAA GAG CTC AAA TTC CAG TGC GGT CAA AAA ACC CTA CGT CCG CGT TTT 336
Glu Glu Leu Lys Phe Gln Cys Gly Gln Lys Thr Leu Arg Pro Arg Phe
            100                 105                 110

AAA ATC ATC GGT GGT GAG TTC ACC ACC ATC GAA AAC CAG CCG TGG TTC 384
Lys Ile Ile Gly Gly Glu Phe Thr Thr Ile Glu Asn Gln Pro Trp Phe
        115                 120                 125

GCT GCT ATC TAC CGT CGT CAC CGT GGT GGT TCT GTT ACC TAC GTT TGC 432
Ala Ala Ile Tyr Arg Arg His Arg Gly Gly Ser Val Thr Tyr Val Cys
    130                 135                 140

GGT GGT TCT CTG ATC TCT CCG TGC TGG GTT ATC TCT GCT ACC CAC TGC 480
Gly Gly Ser Leu Ile Ser Pro Cys Trp Val Ile Ser Ala Thr His Cys
145                 150                 155                 160

TTC ATC GAC TAC CCG AAA AAA GAA GAC TAC ATC GTT TAC CTC GGC CGT 528
Phe Ile Asp Tyr Pro Lys Lys Glu Asp Tyr Ile Val Tyr Leu Gly Arg
                165                 170                 175

TCT CGT TTA AAC TCT AAC ACC CAG GGT GAA ATG AAA TTC GAA GTT GAA 576
Ser Arg Leu Asn Ser Asn Thr Gln Gly Glu Met Lys Phe Glu Val Glu
            180                 185                 190

AAC CTG ATC CTG CAC AAA GAC TAC TCT GCT GAC ACC CTG GCT CAC CAC 624
Asn Leu Ile Leu His Lys Asp Tyr Ser Ala Asp Thr Leu Ala His His
        195                 200                 205

AAC GAC ATC GCT CTG CTA AAA ATC CGT TCT AAA GAA GGT CGT TGC GCT 672
Asn Asp Ile Ala Leu Leu Lys Ile Arg Ser Lys Glu Gly Arg Cys Ala
    210                 215                 220

CAG CCG TCT CGT ACC ATC CAG ACC ATC TGC CTG CCG TCT ATG TAC AAC 720
Gln Pro Ser Arg Thr Ile Gln Thr Ile Cys Leu Pro Ser Met Tyr Asn
225                 230                 235                 240

GAC CCG CAG TTC GGT ACC TCT TGC GAA ATC ACC GGT TTC GGT AAA GAA 768
Asp Pro Gln Phe Gly Thr Ser Cys Glu Ile Thr Gly Phe Gly Lys Glu
                245                 250                 255

AAC TCT ACC GAC TAC CTG TAC CCG GAA CAG CTG AAA ATG ACC GTT GTT 816
Asn Ser Thr Asp Tyr Leu Tyr Pro Glu Gln Leu Lys Met Thr Val Val
            260                 265                 270

AAA CTG ATC TCT CAC CGT GAA TGC CAG CAG CCG CAC TAC TAC GGT TCT 864
Lys Leu Ile Ser His Arg Glu Cys Gln Gln Pro His Tyr Tyr Gly Ser
        275                 280                 285

GAA GTT ACC ACC AAA ATG CTG TGC GCT GCT GAC CCG CAG TGG AAA ACC     912
Glu Val Thr Thr Lys Met Leu Cys Ala Ala Asp Pro Gln Trp Lys Thr
    290                 295                 300

GAC TCT TGC CAA GGT GAC TCT GGT GGT CCA CTA GTT TGC TCT CTC CAG     960
Asp Ser Cys Gln Gly Asp Ser Gly Gly Pro Leu Val Cys Ser Leu Gln
305                 310                 315                 320

GGT CGT ATG ACC CTG ACC GGT ATT GTT TCT TGG GGT CGT GGT TGC GCT     1008
Gly Arg Met Thr Leu Thr Gly Ile Val Ser Trp Gly Arg Gly Cys Ala
                325                 330                 335

CTG AAA GAC AAA CCG GGT GTT TAC ACC CGT GTT TCT CAC TTC CTG CCG     1056
Leu Lys Asp Lys Pro Gly Val Tyr Thr Arg Val Ser His Phe Leu Pro
            340                 345                 350

TGG ATC CGT TCT CAC ACC AAA GAA GAA AAC GGT CTG GCT CTG AGC CCG     1104
Trp Ile Arg Ser His Thr Lys Glu Glu Asn Gly Leu Ala Leu Ser Pro
        355                 360                 365

GTT GAA GTT TTC CCG CGG CCG CTG GGT GGT GGT GGT AAC GGT GAC TTC     1152
Val Glu Val Phe Pro Arg Pro Leu Gly Gly Gly Gly Asn Gly Asp Phe
    370                 375                 380

GAA GAA ATC CCG GAA GAA TAC CTG CAA TAA                             1182
Glu Glu Ile Pro Glu Glu Tyr Leu Gln  *
385                 390

(2) ANGABEN ZU SEQ ID NO: 8:

        (i) SEQUENZKENNZEICHEN:
            (A) LÄNGE: 394 Aminosäuren
            (B) ART: Aminosäure
            (D) TOPOLOGIE: linear

     (ii) ART DES MOLEKÜLS: Protein
     (xi) SEQUENZBESCHREIBUNG: SEQ ID NO: 8:

Met Ser Lys Thr Cys Tyr Glu Gly Asn Gly His Phe Tyr Arg Gly Lys
  1               5                  10                  15

Ala Ser Thr Asp Thr Met Gly Arg Pro Cys Leu Pro Trp Asn Ser Ala
             20                  25                  30

Thr Val Leu Gln Gln Thr Tyr His Ala His Arg Ser Asp Ala Leu Gln
         35                  40                  45

Leu Gly Leu Gly Lys His Asn Tyr Cys Arg Asn Pro Asp Asn Arg Arg
     50                  55                  60

Arg Pro Trp Cys Tyr Val Gln Val Gly Leu Lys Pro Leu Val Gln Glu
 65                  70                  75                  80

Cys Met Val His Asp Cys Ala Asp Gly Lys Lys Pro Ser Ser Pro Pro
                 85                  90                  95

Glu Glu Leu Lys Phe Gln Cys Gly Gln Lys Thr Leu Arg Pro Arg Phe
            100                 105                 110

Lys Ile Ile Gly Gly Glu Phe Thr Thr Ile Glu Asn Gln Pro Trp Phe
        115                 120                 125

Ala Ala Ile Tyr Arg Arg His Arg Gly Gly Ser Val Thr Tyr Val Cys
    130                 135                 140

Gly Gly Ser Leu Ile Ser Pro Cys Trp Val Ile Ser Ala Thr His Cys
145                 150                 155                 160

Phe Ile Asp Tyr Pro Lys Lys Glu Asp Tyr Ile Val Tyr Leu Gly Arg
                165                 170                 175

Ser Arg Leu Asn Ser Asn Thr Gln Gly Glu Met Lys Phe Glu Val Glu
            180                 185                 190

Asn Leu Ile Leu His Lys Asp Tyr Ser Ala Asp Thr Leu Ala His His
        195                 200                 205

Asn Asp Ile Ala Leu Leu Lys Ile Arg Ser Lys Glu Gly Arg Cys Ala
    210                 215                 220

Gln Pro Ser Arg Thr Ile Gln Thr Ile Cys Leu Pro Ser Met Tyr Asn
225                 230                 235                 240

Asp Pro Gln Phe Gly Thr Ser Cys Glu Ile Thr Gly Phe Gly Lys Glu
                245                 250                 255

Asn Ser Thr Asp Tyr Leu Tyr Pro Glu Gln Leu Lys Met Thr Val Val
            260                 265                 270

Lys Leu Ile Ser His Arg Glu Cys Gln Gln Pro His Tyr Tyr Gly Ser
        275                 280                 285

Glu Val Thr Thr Lys Met Leu Cys Ala Ala Asp Pro Gln Trp Lys Thr
    290                 295                 300

Asp Ser Cys Gln Gly Asp Ser Gly Gly Pro Leu Val Cys Ser Leu Gln
305                 310                 315                 320

Gly Arg Met Thr Leu Thr Gly Ile Val Ser Trp Gly Arg Gly Cys Ala
                325                 330                 335

Leu Lys Asp Lys Pro Gly Val Tyr Thr Arg Val Ser His Phe Leu Pro
            340                 345                 350

Trp Ile Arg Ser His Thr Lys Glu Glu Asn Gly Leu Ala Leu Ser Pro
        355                 360                 365

Val Glu Val Phe Pro Arg Pro Leu Gly Gly Gly Gly Asn Gly Asp Phe
    370                 375                 380

Glu Glu Ile Pro Glu Glu Tyr Leu Gln  *
385                 390

(2) ANGABEN ZU SEQ ID NO: 9:

    (i) SEQUENZKENNZEICHEN:
        (A) LÄNGE: 1182 Basenpaare
        (B) ART: Nucleotid
        (C) STRANGFORM: Einzelstrang
        (D) TOPOLOGIE: linear

    (ii) ART DES MOLEKÜLS: Sonstige Nucleinsäure

    (iii) HYPOTHETISCH: NEIN

    (iv) ANTISENSE: NEIN

(ix) MERKMAL:
(A) NAME/SCHLÜSSEL: CDS
(B) LAGE:1..1182
(D) SONSTIGE ANGABEN:/function= "N-term. Methionin wird
in E.coli proteolytisch abgespalten"
/product= "Synthetisches Gen fuer das Protein M28
(pSJ105)"

(xi) SEQUENZBESCHREIBUNG: SEQ ID NO: 9:

ATG AGC AAA ACT TGC TAC GAA GGT AAC GGT CAC TTC TAC CGT GGT AAG 48
Met Ser Lys Thr Cys Tyr Glu Gly Asn Gly His Phe Tyr Arg Gly Lys
1               5                   10                  15

GCT TCT ACC GAC ACC ATG GGT CGT CCG TGC CTG CCG TGG AAC TCT GCT 96
Ala Ser Thr Asp Thr Met Gly Arg Pro Cys Leu Pro Trp Asn Ser Ala
            20                  25                  30

ACC GTT CTG CAG CAG ACC TAC CAC GCT CAC CGT TCT GAT GCA TTG CAG 144
Thr Val Leu Gln Gln Thr Tyr His Ala His Arg Ser Asp Ala Leu Gln
        35                  40                  45

CTG GGT CTG GGT AAA CAC AAC TAC TGC CGT AAC CCG GAC AAC CGT CGT 192
Leu Gly Leu Gly Lys His Asn Tyr Cys Arg Asn Pro Asp Asn Arg Arg
    50                  55                  60

CGT CCG TGG TGC TAC GTT CAG GTT GGT CTG AAA CCG CTA GTT CAG GAA 240
Arg Pro Trp Cys Tyr Val Gln Val Gly Leu Lys Pro Leu Val Gln Glu
65                  70                  75                  80

TGC ATG GTT CAC GAC TGC GCT GAC GGT AAA AAA CCG TCT TCT CCG CCG 288
Cys Met Val His Asp Cys Ala Asp Gly Lys Lys Pro Ser Ser Pro Pro
                85                  90                  95

GAA GAG CTC AAA TTC CAG TGC GGT CAA AAA ACC CTA CGT CCG CGT TTT 336
Glu Glu Leu Lys Phe Gln Cys Gly Gln Lys Thr Leu Arg Pro Arg Phe
            100                 105                 110

AAA ATC ATC GGT GGT GAG TTC ACC ACC ATC GAA AAC CAG CCG TGG TTC 384
Lys Ile Ile Gly Gly Glu Phe Thr Thr Ile Glu Asn Gln Pro Trp Phe
        115                 120                 125

GCT GCT ATC TAC CGT CGT CAC CGT GGT GGT TCT GTT ACC TAC GTT TGC 432
Ala Ala Ile Tyr Arg Arg His Arg Gly Gly Ser Val Thr Tyr Val Cys
    130                 135                 140

GGT GGT TCT CTG ATC TCT CCG TGC TGG GTT ATC TCT GCT ACC CAC TGC 480
Gly Gly Ser Leu Ile Ser Pro Cys Trp Val Ile Ser Ala Thr His Cys
145                 150                 155                 160

TTC ATC GAC TAC CCG AAA AAA GAA GAC TAC ATC GTT TAC CTC GGC CGT 528
Phe Ile Asp Tyr Pro Lys Lys Glu Asp Tyr Ile Val Tyr Leu Gly Arg
                165                 170                 175

TCT CGT TTA AAC TCT AAC ACC CAG GGT GAA ATG AAA TTC GAA GTT GAA 576
Ser Arg Leu Asn Ser Asn Thr Gln Gly Glu Met Lys Phe Glu Val Glu
            180                 185                 190

AAC CTG ATC CTG CAC AAA GAC TAC TCT GCT GAC ACC CTG GCT CAC CAC 624
Asn Leu Ile Leu His Lys Asp Tyr Ser Ala Asp Thr Leu Ala His His
        195                 200                 205

AAC GAC ATC GCT CTG CTA AAA ATC CGT TCT AAA GAA GGT CGT TGC GCT 672
Asn Asp Ile Ala Leu Leu Lys Ile Arg Ser Lys Glu Gly Arg Cys Ala
    210                 215                 220

CAG CCG TCT CGT ACC ATC CAG ACC ATC TGC CTG CCG TCT ATG TAC AAC 720
Gln Pro Ser Arg Thr Ile Gln Thr Ile Cys Leu Pro Ser Met Tyr Asn
225                 230                 235                 240

GAC CCG CAG TTC GGT ACC TCT TGC GAA ATC ACC GGT TTC GGT AAA GAA 768
Asp Pro Gln Phe Gly Thr Ser Cys Glu Ile Thr Gly Phe Gly Lys Glu
                245                 250                 255

AAC TCT ACC GAC TAC CTG TAC CCG GAA CAG CTG AAA ATG ACC GTT GTT 816
Asn Ser Thr Asp Tyr Leu Tyr Pro Glu Gln Leu Lys Met Thr Val Val
            260                 265                 270

AAA CTG ATC TCT CAC CGT GAA TGC CAG CAG CCG CAC TAC TAC GGT TCT 864
Lys Leu Ile Ser His Arg Glu Cys Gln Gln Pro His Tyr Tyr Gly Ser
        275                 280                 285

GAA GTT ACC ACC AAA ATG CTG TGC GCT GCT GAC CCG CAG TGG AAA ACC 912
Glu Val Thr Thr Lys Met Leu Cys Ala Ala Asp Pro Gln Trp Lys Thr
    290                 295                 300

GAC TCT TGC CAA GGT GAC TCT GGT GGT CCA CTA GTT TGC TCT CTC CAG 960
Asp Ser Cys Gln Gly Asp Ser Gly Gly Pro Leu Val Cys Ser Leu Gln
305                 310                 315                 320

GGT CGT ATG ACC CTG ACC GGT ATT GTT TCT TGG GGT CGT GGT TGC GCT 1008
Gly Arg Met Thr Leu Thr Gly Ile Val Ser Trp Gly Arg Gly Cys Ala
                325                 330                 335

CTG AAA GAC AAA CCG GGT GTT TAC ACC CGT GTT TCT CAC TTC CTG CCG 1056
Leu Lys Asp Lys Pro Gly Val Tyr Thr Arg Val Ser His Phe Leu Pro
            340                 345                 350

TGG ATC CGT TCT CAC ACC AAA GAA GAA AAC GGT CTG GCT CTG AGC CCG 1104
Trp Ile Arg Ser His Thr Lys Glu Glu Asn Gly Leu Ala Leu Ser Pro
        355                 360                 365

GTT GTA GTT GTA GTG CGG CCG CTG GGT GGT GGT GGT AAC GGT GAC TTC 1152
Val Val Val Val Val Arg Pro Leu Gly Gly Gly Gly Asn Gly Asp Phe
    370                 375                 380

GAA GAA ATC CCG GAA GAA TAC CTG CAA TAA 1182
Glu Glu Ile Pro Glu Glu Tyr Leu Gln *
385                 390

(2) ANGABEN ZU SEQ ID NO: 10:

(i) SEQUENZKENNZEICHEN:
(A) LÄNGE: 394 Aminosäuren
(B) ART: Aminosäure
(D) TOPOLOGIE: linear

(ii) ART DES MOLEKÜLS: Protein
(xi) SEQUENZBESCHREIBUNG: SEQ ID NO: 10:

Met Ser Lys Thr Cys Tyr Glu Gly Asn Gly His Phe Tyr Arg Gly Lys
1               5                   10                  15

Ala Ser Thr Asp Thr Met Gly Arg Pro Cys Leu Pro Trp Asn Ser Ala
            20                  25                  30

Thr Val Leu Gln Gln Thr Tyr His Ala His Arg Ser Asp Ala Leu Gln
        35                  40                  45

Leu Gly Leu Gly Lys His Asn Tyr Cys Arg Asn Pro Asp Asn Arg Arg
    50                  55                  60

Arg Pro Trp Cys Tyr Val Gln Val Gly Leu Lys Pro Leu Val Gln Glu
65                  70                  75                  80

Cys Met Val His Asp Cys Ala Asp Gly Lys Lys Pro Ser Ser Pro Pro
                85                  90                  95

Glu Glu Leu Lys Phe Gln Cys Gly Gln Lys Thr Leu Arg Pro Arg Phe
            100                 105                 110

Lys Ile Ile Gly Gly Glu Phe Thr Thr Ile Glu Asn Gln Pro Trp Phe
        115                 120                 125

Ala Ala Ile Tyr Arg Arg His Arg Gly Gly Ser Val Thr Tyr Val Cys
    130                 135                 140

Gly Gly Ser Leu Ile Ser Pro Cys Trp Val Ile Ser Ala Thr His Cys
145                 150                 155                 160

Phe Ile Asp Tyr Pro Lys Lys Glu Asp Tyr Ile Val Tyr Leu Gly Arg
                165                 170                 175

Ser Arg Leu Asn Ser Asn Thr Gln Gly Glu Met Lys Phe Glu Val Glu
            180                 185                 190

Asn Leu Ile Leu His Lys Asp Tyr Ser Ala Asp Thr Leu Ala His His
        195                 200                 205

Asn Asp Ile Ala Leu Leu Lys Ile Arg Ser Lys Glu Gly Arg Cys Ala
    210                 215                 220

Gln Pro Ser Arg Thr Ile Gln Thr Ile Cys Leu Pro Ser Met Tyr Asn
225                 230                 235                 240

Asp Pro Gln Phe Gly Thr Ser Cys Glu Ile Thr Gly Phe Gly Lys Glu
                245                 250                 255

Asn Ser Thr Asp Tyr Leu Tyr Pro Glu Gln Leu Lys Met Thr Val Val
            260                 265                 270

Lys Leu Ile Ser His Arg Glu Cys Gln Gln Pro His Tyr Tyr Gly Ser
        275                 280                 285

Glu Val Thr Thr Lys Met Leu Cys Ala Ala Asp Pro Gln Trp Lys Thr
    290                 295                 300

Asp Ser Cys Gln Gly Asp Ser Gly Gly Pro Leu Val Cys Ser Leu Gln
305                 310                 315                 320

Gly Arg Met Thr Leu Thr Gly Ile Val Ser Trp Gly Arg Gly Cys Ala
                325                 330                 335

Leu Lys Asp Lys Pro Gly Val Tyr Thr Arg Val Ser His Phe Leu Pro
            340                 345                 350

Trp Ile Arg Ser His Thr Lys Glu Glu Asn Gly Leu Ala Leu Ser Pro
        355                 360                 365

Val Val Val Val Val Arg Pro Leu Gly Gly Gly Gly Asn Gly Asp Phe
    370                 375                 380

Glu Glu Ile Pro Glu Glu Tyr Leu Gln  *
385                 390

(2) ANGABEN ZU SEQ ID NO: 11:

     (i) SEQUENZKENNZEICHEN:
          (A) LÄNGE: 1191 Basenpaare
          (B) ART: Nucleotid
          (C) STRANGFORM: Einzelstrang
          (D) TOPOLOGIE: linear

    (ii) ART DES MOLEKÜLS: Sonstige Nucleinsäure

   (iii) HYPOTHETISCH: NEIN

    (iv) ANTISENSE: NEIN

    (ix) MERKMAL:
          (A) NAME/SCHLÜSSEL: CDS
          (B) LAGE:1..1191
          (D) SONSTIGE ANGABEN:/function= "N-term. Methionin wird
               in E.coli proteolytisch abgespalten"
               /product= "Synthetisches Gen fuer das Protein M29
               (pSJ 106)"

    (xi) SEQUENZBESCHREIBUNG: SEQ ID NO: 11:

ATG AGC AAA ACT TGC TAC GAA GGT AAC GGT CAC TTC TAC CGT GGT AAG        48
Met Ser Lys Thr Cys Tyr Glu Gly Asn Gly His Phe Tyr Arg Gly Lys
  1               5                  10                  15

GCT TCT ACC GAC ACC ATG GGT CGT CCG TGC CTG CCG TGG AAC TCT GCT        96
Ala Ser Thr Asp Thr Met Gly Arg Pro Cys Leu Pro Trp Asn Ser Ala
             20                  25                  30

ACC GTT CTG CAG CAG ACC TAC CAC GCT CAC CGT TCT GAT GCA TTG CAG       144
Thr Val Leu Gln Gln Thr Tyr His Ala His Arg Ser Asp Ala Leu Gln
         35                  40                  45

CTG GGT CTG GGT AAA CAC AAC TAC TGC CGT AAC CCG GAC AAC CGT CGT       192
Leu Gly Leu Gly Lys His Asn Tyr Cys Arg Asn Pro Asp Asn Arg Arg
     50                  55                  60

CGT CCG TGG TGC TAC GTT CAG GTT GGT CTG AAA CCG CTA GTT CAG GAA       240
Arg Pro Trp Cys Tyr Val Gln Val Gly Leu Lys Pro Leu Val Gln Glu
 65                  70                  75                  80

TGC ATG GTT CAC GAC TGC GCT GAC GGT AAA AAA CCG TCT TCT CCG CCG       288
Cys Met Val His Asp Cys Ala Asp Gly Lys Lys Pro Ser Ser Pro Pro
                 85                  90                  95

GAA GAG CTC AAA TTC CAG TGC GGT CAA AAA ACC CTA CGT CCG CGT TTT       336
Glu Glu Leu Lys Phe Gln Cys Gly Gln Lys Thr Leu Arg Pro Arg Phe
            100                 105                 110

AAA ATC ATC GGT GGT GAG TTC ACC ACC ATC GAA AAC CAG CCG TGG TTC       384
Lys Ile Ile Gly Gly Glu Phe Thr Thr Ile Glu Asn Gln Pro Trp Phe
        115                 120                 125

GCT GCT ATC TAC CGT CGT CAC CGT GGT GGT TCT GTT ACC TAC GTT TGC 432
Ala Ala Ile Tyr Arg Arg His Arg Gly Gly Ser Val Thr Tyr Val Cys
    130                 135                 140

GGT GGT TCT CTG ATC TCT CCG TGC TGG GTT ATC TCT GCT ACC CAC TGC 480
Gly Gly Ser Leu Ile Ser Pro Cys Trp Val Ile Ser Ala Thr His Cys
145                 150                 155                 160

TTC ATC GAC TAC CCG AAA AAA GAA GAC TAC ATC GTT TAC CTC GGC CGT 528
Phe Ile Asp Tyr Pro Lys Lys Glu Asp Tyr Ile Val Tyr Leu Gly Arg
                165                 170                 175

TCT CGT TTA AAC TCT AAC ACC CAG GGT GAA ATG AAA TTC GAA GTT GAA 576
Ser Arg Leu Asn Ser Asn Thr Gln Gly Glu Met Lys Phe Glu Val Glu
            180                 185                 190

AAC CTG ATC CTG CAC AAA GAC TAC TCT GCT GAC ACC CTG GCT CAC CAC 624
Asn Leu Ile Leu His Lys Asp Tyr Ser Ala Asp Thr Leu Ala His His
        195                 200                 205

AAC GAC ATC GCT CTG CTA AAA ATC CGT TCT AAA GAA GGT CGT TGC GCT 672
Asn Asp Ile Ala Leu Leu Lys Ile Arg Ser Lys Glu Gly Arg Cys Ala
    210                 215                 220

CAG CCG TCT CGT ACC ATC CAG ACC ATC TGC CTG CCG TCT ATG TAC AAC 720
Gln Pro Ser Arg Thr Ile Gln Thr Ile Cys Leu Pro Ser Met Tyr Asn
225                 230                 235                 240

GAC CCG CAG TTC GGT ACC TCT TGC GAA ATC ACC GGT TTC GGT AAA GAA 768
Asp Pro Gln Phe Gly Thr Ser Cys Glu Ile Thr Gly Phe Gly Lys Glu
                245                 250                 255

AAC TCT ACC GAC TAC CTG TAC CCG GAA CAG CTG AAA ATG ACC GTT GTT 816
Asn Ser Thr Asp Tyr Leu Tyr Pro Glu Gln Leu Lys Met Thr Val Val
            260                 265                 270

AAA CTG ATC TCT CAC CGT GAA TGC CAG CAG CCG CAC TAC TAC GGT TCT 864
Lys Leu Ile Ser His Arg Glu Cys Gln Gln Pro His Tyr Tyr Gly Ser
        275                 280                 285

GAA GTT ACC ACC AAA ATG CTG TGC GCT GCT GAC CCG CAG TGG AAA ACC 912
Glu Val Thr Thr Lys Met Leu Cys Ala Ala Asp Pro Gln Trp Lys Thr
    290                 295                 300

GAC TCT TGC CAA GGT GAC TCT GGT GGT CCA CTA GTT TGC TCT CTC CAG 960
Asp Ser Cys Gln Gly Asp Ser Gly Gly Pro Leu Val Cys Ser Leu Gln
305                 310                 315                 320

GGT CGT ATG ACC CTG ACC GGT ATT GTT TCT TGG GGT CGT GGT TGC GCT 1008
Gly Arg Met Thr Leu Thr Gly Ile Val Ser Trp Gly Arg Gly Cys Ala
                325                 330                 335

CTG AAA GAC AAA CCG GGT GTT TAC ACC CGT GTT TCT CAC TTC CTG CCG 1056
Leu Lys Asp Lys Pro Gly Val Tyr Thr Arg Val Ser His Phe Leu Pro
            340                 345                 350

TGG ATC CGT TCT CAC ACC AAA GAA GAA AAC GGT CTG GCT CTG AGC CCG 1104
Trp Ile Arg Ser His Thr Lys Glu Glu Asn Gly Leu Ala Leu Ser Pro
        355                 360                 365

GTT GTT GCT TTC CCG CGG CCG TTC CTG CTG CGG AAC CCG AAC GAC AAA 1152
Val Val Ala Phe Pro Arg Pro Phe Leu Leu Arg Asn Pro Asn Asp Lys
    370                 375                 380

TAC GAA CCG TTC TGG GAA GAC GAA GAA AAA AAC GAA TAA 1191
Tyr Glu Pro Phe Trp Glu Asp Glu Glu Lys Asn Glu *
385                 390                 395

(2) ANGABEN ZU SEQ ID NO: 12:

(i) SEQUENZKENNZEICHEN:
(A) LÄNGE: 397 Aminosäuren
(B) ART: Aminosäure
(D) TOPOLOGIE: linear

(ii) ART DES MOLEKÜLS: Protein
(xi) SEQUENZBESCHREIBUNG: SEQ ID NO: 12:

Met Ser Lys Thr Cys Tyr Glu Gly Asn Gly His Phe Tyr Arg Gly Lys
1               5                   10                  15

Ala Ser Thr Asp Thr Met Gly Arg Pro Cys Leu Pro Trp Asn Ser Ala
            20                  25                  30

Thr Val Leu Gln Gln Thr Tyr His Ala His Arg Ser Asp Ala Leu Gln
        35                  40                  45

Leu Gly Leu Gly Lys His Asn Tyr Cys Arg Asn Pro Asp Asn Arg Arg
    50                  55                  60

Arg Pro Trp Cys Tyr Val Gln Val Gly Leu Lys Pro Leu Val Gln Glu
65                  70                  75                  80

Cys Met Val His Asp Cys Ala Asp Gly Lys Lys Pro Ser Ser Pro Pro
                85                  90                  95

Glu Glu Leu Lys Phe Gln Cys Gly Gln Lys Thr Leu Arg Pro Arg Phe
            100                 105                 110

Lys Ile Ile Gly Gly Glu Phe Thr Thr Ile Glu Asn Gln Pro Trp Phe
        115                 120                 125

Ala Ala Ile Tyr Arg Arg His Arg Gly Gly Ser Val Thr Tyr Val Cys
    130                 135                 140

Gly Gly Ser Leu Ile Ser Pro Cys Trp Val Ile Ser Ala Thr His Cys
145                 150                 155                 160

Phe Ile Asp Tyr Pro Lys Lys Glu Asp Tyr Ile Val Tyr Leu Gly Arg
                165                 170                 175

Ser Arg Leu Asn Ser Asn Thr Gln Gly Glu Met Lys Phe Glu Val Glu
            180                 185                 190

Asn Leu Ile Leu His Lys Asp Tyr Ser Ala Asp Thr Leu Ala His His
        195                 200                 205

Asn Asp Ile Ala Leu Leu Lys Ile Arg Ser Lys Glu Gly Arg Cys Ala
    210                 215                 220

Gln Pro Ser Arg Thr Ile Gln Thr Ile Cys Leu Pro Ser Met Tyr Asn
225                 230                 235                 240

Asp Pro Gln Phe Gly Thr Ser Cys Glu Ile Thr Gly Phe Gly Lys Glu
                245                 250                 255

Asn Ser Thr Asp Tyr Leu Tyr Pro Glu Gln Leu Lys Met Thr Val Val
            260                 265                 270

Lys Leu Ile Ser His Arg Glu Cys Gln Gln Pro His Tyr Tyr Gly Ser
        275                 280                 285

Glu Val Thr Thr Lys Met Leu Cys Ala Ala Asp Pro Gln Trp Lys Thr
    290                 295                 300

Asp Ser Cys Gln Gly Asp Ser Gly Gly Pro Leu Val Cys Ser Leu Gln
305                 310                 315                 320

Gly Arg Met Thr Leu Thr Gly Ile Val Ser Trp Gly Arg Gly Cys Ala
                325                 330                 335

Leu Lys Asp Lys Pro Gly Val Tyr Thr Arg Val Ser His Phe Leu Pro
            340                 345                 350

Trp Ile Arg Ser His Thr Lys Glu Glu Asn Gly Leu Ala Leu Ser Pro
        355                 360                 365

Val Val Ala Phe Pro Arg Pro Phe Leu Leu Arg Asn Pro Asn Asp Lys
    370                 375                 380

Tyr Glu Pro Phe Trp Glu Asp Glu Glu Lys Asn Glu *
385                 390                 395

(2) ANGABEN ZU SEQ ID NO: 13:

(i) SEQUENZKENNZEICHEN:
(A) LÄNGE: 1182 Basenpaare
(B) ART: Nucleotid
(C) STRANGFORM: Einzelstrang
(D) TOPOLOGIE: linear

(ii) ART DES MOLEKÜLS: Sonstige Nucleinsäure

(iii) HYPOTHETISCH: NEIN

(iv) ANTISENSE: NEIN

(ix) MERKMAL:
(A) NAME/SCHLÜSSEL: CDS
(B) LAGE:1..1182
(D) SONSTIGE ANGABEN:/function= "N-term. Methionin wird
in E.coli proteolytisch abgespalten"
/product= "Synthetisches Gen fuer das Protein M30
(pSJ109)"

(xi) SEQUENZBESCHREIBUNG: SEQ ID NO: 13:

ATG AGC AAA ACT TGC TAC GAA GGT AAC GGT CAC TTC TAC CGT GGT AAG 48
Met Ser Lys Thr Cys Tyr Glu Gly Asn Gly His Phe Tyr Arg Gly Lys
1               5                   10                  15

GCT TCT ACC GAC ACC ATG GGT CGT CCG TGC CTG CCG TGG AAC TCT GCT 96
Ala Ser Thr Asp Thr Met Gly Arg Pro Cys Leu Pro Trp Asn Ser Ala
            20                  25                  30

ACC GTT CTG CAG CAG ACC TAC CAC GCT CAC CGT TCT GAT GCA TTG CAG 144
Thr Val Leu Gln Gln Thr Tyr His Ala His Arg Ser Asp Ala Leu Gln
        35                  40                  45

CTG GGT CTG GGT AAA CAC AAC TAC TGC CGT AAC CCG GAC AAC CGT CGT 192
Leu Gly Leu Gly Lys His Asn Tyr Cys Arg Asn Pro Asp Asn Arg Arg
    50                  55                  60

CGT CCG TGG TGC TAC GTT CAG GTT GGT CTG AAA CCG CTA GTT CAG GAA 240
Arg Pro Trp Cys Tyr Val Gln Val Gly Leu Lys Pro Leu Val Gln Glu
65                  70                  75                  80

TGC ATG GTT CAC GAC TGC GCT GAC GGT AAA AAA CCG TCT TCT CCG CCG 288
Cys Met Val His Asp Cys Ala Asp Gly Lys Lys Pro Ser Ser Pro Pro
                85                  90                  95

GAA GAG CTC AAA TTC CAG TGC GGT CAA AAA ACC CTA CGT CCG CGT TTT 336
Glu Glu Leu Lys Phe Gln Cys Gly Gln Lys Thr Leu Arg Pro Arg Phe
            100                 105                 110

AAA ATC ATC GGT GGT GAG TTC ACC ACC ATC GAA AAC CAG CCG TGG TTC 384
Lys Ile Ile Gly Gly Glu Phe Thr Thr Ile Glu Asn Gln Pro Trp Phe
        115                 120                 125

GCT GCT ATC TAC CGT CGT CAC CGT GGT GGT TCT GTT ACC TAC GTT TGC 432
Ala Ala Ile Tyr Arg Arg His Arg Gly Gly Ser Val Thr Tyr Val Cys
    130                 135                 140

GGT GGT TCT CTG ATC TCT CCG TGC TGG GTT ATC TCT GCT ACC CAC TGC 480
Gly Gly Ser Leu Ile Ser Pro Cys Trp Val Ile Ser Ala Thr His Cys
145                 150                 155                 160

TTC ATC GAC TAC CCG AAA AAA GAA GAC TAC ATC GTT TAC CTC GGC CGT 528
Phe Ile Asp Tyr Pro Lys Lys Glu Asp Tyr Ile Val Tyr Leu Gly Arg
                165                 170                 175

TCT CGT TTA AAC TCT AAC ACC CAG GGT GAA ATG AAA TTC GAA GTT GAA 576
Ser Arg Leu Asn Ser Asn Thr Gln Gly Glu Met Lys Phe Glu Val Glu
            180                 185                 190

AAC CTG ATC CTG CAC AAA GAC TAC TCT GCT GAC ACC CTG GCT CAC CAC 624
Asn Leu Ile Leu His Lys Asp Tyr Ser Ala Asp Thr Leu Ala His His
        195                 200                 205

AAC GAC ATC GCT CTG CTA AAA ATC CGT TCT AAA GAA GGT CGT TGC GCT 672
Asn Asp Ile Ala Leu Leu Lys Ile Arg Ser Lys Glu Gly Arg Cys Ala
    210                 215                 220

CAG CCG TCT CGT ACC ATC CAG ACC ATC TGC CTG CCG TCT ATG TAC AAC 720
Gln Pro Ser Arg Thr Ile Gln Thr Ile Cys Leu Pro Ser Met Tyr Asn
225                 230                 235                 240

GAC CCG CAG TTC GGT ACC TCT TGC GAA ATC ACC GGT TTC GGT AAA GAA 768
Asp Pro Gln Phe Gly Thr Ser Cys Glu Ile Thr Gly Phe Gly Lys Glu
                245                 250                 255

AAC TCT ACC GAC TAC CTG TAC CCG GAA CAG CTG AAA ATG ACC GTT GTT 816
Asn Ser Thr Asp Tyr Leu Tyr Pro Glu Gln Leu Lys Met Thr Val Val
            260                 265                 270

AAA CTG ATC TCT CAC CGT GAA TGC CAG CAG CCG CAC TAC TAC GGT TCT 864
Lys Leu Ile Ser His Arg Glu Cys Gln Gln Pro His Tyr Tyr Gly Ser
        275                 280                 285

GAA GTT ACC ACC AAA ATG CTG TGC GCT GCT GAC CCG CAG TGG AAA ACC 912
Glu Val Thr Thr Lys Met Leu Cys Ala Ala Asp Pro Gln Trp Lys Thr
    290                 295                 300

GAC TCT TGC CAA GGT GAC TCT GGT GGT CCA CTA GTT TGC TCT CTC CAG 960
Asp Ser Cys Gln Gly Asp Ser Gly Gly Pro Leu Val Cys Ser Leu Gln
305                 310                 315                 320

GGT CGT ATG ACC CTG ACC GGT ATT GTT TCT TGG GGT CGT GGT TGC GCT 1008
Gly Arg Met Thr Leu Thr Gly Ile Val Ser Trp Gly Arg Gly Cys Ala
                325                 330                 335

CTG AAA GAC AAA CCG GGT GTT TAC ACC CGT GTT TCT CAC TTC CTG CCG 1056
Leu Lys Asp Lys Pro Gly Val Tyr Thr Arg Val Ser His Phe Leu Pro
            340                 345                 350

TGG ATC CGT TCT CAC ACC AAA GAA GAA AAC GGT CTG GCT CTG AGC GTG 1104
Trp Ile Arg Ser His Thr Lys Glu Glu Asn Gly Leu Ala Leu Ser Val
        355                 360                 365

GTT GTT GCT TTC CCG CGG CCG CTG GGT GGT GGT GGT AAC GGT GAC TTC 1152
Val Val Ala Phe Pro Arg Pro Leu Gly Gly Gly Gly Asn Gly Asp Phe
    370                 375                 380

GAA GAA ATC CCG GAA GAA TAC CTG CAA TAA 1182
Glu Glu Ile Pro Glu Glu Tyr Leu Gln *
385                 390

(2) ANGABEN ZU SEQ ID NO: 14:

(i) SEQUENZKENNZEICHEN:
(A) LÄNGE: 394 Aminosäuren
(B) ART: Aminosäure
(D) TOPOLOGIE: linear

(ii) ART DES MOLEKÜLS: Protein
(xi) SEQUENZBESCHREIBUNG: SEQ ID NO: 14:

Met Ser Lys Thr Cys Tyr Glu Gly Asn Gly His Phe Tyr Arg Gly Lys
1               5                   10                  15

Ala Ser Thr Asp Thr Met Gly Arg Pro Cys Leu Pro Trp Asn Ser Ala
            20                  25                  30

Thr Val Leu Gln Gln Thr Tyr His Ala His Arg Ser Asp Ala Leu Gln
        35                  40                  45

Leu Gly Leu Gly Lys His Asn Tyr Cys Arg Asn Pro Asp Asn Arg Arg
    50                  55                  60

Arg Pro Trp Cys Tyr Val Gln Val Gly Leu Lys Pro Leu Val Gln Glu
65                  70                  75                  80

Cys Met Val His Asp Cys Ala Asp Gly Lys Lys Pro Ser Ser Pro Pro
                85                  90                  95

Glu Glu Leu Lys Phe Gln Cys Gly Gln Lys Thr Leu Arg Pro Arg Phe
            100                 105                 110

Lys Ile Ile Gly Gly Glu Phe Thr Thr Ile Glu Asn Gln Pro Trp Phe
        115                 120                 125

Ala Ala Ile Tyr Arg Arg His Arg Gly Gly Ser Val Thr Tyr Val Cys
    130                 135                 140

Gly Gly Ser Leu Ile Ser Pro Cys Trp Val Ile Ser Ala Thr His Cys
145                 150                 155                 160

Phe Ile Asp Tyr Pro Lys Lys Glu Asp Tyr Ile Val Tyr Leu Gly Arg
                165                 170                 175

Ser Arg Leu Asn Ser Asn Thr Gln Gly Glu Met Lys Phe Glu Val Glu
            180                 185                 190

Asn Leu Ile Leu His Lys Asp Tyr Ser Ala Asp Thr Leu Ala His His
        195                 200                 205

Asn Asp Ile Ala Leu Leu Lys Ile Arg Ser Lys Glu Gly Arg Cys Ala
    210                 215                 220

Gln Pro Ser Arg Thr Ile Gln Thr Ile Cys Leu Pro Ser Met Tyr Asn
225                 230                 235                 240

Asp Pro Gln Phe Gly Thr Ser Cys Glu Ile Thr Gly Phe Gly Lys Glu
                245                 250                 255

Asn Ser Thr Asp Tyr Leu Tyr Pro Glu Gln Leu Lys Met Thr Val Val
            260                 265                 270

Lys Leu Ile Ser His Arg Glu Cys Gln Gln Pro His Tyr Tyr Gly Ser
        275                 280                 285

Glu Val Thr Thr Lys Met Leu Cys Ala Ala Asp Pro Gln Trp Lys Thr
    290                 295                 300

Asp Ser Cys Gln Gly Asp Ser Gly Gly Pro Leu Val Cys Ser Leu Gln
305                 310                 315                 320

Gly Arg Met Thr Leu Thr Gly Ile Val Ser Trp Gly Arg Gly Cys Ala
                325                 330                 335

Leu Lys Asp Lys Pro Gly Val Tyr Thr Arg Val Ser His Phe Leu Pro
            340                 345                 350

Trp Ile Arg Ser His Thr Lys Glu Glu Asn Gly Leu Ala Leu Ser Val
        355                 360                 365

Val Val Ala Phe Pro Arg Pro Leu Gly Gly Gly Gly Asn Gly Asp Phe
    370                 375                 380

Glu Glu Ile Pro Glu Glu Tyr Leu Gln  *
385                 390

(2) ANGABEN ZU SEQ ID NO: 15:

(i) SEQUENZKENNZEICHEN:
(A) LÄNGE: 1182 Basenpaare
(B) ART: Nucleotid
(C) STRANGFORM: Einzelstrang
(D) TOPOLOGIE: linear

(ii) ART DES MOLEKÜLS: Sonstige Nucleinsäure

(iii) HYPOTHETISCH: NEIN

(iv) ANTISENSE: NEIN

(ix) MERKMAL:
(A) NAME/SCHLÜSSEL: CDS
(B) LAGE:1..1182
(D) SONSTIGE ANGABEN:/function= "N-term. Methionin wird
in E.coli proteolytisch abgespalten"
/product= "Synthetisches Gen fuer das Protein M31
(pSJ111)"

(xi) SEQUENZBESCHREIBUNG: SEQ ID NO: 15:

ATG AGC AAA ACT TGC TAC GAA GGT AAC GGT CAC TTC TAC CGT GGT AAG 48
Met Ser Lys Thr Cys Tyr Glu Gly Asn Gly His Phe Tyr Arg Gly Lys
1               5                   10                  15

GCT TCT ACC GAC ACC ATG GGT CGT CCG TGC CTG CCG TGG AAC TCT GCT 96
Ala Ser Thr Asp Thr Met Gly Arg Pro Cys Leu Pro Trp Asn Ser Ala
            20                  25                  30

ACC GTT CTG CAG CAG ACC TAC CAC GCT CAC CGT TCT GAT GCA TTG CAG 144
Thr Val Leu Gln Gln Thr Tyr His Ala His Arg Ser Asp Ala Leu Gln
        35                  40                  45

CTG GGT CTG GGT AAA CAC AAC TAC TGC CGT AAC CCG GAC AAC CGT CGT 192
Leu Gly Leu Gly Lys His Asn Tyr Cys Arg Asn Pro Asp Asn Arg Arg
    50                  55                  60

CGT CCG TGG TGC TAC GTT CAG GTT GGT CTG AAA CCG CTA GTT CAG GAA 240
Arg Pro Trp Cys Tyr Val Gln Val Gly Leu Lys Pro Leu Val Gln Glu
65                  70                  75                  80

TGC ATG GTT CAC GAC TGC GCT GAC GGT AAA AAA CCG TCT TCT CCG CCG 288
Cys Met Val His Asp Cys Ala Asp Gly Lys Lys Pro Ser Ser Pro Pro
                85                  90                  95

GAA GAG CTC AAA TTC CAG TGC GGT CAA AAA ACC CTA CGT CCG CGT TTT 336
Glu Glu Leu Lys Phe Gln Cys Gly Gln Lys Thr Leu Arg Pro Arg Phe
            100                 105                 110

AAA ATC ATC GGT GGT GAG TTC ACC ACC ATC GAA AAC CAG CCG TGG TTC 384
Lys Ile Ile Gly Gly Glu Phe Thr Thr Ile Glu Asn Gln Pro Trp Phe
        115                 120                 125

GCT GCT ATC TAC CGT CGT CAC CGT GGT GGT TCT GTT ACC TAC GTT TGC 432
Ala Ala Ile Tyr Arg Arg His Arg Gly Gly Ser Val Thr Tyr Val Cys
    130                 135                 140

GGT GGT TCT CTG ATC TCT CCG TGC TGG GTT ATC TCT GCT ACC CAC TGC 480
Gly Gly Ser Leu Ile Ser Pro Cys Trp Val Ile Ser Ala Thr His Cys
145                 150                 155                 160

TTC ATC GAC TAC CCG AAA AAA GAA GAC TAC ATC GTT TAC CTC GGC CGT 528
Phe Ile Asp Tyr Pro Lys Lys Glu Asp Tyr Ile Val Tyr Leu Gly Arg
                165                 170                 175

TCT CGT TTA AAC TCT AAC ACC CAG GGT GAA ATG AAA TTC GAA GTT GAA 576
Ser Arg Leu Asn Ser Asn Thr Gln Gly Glu Met Lys Phe Glu Val Glu
            180                 185                 190

AAC CTG ATC CTG CAC AAA GAC TAC TCT GCT GAC ACC CTG GCT CAC CAC 624
Asn Leu Ile Leu His Lys Asp Tyr Ser Ala Asp Thr Leu Ala His His
        195                 200                 205

AAC GAC ATC GCT CTG CTA AAA ATC CGT TCT AAA GAA GGT CGT TGC GCT 672
Asn Asp Ile Ala Leu Leu Lys Ile Arg Ser Lys Glu Gly Arg Cys Ala
    210                 215                 220

CAG CCG TCT CGT ACC ATC CAG ACC ATC TGC CTG CCG TCT ATG TAC AAC 720
Gln Pro Ser Arg Thr Ile Gln Thr Ile Cys Leu Pro Ser Met Tyr Asn
225                 230                 235                 240

GAC CCG CAG TTC GGT ACC TCT TGC GAA ATC ACC GGT TTC GGT AAA GAA 768
Asp Pro Gln Phe Gly Thr Ser Cys Glu Ile Thr Gly Phe Gly Lys Glu
                245                 250                 255

AAC TCT ACC GAC TAC CTG TAC CCG GAA CAG CTG AAA ATG ACC GTT GTT 816
Asn Ser Thr Asp Tyr Leu Tyr Pro Glu Gln Leu Lys Met Thr Val Val
            260                 265                 270

AAA CTG ATC TCT CAC CGT GAA TGC CAG CAG CCG CAC TAC TAC GGT TCT 864
Lys Leu Ile Ser His Arg Glu Cys Gln Gln Pro His Tyr Tyr Gly Ser
        275                 280                 285

GAA GTT ACC ACC AAA ATG CTG TGC GCT GCT GAC CCG CAG TGG AAA ACC 912
Glu Val Thr Thr Lys Met Leu Cys Ala Ala Asp Pro Gln Trp Lys Thr
    290                 295                 300

GAC TCT TGC CAA GGT GAC TCT GGT GGT CCA CTA GTT TGC TCT CTC CAG 960
Asp Ser Cys Gln Gly Asp Ser Gly Gly Pro Leu Val Cys Ser Leu Gln
305                 310                 315                 320

GGT CGT ATG ACC CTG ACC GGT ATT GTT TCT TGG GGT CGT GGT TGC GCT 1008
Gly Arg Met Thr Leu Thr Gly Ile Val Ser Trp Gly Arg Gly Cys Ala
                325                 330                 335

CTG AAA GAC AAA CCG GGT GTT TAC ACC CGT GTT TCT CAC TTC CTG CCG 1056
Leu Lys Asp Lys Pro Gly Val Tyr Thr Arg Val Ser His Phe Leu Pro
            340                 345                 350

TGG ATC CGT TCT CAC ACC AAA GAA GAA AAC GGT CTG GCT CTG AGC CTG 1104
Trp Ile Arg Ser His Thr Lys Glu Glu Asn Gly Leu Ala Leu Ser Leu
        355                 360                 365

GTT AAA GCT TTC CCG CGG CCG CTG GGT GGT GGT GGT AAC GGT GAC TTC 1152
Val Lys Ala Phe Pro Arg Pro Leu Gly Gly Gly Gly Asn Gly Asp Phe
    370                 375                 380

GAA GAA ATC CCG GAA GAA TAC CTG CAA TAA 1182
Glu Glu Ile Pro Glu Glu Tyr Leu Gln *
385                 390

(2) ANGABEN ZU SEQ ID NO: 16:

(i) SEQUENZKENNZEICHEN:
(A) LÄNGE: 394 Aminosäuren
(B) ART: Aminosäure
(D) TOPOLOGIE: linear

(ii) ART DES MOLEKÜLS: Protein

(xi) SEQUENZBESCHREIBUNG: SEQ ID NO: 16:

Met Ser Lys Thr Cys Tyr Glu Gly Asn Gly His Phe Tyr Arg Gly Lys
1               5                   10                  15

Ala Ser Thr Asp Thr Met Gly Arg Pro Cys Leu Pro Trp Asn Ser Ala
            20                  25                  30

Thr Val Leu Gln Gln Thr Tyr His Ala His Arg Ser Asp Ala Leu Gln
        35                  40                  45

Leu Gly Leu Gly Lys His Asn Tyr Cys Arg Asn Pro Asp Asn Arg Arg
    50                  55                  60

Arg Pro Trp Cys Tyr Val Gln Val Gly Leu Lys Pro Leu Val Gln Glu
65                  70                  75                  80

Cys Met Val His Asp Cys Ala Asp Gly Lys Lys Pro Ser Ser Pro Pro
                85                  90                  95

Glu Glu Leu Lys Phe Gln Cys Gly Gln Lys Thr Leu Arg Pro Arg Phe
            100                 105                 110

Lys Ile Ile Gly Gly Glu Phe Thr Thr Ile Glu Asn Gln Pro Trp Phe
        115                 120                 125

Ala Ala Ile Tyr Arg Arg His Arg Gly Gly Ser Val Thr Tyr Val Cys
    130                 135                 140

Gly Gly Ser Leu Ile Ser Pro Cys Trp Val Ile Ser Ala Thr His Cys
145                 150                 155                 160

Phe Ile Asp Tyr Pro Lys Lys Glu Asp Tyr Ile Val Tyr Leu Gly Arg
                165                 170                 175

Ser Arg Leu Asn Ser Asn Thr Gln Gly Glu Met Lys Phe Glu Val Glu
            180                 185                 190

Asn Leu Ile Leu His Lys Asp Tyr Ser Ala Asp Thr Leu Ala His His
        195                 200                 205

Asn Asp Ile Ala Leu Leu Lys Ile Arg Ser Lys Glu Gly Arg Cys Ala
    210                 215                 220

Gln Pro Ser Arg Thr Ile Gln Thr Ile Cys Leu Pro Ser Met Tyr Asn
225                 230                 235                 240

Asp Pro Gln Phe Gly Thr Ser Cys Glu Ile Thr Gly Phe Gly Lys Glu
                245                 250                 255

Asn Ser Thr Asp Tyr Leu Tyr Pro Glu Gln Leu Lys Met Thr Val Val
            260                 265                 270

Lys Leu Ile Ser His Arg Glu Cys Gln Gln Pro His Tyr Tyr Gly Ser
        275                 280                 285

Glu Val Thr Thr Lys Met Leu Cys Ala Ala Asp Pro Gln Trp Lys Thr
    290                 295                 300

Asp Ser Cys Gln Gly Asp Ser Gly Gly Pro Leu Val Cys Ser Leu Gln
305                 310                 315                 320

Gly Arg Met Thr Leu Thr Gly Ile Val Ser Trp Gly Arg Gly Cys Ala
                325                 330                 335

Leu Lys Asp Lys Pro Gly Val Tyr Thr Arg Val Ser His Phe Leu Pro
            340                 345                 350

Trp Ile Arg Ser His Thr Lys Glu Glu Asn Gly Leu Ala Leu Ser Leu
        355                 360                 365

Val Lys Ala Phe Pro Arg Pro Leu Gly Gly Gly Gly Asn Gly Asp Phe
    370                 375                 380

Glu Glu Ile Pro Glu Glu Tyr Leu Gln  *
385                 390

(2) ANGABEN ZU SEQ ID NO: 17:

(i) SEQUENZKENNZEICHEN:
(A) LÄNGE: 1167 Basenpaare
(B) ART: Nucleotid
(C) STRANGFORM: Einzelstrang
(D) TOPOLOGIE: linear

(ii) ART DES MOLEKÜLS: Sonstige Nucleinsäure

(iii) HYPOTHETISCH: NEIN

(iv) ANTISENSE: NEIN

(ix) MERKMAL:
(A) NAME/SCHLÜSSEL: CDS
(B) LAGE:1..1167
(D) SONSTIGE ANGABEN:/function= "N-term. Methionin wird
in E.coli proteolytisch abgespalten"
/product= "Synthetisches Gen fuer das Protein M33
(pSJ113)"

(xi) SEQUENZBESCHREIBUNG: SEQ ID NO: 17:

ATG AGC AAA ACT TGC TAC GAA GGT AAC GGT CAC TTC TAC CGT GGT AAG      48
Met Ser Lys Thr Cys Tyr Glu Gly Asn Gly His Phe Tyr Arg Gly Lys
  1               5                  10                  15

GCT TCT ACC GAC ACC ATG GGT CGT CCG TGC CTG CCG TGG AAC TCT GCT      96
Ala Ser Thr Asp Thr Met Gly Arg Pro Cys Leu Pro Trp Asn Ser Ala
             20                  25                  30

ACC GTT CTG CAG CAG ACC TAC CAC GCT CAC CGT TCT GAT GCA TTG CAG     144
Thr Val Leu Gln Gln Thr Tyr His Ala His Arg Ser Asp Ala Leu Gln
         35                  40                  45

CTG GGT CTG GGT AAA CAC AAC TAC TGC CGT AAC CCG GAC AAC CGT CGT     192
Leu Gly Leu Gly Lys His Asn Tyr Cys Arg Asn Pro Asp Asn Arg Arg
     50                  55                  60

CGT CCG TGG TGC TAC GTT CAG GTT GGT CTG AAA CCG CTA GTT CAG GAA     240
Arg Pro Trp Cys Tyr Val Gln Val Gly Leu Lys Pro Leu Val Gln Glu
 65                  70                  75                  80

TGC ATG GTT CAC GAC TGC GCT GAC GGT AAA AAA CCG TCT TCT CCG CCG     288
Cys Met Val His Asp Cys Ala Asp Gly Lys Lys Pro Ser Ser Pro Pro
                 85                  90                  95

GAA GAG CTC AAA TTC CAG TGC GGT CAA AAA ACC CTA CGT CCG CGT TTT 336
Glu Glu Leu Lys Phe Gln Cys Gly Gln Lys Thr Leu Arg Pro Arg Phe
            100                 105                 110

AAA ATC ATC GGT GGT GAG TTC ACC ACC ATC GAA AAC CAG CCG TGG TTC 384
Lys Ile Ile Gly Gly Glu Phe Thr Thr Ile Glu Asn Gln Pro Trp Phe
        115                 120                 125

GCT GCT ATC TAC CGT CGT CAC CGT GGT GGT TCT GTT ACC TAC GTT TGC 432
Ala Ala Ile Tyr Arg Arg His Arg Gly Gly Ser Val Thr Tyr Val Cys
    130                 135                 140

GGT GGT TCT CTG ATC TCT CCG TGC TGG GTT ATC TCT GCT ACC CAC TGC 480
Gly Gly Ser Leu Ile Ser Pro Cys Trp Val Ile Ser Ala Thr His Cys
145                 150                 155                 160

TTC ATC GAC TAC CCG AAA AAA GAA GAC TAC ATC GTT TAC CTC GGC CGT 528
Phe Ile Asp Tyr Pro Lys Lys Glu Asp Tyr Ile Val Tyr Leu Gly Arg
                165                 170                 175

TCT CGT TTA AAC TCT AAC ACC CAG GGT GAA ATG AAA TTC GAA GTT GAA 576
Ser Arg Leu Asn Ser Asn Thr Gln Gly Glu Met Lys Phe Glu Val Glu
            180                 185                 190

AAC CTG ATC CTG CAC AAA GAC TAC TCT GCT GAC ACC CTG GCT CAC CAC 624
Asn Leu Ile Leu His Lys Asp Tyr Ser Ala Asp Thr Leu Ala His His
        195                 200                 205

AAC GAC ATC GCT CTG CTA AAA ATC CGT TCT AAA GAA GGT CGT TGC GCT 672
Asn Asp Ile Ala Leu Leu Lys Ile Arg Ser Lys Glu Gly Arg Cys Ala
    210                 215                 220

CAG CCG TCT CGT ACC ATC CAG ACC ATC TGC CTG CCG TCT ATG TAC AAC 720
Gln Pro Ser Arg Thr Ile Gln Thr Ile Cys Leu Pro Ser Met Tyr Asn
225                 230                 235                 240

GAC CCG CAG TTC GGT ACC TCT TGC GAA ATC ACC GGT TTC GGT AAA GAA 768
Asp Pro Gln Phe Gly Thr Ser Cys Glu Ile Thr Gly Phe Gly Lys Glu
                245                 250                 255

AAC TCT ACC GAC TAC CTG TAC CCG GAA CAG CTG AAA ATG ACC GTT GTT 816
Asn Ser Thr Asp Tyr Leu Tyr Pro Glu Gln Leu Lys Met Thr Val Val
            260                 265                 270

AAA CTG ATC TCT CAC CGT GAA TGC CAG CAG CCG CAC TAC TAC GGT TCT 864
Lys Leu Ile Ser His Arg Glu Cys Gln Gln Pro His Tyr Tyr Gly Ser
        275                 280                 285

GAA GTT ACC ACC AAA ATG CTG TGC GCT GCT GAC CCG CAG TGG AAA ACC 912
Glu Val Thr Thr Lys Met Leu Cys Ala Ala Asp Pro Gln Trp Lys Thr
    290                 295                 300

GAC TCT TGC CAA GGT GAC TCT GGT GGT CCA CTA GTT TGC TCT CTC CAG 960
Asp Ser Cys Gln Gly Asp Ser Gly Gly Pro Leu Val Cys Ser Leu Gln
305                 310                 315                 320

GGT CGT ATG ACC CTG ACC GGT ATT GTT TCT TGG GGT CGT GGT TGC GCT 1008
Gly Arg Met Thr Leu Thr Gly Ile Val Ser Trp Gly Arg Gly Cys Ala
                325                 330                 335

CTG AAA GAC AAA CCG GGT GTT TAC ACC CGT GTT TCT CAC TTC CTG CCG 1056
Leu Lys Asp Lys Pro Gly Val Tyr Thr Arg Val Ser His Phe Leu Pro
            340                 345                 350

TGG ATC CGT TCT CAC ACC AAA GAA GAA AAC GGT CTG GCT CTG TTC CCG 1104
Trp Ile Arg Ser His Thr Lys Glu Glu Asn Gly Leu Ala Leu Phe Pro
        355                 360                 365

CGG CCG CTG GGT GGT GGT GGT AAC GGT GAC TTC GAA GAA ATC CCG GAA 1152
Arg Pro Leu Gly Gly Gly Gly Asn Gly Asp Phe Glu Glu Ile Pro Glu
    370                 375                 380

GAA TAC CTG CAA TAA 1167
Glu Tyr Leu Gln *
385

(2) ANGABEN ZU SEQ ID NO: 18:

(i) SEQUENZKENNZEICHEN:
(A) LÄNGE: 389 Aminosäuren
(B) ART: Aminosäure
(D) TOPOLOGIE: linear

(ii) ART DES MOLEKÜLS: Protein
(xi) SEQUENZBESCHREIBUNG: SEQ ID NO: 18:

Met Ser Lys Thr Cys Tyr Glu Gly Asn Gly His Phe Tyr Arg Gly Lys
1               5                   10                  15

Ala Ser Thr Asp Thr Met Gly Arg Pro Cys Leu Pro Trp Asn Ser Ala
            20                  25                  30

Thr Val Leu Gln Gln Thr Tyr His Ala His Arg Ser Asp Ala Leu Gln
        35                  40                  45

Leu Gly Leu Gly Lys His Asn Tyr Cys Arg Asn Pro Asp Asn Arg Arg
    50                  55                  60

Arg Pro Trp Cys Tyr Val Gln Val Gly Leu Lys Pro Leu Val Gln Glu
65                  70                  75                  80

Cys Met Val His Asp Cys Ala Asp Gly Lys Lys Pro Ser Ser Pro Pro
                85                  90                  95

Glu Glu Leu Lys Phe Gln Cys Gly Gln Lys Thr Leu Arg Pro Arg Phe
            100                 105                 110

Lys Ile Ile Gly Gly Glu Phe Thr Thr Ile Glu Asn Gln Pro Trp Phe
        115                 120                 125

Ala Ala Ile Tyr Arg Arg His Arg Gly Gly Ser Val Thr Tyr Val Cys
    130                 135                 140

Gly Gly Ser Leu Ile Ser Pro Cys Trp Val Ile Ser Ala Thr His Cys
145                 150                 155                 160

Phe Ile Asp Tyr Pro Lys Lys Glu Asp Tyr Ile Val Tyr Leu Gly Arg
                165                 170                 175

Ser Arg Leu Asn Ser Asn Thr Gln Gly Glu Met Lys Phe Glu Val Glu
            180                 185                 190

Asn Leu Ile Leu His Lys Asp Tyr Ser Ala Asp Thr Leu Ala His His
        195                 200                 205

Asn Asp Ile Ala Leu Leu Lys Ile Arg Ser Lys Glu Gly Arg Cys Ala
    210                 215                 220

Gln Pro Ser Arg Thr Ile Gln Thr Ile Cys Leu Pro Ser Met Tyr Asn
225                 230                 235                 240

Asp Pro Gln Phe Gly Thr Ser Cys Glu Ile Thr Gly Phe Gly Lys Glu
                245                 250                 255

Asn Ser Thr Asp Tyr Leu Tyr Pro Glu Gln Leu Lys Met Thr Val Val
            260                 265                 270

Lys Leu Ile Ser His Arg Glu Cys Gln Gln Pro His Tyr Tyr Gly Ser
        275                 280                 285

Glu Val Thr Thr Lys Met Leu Cys Ala Ala Asp Pro Gln Trp Lys Thr
    290                 295                 300

Asp Ser Cys Gln Gly Asp Ser Gly Gly Pro Leu Val Cys Ser Leu Gln
305                 310                 315                 320

Gly Arg Met Thr Leu Thr Gly Ile Val Ser Trp Gly Arg Gly Cys Ala
                325                 330                 335

Leu Lys Asp Lys Pro Gly Val Tyr Thr Arg Val Ser His Phe Leu Pro
            340                 345                 350

Trp Ile Arg Ser His Thr Lys Glu Glu Asn Gly Leu Ala Leu Phe Pro
        355                 360                 365

Arg Pro Leu Gly Gly Gly Gly Asn Gly Asp Phe Glu Glu Ile Pro Glu
    370                 375                 380

Glu Tyr Leu Gln  *
385

(2) ANGABEN ZU SEQ ID NO: 19:

(i) SEQUENZKENNZEICHEN:
(A) LÄNGE: 1191 Basenpaare
(B) ART: Nucleotid
(C) STRANGFORM: Einzelstrang
(D) TOPOLOGIE: linear

(ii) ART DES MOLEKÜLS: Sonstige Nucleinsäure

(iii) HYPOTHETISCH: NEIN

(iv) ANTISENSE: NEIN

(ix) MERKMAL:
(A) NAME/SCHLÜSSEL: CDS
(B) LAGE:1..1191
(D) SONSTIGE ANGABEN:/function= "N-term. Methionin wird
in E.coli proteolytisch abgespalten"
/product= "Synthetisches Gen fuer das Protein M32
(pSJ114)"

(xi) SEQUENZBESCHREIBUNG: SEQ ID NO: 19:

ATG AGC AAA ACT TGC TAC GAA GGT AAC GGT CAC TTC TAC CGT GGT AAG      48
Met Ser Lys Thr Cys Tyr Glu Gly Asn Gly His Phe Tyr Arg Gly Lys
  1               5                  10                  15

GCT TCT ACC GAC ACC ATG GGT CGT CCG TGC CTG CCG TGG AAC TCT GCT 96
Ala Ser Thr Asp Thr Met Gly Arg Pro Cys Leu Pro Trp Asn Ser Ala
            20                  25                  30

ACC GTT CTG CAG CAG ACC TAC CAC GCT CAC CGT TCT GAT GCA TTG CAG 144
Thr Val Leu Gln Gln Thr Tyr His Ala His Arg Ser Asp Ala Leu Gln
        35                  40                  45

CTG GGT CTG GGT AAA CAC AAC TAC TGC CGT AAC CCG GAC AAC CGT CGT 192
Leu Gly Leu Gly Lys His Asn Tyr Cys Arg Asn Pro Asp Asn Arg Arg
    50                  55                  60

CGT CCG TGG TGC TAC GTT CAG GTT GGT CTG AAA CCG CTA GTT CAG GAA 240
Arg Pro Trp Cys Tyr Val Gln Val Gly Leu Lys Pro Leu Val Gln Glu
65                  70                  75                  80

TGC ATG GTT CAC GAC TGC GCT GAC GGT AAA AAA CCG TCT TCT CCG CCG 288
Cys Met Val His Asp Cys Ala Asp Gly Lys Lys Pro Ser Ser Pro Pro
                85                  90                  95

GAA GAG CTC AAA TTC CAG TGC GGT CAA AAA ACC CTA CGT CCG CGT TTT 336
Glu Glu Leu Lys Phe Gln Cys Gly Gln Lys Thr Leu Arg Pro Arg Phe
            100                 105                 110

AAA ATC ATC GGT GGT GAG TTC ACC ACC ATC GAA AAC CAG CCG TGG TTC 384
Lys Ile Ile Gly Gly Glu Phe Thr Thr Ile Glu Asn Gln Pro Trp Phe
        115                 120                 125

GCT GCT ATC TAC CGT CGT CAC CGT GGT GGT TCT GTT ACC TAC GTT TGC 432
Ala Ala Ile Tyr Arg Arg His Arg Gly Gly Ser Val Thr Tyr Val Cys
    130                 135                 140

GGT GGT TCT CTG ATC TCT CCG TGC TGG GTT ATC TCT GCT ACC CAC TGC 480
Gly Gly Ser Leu Ile Ser Pro Cys Trp Val Ile Ser Ala Thr His Cys
145                 150                 155                 160

TTC ATC GAC TAC CCG AAA AAA GAA GAC TAC ATC GTT TAC CTC GGC CGT 528
Phe Ile Asp Tyr Pro Lys Lys Glu Asp Tyr Ile Val Tyr Leu Gly Arg
                165                 170                 175

TCT CGT TTA AAC TCT AAC ACC CAG GGT GAA ATG AAA TTC GAA GTT GAA 576
Ser Arg Leu Asn Ser Asn Thr Gln Gly Glu Met Lys Phe Glu Val Glu
            180                 185                 190

AAC CTG ATC CTG CAC AAA GAC TAC TCT GCT GAC ACC CTG GCT CAC CAC 624
Asn Leu Ile Leu His Lys Asp Tyr Ser Ala Asp Thr Leu Ala His His
        195                 200                 205

AAC GAC ATC GCT CTG CTA AAA ATC CGT TCT AAA GAA GGT CGT TGC GCT 672
Asn Asp Ile Ala Leu Leu Lys Ile Arg Ser Lys Glu Gly Arg Cys Ala
    210                 215                 220

CAG CCG TCT CGT ACC ATC CAG ACC ATC TGC CTG CCG TCT ATG TAC AAC 720
Gln Pro Ser Arg Thr Ile Gln Thr Ile Cys Leu Pro Ser Met Tyr Asn
225                 230                 235                 240

GAC CCG CAG TTC GGT ACC TCT TGC GAA ATC ACC GGT TTC GGT AAA GAA 768
Asp Pro Gln Phe Gly Thr Ser Cys Glu Ile Thr Gly Phe Gly Lys Glu
                245                 250                 255

AAC TCT ACC GAC TAC CTG TAC CCG GAA CAG CTG AAA ATG ACC GTT GTT 816
Asn Ser Thr Asp Tyr Leu Tyr Pro Glu Gln Leu Lys Met Thr Val Val
            260                 265                 270

AAA CTG ATC TCT CAC CGT GAA TGC CAG CAG CCG CAC TAC TAC GGT TCT 864
Lys Leu Ile Ser His Arg Glu Cys Gln Gln Pro His Tyr Tyr Gly Ser
        275                 280                 285

GAA GTT ACC ACC AAA ATG CTG TGC GCT GCT GAC CCG CAG TGG AAA ACC 912
Glu Val Thr Thr Lys Met Leu Cys Ala Ala Asp Pro Gln Trp Lys Thr
    290                 295                 300

GAC TCT TGC CAA GGT GAC TCT GGT GGT CCA CTA GTT TGC TCT CTC CAG 960
Asp Ser Cys Gln Gly Asp Ser Gly Gly Pro Leu Val Cys Ser Leu Gln
305                 310                 315                 320

GGT CGT ATG ACC CTG ACC GGT ATT GTT TCT TGG GGT CGT GGT TGC GCT 1008
Gly Arg Met Thr Leu Thr Gly Ile Val Ser Trp Gly Arg Gly Cys Ala
                325                 330                 335

CTG AAA GAC AAA CCG GGT GTT TAC ACC CGT GTT TCT CAC TTC CTG CCG 1056
Leu Lys Asp Lys Pro Gly Val Tyr Thr Arg Val Ser His Phe Leu Pro
            340                 345                 350

TGG ATC CGT TCT CAC ACC AAA GAA GAA AAC GGT CTG GCT CTG AGC CCG 1104
Trp Ile Arg Ser His Thr Lys Glu Glu Asn Gly Leu Ala Leu Ser Pro
        355                 360                 365

GTT AAG GCT TTC CCG CGG CCG TTC CTG CTG CGG AAC CCG AAC GAC AAA 1152
Val Lys Ala Phe Pro Arg Pro Phe Leu Leu Arg Asn Pro Asn Asp Lys
    370                 375                 380

TAC GAA CCG TTC TGG GAA GAC GAA GAA AAA AAC GAA TAA 1191
Tyr Glu Pro Phe Trp Glu Asp Glu Glu Lys Asn Glu *
385                 390                 395

(2) ANGABEN ZU SEQ ID NO: 20:

(i) SEQUENZKENNZEICHEN:
(A) LÄNGE: 397 Aminosäuren
(B) ART: Aminosäure
(D) TOPOLOGIE: linear

(ii) ART DES MOLEKÜLS: Protein
(xi) SEQUENZBESCHREIBUNG: SEQ ID NO: 20:

Met Ser Lys Thr Cys Tyr Glu Gly Asn Gly His Phe Tyr Arg Gly Lys
1               5                   10                  15

Ala Ser Thr Asp Thr Met Gly Arg Pro Cys Leu Pro Trp Asn Ser Ala
            20                  25                  30

Thr Val Leu Gln Gln Thr Tyr His Ala His Arg Ser Asp Ala Leu Gln
        35                  40                  45

Leu Gly Leu Gly Lys His Asn Tyr Cys Arg Asn Pro Asp Asn Arg Arg
    50                  55                  60

Arg Pro Trp Cys Tyr Val Gln Val Gly Leu Lys Pro Leu Val Gln Glu
65                  70                  75                  80

Cys Met Val His Asp Cys Ala Asp Gly Lys Lys Pro Ser Ser Pro Pro
                85                  90                  95

Glu Glu Leu Lys Phe Gln Cys Gly Gln Lys Thr Leu Arg Pro Arg Phe
            100                 105                 110

Lys Ile Ile Gly Gly Glu Phe Thr Thr Ile Glu Asn Gln Pro Trp Phe
        115                 120                 125

Ala Ala Ile Tyr Arg Arg His Arg Gly Gly Ser Val Thr Tyr Val Cys
    130                 135                 140

Gly Gly Ser Leu Ile Ser Pro Cys Trp Val Ile Ser Ala Thr His Cys
145                 150                 155                 160

Phe Ile Asp Tyr Pro Lys Lys Glu Asp Tyr Ile Val Tyr Leu Gly Arg
                165                 170                 175

Ser Arg Leu Asn Ser Asn Thr Gln Gly Glu Met Lys Phe Glu Val Glu
            180                 185                 190

Asn Leu Ile Leu His Lys Asp Tyr Ser Ala Asp Thr Leu Ala His His
        195                 200                 205

Asn Asp Ile Ala Leu Leu Lys Ile Arg Ser Lys Glu Gly Arg Cys Ala
    210                 215                 220

Gln Pro Ser Arg Thr Ile Gln Thr Ile Cys Leu Pro Ser Met Tyr Asn
225                 230                 235                 240

Asp Pro Gln Phe Gly Thr Ser Cys Glu Ile Thr Gly Phe Gly Lys Glu
                245                 250                 255

Asn Ser Thr Asp Tyr Leu Tyr Pro Glu Gln Leu Lys Met Thr Val Val
            260                 265                 270

Lys Leu Ile Ser His Arg Glu Cys Gln Gln Pro His Tyr Tyr Gly Ser
        275                 280                 285

Glu Val Thr Thr Lys Met Leu Cys Ala Ala Asp Pro Gln Trp Lys Thr
    290                 295                 300

Asp Ser Cys Gln Gly Asp Ser Gly Gly Pro Leu Val Cys Ser Leu Gln
305                 310                 315                 320

Gly Arg Met Thr Leu Thr Gly Ile Val Ser Trp Gly Arg Gly Cys Ala
                325                 330                 335

Leu Lys Asp Lys Pro Gly Val Tyr Thr Arg Val Ser His Phe Leu Pro
            340                 345                 350

Trp Ile Arg Ser His Thr Lys Glu Glu Asn Gly Leu Ala Leu Ser Pro
        355                 360                 365

Val Lys Ala Phe Pro Arg Pro Phe Leu Leu Arg Asn Pro Asn Asp Lys
    370                 375                 380

Tyr Glu Pro Phe Trp Glu Asp Glu Glu Lys Asn Glu *
385                 390                 395

(2) ANGABEN ZU SEQ ID NO: 21:

(i) SEQUENZKENNZEICHEN:
(A) LÄNGE: 1098 Basenpaare
(B) ART: Nucleotid
(C) STRANGFORM: Einzelstrang
(D) TOPOLOGIE: linear

(ii) ART DES MOLEKÜLS: Sonstige Nucleinsäure

(iii) HYPOTHETISCH: NEIN

(iv) ANTISENSE: NEIN

(ix) MERKMAL:
(A) NAME/SCHLÜSSEL: CDS
(B) LAGE:1..1098
(D) SONSTIGE ANGABEN:/function= "N-term. Methionin wird
in E.coli proteolytisch abgespalten"
/product= "Synthetisches Gen fuer das Protein M4
(pSJ41)"

(xi) SEQUENZBESCHREIBUNG: SEQ ID NO: 21:

ATG AGC AAA ACT TGC TAC GAA GGT AAC GGT CAC TTC TAC CGT GGT AAG 48
Met Ser Lys Thr Cys Tyr Glu Gly Asn Gly His Phe Tyr Arg Gly Lys
1               5                   10                  15

GCT TCT ACC GAC ACC ATG GGT CGT CCG TGC CTG CCG TGG AAC TCT GCT 96
Ala Ser Thr Asp Thr Met Gly Arg Pro Cys Leu Pro Trp Asn Ser Ala
            20                  25                  30

ACC GTT CTG CAG CAG ACC TAC CAC GCT CAC CGT TCT GAT GCA TTG CAG 144
Thr Val Leu Gln Gln Thr Tyr His Ala His Arg Ser Asp Ala Leu Gln
        35                  40                  45

CTG GGT CTG GGT AAA CAC AAC TAC TGC CGT AAC CCG GAC AAC CGT CGT 192
Leu Gly Leu Gly Lys His Asn Tyr Cys Arg Asn Pro Asp Asn Arg Arg
    50                  55                  60

CGT CCG TGG TGC TAC GTT CAG GTT GGT CTG AAA CCG CTA GTT CAG GAA 240
Arg Pro Trp Cys Tyr Val Gln Val Gly Leu Lys Pro Leu Val Gln Glu
65                  70                  75                  80

TGC ATG GTT CAC GAC TGC GCT GAC GGT AAA AAA CCG TCT TCT CCG CCG 288
Cys Met Val His Asp Cys Ala Asp Gly Lys Lys Pro Ser Ser Pro Pro
                85                  90                  95

GAA GAG CTC AAA TTC CAG TGC GGT CAA AAA ACC CTA CGT CCG CGT TTT 336
Glu Glu Leu Lys Phe Gln Cys Gly Gln Lys Thr Leu Arg Pro Arg Phe
            100                 105                 110

AAA ATC ATC GGT GGT GAG TTC ACC ACC ATC GAA AAC CAG CCG TGG TTC 384
Lys Ile Ile Gly Gly Glu Phe Thr Thr Ile Glu Asn Gln Pro Trp Phe
        115                 120                 125

GCT GCT ATC TAC CGT CGT CAC CGT GGT GGT TCT GTT ACC TAC GTT TGC 432
Ala Ala Ile Tyr Arg Arg His Arg Gly Gly Ser Val Thr Tyr Val Cys
    130                 135                 140

GGT GGT TCT CTG ATC TCT CCG TGC TGG GTT ATC TCT GCT ACC CAC TGC 480
Gly Gly Ser Leu Ile Ser Pro Cys Trp Val Ile Ser Ala Thr His Cys
145                 150                 155                 160

TTC ATC GAC TAC CCG AAA AAA GAA GAC TAC ATC GTT TAC CTC GGC CGT 528
Phe Ile Asp Tyr Pro Lys Lys Glu Asp Tyr Ile Val Tyr Leu Gly Arg
                165                 170                 175

TCT CGT TTA AAC TCT AAC ACC CAG GGT GAA ATG AAA TTC GAA GTT GAA 576
Ser Arg Leu Asn Ser Asn Thr Gln Gly Glu Met Lys Phe Glu Val Glu
            180                 185                 190

AAC CTG ATC CTG CAC AAA GAC TAC TCT GCT GAC ACC CTG GCT CAC CAC 624
Asn Leu Ile Leu His Lys Asp Tyr Ser Ala Asp Thr Leu Ala His His
        195                 200                 205

AAC GAC ATC GCT CTG CTA AAA ATC CGT TCT AAA GAA GGT CGT TGC GCT 672
Asn Asp Ile Ala Leu Leu Lys Ile Arg Ser Lys Glu Gly Arg Cys Ala
    210                 215                 220

CAG CCG TCT CGT ACC ATC CAG ACC ATC TGC CTG CCG TCT ATG TAC AAC 720
Gln Pro Ser Arg Thr Ile Gln Thr Ile Cys Leu Pro Ser Met Tyr Asn
225                 230                 235                 240

GAC CCG CAG TTC GGT ACC TCT TGC GAA ATC ACC GGT TTC GGT AAA GAA 768
Asp Pro Gln Phe Gly Thr Ser Cys Glu Ile Thr Gly Phe Gly Lys Glu
                245                 250                 255

AAC TCT ACC GAC TAC CTG TAC CCG GAA CAG CTG AAA ATG ACC GTT GTT 816
Asn Ser Thr Asp Tyr Leu Tyr Pro Glu Gln Leu Lys Met Thr Val Val
            260                 265                 270

AAA CTG ATC TCT CAC CGT GAA TGC CAG CAG CCG CAC TAC TAC GGT TCT 864
Lys Leu Ile Ser His Arg Glu Cys Gln Gln Pro His Tyr Tyr Gly Ser
        275                 280                 285

GAA GTT ACC ACC AAA ATG CTG TGC GCT GCT GAC CCG CAG TGG AAA ACC 912
Glu Val Thr Thr Lys Met Leu Cys Ala Ala Asp Pro Gln Trp Lys Thr
    290                 295                 300

GAC TCT TGC CAA GGT GAC TCT GGT GGT CCA CTA GTT TGC TCT CTC CAG 960
Asp Ser Cys Gln Gly Asp Ser Gly Gly Pro Leu Val Cys Ser Leu Gln
305                 310                 315                 320

GGT CGT ATG ACC CTG ACC GGT ATT GTT TCT TGG GGT CGT GGT TGC GCT 1008
Gly Arg Met Thr Leu Thr Gly Ile Val Ser Trp Gly Arg Gly Cys Ala
                325                 330                 335

CTG AAA GAC AAA CCG GGT GTT TAC ACC CGT GTT TCT CAC TTC CTG CCG 1056
Leu Lys Asp Lys Pro Gly Val Tyr Thr Arg Val Ser His Phe Leu Pro
            340                 345                 350

TGG ATC CGT TCT CAC ACC AAA GAA GAA AAC GGT CTG GCT CTG 1098
Trp Ile Arg Ser His Thr Lys Glu Glu Asn Gly Leu Ala Leu
        355                 360                 365

(2) ANGABEN ZU SEQ ID NO: 22:

(i) SEQUENZKENNZEICHEN:
(A) LÄNGE: 366 Aminosäuren
(B) ART: Aminosäure
(D) TOPOLOGIE: linear

(ii) ART DES MOLEKÜLS: Protein
(xi) SEQUENZBESCHREIBUNG: SEQ ID NO: 22:

Met Ser Lys Thr Cys Tyr Glu Gly Asn Gly His Phe Tyr Arg Gly Lys
1               5                   10                  15

Ala Ser Thr Asp Thr Met Gly Arg Pro Cys Leu Pro Trp Asn Ser Ala
            20                  25                  30

Thr Val Leu Gln Gln Thr Tyr His Ala His Arg Ser Asp Ala Leu Gln
        35                  40                  45

Leu Gly Leu Gly Lys His Asn Tyr Cys Arg Asn Pro Asp Asn Arg Arg
    50                  55                  60

Arg Pro Trp Cys Tyr Val Gln Val Gly Leu Lys Pro Leu Val Gln Glu
65                  70                  75                  80

Cys Met Val His Asp Cys Ala Asp Gly Lys Lys Pro Ser Ser Pro Pro
                85                  90                  95

Glu Glu Leu Lys Phe Gln Cys Gly Gln Lys Thr Leu Arg Pro Arg Phe
            100                 105                 110

Lys Ile Ile Gly Gly Glu Phe Thr Thr Ile Glu Asn Gln Pro Trp Phe
        115                 120                 125

Ala Ala Ile Tyr Arg Arg His Arg Gly Gly Ser Val Thr Tyr Val Cys
    130                 135                 140

Gly Gly Ser Leu Ile Ser Pro Cys Trp Val Ile Ser Ala Thr His Cys
145                 150                 155                 160

Phe Ile Asp Tyr Pro Lys Lys Glu Asp Tyr Ile Val Tyr Leu Gly Arg
                165                 170                 175

Ser Arg Leu Asn Ser Asn Thr Gln Gly Glu Met Lys Phe Glu Val Glu
            180                 185                 190

Asn Leu Ile Leu His Lys Asp Tyr Ser Ala Asp Thr Leu Ala His His
        195                 200                 205

Asn Asp Ile Ala Leu Leu Lys Ile Arg Ser Lys Glu Gly Arg Cys Ala
    210                 215                 220

Gln Pro Ser Arg Thr Ile Gln Thr Ile Cys Leu Pro Ser Met Tyr Asn
225                 230                 235                 240

Asp Pro Gln Phe Gly Thr Ser Cys Glu Ile Thr Gly Phe Gly Lys Glu
                245                 250                 255

Asn Ser Thr Asp Tyr Leu Tyr Pro Glu Gln Leu Lys Met Thr Val Val
            260                 265                 270

Lys Leu Ile Ser His Arg Glu Cys Gln Gln Pro His Tyr Tyr Gly Ser
        275                 280                 285

Glu Val Thr Thr Lys Met Leu Cys Ala Ala Asp Pro Gln Trp Lys Thr
    290                 295                 300

Asp Ser Cys Gln Gly Asp Ser Gly Gly Pro Leu Val Cys Ser Leu Gln
305                 310                 315                 320

Gly Arg Met Thr Leu Thr Gly Ile Val Ser Trp Gly Arg Gly Cys Ala
                325                 330                 335

Leu Lys Asp Lys Pro Gly Val Tyr Thr Arg Val Ser His Phe Leu Pro
            340                 345                 350

Trp Ile Arg Ser His Thr Lys Glu Glu Asn Gly Leu Ala Leu
        355                 360                 365

(2) ANGABEN ZU SEQ ID NO: 23:

(i) SEQUENZKENNZEICHEN:
(A) LÄNGE: 1185 Basenpaare
(B) ART: Nucleotid
(C) STRANGFORM: Einzelstrang
(D) TOPOLOGIE: linear

(ii) ART DES MOLEKÜLS: Sonstige Nucleinsäure

(iii) HYPOTHETISCH: NEIN

(iv) ANTISENSE: NEIN

(ix) MERKMAL:
(A) NAME/SCHLÜSSEL: CDS
(B) LAGE:1..1185
(D) SONSTIGE ANGABEN:/function= "N-term. Methionin wird
in E.coli proteolytisch abgespalten"
/product= "Synthetisches Gen fuer das Protein M11
(pSJ69)'"

(xi) SEQUENZBESCHREIBUNG: SEQ ID NO: 23:

ATG AGC AAA ACT TGC TAC GAA GGT AAC GGT CAC TTC TAC CGT GGT AAG 48
Met Ser Lys Thr Cys Tyr Glu Gly Asn Gly His Phe Tyr Arg Gly Lys
1               5                   10                  15

GCT TCT ACC GAC ACC ATG GGT CGT CCG TGC CTG CCG TGG AAC TCT GCT 96
Ala Ser Thr Asp Thr Met Gly Arg Pro Cys Leu Pro Trp Asn Ser Ala
            20                  25                  30

ACC GTT CTG CAG CAG ACC TAC CAC GCT CAC CGT TCT GAT GCA TTG CAG 144
Thr Val Leu Gln Gln Thr Tyr His Ala His Arg Ser Asp Ala Leu Gln
        35                  40                  45

CTG GGT CTG GGT AAA CAC AAC TAC TGC CGT AAC CCG GAC AAC CGT CGT 192
Leu Gly Leu Gly Lys His Asn Tyr Cys Arg Asn Pro Asp Asn Arg Arg
    50                  55                  60

CGT CCG TGG TGC TAC GTT CAG GTT GGT CTG AAA CCG CTA GTT CAG GAA 240
Arg Pro Trp Cys Tyr Val Gln Val Gly Leu Lys Pro Leu Val Gln Glu
65                  70                  75                  80

TGC ATG GTT CAC GAC TGC GCT GAC GGT AAA AAA CCG TCT TCT CCG CCG 288
Cys Met Val His Asp Cys Ala Asp Gly Lys Lys Pro Ser Ser Pro Pro
                85                  90                  95

GAA GAG CTC AAA TTC CAG TGC GGT CAA AAA ACC CTA CGT CCG CGT TTT 336
Glu Glu Leu Lys Phe Gln Cys Gly Gln Lys Thr Leu Arg Pro Arg Phe
            100                 105                 110

AAA ATC ATC GGT GGT GAG TTC ACC ACC ATC GAA AAC CAG CCG TGG TTC 384
Lys Ile Ile Gly Gly Glu Phe Thr Thr Ile Glu Asn Gln Pro Trp Phe
        115                 120                 125

GCT GCT ATC TAC CGT CGT CAC CGT GGT GGT TCT GTT ACC TAC GTT TGC 432
Ala Ala Ile Tyr Arg Arg His Arg Gly Gly Ser Val Thr Tyr Val Cys
    130                 135                 140

GGT GGT TCT CTG ATC TCT CCG TGC TGG GTT ATC TCT GCT ACC CAC TGC 480
Gly Gly Ser Leu Ile Ser Pro Cys Trp Val Ile Ser Ala Thr His Cys
145                 150                 155                 160

TTC ATC GAC TAC CCG AAA AAA GAA GAC TAC ATC GTT TAC CTC GGC CGT 528
Phe Ile Asp Tyr Pro Lys Lys Glu Asp Tyr Ile Val Tyr Leu Gly Arg
                165                 170                 175

TCT CGT TTA AAC TCT AAC ACC CAG GGT GAA ATG AAA TTC GAA GTT GAA 576
Ser Arg Leu Asn Ser Asn Thr Gln Gly Glu Met Lys Phe Glu Val Glu
            180                 185                 190

AAC CTG ATC CTG CAC AAA GAC TAC TCT GCT GAC ACC CTG GCT CAC CAC 624
Asn Leu Ile Leu His Lys Asp Tyr Ser Ala Asp Thr Leu Ala His His
        195                 200                 205

AAC GAC ATC GCT CTG CTA AAA ATC CGT TCT AAA GAA GGT CGT TGC GCT 672
Asn Asp Ile Ala Leu Leu Lys Ile Arg Ser Lys Glu Gly Arg Cys Ala
    210                 215                 220

CAG CCG TCT CGT ACC ATC CAG ACC ATC TGC CTG CCG TCT ATG TAC AAC 720
Gln Pro Ser Arg Thr Ile Gln Thr Ile Cys Leu Pro Ser Met Tyr Asn
225                 230                 235                 240

GAC CCG CAG TTC GGT ACC TCT TGC GAA ATC ACC GGT TTC GGT AAA GAA 768
Asp Pro Gln Phe Gly Thr Ser Cys Glu Ile Thr Gly Phe Gly Lys Glu
                245                 250                 255

AAC TCT ACC GAC TAC CTG TAC CCG GAA CAG CTG AAA ATG ACC GTT GTT 816
Asn Ser Thr Asp Tyr Leu Tyr Pro Glu Gln Leu Lys Met Thr Val Val
            260                 265                 270

AAA CTG ATC TCT CAC CGT GAA TGC CAG CAG CCG CAC TAC TAC GGT TCT 864
Lys Leu Ile Ser His Arg Glu Cys Gln Gln Pro His Tyr Tyr Gly Ser
        275                 280                 285

GAA GTT ACC ACC AAA ATG CTG TGC GCT GCT GAC CCG CAG TGG AAA ACC 912
Glu Val Thr Thr Lys Met Leu Cys Ala Ala Asp Pro Gln Trp Lys Thr
    290                 295                 300

GAC TCT TGC CAA GGT GAC TCT GGT GGT CCA CTA GTT TGC TCT CTC CAG 960
Asp Ser Cys Gln Gly Asp Ser Gly Gly Pro Leu Val Cys Ser Leu Gln
305                 310                 315                 320

GGT CGT ATG ACC CTG ACC GGT ATT GTT TCT TGG GGT CGT GGT TGC GCT 1008
Gly Arg Met Thr Leu Thr Gly Ile Val Ser Trp Gly Arg Gly Cys Ala
                325                 330                 335

CTG AAA GAC AAA CCG GGT GTT TAC ACC CGT GTT TCT CAC TTC CTG CCG 1056
Leu Lys Asp Lys Pro Gly Val Tyr Thr Arg Val Ser His Phe Leu Pro
            340                 345                 350

TGG ATC CGT TCT CAC ACC AAA GAA GAA AAC GGT CTG GCT CTG AGC CCG 1104
Trp Ile Arg Ser His Thr Lys Glu Glu Asn Gly Leu Ala Leu Ser Pro
        355                 360                 365

CCG TCT CCG CCG GGT GGT TTC CCG CGT CCG GGT GGT GGT GGT AAC GGT 1152
Pro Ser Pro Pro Gly Gly Phe Pro Arg Pro Gly Gly Gly Gly Asn Gly
    370                 375                 380

GAC TTC GAA GAA ATC CCG GAA GAA TAC CTG TAA 1185
Asp Phe Glu Glu Ile Pro Glu Glu Tyr Leu *
385                 390                 395

(2) ANGABEN ZU SEQ ID NO: 24:

(i) SEQUENZKENNZEICHEN:
(A) LÄNGE: 395 Aminosäuren
(B) ART: Aminosäure
(D) TOPOLOGIE: linear

(ii) ART DES MOLEKÜLS: Protein
(xi) SEQUENZBESCHREIBUNG: SEQ ID NO: 24:

Met Ser Lys Thr Cys Tyr Glu Gly Asn Gly His Phe Tyr Arg Gly Lys
1               5                   10                  15

Ala Ser Thr Asp Thr Met Gly Arg Pro Cys Leu Pro Trp Asn Ser Ala
            20                  25                  30

Thr Val Leu Gln Gln Thr Tyr His Ala His Arg Ser Asp Ala Leu Gln
        35                  40                  45

Leu Gly Leu Gly Lys His Asn Tyr Cys Arg Asn Pro Asp Asn Arg Arg
    50                  55                  60

Arg Pro Trp Cys Tyr Val Gln Val Gly Leu Lys Pro Leu Val Gln Glu
65                  70                  75                  80

Cys Met Val His Asp Cys Ala Asp Gly Lys Lys Pro Ser Ser Pro Pro
                85                  90                  95

Glu Glu Leu Lys Phe Gln Cys Gly Gln Lys Thr Leu Arg Pro Arg Phe
            100                 105                 110

Lys Ile Ile Gly Gly Glu Phe Thr Thr Ile Glu Asn Gln Pro Trp Phe
        115                 120                 125

Ala Ala Ile Tyr Arg Arg His Arg Gly Gly Ser Val Thr Tyr Val Cys
    130                 135                 140

Gly Gly Ser Leu Ile Ser Pro Cys Trp Val Ile Ser Ala Thr His Cys
145                 150                 155                 160

Phe Ile Asp Tyr Pro Lys Lys Glu Asp Tyr Ile Val Tyr Leu Gly Arg
                165                 170                 175

Ser Arg Leu Asn Ser Asn Thr Gln Gly Glu Met Lys Phe Glu Val Glu
            180                 185                 190

Asn Leu Ile Leu His Lys Asp Tyr Ser Ala Asp Thr Leu Ala His His
        195                 200                 205

Asn Asp Ile Ala Leu Leu Lys Ile Arg Ser Lys Glu Gly Arg Cys Ala
    210                 215                 220

Gln Pro Ser Arg Thr Ile Gln Thr Ile Cys Leu Pro Ser Met Tyr Asn
225                 230                 235                 240

Asp Pro Gln Phe Gly Thr Ser Cys Glu Ile Thr Gly Phe Gly Lys Glu
                245                 250                 255

Asn Ser Thr Asp Tyr Leu Tyr Pro Glu Gln Leu Lys Met Thr Val Val
            260                 265                 270

Lys Leu Ile Ser His Arg Glu Cys Gln Gln Pro His Tyr Tyr Gly Ser
        275                 280                 285

Glu Val Thr Thr Lys Met Leu Cys Ala Ala Asp Pro Gln Trp Lys Thr
    290                 295                 300

Asp Ser Cys Gln Gly Asp Ser Gly Gly Pro Leu Val Cys Ser Leu Gln
305                 310                 315                 320

Gly Arg Met Thr Leu Thr Gly Ile Val Ser Trp Gly Arg Gly Cys Ala
                325                 330                 335

Leu Lys Asp Lys Pro Gly Val Tyr Thr Arg Val Ser His Phe Leu Pro
            340                 345                 350

Trp Ile Arg Ser His Thr Lys Glu Glu Asn Gly Leu Ala Leu Ser Pro
        355                 360                 365

Pro Ser Pro Pro Gly Gly Phe Pro Arg Pro Gly Gly Gly Gly Asn Gly
    370                 375                 380

Asp Phe Glu Glu Ile Pro Glu Glu Tyr Leu  *
385                 390                 395

(2) ANGABEN ZU SEQ ID NO: 25:

(i) SEQUENZKENNZEICHEN:
(A) LÄNGE: 1176 Basenpaare
(B) ART: Nucleotid
(C) STRANGFORM: Einzelstrang
(D) TOPOLOGIE: linear

(ii) ART DES MOLEKÜLS: Sonstige Nucleinsäure

(iii) HYPOTHETISCH: NEIN

(iv) ANTISENSE: NEIN

(ix) MERKMAL:
(A) NAME/SCHLÜSSEL: CDS
(B) LAGE:1..1176
(D) SONSTIGE ANGABEN:/function= "N-term. Methionin wird
in E.coli proteolytisch abgespalten"
/product= "Synthetisches Gen fuer das Protein M12
(pSJ76)"

(xi) SEQUENZBESCHREIBUNG: SEQ ID NO: 25:

ATG AGC AAA ACT TGC TAC GAA GGT AAC GGT CAC TTC TAC CGT GGT AAG      48
Met Ser Lys Thr Cys Tyr Glu Gly Asn Gly His Phe Tyr Arg Gly Lys
  1               5                  10                  15

GCT TCT ACC GAC ACC ATG GGT CGT CCG TGC CTG CCG TGG AAC TCT GCT      96
Ala Ser Thr Asp Thr Met Gly Arg Pro Cys Leu Pro Trp Asn Ser Ala
             20                  25                  30

ACC GTT CTG CAG CAG ACC TAC CAC GCT CAC CGT TCT GAT GCA TTG CAG     144
Thr Val Leu Gln Gln Thr Tyr His Ala His Arg Ser Asp Ala Leu Gln
         35                  40                  45

CTG GGT CTG GGT AAA CAC AAC TAC TGC CGT AAC CCG GAC AAC CGT CGT     192
Leu Gly Leu Gly Lys His Asn Tyr Cys Arg Asn Pro Asp Asn Arg Arg
     50                  55                  60

CGT CCG TGG TGC TAC GTT CAG GTT GGT CTG AAA CCG CTA GTT CAG GAA 240
Arg Pro Trp Cys Tyr Val Gln Val Gly Leu Lys Pro Leu Val Gln Glu
65                  70                  75                  80

TGC ATG GTT CAC GAC TGC GCT GAC GGT AAA AAA CCG TCT TCT CCG CCG 288
Cys Met Val His Asp Cys Ala Asp Gly Lys Lys Pro Ser Ser Pro Pro
                85                  90                  95

GAA GAG CTC AAA TTC CAG TGC GGT CAA AAA ACC CTA CGT CCG CGT TTT 336
Glu Glu Leu Lys Phe Gln Cys Gly Gln Lys Thr Leu Arg Pro Arg Phe
            100                 105                 110

AAA ATC ATC GGT GGT GAG TTC ACC ACC ATC GAA AAC CAG CCG TGG TTC 384
Lys Ile Ile Gly Gly Glu Phe Thr Thr Ile Glu Asn Gln Pro Trp Phe
        115                 120                 125

GCT GCT ATC TAC CGT CGT CAC CGT GGT GGT TCT GTT ACC TAC GTT TGC 432
Ala Ala Ile Tyr Arg Arg His Arg Gly Gly Ser Val Thr Tyr Val Cys
    130                 135                 140

GGT GGT TCT CTG ATC TCT CCG TGC TGG GTT ATC TCT GCT ACC CAC TGC 480
Gly Gly Ser Leu Ile Ser Pro Cys Trp Val Ile Ser Ala Thr His Cys
145                 150                 155                 160

TTC ATC GAC TAC CCG AAA AAA GAA GAC TAC ATC GTT TAC CTC GGC CGT 528
Phe Ile Asp Tyr Pro Lys Lys Glu Asp Tyr Ile Val Tyr Leu Gly Arg
                165                 170                 175

TCT CGT TTA AAC TCT AAC ACC CAG GGT GAA ATG AAA TTC GAA GTT GAA 576
Ser Arg Leu Asn Ser Asn Thr Gln Gly Glu Met Lys Phe Glu Val Glu
            180                 185                 190

AAC CTG ATC CTG CAC AAA GAC TAC TCT GCT GAC ACC CTG GCT CAC CAC 624
Asn Leu Ile Leu His Lys Asp Tyr Ser Ala Asp Thr Leu Ala His His
        195                 200                 205

AAC GAC ATC GCT CTG CTA AAA ATC CGT TCT AAA GAA GGT CGT TGC GCT 672
Asn Asp Ile Ala Leu Leu Lys Ile Arg Ser Lys Glu Gly Arg Cys Ala
    210                 215                 220

CAG CCG TCT CGT ACC ATC CAG ACC ATC TGC CTG CCG TCT ATG TAC AAC 720
Gln Pro Ser Arg Thr Ile Gln Thr Ile Cys Leu Pro Ser Met Tyr Asn
225                 230                 235                 240

GAC CCG CAG TTC GGT ACC TCT TGC GAA ATC ACC GGT TTC GGT AAA GAA 768
Asp Pro Gln Phe Gly Thr Ser Cys Glu Ile Thr Gly Phe Gly Lys Glu
                245                 250                 255

AAC TCT ACC GAC TAC CTG TAC CCG GAA CAG CTG AAA ATG ACC GTT GTT 816
Asn Ser Thr Asp Tyr Leu Tyr Pro Glu Gln Leu Lys Met Thr Val Val
            260                 265                 270

AAA CTG ATC TCT CAC CGT GAA TGC CAG CAG CCG CAC TAC TAC GGT TCT 864
Lys Leu Ile Ser His Arg Glu Cys Gln Gln Pro His Tyr Tyr Gly Ser
        275                 280                 285

GAA GTT ACC ACC AAA ATG CTG TGC GCT GCT GAC CCG CAG TGG AAA ACC 912
Glu Val Thr Thr Lys Met Leu Cys Ala Ala Asp Pro Gln Trp Lys Thr
    290                 295                 300

GAC TCT TGC CAA GGT GAC TCT GGT GGT CCA CTA GTT TGC TCT CTC CAG 960
Asp Ser Cys Gln Gly Asp Ser Gly Gly Pro Leu Val Cys Ser Leu Gln
305                 310                 315                 320

GGT CGT ATG ACC CTG ACC GGT ATT GTT TCT TGG GGT CGT GGT TGC GCT 1008
Gly Arg Met Thr Leu Thr Gly Ile Val Ser Trp Gly Arg Gly Cys Ala
                325                 330                 335

CTG AAA GAC AAA CCG GGT GTT TAC ACC CGT GTT TCT CAC TTC CTG CCG 1056
Leu Lys Asp Lys Pro Gly Val Tyr Thr Arg Val Ser His Phe Leu Pro
            340                 345                 350

TGG ATC CGT TCT CAC ACC AAA GAA GAA AAC GGT CTG GCT CTG AGC CCG 1104
Trp Ile Arg Ser His Thr Lys Glu Glu Asn Gly Leu Ala Leu Ser Pro
        355                 360                 365

GTT AAG GCT TTC CCG AGG CCT GGT GGT GGT GGT AAC GGT GAC TTC GAA 1152
Val Lys Ala Phe Pro Arg Pro Gly Gly Gly Gly Asn Gly Asp Phe Glu
    370                 375                 380

GAA ATC CCG GAA GAA TAC CTG TGA 1176
Glu Ile Pro Glu Glu Tyr Leu *
385                 390

(2) ANGABEN ZU SEQ ID NO: 26:

(i) SEQUENZKENNZEICHEN:
(A) LÄNGE: 392 Aminosäuren
(B) ART: Aminosäure
(D) TOPOLOGIE: linear

(ii) ART DES MOLEKÜLS: Protein
(xi) SEQUENZBESCHREIBUNG: SEQ ID NO: 26:

Met Ser Lys Thr Cys Tyr Glu Gly Asn Gly His Phe Tyr Arg Gly Lys
1               5                   10                  15

Ala Ser Thr Asp Thr Met Gly Arg Pro Cys Leu Pro Trp Asn Ser Ala
            20                  25                  30

Thr Val Leu Gln Gln Thr Tyr His Ala His Arg Ser Asp Ala Leu Gln
        35                  40                  45

Leu Gly Leu Gly Lys His Asn Tyr Cys Arg Asn Pro Asp Asn Arg Arg
    50                  55                  60

Arg Pro Trp Cys Tyr Val Gln Val Gly Leu Lys Pro Leu Val Gln Glu
65                  70                  75                  80

Cys Met Val His Asp Cys Ala Asp Gly Lys Lys Pro Ser Ser Pro Pro
                85                  90                  95

Glu Glu Leu Lys Phe Gln Cys Gly Gln Lys Thr Leu Arg Pro Arg Phe
            100                 105                 110

Lys Ile Ile Gly Gly Glu Phe Thr Thr Ile Glu Asn Gln Pro Trp Phe
        115                 120                 125

Ala Ala Ile Tyr Arg Arg His Arg Gly Gly Ser Val Thr Tyr Val Cys
    130                 135                 140

Gly Gly Ser Leu Ile Ser Pro Cys Trp Val Ile Ser Ala Thr His Cys
145                 150                 155                 160

Phe Ile Asp Tyr Pro Lys Lys Glu Asp Tyr Ile Val Tyr Leu Gly Arg
                165                 170                 175

Ser Arg Leu Asn Ser Asn Thr Gln Gly Glu Met Lys Phe Glu Val Glu
            180                 185                 190

Asn Leu Ile Leu His Lys Asp Tyr Ser Ala Asp Thr Leu Ala His His
        195                 200                 205

Asn Asp Ile Ala Leu Leu Lys Ile Arg Ser Lys Glu Gly Arg Cys Ala
    210                 215                 220

Gln Pro Ser Arg Thr Ile Gln Thr Ile Cys Leu Pro Ser Met Tyr Asn
225                 230                 235                 240

Asp Pro Gln Phe Gly Thr Ser Cys Glu Ile Thr Gly Phe Gly Lys Glu
                245                 250                 255

Asn Ser Thr Asp Tyr Leu Tyr Pro Glu Gln Leu Lys Met Thr Val Val
            260                 265                 270

Lys Leu Ile Ser His Arg Glu Cys Gln Gln Pro His Tyr Tyr Gly Ser
        275                 280                 285

Glu Val Thr Thr Lys Met Leu Cys Ala Ala Asp Pro Gln Trp Lys Thr
    290                 295                 300

Asp Ser Cys Gln Gly Asp Ser Gly Gly Pro Leu Val Cys Ser Leu Gln
305                 310                 315                 320

Gly Arg Met Thr Leu Thr Gly Ile Val Ser Trp Gly Arg Gly Cys Ala
                325                 330                 335

Leu Lys Asp Lys Pro Gly Val Tyr Thr Arg Val Ser His Phe Leu Pro
            340                 345                 350

Trp Ile Arg Ser His Thr Lys Glu Glu Asn Gly Leu Ala Leu Ser Pro
        355                 360                 365

Val Lys Ala Phe Pro Arg Pro Gly Gly Gly Gly Asn Gly Asp Phe Glu
    370                 375                 380

Glu Ile Pro Glu Glu Tyr Leu  *
385                 390

(2) ANGABEN ZU SEQ ID NO: 27:

    (i) SEQUENZKENNZEICHEN:
        (A) LÄNGE: 1194 Basenpaare
        (B) ART: Nucleotid
        (C) STRANGFORM: Einzelstrang
        (D) TOPOLOGIE: linear

    (ii) ART DES MOLEKÜLS: Sonstige Nucleinsäure

    (iii) HYPOTHETISCH: NEIN

    (iv) ANTISENSE: NEIN

    (ix) MERKMAL:
        (A) NAME/SCHLÜSSEL: CDS
        (B) LAGE:1..1194
        (D) SONSTIGE ANGABEN:/function= "N-term. Methionin wird
            in E.coli proteolytisch abgespalten"
            /product= "Synthetisches Gen fuer das Protein M13
            (pSJ77)"

(xi) SEQUENZBESCHREIBUNG: SEQ ID NO: 27:

ATG AGC AAA ACT TGC TAC GAA GGT AAC GGT CAC TTC TAC CGT GGT AAG 48
Met Ser Lys Thr Cys Tyr Glu Gly Asn Gly His Phe Tyr Arg Gly Lys
1               5                   10                  15

GCT TCT ACC GAC ACC ATG GGT CGT CCG TGC CTG CCG TGG AAC TCT GCT 96
Ala Ser Thr Asp Thr Met Gly Arg Pro Cys Leu Pro Trp Asn Ser Ala
            20                  25                  30

ACC GTT CTG CAG CAG ACC TAC CAC GCT CAC CGT TCT GAT GCA TTG CAG 144
Thr Val Leu Gln Gln Thr Tyr His Ala His Arg Ser Asp Ala Leu Gln
        35                  40                  45

CTG GGT CTG GGT AAA CAC AAC TAC TGC CGT AAC CCG GAC AAC CGT CGT 192
Leu Gly Leu Gly Lys His Asn Tyr Cys Arg Asn Pro Asp Asn Arg Arg
    50                  55                  60

CGT CCG TGG TGC TAC GTT CAG GTT GGT CTG AAA CCG CTA GTT CAG GAA 240
Arg Pro Trp Cys Tyr Val Gln Val Gly Leu Lys Pro Leu Val Gln Glu
65                  70                  75                  80

TGC ATG GTT CAC GAC TGC GCT GAC GGT AAA AAA CCG TCT TCT CCG CCG 288
Cys Met Val His Asp Cys Ala Asp Gly Lys Lys Pro Ser Ser Pro Pro
                85                  90                  95

GAA GAG CTC AAA TTC CAG TGC GGT CAA AAA ACC CTA CGT CCG CGT TTT 336
Glu Glu Leu Lys Phe Gln Cys Gly Gln Lys Thr Leu Arg Pro Arg Phe
            100                 105                 110

AAA ATC ATC GGT GGT GAG TTC ACC ACC ATC GAA AAC CAG CCG TGG TTC 384
Lys Ile Ile Gly Gly Glu Phe Thr Thr Ile Glu Asn Gln Pro Trp Phe
        115                 120                 125

GCT GCT ATC TAC CGT CGT CAC CGT GGT GGT TCT GTT ACC TAC GTT TGC 432
Ala Ala Ile Tyr Arg Arg His Arg Gly Gly Ser Val Thr Tyr Val Cys
    130                 135                 140

GGT GGT TCT CTG ATC TCT CCG TGC TGG GTT ATC TCT GCT ACC CAC TGC 480
Gly Gly Ser Leu Ile Ser Pro Cys Trp Val Ile Ser Ala Thr His Cys
145                 150                 155                 160

TTC ATC GAC TAC CCG AAA AAA GAA GAC TAC ATC GTT TAC CTC GGC CGT 528
Phe Ile Asp Tyr Pro Lys Lys Glu Asp Tyr Ile Val Tyr Leu Gly Arg
                165                 170                 175

TCT CGT TTA AAC TCT AAC ACC CAG GGT GAA ATG AAA TTC GAA GTT GAA 576
Ser Arg Leu Asn Ser Asn Thr Gln Gly Glu Met Lys Phe Glu Val Glu
            180                 185                 190

AAC CTG ATC CTG CAC AAA GAC TAC TCT GCT GAC ACC CTG GCT CAC CAC 624
Asn Leu Ile Leu His Lys Asp Tyr Ser Ala Asp Thr Leu Ala His His
        195                 200                 205

AAC GAC ATC GCT CTG CTA AAA ATC CGT TCT AAA GAA GGT CGT TGC GCT 672
Asn Asp Ile Ala Leu Leu Lys Ile Arg Ser Lys Glu Gly Arg Cys Ala
    210                 215                 220

CAG CCG TCT CGT ACC ATC CAG ACC ATC TGC CTG CCG TCT ATG TAC AAC 720
Gln Pro Ser Arg Thr Ile Gln Thr Ile Cys Leu Pro Ser Met Tyr Asn
225                 230                 235                 240

GAC CCG CAG TTC GGT ACC TCT TGC GAA ATC ACC GGT TTC GGT AAA GAA 768
Asp Pro Gln Phe Gly Thr Ser Cys Glu Ile Thr Gly Phe Gly Lys Glu
                245                 250                 255

AAC TCT ACC GAC TAC CTG TAC CCG GAA CAG CTG AAA ATG ACC GTT GTT 816
Asn Ser Thr Asp Tyr Leu Tyr Pro Glu Gln Leu Lys Met Thr Val Val
            260                 265                 270

AAA CTG ATC TCT CAC CGT GAA TGC CAG CAG CCG CAC TAC TAC GGT TCT 864
Lys Leu Ile Ser His Arg Glu Cys Gln Gln Pro His Tyr Tyr Gly Ser
        275                 280                 285

GAA GTT ACC ACC AAA ATG CTG TGC GCT GCT GAC CCG CAG TGG AAA ACC 912
Glu Val Thr Thr Lys Met Leu Cys Ala Ala Asp Pro Gln Trp Lys Thr
    290                 295                 300

GAC TCT TGC CAA GGT GAC TCT GGT GGT CCA CTA GTT TGC TCT CTC CAG 960
Asp Ser Cys Gln Gly Asp Ser Gly Gly Pro Leu Val Cys Ser Leu Gln
305                 310                 315                 320

GGT CGT ATG ACC CTG ACC GGT ATT GTT TCT TGG GGT CGT GGT TGC GCT 1008
Gly Arg Met Thr Leu Thr Gly Ile Val Ser Trp Gly Arg Gly Cys Ala
                325                 330                 335

CTG AAA GAC AAA CCG GGT GTT TAC ACC CGT GTT TCT CAC TTC CTG CCG 1056
Leu Lys Asp Lys Pro Gly Val Tyr Thr Arg Val Ser His Phe Leu Pro
            340                 345                 350

TGG ATC CGT TCT CAC ACC AAA GAA GAA AAC GGT CTG GCT CTG AGC CCG 1104
Trp Ile Arg Ser His Thr Lys Glu Glu Asn Gly Leu Ala Leu Ser Pro
        355                 360                 365

CCG AGC CCG CCG AGC CCG CCG GGT GGT TTC CCG AGG CCT GGT GGT GGT 1152
Pro Ser Pro Pro Ser Pro Pro Gly Gly Phe Pro Arg Pro Gly Gly Gly
    370                 375                 380

GGT AAC GGT GAC TTC GAA GAA ATC CCG GAA GAG TAC CTG TGA 1194
Gly Asn Gly Asp Phe Glu Glu Ile Pro Glu Glu Tyr Leu *
385                 390                 395

(2) ANGABEN ZU SEQ ID NO: 28:

(i) SEQUENZKENNZEICHEN:
(A) LÄNGE: 398 Aminosäuren
(B) ART: Aminosäure
(D) TOPOLOGIE: linear

(ii) ART DES MOLEKÜLS: Protein
(xi) SEQUENZBESCHREIBUNG: SEQ ID NO: 28:

Met Ser Lys Thr Cys Tyr Glu Gly Asn Gly His Phe Tyr Arg Gly Lys
1               5                   10                  15

Ala Ser Thr Asp Thr Met Gly Arg Pro Cys Leu Pro Trp Asn Ser Ala
            20                  25                  30

Thr Val Leu Gln Gln Thr Tyr His Ala His Arg Ser Asp Ala Leu Gln
        35                  40                  45

Leu Gly Leu Gly Lys His Asn Tyr Cys Arg Asn Pro Asp Asn Arg Arg
    50                  55                  60

Arg Pro Trp Cys Tyr Val Gln Val Gly Leu Lys Pro Leu Val Gln Glu
65                  70                  75                  80

Cys Met Val His Asp Cys Ala Asp Gly Lys Lys Pro Ser Ser Pro Pro
                85                  90                  95

Glu Glu Leu Lys Phe Gln Cys Gly Gln Lys Thr Leu Arg Pro Arg Phe
            100                 105                 110

Lys Ile Ile Gly Gly Glu Phe Thr Thr Ile Glu Asn Gln Pro Trp Phe
        115                 120                 125

Ala Ala Ile Tyr Arg Arg His Arg Gly Gly Ser Val Thr Tyr Val Cys
    130                 135                 140

Gly Gly Ser Leu Ile Ser Pro Cys Trp Val Ile Ser Ala Thr His Cys
145                 150                 155                 160

Phe Ile Asp Tyr Pro Lys Lys Glu Asp Tyr Ile Val Tyr Leu Gly Arg
                165                 170                 175

Ser Arg Leu Asn Ser Asn Thr Gln Gly Glu Met Lys Phe Glu Val Glu
            180                 185                 190

Asn Leu Ile Leu His Lys Asp Tyr Ser Ala Asp Thr Leu Ala His His
        195                 200                 205

Asn Asp Ile Ala Leu Leu Lys Ile Arg Ser Lys Glu Gly Arg Cys Ala
    210                 215                 220

Gln Pro Ser Arg Thr Ile Gln Thr Ile Cys Leu Pro Ser Met Tyr Asn
225                 230                 235                 240

Asp Pro Gln Phe Gly Thr Ser Cys Glu Ile Thr Gly Phe Gly Lys Glu
                245                 250                 255

Asn Ser Thr Asp Tyr Leu Tyr Pro Glu Gln Leu Lys Met Thr Val Val
            260                 265                 270

Lys Leu Ile Ser His Arg Glu Cys Gln Gln Pro His Tyr Tyr Gly Ser
        275                 280                 285

Glu Val Thr Thr Lys Met Leu Cys Ala Ala Asp Pro Gln Trp Lys Thr
    290                 295                 300

Asp Ser Cys Gln Gly Asp Ser Gly Gly Pro Leu Val Cys Ser Leu Gln
305                 310                 315                 320

Gly Arg Met Thr Leu Thr Gly Ile Val Ser Trp Gly Arg Gly Cys Ala
                325                 330                 335

Leu Lys Asp Lys Pro Gly Val Tyr Thr Arg Val Ser His Phe Leu Pro
            340                 345                 350

Trp Ile Arg Ser His Thr Lys Glu Glu Asn Gly Leu Ala Leu Ser Pro
        355                 360                 365

Pro Ser Pro Pro Ser Pro Pro Gly Gly Phe Pro Arg Pro Gly Gly Gly
    370                 375                 380

Gly Asn Gly Asp Phe Glu Glu Ile Pro Glu Glu Tyr Leu  *
385                 390                 395

(2) ANGABEN ZU SEQ ID NO: 29:

(i) SEQUENZKENNZEICHEN:
(A) LÄNGE: 1176 Basenpaare
(B) ART: Nucleotid
(C) STRANGFORM: Einzelstrang
(D) TOPOLOGIE: linear

(ii) ART DES MOLEKÜLS: Sonstige Nucleinsäure

(iii) HYPOTHETISCH: NEIN

(iv) ANTISENSE: NEIN

(ix) MERKMAL:
(A) NAME/SCHLÜSSEL: CDS
(B) LAGE:1..1176
(D) SONSTIGE ANGABEN:/function= "N-term. Methionin wird
in E.coli proteolytisch abgespalten"
/product= "Synthetisches Gen fuer das Protein M14
(pSJ78)"

(xi) SEQUENZBESCHREIBUNG: SEQ ID NO: 29:

ATG AGC AAA ACT TGC TAC GAA GGT AAC GGT CAC TTC TAC CGT GGT AAG 48
Met Ser Lys Thr Cys Tyr Glu Gly Asn Gly His Phe Tyr Arg Gly Lys
1               5                   10                  15

GCT TCT ACC GAC ACC ATG GGT CGT CCG TGC CTG CCG TGG AAC TCT GCT 96
Ala Ser Thr Asp Thr Met Gly Arg Pro Cys Leu Pro Trp Asn Ser Ala
            20                  25                  30

ACC GTT CTG CAG CAG ACC TAC CAC GCT CAC CGT TCT GAT GCA TTG CAG 144
Thr Val Leu Gln Gln Thr Tyr His Ala His Arg Ser Asp Ala Leu Gln
        35                  40                  45

CTG GGT CTG GGT AAA CAC AAC TAC TGC CGT AAC CCG GAC AAC CGT CGT 192
Leu Gly Leu Gly Lys His Asn Tyr Cys Arg Asn Pro Asp Asn Arg Arg
    50                  55                  60

CGT CCG TGG TGC TAC GTT CAG GTT GGT CTG AAA CCG CTA GTT CAG GAA 240
Arg Pro Trp Cys Tyr Val Gln Val Gly Leu Lys Pro Leu Val Gln Glu
65                  70                  75                  80

TGC ATG GTT CAC GAC TGC GCT GAC GGT AAA AAA CCG TCT TCT CCG CCG 288
Cys Met Val His Asp Cys Ala Asp Gly Lys Lys Pro Ser Ser Pro Pro
                85                  90                  95

GAA GAG CTC AAA TTC CAG TGC GGT CAA AAA ACC CTA CGT CCG CGT TTT 336
Glu Glu Leu Lys Phe Gln Cys Gly Gln Lys Thr Leu Arg Pro Arg Phe
            100                 105                 110

AAA ATC ATC GGT GGT GAG TTC ACC ACC ATC GAA AAC CAG CCG TGG TTC 384
Lys Ile Ile Gly Gly Glu Phe Thr Thr Ile Glu Asn Gln Pro Trp Phe
        115                 120                 125

GCT GCT ATC TAC CGT CGT CAC CGT GGT GGT TCT GTT ACC TAC GTT TGC 432
Ala Ala Ile Tyr Arg Arg His Arg Gly Gly Ser Val Thr Tyr Val Cys
    130                 135                 140

GGT GGT TCT CTG ATC TCT CCG TGC TGG GTT ATC TCT GCT ACC CAC TGC 480
Gly Gly Ser Leu Ile Ser Pro Cys Trp Val Ile Ser Ala Thr His Cys
145                 150                 155                 160

TTC ATC GAC TAC CCG AAA AAA GAA GAC TAC ATC GTT TAC CTC GGC CGT 528
Phe Ile Asp Tyr Pro Lys Lys Glu Asp Tyr Ile Val Tyr Leu Gly Arg
                165                 170                 175

TCT CGT TTA AAC TCT AAC ACC CAG GGT GAA ATG AAA TTC GAA GTT GAA 576
Ser Arg Leu Asn Ser Asn Thr Gln Gly Glu Met Lys Phe Glu Val Glu
            180                 185                 190

AAC CTG ATC CTG CAC AAA GAC TAC TCT GCT GAC ACC CTG GCT CAC CAC 624
Asn Leu Ile Leu His Lys Asp Tyr Ser Ala Asp Thr Leu Ala His His
        195                 200                 205

AAC GAC ATC GCT CTG CTA AAA ATC CGT TCT AAA GAA GGT CGT TGC GCT 672
Asn Asp Ile Ala Leu Leu Lys Ile Arg Ser Lys Glu Gly Arg Cys Ala
    210                 215                 220

CAG CCG TCT CGT ACC ATC CAG ACC ATC TGC CTG CCG TCT ATG TAC AAC 720
Gln Pro Ser Arg Thr Ile Gln Thr Ile Cys Leu Pro Ser Met Tyr Asn
225                 230                 235                 240

GAC CCG CAG TTC GGT ACC TCT TGC GAA ATC ACC GGT TTC GGT AAA GAA 768
Asp Pro Gln Phe Gly Thr Ser Cys Glu Ile Thr Gly Phe Gly Lys Glu
                245                 250                 255

AAC TCT ACC GAC TAC CTG TAC CCG GAA CAG CTG AAA ATG ACC GTT GTT 816
Asn Ser Thr Asp Tyr Leu Tyr Pro Glu Gln Leu Lys Met Thr Val Val
            260                 265                 270

AAA CTG ATC TCT CAC CGT GAA TGC CAG CAG CCG CAC TAC TAC GGT TCT 864
Lys Leu Ile Ser His Arg Glu Cys Gln Gln Pro His Tyr Tyr Gly Ser
        275                 280                 285

GAA GTT ACC ACC AAA ATG CTG TGC GCT GCT GAC CCG CAG TGG AAA ACC 912
Glu Val Thr Thr Lys Met Leu Cys Ala Ala Asp Pro Gln Trp Lys Thr
    290                 295                 300

GAC TCT TGC CAA GGT GAC TCT GGT GGT CCA CTA GTT TGC TCT CTC CAG 960
Asp Ser Cys Gln Gly Asp Ser Gly Gly Pro Leu Val Cys Ser Leu Gln
305                 310                 315                 320

GGT CGT ATG ACC CTG ACC GGT ATT GTT TCT TGG GGT CGT GGT TGC GCT 1008
Gly Arg Met Thr Leu Thr Gly Ile Val Ser Trp Gly Arg Gly Cys Ala
                325                 330                 335

CTG AAA GAC AAA CCG GGT GTT TAC ACC CGT GTT TCT CAC TTC CTG CCG 1056
Leu Lys Asp Lys Pro Gly Val Tyr Thr Arg Val Ser His Phe Leu Pro
            340                 345                 350

TGG ATC CGT TCT CAC ACC AAA GAA GAA AAC GGT CTG GCT CTG AGC CCG 1104
Trp Ile Arg Ser His Thr Lys Glu Glu Asn Gly Leu Ala Leu Ser Pro
        355                 360                 365

CCG GGT GGT TTC CCG AGG CCT GGT GGT GGT GGT AAC GGT GAC TTC GAA 1152
Pro Gly Gly Phe Pro Arg Pro Gly Gly Gly Gly Asn Gly Asp Phe Glu
    370                 375                 380

GAA ATC CCG GAA GAG TAC CTG TGA 1176
Glu Ile Pro Glu Glu Tyr Leu *
385                 390

(2) ANGABEN ZU SEQ ID NO: 30:

(i) SEQUENZKENNZEICHEN:
(A) LÄNGE: 392 Aminosäuren
(B) ART: Aminosäure
(D) TOPOLOGIE: linear

(ii) ART DES MOLEKÜLS: Protein
(xi) SEQUENZBESCHREIBUNG: SEQ ID NO: 30:

Met Ser Lys Thr Cys Tyr Glu Gly Asn Gly His Phe Tyr Arg Gly Lys
1               5                   10                  15

Ala Ser Thr Asp Thr Met Gly Arg Pro Cys Leu Pro Trp Asn Ser Ala
            20                  25                  30

Thr Val Leu Gln Gln Thr Tyr His Ala His Arg Ser Asp Ala Leu Gln
        35                  40                  45

Leu Gly Leu Gly Lys His Asn Tyr Cys Arg Asn Pro Asp Asn Arg Arg
    50                  55                  60

Arg Pro Trp Cys Tyr Val Gln Val Gly Leu Lys Pro Leu Val Gln Glu
65                  70                  75                  80

Cys Met Val His Asp Cys Ala Asp Gly Lys Lys Pro Ser Ser Pro Pro
                85                  90                  95

Glu Glu Leu Lys Phe Gln Cys Gly Gln Lys Thr Leu Arg Pro Arg Phe
            100                 105                 110

Lys Ile Ile Gly Gly Glu Phe Thr Thr Ile Glu Asn Gln Pro Trp Phe
        115                 120                 125

Ala Ala Ile Tyr Arg Arg His Arg Gly Gly Ser Val Thr Tyr Val Cys
    130                 135                 140

Gly Gly Ser Leu Ile Ser Pro Cys Trp Val Ile Ser Ala Thr His Cys
145                 150                 155                 160

Phe Ile Asp Tyr Pro Lys Lys Glu Asp Tyr Ile Val Tyr Leu Gly Arg
                165                 170                 175

Ser Arg Leu Asn Ser Asn Thr Gln Gly Glu Met Lys Phe Glu Val Glu
            180                 185                 190

Asn Leu Ile Leu His Lys Asp Tyr Ser Ala Asp Thr Leu Ala His His
        195                 200                 205

Asn Asp Ile Ala Leu Leu Lys Ile Arg Ser Lys Glu Gly Arg Cys Ala
    210                 215                 220

Gln Pro Ser Arg Thr Ile Gln Thr Ile Cys Leu Pro Ser Met Tyr Asn
225                 230                 235                 240

Asp Pro Gln Phe Gly Thr Ser Cys Glu Ile Thr Gly Phe Gly Lys Glu
                245                 250                 255

Asn Ser Thr Asp Tyr Leu Tyr Pro Glu Gln Leu Lys Met Thr Val Val
            260                 265                 270

Lys Leu Ile Ser His Arg Glu Cys Gln Gln Pro His Tyr Tyr Gly Ser
        275                 280                 285

Glu Val Thr Thr Lys Met Leu Cys Ala Ala Asp Pro Gln Trp Lys Thr
    290                 295                 300

Asp Ser Cys Gln Gly Asp Ser Gly Gly Pro Leu Val Cys Ser Leu Gln
305                 310                 315                 320

Gly Arg Met Thr Leu Thr Gly Ile Val Ser Trp Gly Arg Gly Cys Ala
                325                 330                 335

Leu Lys Asp Lys Pro Gly Val Tyr Thr Arg Val Ser His Phe Leu Pro
            340                 345                 350

Trp Ile Arg Ser His Thr Lys Glu Glu Asn Gly Leu Ala Leu Ser Pro
        355                 360                 365

Pro Gly Gly Phe Pro Arg Pro Gly Gly Gly Gly Asn Gly Asp Phe Glu
    370                 375                 380

Glu Ile Pro Glu Glu Tyr Leu  *
385                 390

(2) ANGABEN ZU SEQ ID NO: 31:

(i) SEQUENZKENNZEICHEN:
(A) LÄNGE: 1197 Basenpaare
(B) ART: Nucleotid
(C) STRANGFORM: Einzelstrang
(D) TOPOLOGIE: linear

(ii) ART DES MOLEKÜLS: Sonstige Nucleinsäure

(iii) HYPOTHETISCH: NEIN

(iv) ANTISENSE: NEIN

(ix) MERKMAL:
(A) NAME/SCHLÜSSEL: CDS
(B) LAGE:1..1197
(D) SONSTIGE ANGABEN:/function= "N-term. Methionin wird
in E.coli proteolytisch abgespalten"
/product= "Synthetisches Gen fuer das Protein M15
(pSJ79)"

(xi) SEQUENZBESCHREIBUNG: SEQ ID NO: 31:

ATG AGC AAA ACT TGC TAC GAA GGT AAC GGT CAC TTC TAC CGT GGT AAG      48
Met Ser Lys Thr Cys Tyr Glu Gly Asn Gly His Phe Tyr Arg Gly Lys
  1               5                  10                  15

GCT TCT ACC GAC ACC ATG GGT CGT CCG TGC CTG CCG TGG AAC TCT GCT      96
Ala Ser Thr Asp Thr Met Gly Arg Pro Cys Leu Pro Trp Asn Ser Ala
             20                  25                  30

ACC GTT CTG CAG CAG ACC TAC CAC GCT CAC CGT TCT GAT GCA TTG CAG     144
Thr Val Leu Gln Gln Thr Tyr His Ala His Arg Ser Asp Ala Leu Gln
         35                  40                  45

CTG GGT CTG GGT AAA CAC AAC TAC TGC CGT AAC CCG GAC AAC CGT CGT     192
Leu Gly Leu Gly Lys His Asn Tyr Cys Arg Asn Pro Asp Asn Arg Arg
     50                  55                  60

CGT CCG TGG TGC TAC GTT CAG GTT GGT CTG AAA CCG CTA GTT CAG GAA 240
Arg Pro Trp Cys Tyr Val Gln Val Gly Leu Lys Pro Leu Val Gln Glu
65                  70                  75                  80

TGC ATG GTT CAC GAC TGC GCT GAC GGT AAA AAA CCG TCT TCT CCG CCG 288
Cys Met Val His Asp Cys Ala Asp Gly Lys Lys Pro Ser Ser Pro Pro
                85                  90                  95

GAA GAG CTC AAA TTC CAG TGC GGT CAA AAA ACC CTA CGT CCG CGT TTT 336
Glu Glu Leu Lys Phe Gln Cys Gly Gln Lys Thr Leu Arg Pro Arg Phe
            100                 105                 110

AAA ATC ATC GGT GGT GAG TTC ACC ACC ATC GAA AAC CAG CCG TGG TTC 384
Lys Ile Ile Gly Gly Glu Phe Thr Thr Ile Glu Asn Gln Pro Trp Phe
        115                 120                 125

GCT GCT ATC TAC CGT CGT CAC CGT GGT GGT TCT GTT ACC TAC GTT TGC 432
Ala Ala Ile Tyr Arg Arg His Arg Gly Gly Ser Val Thr Tyr Val Cys
    130                 135                 140

GGT GGT TCT CTG ATC TCT CCG TGC TGG GTT ATC TCT GCT ACC CAC TGC 480
Gly Gly Ser Leu Ile Ser Pro Cys Trp Val Ile Ser Ala Thr His Cys
145                 150                 155                 160

TTC ATC GAC TAC CCG AAA AAA GAA GAC TAC ATC GTT TAC CTC GGC CGT 528
Phe Ile Asp Tyr Pro Lys Lys Glu Asp Tyr Ile Val Tyr Leu Gly Arg
                165                 170                 175

TCT CGT TTA AAC TCT AAC ACC CAG GGT GAA ATG AAA TTC GAA GTT GAA 576
Ser Arg Leu Asn Ser Asn Thr Gln Gly Glu Met Lys Phe Glu Val Glu
            180                 185                 190

AAC CTG ATC CTG CAC AAA GAC TAC TCT GCT GAC ACC CTG GCT CAC CAC 624
Asn Leu Ile Leu His Lys Asp Tyr Ser Ala Asp Thr Leu Ala His His
        195                 200                 205

AAC GAC ATC GCT CTG CTA AAA ATC CGT TCT AAA GAA GGT CGT TGC GCT 672
Asn Asp Ile Ala Leu Leu Lys Ile Arg Ser Lys Glu Gly Arg Cys Ala
    210                 215                 220

CAG CCG TCT CGT ACC ATC CAG ACC ATC TGC CTG CCG TCT ATG TAC AAC 720
Gln Pro Ser Arg Thr Ile Gln Thr Ile Cys Leu Pro Ser Met Tyr Asn
225                 230                 235                 240

GAC CCG CAG TTC GGT ACC TCT TGC GAA ATC ACC GGT TTC GGT AAA GAA 768
Asp Pro Gln Phe Gly Thr Ser Cys Glu Ile Thr Gly Phe Gly Lys Glu
                245                 250                 255

AAC TCT ACC GAC TAC CTG TAC CCG GAA CAG CTG AAA ATG ACC GTT GTT 816
Asn Ser Thr Asp Tyr Leu Tyr Pro Glu Gln Leu Lys Met Thr Val Val
            260                 265                 270

AAA CTG ATC TCT CAC CGT GAA TGC CAG CAG CCG CAC TAC TAC GGT TCT 864
Lys Leu Ile Ser His Arg Glu Cys Gln Gln Pro His Tyr Tyr Gly Ser
        275                 280                 285

GAA GTT ACC ACC AAA ATG CTG TGC GCT GCT GAC CCG CAG TGG AAA ACC 912
Glu Val Thr Thr Lys Met Leu Cys Ala Ala Asp Pro Gln Trp Lys Thr
    290                 295                 300

GAC TCT TGC CAA GGT GAC TCT GGT GGT CCA CTA GTT TGC TCT CTC CAG 960
Asp Ser Cys Gln Gly Asp Ser Gly Gly Pro Leu Val Cys Ser Leu Gln
305                 310                 315                 320

GGT CGT ATG ACC CTG ACC GGT ATT GTT TCT TGG GGT CGT GGT TGC GCT 1008
Gly Arg Met Thr Leu Thr Gly Ile Val Ser Trp Gly Arg Gly Cys Ala
                325                 330                 335

CTG AAA GAC AAA CCG GGT GTT TAC ACC CGT GTT TCT CAC TTC CTG CCG 1056
Leu Lys Asp Lys Pro Gly Val Tyr Thr Arg Val Ser His Phe Leu Pro
            340                 345                 350

TGG ATC CGT TCT CAC ACC AAA GAA GAA AAC GGT CTG GCT CTG AGC CCG 1104
Trp Ile Arg Ser His Thr Lys Glu Glu Asn Gly Leu Ala Leu Ser Pro
        355                 360                 365

CCG AGC CCG CCG AGC CCG CCG GGT GGT TTC GGT CCG AGG CCT GGT GGT 1152
Pro Ser Pro Pro Ser Pro Pro Gly Gly Phe Gly Pro Arg Pro Gly Gly
    370                 375                 380

GGT GGT AAC GGT GAC TTC GAA GAA ATC CCG GAA GAG TAC CTG TGA 1197
Gly Gly Asn Gly Asp Phe Glu Glu Ile Pro Glu Glu Tyr Leu *
385                 390                 395

(2) ANGABEN ZU SEQ ID NO: 32:

(i) SEQUENZKENNZEICHEN:
(A) LÄNGE: 399 Aminosäuren
(B) ART: Aminosäure
(D) TOPOLOGIE: linear

(ii) ART DES MOLEKÜLS: Protein
(xi) SEQUENZBESCHREIBUNG: SEQ ID NO: 32:

Met Ser Lys Thr Cys Tyr Glu Gly Asn Gly His Phe Tyr Arg Gly Lys
1               5                   10                  15

Ala Ser Thr Asp Thr Met Gly Arg Pro Cys Leu Pro Trp Asn Ser Ala
            20                  25                  30

Thr Val Leu Gln Gln Thr Tyr His Ala His Arg Ser Asp Ala Leu Gln
        35                  40                  45

Leu Gly Leu Gly Lys His Asn Tyr Cys Arg Asn Pro Asp Asn Arg Arg
    50                  55                  60

Arg Pro Trp Cys Tyr Val Gln Val Gly Leu Lys Pro Leu Val Gln Glu
65                  70                  75                  80

Cys Met Val His Asp Cys Ala Asp Gly Lys Lys Pro Ser Ser Pro Pro
                85                  90                  95

Glu Glu Leu Lys Phe Gln Cys Gly Gln Lys Thr Leu Arg Pro Arg Phe
            100                 105                 110

Lys Ile Ile Gly Gly Glu Phe Thr Thr Ile Glu Asn Gln Pro Trp Phe
        115                 120                 125

Ala Ala Ile Tyr Arg Arg His Arg Gly Gly Ser Val Thr Tyr Val Cys
    130                 135                 140

Gly Gly Ser Leu Ile Ser Pro Cys Trp Val Ile Ser Ala Thr His Cys
145                 150                 155                 160

Phe Ile Asp Tyr Pro Lys Lys Glu Asp Tyr Ile Val Tyr Leu Gly Arg
                165                 170                 175

Ser Arg Leu Asn Ser Asn Thr Gln Gly Glu Met Lys Phe Glu Val Glu
            180                 185                 190

Asn Leu Ile Leu His Lys Asp Tyr Ser Ala Asp Thr Leu Ala His His
        195                 200                 205

Asn Asp Ile Ala Leu Leu Lys Ile Arg Ser Lys Glu Gly Arg Cys Ala
    210                 215                 220

Gln Pro Ser Arg Thr Ile Gln Thr Ile Cys Leu Pro Ser Met Tyr Asn
225                 230                 235                 240

Asp Pro Gln Phe Gly Thr Ser Cys Glu Ile Thr Gly Phe Gly Lys Glu
                245                 250                 255

Asn Ser Thr Asp Tyr Leu Tyr Pro Glu Gln Leu Lys Met Thr Val Val
            260                 265                 270

Lys Leu Ile Ser His Arg Glu Cys Gln Gln Pro His Tyr Tyr Gly Ser
        275                 280                 285

Glu Val Thr Thr Lys Met Leu Cys Ala Ala Asp Pro Gln Trp Lys Thr
    290                 295                 300

Asp Ser Cys Gln Gly Asp Ser Gly Gly Pro Leu Val Cys Ser Leu Gln
305                 310                 315                 320

Gly Arg Met Thr Leu Thr Gly Ile Val Ser Trp Gly Arg Gly Cys Ala
                325                 330                 335

Leu Lys Asp Lys Pro Gly Val Tyr Thr Arg Val Ser His Phe Leu Pro
            340                 345                 350

Trp Ile Arg Ser His Thr Lys Glu Glu Asn Gly Leu Ala Leu Ser Pro
        355                 360                 365

Pro Ser Pro Pro Ser Pro Pro Gly Gly Phe Gly Pro Arg Pro Gly Gly
    370                 375                 380

Gly Gly Asn Gly Asp Phe Glu Glu Ile Pro Glu Glu Tyr Leu  *
385                 390                 395

(2) ANGABEN ZU SEQ ID NO: 33:

    (i) SEQUENZKENNZEICHEN:
        (A) LÄNGE: 1185 Basenpaare
        (B) ART: Nucleotid
        (C) STRANGFORM: Einzelstrang
        (D) TOPOLOGIE: linear

    (ii) ART DES MOLEKÜLS: Sonstige Nucleinsäure

    (iii) HYPOTHETISCH: NEIN

    (iv) ANTISENSE: NEIN

(ix) MERKMAL:
(A) NAME/SCHLÜSSEL: CDS
(B) LAGE:1..1185
(D) SONSTIGE ANGABEN:/function= "N-term. Methionin wird
in E.coli proteolytisch abgespalten"
/product= "Synthetisches Gen fuer das Protein M16
(pSJ81)"

(xi) SEQUENZBESCHREIBUNG: SEQ ID NO: 33:

ATG AGC AAA ACT TGC TAC GAA GGT AAC GGT CAC TTC TAC CGT GGT AAG 48
Met Ser Lys Thr Cys Tyr Glu Gly Asn Gly His Phe Tyr Arg Gly Lys
1               5                   10                  15

GCT TCT ACC GAC ACC ATG GGT CGT CCG TGC CTG CCG TGG AAC TCT GCT 96
Ala Ser Thr Asp Thr Met Gly Arg Pro Cys Leu Pro Trp Asn Ser Ala
            20                  25                  30

ACC GTT CTG CAG CAG ACC TAC CAC GCT CAC CGT TCT GAT GCA TTG CAG 144
Thr Val Leu Gln Gln Thr Tyr His Ala His Arg Ser Asp Ala Leu Gln
        35                  40                  45

CTG GGT CTG GGT AAA CAC AAC TAC TGC CGT AAC CCG GAC AAC CGT CGT 192
Leu Gly Leu Gly Lys His Asn Tyr Cys Arg Asn Pro Asp Asn Arg Arg
    50                  55                  60

CGT CCG TGG TGC TAC GTT CAG GTT GGT CTG AAA CCG CTA GTT CAG GAA 240
Arg Pro Trp Cys Tyr Val Gln Val Gly Leu Lys Pro Leu Val Gln Glu
65                  70                  75                  80

TGC ATG GTT CAC GAC TGC GCT GAC GGT AAA AAA CCG TCT TCT CCG CCG 288
Cys Met Val His Asp Cys Ala Asp Gly Lys Lys Pro Ser Ser Pro Pro
                85                  90                  95

GAA GAG CTC AAA TTC CAG TGC GGT CAA AAA ACC CTA CGT CCG CGT TTT 336
Glu Glu Leu Lys Phe Gln Cys Gly Gln Lys Thr Leu Arg Pro Arg Phe
            100                 105                 110

AAA ATC ATC GGT GGT GAG TTC ACC ACC ATC GAA AAC CAG CCG TGG TTC 384
Lys Ile Ile Gly Gly Glu Phe Thr Thr Ile Glu Asn Gln Pro Trp Phe
        115                 120                 125

GCT GCT ATC TAC CGT CGT CAC CGT GGT GGT TCT GTT ACC TAC GTT TGC 432
Ala Ala Ile Tyr Arg Arg His Arg Gly Gly Ser Val Thr Tyr Val Cys
    130                 135                 140

GGT GGT TCT CTG ATC TCT CCG TGC TGG GTT ATC TCT GCT ACC CAC TGC 480
Gly Gly Ser Leu Ile Ser Pro Cys Trp Val Ile Ser Ala Thr His Cys
145                 150                 155                 160

TTC ATC GAC TAC CCG AAA AAA GAA GAC TAC ATC GTT TAC CTC GGC CGT 528
Phe Ile Asp Tyr Pro Lys Lys Glu Asp Tyr Ile Val Tyr Leu Gly Arg
                165                 170                 175

TCT CGT TTA AAC TCT AAC ACC CAG GGT GAA ATG AAA TTC GAA GTT GAA 576
Ser Arg Leu Asn Ser Asn Thr Gln Gly Glu Met Lys Phe Glu Val Glu
            180                 185                 190

AAC CTG ATC CTG CAC AAA GAC TAC TCT GCT GAC ACC CTG GCT CAC CAC 624
Asn Leu Ile Leu His Lys Asp Tyr Ser Ala Asp Thr Leu Ala His His
        195                 200                 205

AAC GAC ATC GCT CTG CTA AAA ATC CGT TCT AAA GAA GGT CGT TGC GCT 672
Asn Asp Ile Ala Leu Leu Lys Ile Arg Ser Lys Glu Gly Arg Cys Ala
    210                 215                 220

CAG CCG TCT CGT ACC ATC CAG ACC ATC TGC CTG CCG TCT ATG TAC AAC 720
Gln Pro Ser Arg Thr Ile Gln Thr Ile Cys Leu Pro Ser Met Tyr Asn
225                 230                 235                 240

GAC CCG CAG TTC GGT ACC TCT TGC GAA ATC ACC GGT TTC GGT AAA GAA 768
Asp Pro Gln Phe Gly Thr Ser Cys Glu Ile Thr Gly Phe Gly Lys Glu
                245                 250                 255

AAC TCT ACC GAC TAC CTG TAC CCG GAA CAG CTG AAA ATG ACC GTT GTT 816
Asn Ser Thr Asp Tyr Leu Tyr Pro Glu Gln Leu Lys Met Thr Val Val
            260                 265                 270

AAA CTG ATC TCT CAC CGT GAA TGC CAG CAG CCG CAC TAC TAC GGT TCT 864
Lys Leu Ile Ser His Arg Glu Cys Gln Gln Pro His Tyr Tyr Gly Ser
        275                 280                 285

GAA GTT ACC ACC AAA ATG CTG TGC GCT GCT GAC CCG CAG TGG AAA ACC 912
Glu Val Thr Thr Lys Met Leu Cys Ala Ala Asp Pro Gln Trp Lys Thr
    290                 295                 300

GAC TCT TGC CAA GGT GAC TCT GGT GGT CCA CTA GTT TGC TCT CTC CAG 960
Asp Ser Cys Gln Gly Asp Ser Gly Gly Pro Leu Val Cys Ser Leu Gln
305                 310                 315                 320

GGT CGT ATG ACC CTG ACC GGT ATT GTT TCT TGG GGT CGT GGT TGC GCT 1008
Gly Arg Met Thr Leu Thr Gly Ile Val Ser Trp Gly Arg Gly Cys Ala
                325                 330                 335

CTG AAA GAC AAA CCG GGT GTT TAC ACC CGT GTT TCT CAC TTC CTG CCG 1056
Leu Lys Asp Lys Pro Gly Val Tyr Thr Arg Val Ser His Phe Leu Pro
            340                 345                 350

TGG ATC CGT TCT CAC ACC AAA GAA GAA AAC GGT CTG GCT CTG AGC CCG 1104
Trp Ile Arg Ser His Thr Lys Glu Glu Asn Gly Leu Ala Leu Ser Pro
        355                 360                 365

GTT AAG GCT TGG CCG GGA CGG CCG CTG GGT GGT GGT GGT AAC GGT GAC 1152
Val Lys Ala Trp Pro Gly Arg Pro Leu Gly Gly Gly Gly Asn Gly Asp
    370                 375                 380

TTC GAA GAA ATC CCG GAA GAA TAC CTG CAA TAA 1185
Phe Glu Glu Ile Pro Glu Glu Tyr Leu Gln *
385                 390                 395

(2) ANGABEN ZU SEQ ID NO: 34:

(i) SEQUENZKENNZEICHEN:
(A) LÄNGE: 395 Aminosäuren
(B) ART: Aminosäure
(D) TOPOLOGIE: linear

(ii) ART DES MOLEKÜLS: Protein
(xi) SEQUENZBESCHREIBUNG: SEQ ID NO: 34:

Met Ser Lys Thr Cys Tyr Glu Gly Asn Gly His Phe Tyr Arg Gly Lys
1               5                   10                  15

Ala Ser Thr Asp Thr Met Gly Arg Pro Cys Leu Pro Trp Asn Ser Ala
            20                  25                  30

Thr Val Leu Gln Gln Thr Tyr His Ala His Arg Ser Asp Ala Leu Gln
        35                  40                  45

Leu Gly Leu Gly Lys His Asn Tyr Cys Arg Asn Pro Asp Asn Arg Arg
    50                  55                  60

Arg Pro Trp Cys Tyr Val Gln Val Gly Leu Lys Pro Leu Val Gln Glu
65                  70                  75                  80

Cys Met Val His Asp Cys Ala Asp Gly Lys Lys Pro Ser Ser Pro Pro
                85                  90                  95

Glu Glu Leu Lys Phe Gln Cys Gly Gln Lys Thr Leu Arg Pro Arg Phe
            100                 105                 110

Lys Ile Ile Gly Gly Glu Phe Thr Thr Ile Glu Asn Gln Pro Trp Phe
        115                 120                 125

Ala Ala Ile Tyr Arg Arg His Arg Gly Gly Ser Val Thr Tyr Val Cys
    130                 135                 140

Gly Gly Ser Leu Ile Ser Pro Cys Trp Val Ile Ser Ala Thr His Cys
145                 150                 155                 160

Phe Ile Asp Tyr Pro Lys Lys Glu Asp Tyr Ile Val Tyr Leu Gly Arg
                165                 170                 175

Ser Arg Leu Asn Ser Asn Thr Gln Gly Glu Met Lys Phe Glu Val Glu
            180                 185                 190

Asn Leu Ile Leu His Lys Asp Tyr Ser Ala Asp Thr Leu Ala His His
        195                 200                 205

Asn Asp Ile Ala Leu Leu Lys Ile Arg Ser Lys Glu Gly Arg Cys Ala
    210                 215                 220

Gln Pro Ser Arg Thr Ile Gln Thr Ile Cys Leu Pro Ser Met Tyr Asn
225                 230                 235                 240

Asp Pro Gln Phe Gly Thr Ser Cys Glu Ile Thr Gly Phe Gly Lys Glu
                245                 250                 255

Asn Ser Thr Asp Tyr Leu Tyr Pro Glu Gln Leu Lys Met Thr Val Val
            260                 265                 270

Lys Leu Ile Ser His Arg Glu Cys Gln Gln Pro His Tyr Tyr Gly Ser
        275                 280                 285

Glu Val Thr Thr Lys Met Leu Cys Ala Ala Asp Pro Gln Trp Lys Thr
    290                 295                 300

Asp Ser Cys Gln Gly Asp Ser Gly Gly Pro Leu Val Cys Ser Leu Gln
305                 310                 315                 320

Gly Arg Met Thr Leu Thr Gly Ile Val Ser Trp Gly Arg Gly Cys Ala
                325                 330                 335

Leu Lys Asp Lys Pro Gly Val Tyr Thr Arg Val Ser His Phe Leu Pro
            340                 345                 350

Trp Ile Arg Ser His Thr Lys Glu Glu Asn Gly Leu Ala Leu Ser Pro
        355                 360                 365

Val Lys Ala Trp Pro Gly Arg Pro Leu Gly Gly Gly Gly Asn Gly Asp
    370                 375                 380

Phe Glu Glu Ile Pro Glu Glu Tyr Leu Gln  *
385                 390                 395

(2) ANGABEN ZU SEQ ID NO: 35:

     (i) SEQUENZKENNZEICHEN:
          (A) LÄNGE: 1185 Basenpaare
          (B) ART: Nucleotid
          (C) STRANGFORM: Einzelstrang
          (D) TOPOLOGIE: linear

    (ii) ART DES MOLEKÜLS: Sonstige Nucleinsäure

   (iii) HYPOTHETISCH: NEIN

    (iv) ANTISENSE: NEIN

    (ix) MERKMAL:
          (A) NAME/SCHLÜSSEL: CDS
          (B) LAGE:1..1185
          (D) SONSTIGE ANGABEN:/function= "N-term. Methionin wird
               in E.coli proteolytisch abgespalten"
               /product= "Synthetisches Gen fuer das Protein M17
               (pSJ83)"

    (xi) SEQUENZBESCHREIBUNG: SEQ ID NO: 35:

ATG AGC AAA ACT TGC TAC GAA GGT AAC GGT CAC TTC TAC CGT GGT AAG        48
Met Ser Lys Thr Cys Tyr Glu Gly Asn Gly His Phe Tyr Arg Gly Lys
  1               5                  10                  15

GCT TCT ACC GAC ACC ATG GGT CGT CCG TGC CTG CCG TGG AAC TCT GCT        96
Ala Ser Thr Asp Thr Met Gly Arg Pro Cys Leu Pro Trp Asn Ser Ala
             20                  25                  30

ACC GTT CTG CAG CAG ACC TAC CAC GCT CAC CGT TCT GAT GCA TTG CAG       144
Thr Val Leu Gln Gln Thr Tyr His Ala His Arg Ser Asp Ala Leu Gln
         35                  40                  45

CTG GGT CTG GGT AAA CAC AAC TAC TGC CGT AAC CCG GAC AAC CGT CGT       192
Leu Gly Leu Gly Lys His Asn Tyr Cys Arg Asn Pro Asp Asn Arg Arg
     50                  55                  60

CGT CCG TGG TGC TAC GTT CAG GTT GGT CTG AAA CCG CTA GTT CAG GAA       240
Arg Pro Trp Cys Tyr Val Gln Val Gly Leu Lys Pro Leu Val Gln Glu
 65                  70                  75                  80

TGC ATG GTT CAC GAC TGC GCT GAC GGT AAA AAA CCG TCT TCT CCG CCG       288
Cys Met Val His Asp Cys Ala Asp Gly Lys Lys Pro Ser Ser Pro Pro
                 85                  90                  95

GAA GAG CTC AAA TTC CAG TGC GGT CAA AAA ACC CTA CGT CCG CGT TTT       336
Glu Glu Leu Lys Phe Gln Cys Gly Gln Lys Thr Leu Arg Pro Arg Phe
            100                 105                 110

AAA ATC ATC GGT GGT GAG TTC ACC ACC ATC GAA AAC CAG CCG TGG TTC       384
Lys Ile Ile Gly Gly Glu Phe Thr Thr Ile Glu Asn Gln Pro Trp Phe
        115                 120                 125

GCT GCT ATC TAC CGT CGT CAC CGT GGT GGT TCT GTT ACC TAC GTT TGC 432
Ala Ala Ile Tyr Arg Arg His Arg Gly Gly Ser Val Thr Tyr Val Cys
    130                 135                 140

GGT GGT TCT CTG ATC TCT CCG TGC TGG GTT ATC TCT GCT ACC CAC TGC 480
Gly Gly Ser Leu Ile Ser Pro Cys Trp Val Ile Ser Ala Thr His Cys
145                 150                 155                 160

TTC ATC GAC TAC CCG AAA AAA GAA GAC TAC ATC GTT TAC CTC GGC CGT 528
Phe Ile Asp Tyr Pro Lys Lys Glu Asp Tyr Ile Val Tyr Leu Gly Arg
                165                 170                 175

TCT CGT TTA AAC TCT AAC ACC CAG GGT GAA ATG AAA TTC GAA GTT GAA 576
Ser Arg Leu Asn Ser Asn Thr Gln Gly Glu Met Lys Phe Glu Val Glu
            180                 185                 190

AAC CTG ATC CTG CAC AAA GAC TAC TCT GCT GAC ACC CTG GCT CAC CAC 624
Asn Leu Ile Leu His Lys Asp Tyr Ser Ala Asp Thr Leu Ala His His
195 200 206

AAC GAC ATC GCT CTG CTA AAA ATC CGT TCT AAA GAA GGT CGT TGC GCT 672
Asn Asp Ile Ala Leu Leu Lys Ile Arg Ser Lys Glu Gly Arg Cys Ala
    210                 215                 220

CAG CCG TCT CGT ACC ATC CAG ACC ATC TGC CTG CCG TCT ATG TAC AAC 720
Gln Pro Ser Arg Thr Ile Gln Thr Ile Cys Leu Pro Ser Met Tyr Asn
225                 230                 235                 240

GAC CCG CAG TTC GGT ACC TCT TGC GAA ATC ACC GGT TTC GGT AAA GAA 768
Asp Pro Gln Phe Gly Thr Ser Cys Glu Ile Thr Gly Phe Gly Lys Glu
                245                 250                 255

AAC TCT ACC GAC TAC CTG TAC CCG GAA CAG CTG AAA ATG ACC GTT GTT 816
Asn Ser Thr Asp Tyr Leu Tyr Pro Glu Gln Leu Lys Met Thr Val Val
            260                 265                 270

AAA CTG ATC TCT CAC CGT GAA TGC CAG CAG CCG CAC TAC TAC GGT TCT 864
Lys Leu Ile Ser His Arg Glu Cys Gln Gln Pro His Tyr Tyr Gly Ser
        275                 280                 285

GAA GTT ACC ACC AAA ATG CTG TGC GCT GCT GAC CCG CAG TGG AAA ACC 912
Glu Val Thr Thr Lys Met Leu Cys Ala Ala Asp Pro Gln Trp Lys Thr
    290                 295                 300

GAC TCT TGC CAA GGT GAC TCT GGT GGT CCA CTA GTT TGC TCT CTC CAG 960
Asp Ser Cys Gln Gly Asp Ser Gly Gly Pro Leu Val Cys Ser Leu Gln
305                 310                 315                 320

GGT CGT ATG ACC CTG ACC GGT ATT GTT TCT TGG GGT CGT GGT TGC GCT 1008
Gly Arg Met Thr Leu Thr Gly Ile Val Ser Trp Gly Arg Gly Cys Ala
                325                 330                 335

CTG AAA GAC AAA CCG GGT GTT TAC ACC CGT GTT TCT CAC TTC CTG CCG 1056
Leu Lys Asp Lys Pro Gly Val Tyr Thr Arg Val Ser His Phe Leu Pro
            340                 345                 350

TGG ATC CGT TCT CAC ACC AAA GAA GAA AAC GGT CTG GCT CTG AGC CCG 1104
Trp Ile Arg Ser His Thr Lys Glu Glu Asn Gly Leu Ala Leu Ser Pro
        355                 360                 365

GTT AAG GCT TTC GGA CCG CGG CCG CTG GGT GGT GGT GGT AAC GGT GAC 1152
Val Lys Ala Phe Gly Pro Arg Pro Leu Gly Gly Gly Gly Asn Gly Asp
    370                 375                 380

TTC GAA GAA ATC CCG GAA GAA TAC CTG CAA TAA
Phe Glu Glu Ile Pro Glu Glu Tyr Leu Gln  *
385                 390                 395

(2) ANGABEN ZU SEQ ID NO: 36:

(i) SEQUENZKENNZEICHEN:
(A) LÄNGE: 395 Aminosäuren
(B) ART: Aminosäure
(D) TOPOLOGIE: linear

(ii) ART DES MOLEKÜLS: Protein
(xi) SEQUENZBESCHREIBUNG: SEQ ID NO: 36:

Met Ser Lys Thr Cys Tyr Glu Gly Asn Gly His Phe Tyr Arg Gly Lys
1               5                   10                  15

Ala Ser Thr Asp Thr Met Gly Arg Pro Cys Leu Pro Trp Asn Ser Ala
            20                  25                  30

Thr Val Leu Gln Gln Thr Tyr His Ala His Arg Ser Asp Ala Leu Gln
        35                  40                  45

Leu Gly Leu Gly Lys His Asn Tyr Cys Arg Asn Pro Asp Asn Arg Arg
    50                  55                  60

Arg Pro Trp Cys Tyr Val Gln Val Gly Leu Lys Pro Leu Val Gln Glu
65                  70                  75                  80

Cys Met Val His Asp Cys Ala Asp Gly Lys Lys Pro Ser Ser Pro Pro
                85                  90                  95

Glu Glu Leu Lys Phe Gln Cys Gly Gln Lys Thr Leu Arg Pro Arg Phe
            100                 105                 110

Lys Ile Ile Gly Gly Glu Phe Thr Thr Ile Glu Asn Gln Pro Trp Phe
        115                 120                 125

Ala Ala Ile Tyr Arg Arg His Arg Gly Gly Ser Val Thr Tyr Val Cys
    130                 135                 140

Gly Gly Ser Leu Ile Ser Pro Cys Trp Val Ile Ser Ala Thr His Cys
145                 150                 155                 160

Phe Ile Asp Tyr Pro Lys Lys Glu Asp Tyr Ile Val Tyr Leu Gly Arg
                165                 170                 175

Ser Arg Leu Asn Ser Asn Thr Gln Gly Glu Met Lys Phe Glu Val Glu
            180                 185                 190

Asn Leu Ile Leu His Lys Asp Tyr Ser Ala Asp Thr Leu Ala His His
        195                 200                 205

Asn Asp Ile Ala Leu Leu Lys Ile Arg Ser Lys Glu Gly Arg Cys Ala
    210                 215                 220

Gln Pro Ser Arg Thr Ile Gln Thr Ile Cys Leu Pro Ser Met Tyr Asn
225                 230                 235                 240

Asp Pro Gln Phe Gly Thr Ser Cys Glu Ile Thr Gly Phe Gly Lys Glu
                245                 250                 255

Asn Ser Thr Asp Tyr Leu Tyr Pro Glu Gln Leu Lys Met Thr Val Val
            260                 265                 270

Lys Leu Ile Ser His Arg Glu Cys Gln Gln Pro His Tyr Tyr Gly Ser
        275                 280                 285

Glu Val Thr Thr Lys Met Leu Cys Ala Ala Asp Pro Gln Trp Lys Thr
    290                 295                 300

Asp Ser Cys Gln Gly Asp Ser Gly Gly Pro Leu Val Cys Ser Leu Gln
305                 310                 315                 320

Gly Arg Met Thr Leu Thr Gly Ile Val Ser Trp Gly Arg Gly Cys Ala
                325                 330                 335

Leu Lys Asp Lys Pro Gly Val Tyr Thr Arg Val Ser His Phe Leu Pro
            340                 345                 350

Trp Ile Arg Ser His Thr Lys Glu Glu Asn Gly Leu Ala Leu Ser Pro
        355                 360                 365

Val Lys Ala Phe Gly Pro Arg Pro Leu Gly Gly Gly Gly Asn Gly Asp
    370                 375                 380

Phe Glu Glu Ile Pro Glu Glu Tyr Leu Gln *
385                 390                 395

(2) ANGABEN ZU SEQ ID NO: 37:

(i) SEQUENZKENNZEICHEN:
(A) LÄNGE: 1185 Basenpaare
(B) ART: Nucleotid
(C) STRANGFORM: Einzelstrang
(D) TOPOLOGIE: linear

(ii) ART DES MOLEKÜLS: Sonstige Nucleinsäure

(iii) HYPOTHETISCH: NEIN

(iv) ANTISENSE: NEIN

(ix) MERKMAL:
(A) NAME/SCHLÜSSEL: CDS
(B) LAGE:1..1185
(D) SONSTIGE ANGABEN:/function= "N-term. Methionin wird
in E.coli proteolytisch abgespalten"
/product= "Synthetisches Gen fuer das Protein M18
(pSJ90)"

(xi) SEQUENZBESCHREIBUNG: SEQ ID NO: 37:

ATG AGC AAA ACT TGC TAC GAA GGT AAC GGT CAC TTC TAC CGT GGT AAG 48
Met Ser Lys Thr Cys Tyr Glu Gly Asn Gly His Phe Tyr Arg Gly Lys
1               5                   10                  15

GCT TCT ACC GAC ACC ATG GGT CGT CCG TGC CTG CCG TGG AAC TCT GCT 96
Ala Ser Thr Asp Thr Met Gly Arg Pro Cys Leu Pro Trp Asn Ser Ala
            20                  25                  30

ACC GTT CTG CAG CAG ACC TAC CAC GCT CAC CGT TCT GAT GCA TTG CAG 144
Thr Val Leu Gln Gln Thr Tyr His Ala His Arg Ser Asp Ala Leu Gln
        35                  40                  45

CTG GGT CTG GGT AAA CAC AAC TAC TGC CGT AAC CCG GAC AAC CGT CGT 192
Leu Gly Leu Gly Lys His Asn Tyr Cys Arg Asn Pro Asp Asn Arg Arg
    50                  55                  60

CGT CCG TGG TGC TAC GTT CAG GTT GGT CTG AAA CCG CTA GTT CAG GAA 240
Arg Pro Trp Cys Tyr Val Gln Val Gly Leu Lys Pro Leu Val Gln Glu
65                  70                  75                  80

TGC ATG GTT CAC GAC TGC GCT GAC GGT AAA AAA CCG TCT TCT CCG CCG 288
Cys Met Val His Asp Cys Ala Asp Gly Lys Lys Pro Ser Ser Pro Pro
                85                  90                  95

GAA GAG CTC AAA TTC CAG TGC GGT CAA AAA ACC CTA CGT CCG CGT TTT 336
Glu Glu Leu Lys Phe Gln Cys Gly Gln Lys Thr Leu Arg Pro Arg Phe
            100                 105                 110

AAA ATC ATC GGT GGT GAG TTC ACC ACC ATC GAA AAC CAG CCG TGG TTC 384
Lys Ile Ile Gly Gly Glu Phe Thr Thr Ile Glu Asn Gln Pro Trp Phe
        115                 120                 125

GCT GCT ATC TAC CGT CGT CAC CGT GGT GGT TCT GTT ACC TAC GTT TGC 432
Ala Ala Ile Tyr Arg Arg His Arg Gly Gly Ser Val Thr Tyr Val Cys
    130                 135                 140

GGT GGT TCT CTG ATC TCT CCG TGC TGG GTT ATC TCT GCT ACC CAC TGC 480
Gly Gly Ser Leu Ile Ser Pro Cys Trp Val Ile Ser Ala Thr His Cys
145                 150                 155                 160

TTC ATC GAC TAC CCG AAA AAA GAA GAC TAC ATC GTT TAC CTC GGC CGT 528
Phe Ile Asp Tyr Pro Lys Lys Glu Asp Tyr Ile Val Tyr Leu Gly Arg
                165                 170                 175

TCT CGT TTA AAC TCT AAC ACC CAG GGT GAA ATG AAA TTC GAA GTT GAA 576
Ser Arg Leu Asn Ser Asn Thr Gln Gly Glu Met Lys Phe Glu Val Glu
            180                 185                 190

AAC CTG ATC CTG CAC AAA GAC TAC TCT GCT GAC ACC CTG GCT CAC CAC 624
Asn Leu Ile Leu His Lys Asp Tyr Ser Ala Asp Thr Leu Ala His His
        195                 200                 205

AAC GAC ATC GCT CTG CTA AAA ATC CGT TCT AAA GAA GGT CGT TGC GCT 672
Asn Asp Ile Ala Leu Leu Lys Ile Arg Ser Lys Glu Gly Arg Cys Ala
    210                 215                 220

CAG CCG TCT CGT ACC ATC CAG ACC ATC TGC CTG CCG TCT ATG TAC AAC 720
Gln Pro Ser Arg Thr Ile Gln Thr Ile Cys Leu Pro Ser Met Tyr Asn
225                 230                 235                 240

GAC CCG CAG TTC GGT ACC TCT TGC GAA ATC ACC GGT TTC GGT AAA GAA 768
Asp Pro Gln Phe Gly Thr Ser Cys Glu Ile Thr Gly Phe Gly Lys Glu
                245                 250                 255

AAC TCT ACC GAC TAC CTG TAC CCG GAA CAG CTG AAA ATG ACC GTT GTT 816
Asn Ser Thr Asp Tyr Leu Tyr Pro Glu Gln Leu Lys Met Thr Val Val
            260                 265                 270

AAA CTG ATC TCT CAC CGT GAA TGC CAG CAG CCG CAC TAC TAC GGT TCT 864
Lys Leu Ile Ser His Arg Glu Cys Gln Gln Pro His Tyr Tyr Gly Ser
        275                 280                 285

GAA GTT ACC ACC AAA ATG CTG TGC GCT GCT GAC CCG CAG TGG AAA ACC 912
Glu Val Thr Thr Lys Met Leu Cys Ala Ala Asp Pro Gln Trp Lys Thr
    290                 295                 300

GAC TCT TGC CAA GGT GAC TCT GGT GGT CCA CTA GTT TGC TCT CTC CAG          960
Asp Ser Cys Gln Gly Asp Ser Gly Gly Pro Leu Val Cys Ser Leu Gln
305                 310                 315                 320

GGT CGT ATG ACC CTG ACC GGT ATT GTT TCT TGG GGT CGT GGT TGC GCT         1008
Gly Arg Met Thr Leu Thr Gly Ile Val Ser Trp Gly Arg Gly Cys Ala
                325                 330                 335

CTG AAA GAC AAA CCG GGT GTT TAC ACC CGT GTT TCT CAC TTC CTG CCG         1056
Leu Lys Asp Lys Pro Gly Val Tyr Thr Arg Val Ser His Phe Leu Pro
            340                 345                 350

TGG ATC CGT TCT CAC ACC AAA GAA GAA AAC GGT CTG GCT CTG AGC CCG         1104
Trp Ile Arg Ser His Thr Lys Glu Glu Asn Gly Leu Ala Leu Ser Pro
        355                 360                 365

GTT CGG GCT TTC GGA CCG CGG CCG CTG GGT GGT GGT GGT AAC GGT GAC         1152
Val Arg Ala Phe Gly Pro Arg Pro Leu Gly Gly Gly Gly Asn Gly Asp
    370                 375                 380

TTC GAA GAA ATC CCG GAA GAA TAC CTG CAA TAA                             1185
Phe Glu Glu Ile Pro Glu Glu Tyr Leu Gln  *
385                 390                 395

(2) ANGABEN ZU SEQ ID NO: 38:

(i) SEQUENZKENNZEICHEN:
(A) LÄNGE: 395 Aminosäuren
(B) ART: Aminosäure
(D) TOPOLOGIE: linear

(ii) ART DES MOLEKÜLS: Protein
(xi) SEQUENZBESCHREIBUNG: SEQ ID NO: 38:
   Met Ser Lys Thr Cys Tyr Glu Gly Asn Gly His Phe Tyr Arg Gly Lys
     1               5                  10                  15
   Ala Ser Thr Asp Thr Met Gly Arg Pro Cys Leu Pro Trp Asn Ser Ala
                20                  25                  30
   Thr Val Leu Gln Gln Thr Tyr His Ala His Arg Ser Asp Ala Leu Gln
            35                  40                  45
   Leu Gly Leu Gly Lys His Asn Tyr Cys Arg Asn Pro Asp Asn Arg Arg
        50                  55                  60
   Arg Pro Trp Cys Tyr Val Gln Val Gly Leu Lys Pro Leu Val Gln Glu
    65                  70                  75                  80
   Cys Met Val His Asp Cys Ala Asp Gly Lys Lys Pro Ser Ser Pro Pro
                    85                  90                  95
   Glu Glu Leu Lys Phe Gln Cys Gly Gln Lys Thr Leu Arg Pro Arg Phe
               100                 105                 110
   Lys Ile Ile Gly Gly Glu Phe Thr Thr Ile Glu Asn Gln Pro Trp Phe
           115                 120                 125
   Ala Ala Ile Tyr Arg Arg His Arg Gly Gly Ser Val Thr Tyr Val Cys
       130                 135                 140
   Gly Gly Ser Leu Ile Ser Pro Cys Trp Val Ile Ser Ala Thr His Cys
   145                 150                 155                 160
   Phe Ile Asp Tyr Pro Lys Lys Glu Asp Tyr Ile Val Tyr Leu Gly Arg
                   165                 170                 175
   Ser Arg Leu Asn Ser Asn Thr Gln Gly Glu Met Lys Phe Glu Val Glu
               180                 185                 190
   Asn Leu Ile Leu His Lys Asp Tyr Ser Ala Asp Thr Leu Ala His His
           195                 200                 205
   Asn Asp Ile Ala Leu Leu Lys Ile Arg Ser Lys Glu Gly Arg Cys Ala
       210                 215                 220
   Gln Pro Ser Arg Thr Ile Gln Thr Ile Cys Leu Pro Ser Met Tyr Asn
   225                 230                 235                 240
   Asp Pro Gln Phe Gly Thr Ser Cys Glu Ile Thr Gly Phe Gly Lys Glu
                   245                 250                 255
   Asn Ser Thr Asp Tyr Leu Tyr Pro Glu Gln Leu Lys Met Thr Val Val
               260                 265                 270
   Lys Leu Ile Ser His Arg Glu Cys Gln Gln Pro His Tyr Tyr Gly Ser
           275                 280                 285
   Glu Val Thr Thr Lys Met Leu Cys Ala Ala Asp Pro Gln Trp Lys Thr
       290                 295                 300
   Asp Ser Cys Gln Gly Asp Ser Gly Gly Pro Leu Val Cys Ser Leu Gln
   305                 310                 315                 320
   Gly Arg Met Thr Leu Thr Gly Ile Val Ser Trp Gly Arg Gly Cys Ala
                   325                 330                 335
   Leu Lys Asp Lys Pro Gly Val Tyr Thr Arg Val Ser His Phe Leu Pro
               340                 345                 350
   Trp Ile Arg Ser His Thr Lys Glu Glu Asn Gly Leu Ala Leu Ser Pro
           355                 360                 365
   Val Arg Ala Phe Gly Pro Arg Pro Leu Gly Gly Gly Gly Asn Gly Asp
       370                 375                 380
   Phe Glu Glu Ile Pro Glu Glu Tyr Leu Gln  *
   385                 390                 395

(2) ANGABEN ZU SEQ ID NO: 39:

     (i) SEQUENZKENNZEICHEN:
          (A) LÄNGE: 1185 Basenpaare
          (B) ART: Nucleotid
          (C) STRANGFORM: Einzelstrang
          (D) TOPOLOGIE: linear

    (ii) ART DES MOLEKÜLS: Sonstige Nucleinsäure

   (iii) HYPOTHETISCH: NEIN

    (iv) ANTISENSE: NEIN

(ix) MERKMAL:
(A) NAME/SCHLÜSSEL: CDS
(B) LAGE:1..1185
(D) SONSTIGE ANGABEN:/function= "N-term. Methionin wird
in E.coli proteolytisch abgespalten"
/product= "Synthetisches Gen fuer das Protein M19
(pSJ91)"

(xi) SEQUENZBESCHREIBUNG: SEQ ID NO: 39:

ATG AGC AAA ACT TGC TAC GAA GGT AAC GGT CAC TTC TAC CGT GGT AAG 48
Met Ser Lys Thr Cys Tyr Glu Gly Asn Gly His Phe Tyr Arg Gly Lys
1               5                   10                  15

GCT TCT ACC GAC ACC ATG GGT CGT CCG TGC CTG CCG TGG AAC TCT GCT 96
Ala Ser Thr Asp Thr Met Gly Arg Pro Cys Leu Pro Trp Asn Ser Ala
            20                  25                  30

ACC GTT CTG CAG CAG ACC TAC CAC GCT CAC CGT TCT GAT GCA TTG CAG 144
Thr Val Leu Gln Gln Thr Tyr His Ala His Arg Ser Asp Ala Leu Gln
        35                  40                  45

CTG GGT CTG GGT AAA CAC AAC TAC TGC CGT AAC CCG GAC AAC CGT CGT 192
Leu Gly Leu Gly Lys His Asn Tyr Cys Arg Asn Pro Asp Asn Arg Arg
    50                  55                  60

CGT CCG TGG TGC TAC GTT CAG GTT GGT CTG AAA CCG CTA GTT CAG GAA 240
Arg Pro Trp Cys Tyr Val Gln Val Gly Leu Lys Pro Leu Val Gln Glu
65                  70                  75                  80

TGC ATG GTT CAC GAC TGC GCT GAC GGT AAA AAA CCG TCT TCT CCG CCG 288
Cys Met Val His Asp Cys Ala Asp Gly Lys Lys Pro Ser Ser Pro Pro
                85                  90                  95

GAA GAG CTC AAA TTC CAG TGC GGT CAA AAA ACC CTA CGT CCG CGT TTT 336
Glu Glu Leu Lys Phe Gln Cys Gly Gln Lys Thr Leu Arg Pro Arg Phe
            100                 105                 110

AAA ATC ATC GGT GGT GAG TTC ACC ACC ATC GAA AAC CAG CCG TGG TTC 384
Lys Ile Ile Gly Gly Glu Phe Thr Thr Ile Glu Asn Gln Pro Trp Phe
        115                 120                 125

GCT GCT ATC TAC CGT CGT CAC CGT GGT GGT TCT GTT ACC TAC GTT TGC 432
Ala Ala Ile Tyr Arg Arg His Arg Gly Gly Ser Val Thr Tyr Val Cys
    130                 135                 140

GGT GGT TCT CTG ATC TCT CCG TGC TGG GTT ATC TCT GCT ACC CAC TGC 480
Gly Gly Ser Leu Ile Ser Pro Cys Trp Val Ile Ser Ala Thr His Cys
145                 150                 155                 160

TTC ATC GAC TAC CCG AAA AAA GAA GAC TAC ATC GTT TAC CTC GGC CGT 528
Phe Ile Asp Tyr Pro Lys Lys Glu Asp Tyr Ile Val Tyr Leu Gly Arg
                165                 170                 175

TCT CGT TTA AAC TCT AAC ACC CAG GGT GAA ATG AAA TTC GAA GTT GAA 576
Ser Arg Leu Asn Ser Asn Thr Gln Gly Glu Met Lys Phe Glu Val Glu
            180                 185                 190

AAC CTG ATC CTG CAC AAA GAC TAC TCT GCT GAC ACC CTG GCT CAC CAC 624
Asn Leu Ile Leu His Lys Asp Tyr Ser Ala Asp Thr Leu Ala His His
        195                 200                 205

AAC GAC ATC GCT CTG CTA AAA ATC CGT TCT AAA GAA GGT CGT TGC GCT 672
Asn Asp Ile Ala Leu Leu Lys Ile Arg Ser Lys Glu Gly Arg Cys Ala
    210                 215                 220

CAG CCG TCT CGT ACC ATC CAG ACC ATC TGC CTG CCG TCT ATG TAC AAC 720
Gln Pro Ser Arg Thr Ile Gln Thr Ile Cys Leu Pro Ser Met Tyr Asn
225                 230                 235                 240

GAC CCG CAG TTC GGT ACC TCT TGC GAA ATC ACC GGT TTC GGT AAA GAA 768
Asp Pro Gln Phe Gly Thr Ser Cys Glu Ile Thr Gly Phe Gly Lys Glu
                245                 250                 255

AAC TCT ACC GAC TAC CTG TAC CCG GAA CAG CTG AAA ATG ACC GTT GTT 816
Asn Ser Thr Asp Tyr Leu Tyr Pro Glu Gln Leu Lys Met Thr Val Val
            260                 265                 270

AAA CTG ATC TCT CAC CGT GAA TGC CAG CAG CCG CAC TAC TAC GGT TCT 864
Lys Leu Ile Ser His Arg Glu Cys Gln Gln Pro His Tyr Tyr Gly Ser
        275                 280                 285

GAA GTT ACC ACC AAA ATG CTG TGC GCT GCT GAC CCG CAG TGG AAA ACC 912
Glu Val Thr Thr Lys Met Leu Cys Ala Ala Asp Pro Gln Trp Lys Thr
    290                 295                 300

GAC TCT TGC CAA GGT GAC TCT GGT GGT CCA CTA GTT TGC TCT CTC CAG 960
Asp Ser Cys Gln Gly Asp Ser Gly Gly Pro Leu Val Cys Ser Leu Gln
305                 310                 315                 320

GGT CGT ATG ACC CTG ACC GGT ATT GTT TCT TGG GGT CGT GGT TGC GCT 1008
Gly Arg Met Thr Leu Thr Gly Ile Val Ser Trp Gly Arg Gly Cys Ala
                325                 330                 335

CTG AAA GAC AAA CCG GGT GTT TAC ACC CGT GTT TCT CAC TTC CTG CCG 1056
Leu Lys Asp Lys Pro Gly Val Tyr Thr Arg Val Ser His Phe Leu Pro
            340                 345                 350

TGG ATC CGT TCT CAC ACC AAA GAA GAA AAC GGT CTG GCT CTG AGC CCG 1104
Trp Ile Arg Ser His Thr Lys Glu Glu Asn Gly Leu Ala Leu Ser Pro
        355                 360                 365

GTT AAG GCT TAC GGA CCG CGG CCG CTG GGT GGT GGT GGT AAC GGT GAC 1152
Val Lys Ala Tyr Gly Pro Arg Pro Leu Gly Gly Gly Gly Asn Gly Asp
    370                 375                 380

TTC GAA GAA ATC CCG GAA GAA TAC CTG CAA TAA 1185
Phe Glu Glu Ile Pro Glu Glu Tyr Leu Gln *
385                 390                 395

(2) ANGABEN ZU SEQ ID NO: 40:

(i) SEQUENZKENNZEICHEN:
(A) LÄNGE: 395 Aminosäuren
(B) ART: Aminosäure
(D) TOPOLOGIE: linear

(ii) ART DES MOLEKÜLS: Protein
(xi) SEQUENZBESCHREIBUNG: SEQ ID NO: 40:

Met Ser Lys Thr Cys Tyr Glu Gly Asn Gly His Phe Tyr Arg Gly Lys
1               5                   10                  15

Ala Ser Thr Asp Thr Met Gly Arg Pro Cys Leu Pro Trp Asn Ser Ala
            20                  25                  30

Thr Val Leu Gln Gln Thr Tyr His Ala His Arg Ser Asp Ala Leu Gln
        35                  40                  45

Leu Gly Leu Gly Lys His Asn Tyr Cys Arg Asn Pro Asp Asn Arg Arg
    50                  55                  60

Arg Pro Trp Cys Tyr Val Gln Val Gly Leu Lys Pro Leu Val Gln Glu
65                  70                  75                  80

Cys Met Val His Asp Cys Ala Asp Gly Lys Lys Pro Ser Ser Pro Pro
                85                  90                  95

Glu Glu Leu Lys Phe Gln Cys Gly Gln Lys Thr Leu Arg Pro Arg Phe
            100                 105                 110

Lys Ile Ile Gly Gly Glu Phe Thr Thr Ile Glu Asn Gln Pro Trp Phe
        115                 120                 125

Ala Ala Ile Tyr Arg Arg His Arg Gly Gly Ser Val Thr Tyr Val Cys
    130                 135                 140

Gly Gly Ser Leu Ile Ser Pro Cys Trp Val Ile Ser Ala Thr His Cys
145                 150                 155                 160

Phe Ile Asp Tyr Pro Lys Lys Glu Asp Tyr Ile Val Tyr Leu Gly Arg
                165                 170                 175

Ser Arg Leu Asn Ser Asn Thr Gln Gly Glu Met Lys Phe Glu Val Glu
            180                 185                 190

Asn Leu Ile Leu His Lys Asp Tyr Ser Ala Asp Thr Leu Ala His His
        195                 200                 205

Asn Asp Ile Ala Leu Leu Lys Ile Arg Ser Lys Glu Gly Arg Cys Ala
    210                 215                 220

Gln Pro Ser Arg Thr Ile Gln Thr Ile Cys Leu Pro Ser Met Tyr Asn
225                 230                 235                 240

Asp Pro Gln Phe Gly Thr Ser Cys Glu Ile Thr Gly Phe Gly Lys Glu
                245                 250                 255

Asn Ser Thr Asp Tyr Leu Tyr Pro Glu Gln Leu Lys Met Thr Val Val
            260                 265                 270

Lys Leu Ile Ser His Arg Glu Cys Gln Gln Pro His Tyr Tyr Gly Ser
        275                 280                 285

Glu Val Thr Thr Lys Met Leu Cys Ala Ala Asp Pro Gln Trp Lys Thr
    290                 295                 300

Asp Ser Cys Gln Gly Asp Ser Gly Gly Pro Leu Val Cys Ser Leu Gln
305                 310                 315                 320

Gly Arg Met Thr Leu Thr Gly Ile Val Ser Trp Gly Arg Gly Cys Ala
                325                 330                 335

Leu Lys Asp Lys Pro Gly Val Tyr Thr Arg Val Ser His Phe Leu Pro
            340                 345                 350

Trp Ile Arg Ser His Thr Lys Glu Glu Asn Gly Leu Ala Leu Ser Pro
        355                 360                 365

Val Lys Ala Tyr Gly Pro Arg Pro Leu Gly Gly Gly Gly Asn Gly Asp
    370                 375                 380

Phe Glu Glu Ile Pro Glu Glu Tyr Leu Gln  *
385                 390                 395

(2) ANGABEN ZU SEQ ID NO: 41:

     (i) SEQUENZKENNZEICHEN:
          (A) LÄNGE: 1185 Basenpaare
          (B) ART: Nucleotid
          (C) STRANGFORM: Einzelstrang
          (D) TOPOLOGIE: linear

    (ii) ART DES MOLEKÜLS: Sonstige Nucleinsäure

   (iii) HYPOTHETISCH: NEIN

    (iv) ANTISENSE: NEIN

    (ix) MERKMAL:
          (A) NAME/SCHLÜSSEL: CDS
          (B) LAGE:1..1185
          (D) SONSTIGE ANGABEN:/function= "N-term. Methionin wird
               in E.coli proteolytisch abgespalten"
               /product= "Synthetisches Gen fuer das Protein M20
               (pSJ92)"

    (xi) SEQUENZBESCHREIBUNG: SEQ ID NO: 41:

ATG AGC AAA ACT TGC TAC GAA GGT AAC GGT CAC TTC TAC CGT GGT AAG      48
Met Ser Lys Thr Cys Tyr Glu Gly Asn Gly His Phe Tyr Arg Gly Lys
  1               5                  10                  15

GCT TCT ACC GAC ACC ATG GGT CGT CCG TGC CTG CCG TGG AAC TCT GCT      96
Ala Ser Thr Asp Thr Met Gly Arg Pro Cys Leu Pro Trp Asn Ser Ala
             20                  25                  30

ACC GTT CTG CAG CAG ACC TAC CAC GCT CAC CGT TCT GAT GCA TTG CAG     144
Thr Val Leu Gln Gln Thr Tyr His Ala His Arg Ser Asp Ala Leu Gln
         35                  40                  45

CTG GGT CTG GGT AAA CAC AAC TAC TGC CGT AAC CCG GAC AAC CGT CGT     192
Leu Gly Leu Gly Lys His Asn Tyr Cys Arg Asn Pro Asp Asn Arg Arg
     50                  55                  60

CGT CCG TGG TGC TAC GTT CAG GTT GGT CTG AAA CCG CTA GTT CAG GAA     240
Arg Pro Trp Cys Tyr Val Gln Val Gly Leu Lys Pro Leu Val Gln Glu
 65                  70                  75                  80

TGC ATG GTT CAC GAC TGC GCT GAC GGT AAA AAA CCG TCT TCT CCG CCG     288
Cys Met Val His Asp Cys Ala Asp Gly Lys Lys Pro Ser Ser Pro Pro
                 85                  90                  95

GAA GAG CTC AAA TTC CAG TGC GGT CAA AAA ACC CTA CGT CCG CGT TTT     336
Glu Glu Leu Lys Phe Gln Cys Gly Gln Lys Thr Leu Arg Pro Arg Phe
            100                 105                 110

AAA ATC ATC GGT GGT GAG TTC ACC ACC ATC GAA AAC CAG CCG TGG TTC     384
Lys Ile Ile Gly Gly Glu Phe Thr Thr Ile Glu Asn Gln Pro Trp Phe
        115                 120                 125

GCT GCT ATC TAC CGT CGT CAC CGT GGT GGT TCT GTT ACC TAC GTT TGC 432
Ala Ala Ile Tyr Arg Arg His Arg Gly Gly Ser Val Thr Tyr Val Cys
    130                 135                 140

GGT GGT TCT CTG ATC TCT CCG TGC TGG GTT ATC TCT GCT ACC CAC TGC 480
Gly Gly Ser Leu Ile Ser Pro Cys Trp Val Ile Ser Ala Thr His Cys
145                 150                 155                 160

TTC ATC GAC TAC CCG AAA AAA GAA GAC TAC ATC GTT TAC CTC GGC CGT 528
Phe Ile Asp Tyr Pro Lys Lys Glu Asp Tyr Ile Val Tyr Leu Gly Arg
                165                 170                 175

TCT CGT TTA AAC TCT AAC ACC CAG GGT GAA ATG AAA TTC GAA GTT GAA 576
Ser Arg Leu Asn Ser Asn Thr Gln Gly Glu Met Lys Phe Glu Val Glu
            180                 185                 190

AAC CTG ATC CTG CAC AAA GAC TAC TCT GCT GAC ACC CTG GCT CAC CAC 624
Asn Leu Ile Leu His Lys Asp Tyr Ser Ala Asp Thr Leu Ala His His
        195                 200                 205

AAC GAC ATC GCT CTG CTA AAA ATC CGT TCT AAA GAA GGT CGT TGC GCT 672
Asn Asp Ile Ala Leu Leu Lys Ile Arg Ser Lys Glu Gly Arg Cys Ala
    210                 215                 220

CAG CCG TCT CGT ACC ATC CAG ACC ATC TGC CTG CCG TCT ATG TAC AAC 720
Gln Pro Ser Arg Thr Ile Gln Thr Ile Cys Leu Pro Ser Met Tyr Asn
225                 230                 235                 240

GAC CCG CAG TTC GGT ACC TCT TGC GAA ATC ACC GGT TTC GGT AAA GAA 768
Asp Pro Gln Phe Gly Thr Ser Cys Glu Ile Thr Gly Phe Gly Lys Glu
                245                 250                 255

AAC TCT ACC GAC TAC CTG TAC CCG GAA CAG CTG AAA ATG ACC GTT GTT 816
Asn Ser Thr Asp Tyr Leu Tyr Pro Glu Gln Leu Lys Met Thr Val Val
            260                 265                 270

AAA CTG ATC TCT CAC CGT GAA TGC CAG CAG CCG CAC TAC TAC GGT TCT 864
Lys Leu Ile Ser His Arg Glu Cys Gln Gln Pro His Tyr Tyr Gly Ser
        275                 280                 285

GAA GTT ACC ACC AAA ATG CTG TGC GCT GCT GAC CCG CAG TGG AAA ACC 912
Glu Val Thr Thr Lys Met Leu Cys Ala Ala Asp Pro Gln Trp Lys Thr
    290                 295                 300

GAC TCT TGC CAA GGT GAC TCT GGT GGT CCA CTA GTT TGC TCT CTC CAG 960
Asp Ser Cys Gln Gly Asp Ser Gly Gly Pro Leu Val Cys Ser Leu Gln
305                 310                 315                 320

GGT CGT ATG ACC CTG ACC GGT ATT GTT TCT TGG GGT CGT GGT TGC GCT 1008
Gly Arg Met Thr Leu Thr Gly Ile Val Ser Trp Gly Arg Gly Cys Ala
                325                 330                 335

CTG AAA GAC AAA CCG GGT GTT TAC ACC CGT GTT TCT CAC TTC CTG CCG 1056
Leu Lys Asp Lys Pro Gly Val Tyr Thr Arg Val Ser His Phe Leu Pro
            340                 345                 350

TGG ATC CGT TCT CAC ACC AAA GAA GAA AAC GGT CTG GCT CTG AGC CCG 1104
Trp Ile Arg Ser His Thr Lys Glu Glu Asn Gly Leu Ala Leu Ser Pro
        355                 360                 365

GTT GTT GCT TTC GGA CCG CGG CCG CTG GGT GGT GGT GGT AAC GGT GAC 1152
Val Val Ala Phe Gly Pro Arg Pro Leu Gly Gly Gly Gly Asn Gly Asp
    370                 375                 380

TTC GAA GAA ATC CCG GAA GAA TAC CTG CAA TAA 1185
Phe Glu Glu Ile Pro Glu Glu Tyr Leu Gln *
385                 390                 395

(2) ANGABEN ZU SEQ ID NO: 42:

(i) SEQUENZKENNZEICHEN:
(A) LÄNGE: 395 Aminosäuren
(B) ART: Aminosäure
(D) TOPOLOGIE: linear

(ii) ART DES MOLEKÜLS: Protein
(xi) SEQUENZBESCHREIBUNG: SEQ ID NO: 42:

Met Ser Lys Thr Cys Tyr Glu Gly Asn Gly His Phe Tyr Arg Gly Lys
1               5                   10                  15

Ala Ser Thr Asp Thr Met Gly Arg Pro Cys Leu Pro Trp Asn Ser Ala
            20                  25                  30

Thr Val Leu Gln Gln Thr Tyr His Ala His Arg Ser Asp Ala Leu Gln
        35                  40                  45

Leu Gly Leu Gly Lys His Asn Tyr Cys Arg Asn Pro Asp Asn Arg Arg
    50                  55                  60

Arg Pro Trp Cys Tyr Val Gln Val Gly Leu Lys Pro Leu Val Gln Glu
65                  70                  75                  80

Cys Met Val His Asp Cys Ala Asp Gly Lys Lys Pro Ser Ser Pro Pro
                85                  90                  95

Glu Glu Leu Lys Phe Gln Cys Gly Gln Lys Thr Leu Arg Pro Arg Phe
            100                 105                 110

Lys Ile Ile Gly Gly Glu Phe Thr Thr Ile Glu Asn Gln Pro Trp Phe
        115                 120                 125

Ala Ala Ile Tyr Arg Arg His Arg Gly Gly Ser Val Thr Tyr Val Cys
    130                 135                 140

Gly Gly Ser Leu Ile Ser Pro Cys Trp Val Ile Ser Ala Thr His Cys
145                 150                 155                 160

Phe Ile Asp Tyr Pro Lys Lys Glu Asp Tyr Ile Val Tyr Leu Gly Arg
                165                 170                 175

Ser Arg Leu Asn Ser Asn Thr Gln Gly Glu Met Lys Phe Glu Val Glu
            180                 185                 190

Asn Leu Ile Leu His Lys Asp Tyr Ser Ala Asp Thr Leu Ala His His
        195                 200                 205

Asn Asp Ile Ala Leu Leu Lys Ile Arg Ser Lys Glu Gly Arg Cys Ala
    210                 215                 220

Gln Pro Ser Arg Thr Ile Gln Thr Ile Cys Leu Pro Ser Met Tyr Asn
225                 230                 235                 240

Asp Pro Gln Phe Gly Thr Ser Cys Glu Ile Thr Gly Phe Gly Lys Glu
                245                 250                 255

Asn Ser Thr Asp Tyr Leu Tyr Pro Glu Gln Leu Lys Met Thr Val Val
            260                 265                 270

Lys Leu Ile Ser His Arg Glu Cys Gln Gln Pro His Tyr Tyr Gly Ser
        275                 280                 285

Glu Val Thr Thr Lys Met Leu Cys Ala Ala Asp Pro Gln Trp Lys Thr
    290                 295                 300

Asp Ser Cys Gln Gly Asp Ser Gly Gly Pro Leu Val Cys Ser Leu Gln
305                 310                 315                 320

Gly Arg Met Thr Leu Thr Gly Ile Val Ser Trp Gly Arg Gly Cys Ala
                325                 330                 335

Leu Lys Asp Lys Pro Gly Val Tyr Thr Arg Val Ser His Phe Leu Pro
            340                 345                 350

Trp Ile Arg Ser His Thr Lys Glu Glu Asn Gly Leu Ala Leu Ser Pro
        355                 360                 365

Val Val Ala Phe Gly Pro Arg Pro Leu Gly Gly Gly Gly Asn Gly Asp
    370                 375                 380

Phe Glu Glu Ile Pro Glu Glu Tyr Leu Gln  *
385                 390                 395

(2) ANGABEN ZU SEQ ID NO: 43:

(i) SEQUENZKENNZEICHEN:
(A) LÄNGE: 1182 Basenpaare
(B) ART: Nucleotid
(C) STRANGFORM: Einzelstrang
(D) TOPOLOGIE: linear

(ii) ART DES MOLEKÜLS: Sonstige Nucleinsäure

(iii) HYPOTHETISCH: NEIN

(iv) ANTISENSE: NEIN

(ix) MERKMAL:
(A) NAME/SCHLÜSSEL: CDS
(B) LAGE:1..1182
(D) SONSTIGE ANGABEN:/function= "N-term. Methionin wird
in E.coli proteolytisch abgespalten"
/product= "Synthetisches Gen fuer das Protein M21
(pSJ93)"

(xi) SEQUENZBESCHREIBUNG: SEQ ID NO: 43:

ATG AGC AAA ACT TGC TAC GAA GGT AAC GGT CAC TTC TAC CGT GGT AAG      48
Met Ser Lys Thr Cys Tyr Glu Gly Asn Gly His Phe Tyr Arg Gly Lys
  1               5                  10                  15

GCT TCT ACC GAC ACC ATG GGT CGT CCG TGC CTG CCG TGG AAC TCT GCT      96
Ala Ser Thr Asp Thr Met Gly Arg Pro Cys Leu Pro Trp Asn Ser Ala
             20                  25                  30

ACC GTT CTG CAG CAG ACC TAC CAC GCT CAC CGT TCT GAT GCA TTG CAG     144
Thr Val Leu Gln Gln Thr Tyr His Ala His Arg Ser Asp Ala Leu Gln
         35                  40                  45

CTG GGT CTG GGT AAA CAC AAC TAC TGC CGT AAC CCG GAC AAC CGT CGT 192
Leu Gly Leu Gly Lys His Asn Tyr Cys Arg Asn Pro Asp Asn Arg Arg
    50                  55                  60

CGT CCG TGG TGC TAC GTT CAG GTT GGT CTG AAA CCG CTA GTT CAG GAA 240
Arg Pro Trp Cys Tyr Val Gln Val Gly Leu Lys Pro Leu Val Gln Glu
65                  70                  75                  80

TGC ATG GTT CAC GAC TGC GCT GAC GGT AAA AAA CCG TCT TCT CCG CCG 288
Cys Met Val His Asp Cys Ala Asp Gly Lys Lys Pro Ser Ser Pro Pro
                85                  90                  95

GAA GAG CTC AAA TTC CAG TGC GGT CAA AAA ACC CTA CGT CCG CGT TTT 336
Glu Glu Leu Lys Phe Gln Cys Gly Gln Lys Thr Leu Arg Pro Arg Phe
            100                 105                 110

AAA ATC ATC GGT GGT GAG TTC ACC ACC ATC GAA AAC CAG CCG TGG TTC 384
Lys Ile Ile Gly Gly Glu Phe Thr Thr Ile Glu Asn Gln Pro Trp Phe
        115                 120                 125

GCT GCT ATC TAC CGT CGT CAC CGT GGT GGT TCT GTT ACC TAC GTT TGC 432
Ala Ala Ile Tyr Arg Arg His Arg Gly Gly Ser Val Thr Tyr Val Cys
    130                 135                 140

GGT GGT TCT CTG ATC TCT CCG TGC TGG GTT ATC TCT GCT ACC CAC TGC 480
Gly Gly Ser Leu Ile Ser Pro Cys Trp Val Ile Ser Ala Thr His Cys
145                 150                 155                 160

TTC ATC GAC TAC CCG AAA AAA GAA GAC TAC ATC GTT TAC CTC GGC CGT 528
Phe Ile Asp Tyr Pro Lys Lys Glu Asp Tyr Ile Val Tyr Leu Gly Arg
                165                 170                 175

TCT CGT TTA AAC TCT AAC ACC CAG GGT GAA ATG AAA TTC GAA GTT GAA 576
Ser Arg Leu Asn Ser Asn Thr Gln Gly Glu Met Lys Phe Glu Val Glu
            180                 185                 190

AAC CTG ATC CTG CAC AAA GAC TAC TCT GCT GAC ACC CTG GCT CAC CAC 624
Asn Leu Ile Leu His Lys Asp Tyr Ser Ala Asp Thr Leu Ala His His
        195                 200                 205

AAC GAC ATC GCT CTG CTA AAA ATC CGT TCT AAA GAA GGT CGT TGC GCT 672
Asn Asp Ile Ala Leu Leu Lys Ile Arg Ser Lys Glu Gly Arg Cys Ala
    210                 215                 220

CAG CCG TCT CGT ACC ATC CAG ACC ATC TGC CTG CCG TCT ATG TAC AAC 720
Gln Pro Ser Arg Thr Ile Gln Thr Ile Cys Leu Pro Ser Met Tyr Asn
225                 230                 235                 240

GAC CCG CAG TTC GGT ACC TCT TGC GAA ATC ACC GGT TTC GGT AAA GAA 768
Asp Pro Gln Phe Gly Thr Ser Cys Glu Ile Thr Gly Phe Gly Lys Glu
                245                 250                 255

AAC TCT ACC GAC TAC CTG TAC CCG GAA CAG CTG AAA ATG ACC GTT GTT 816
Asn Ser Thr Asp Tyr Leu Tyr Pro Glu Gln Leu Lys Met Thr Val Val
            260                 265                 270

AAA CTG ATC TCT CAC CGT GAA TGC CAG CAG CCG CAC TAC TAC GGT TCT 864
Lys Leu Ile Ser His Arg Glu Cys Gln Gln Pro His Tyr Tyr Gly Ser
        275                 280                 285

GAA GTT ACC ACC AAA ATG CTG TGC GCT GCT GAC CCG CAG TGG AAA ACC 912
Glu Val Thr Thr Lys Met Leu Cys Ala Ala Asp Pro Gln Trp Lys Thr
    290                 295                 300

GAC TCT TGC CAA GGT GAC TCT GGT GGT CCA CTA GTT TGC TCT CTC CAG 960
Asp Ser Cys Gln Gly Asp Ser Gly Gly Pro Leu Val Cys Ser Leu Gln
305                 310                 315                 320

GGT CGT ATG ACC CTG ACC GGT ATT GTT TCT TGG GGT CGT GGT TGC GCT 1008
Gly Arg Met Thr Leu Thr Gly Ile Val Ser Trp Gly Arg Gly Cys Ala
                325                 330                 335

CTG AAA GAC AAA CCG GGT GTT TAC ACC CGT GTT TCT CAC TTC CTG CCG 1056
Leu Lys Asp Lys Pro Gly Val Tyr Thr Arg Val Ser His Phe Leu Pro
            340                 345                 350

TGG ATC CGT TCT CAC ACC AAA GAA GAA AAC GGT CTG GCT CTG AGC CCG 1104
Trp Ile Arg Ser His Thr Lys Glu Glu Asn Gly Leu Ala Leu Ser Pro
        355                 360                 365

GTT CGG GCT TTC CCG CGG CCG CTG GGT GGT GGT GGT AAC GGT GAC TTC 1152
Val Arg Ala Phe Pro Arg Pro Leu Gly Gly Gly Gly Asn Gly Asp Phe
    370                 375                 380

GAA GAA ATC CCG GAA GAA TAC CTG CAA TAA 1182
Glu Glu Ile Pro Glu Glu Tyr Leu Gln *
385                 390

(2) ANGABEN ZU SEQ ID NO: 44:

(i) SEQUENZKENNZEICHEN:
(A) LÄNGE: 394 Aminosäuren
(B) ART: Aminosäure
(D) TOPOLOGIE: linear

(ii) ART DES MOLEKÜLS: Protein
(xi) SEQUENZBESCHREIBUNG: SEQ ID NO: 44:

Met Ser Lys Thr Cys Tyr Glu Gly Asn Gly His Phe Tyr Arg Gly Lys
1               5                   10                  15

Ala Ser Thr Asp Thr Met Gly Arg Pro Cys Leu Pro Trp Asn Ser Ala
            20                  25                  30

Thr Val Leu Gln Gln Thr Tyr His Ala His Arg Ser Asp Ala Leu Gln
        35                  40                  45

Leu Gly Leu Gly Lys His Asn Tyr Cys Arg Asn Pro Asp Asn Arg Arg
    50                  55                  60

Arg Pro Trp Cys Tyr Val Gln Val Gly Leu Lys Pro Leu Val Gln Glu
65                  70                  75                  80

Cys Met Val His Asp Cys Ala Asp Gly Lys Lys Pro Ser Ser Pro Pro
                85                  90                  95

Glu Glu Leu Lys Phe Gln Cys Gly Gln Lys Thr Leu Arg Pro Arg Phe
            100                 105                 110

Lys Ile Ile Gly Gly Glu Phe Thr Thr Ile Glu Asn Gln Pro Trp Phe
        115                 120                 125

Ala Ala Ile Tyr Arg Arg His Arg Gly Gly Ser Val Thr Tyr Val Cys
    130                 135                 140

Gly Gly Ser Leu Ile Ser Pro Cys Trp Val Ile Ser Ala Thr His Cys
145                 150                 155                 160

Phe Ile Asp Tyr Pro Lys Lys Glu Asp Tyr Ile Val Tyr Leu Gly Arg
                165                 170                 175

Ser Arg Leu Asn Ser Asn Thr Gln Gly Glu Met Lys Phe Glu Val Glu
            180                 185                 190

Asn Leu Ile Leu His Lys Asp Tyr Ser Ala Asp Thr Leu Ala His His
        195                 200                 205

Asn Asp Ile Ala Leu Leu Lys Ile Arg Ser Lys Glu Gly Arg Cys Ala
    210                 215                 220

Gln Pro Ser Arg Thr Ile Gln Thr Ile Cys Leu Pro Ser Met Tyr Asn
225                 230                 235                 240

Asp Pro Gln Phe Gly Thr Ser Cys Glu Ile Thr Gly Phe Gly Lys Glu
                245                 250                 255

Asn Ser Thr Asp Tyr Leu Tyr Pro Glu Gln Leu Lys Met Thr Val Val
            260                 265                 270

Lys Leu Ile Ser His Arg Glu Cys Gln Gln Pro His Tyr Tyr Gly Ser
        275                 280                 285

Glu Val Thr Thr Lys Met Leu Cys Ala Ala Asp Pro Gln Trp Lys Thr
    290                 295                 300

Asp Ser Cys Gln Gly Asp Ser Gly Gly Pro Leu Val Cys Ser Leu Gln
305                 310                 315                 320

Gly Arg Met Thr Leu Thr Gly Ile Val Ser Trp Gly Arg Gly Cys Ala
                325                 330                 335

Leu Lys Asp Lys Pro Gly Val Tyr Thr Arg Val Ser His Phe Leu Pro
            340                 345                 350

Trp Ile Arg Ser His Thr Lys Glu Glu Asn Gly Leu Ala Leu Ser Pro
        355                 360                 365

Val Arg Ala Phe Pro Arg Pro Leu Gly Gly Gly Gly Asn Gly Asp Phe
    370                 375                 380

Glu Glu Ile Pro Glu Glu Tyr Leu Gln  *
385                 390

(2) ANGABEN ZU SEQ ID NO: 45:

     (i) SEQUENZKENNZEICHEN:
          (A) LÄNGE: 1182 Basenpaare
          (B) ART: Nucleotid
          (C) STRANGFORM: Einzelstrang
          (D) TOPOLOGIE: linear

    (ii) ART DES MOLEKÜLS: Sonstige Nucleinsäure

   (iii) HYPOTHETISCH: NEIN

    (iv) ANTISENSE: NEIN

(ix) MERKMAL:
(A) NAME/SCHLÜSSEL: CDS
(B) LAGE:1..1182
(D) SONSTIGE ANGABEN:/function= "N-term. Methionin wird
in E.coli proteolytisch abgespalten"
/product= "Synthetisches Gen fuer das Protein M22
(pSJ94)"

(xi) SEQUENZBESCHREIBUNG: SEQ ID NO: 45:

ATG AGC AAA ACT TGC TAC GAA GGT AAC GGT CAC TTC TAC CGT GGT AAG 48
Met Ser Lys Thr Cys Tyr Glu Gly Asn Gly His Phe Tyr Arg Gly Lys
1               5                   10                  15

GCT TCT ACC GAC ACC ATG GGT CGT CCG TGC CTG CCG TGG AAC TCT GCT 96
Ala Ser Thr Asp Thr Met Gly Arg Pro Cys Leu Pro Trp Asn Ser Ala
            20                  25                  30

ACC GTT CTG CAG CAG ACC TAC CAC GCT CAC CGT TCT GAT GCA TTG CAG 144
Thr Val Leu Gln Gln Thr Tyr His Ala His Arg Ser Asp Ala Leu Gln
        35                  40                  45

CTG GGT CTG GGT AAA CAC AAC TAC TGC CGT AAC CCG GAC AAC CGT CGT 192
Leu Gly Leu Gly Lys His Asn Tyr Cys Arg Asn Pro Asp Asn Arg Arg
    50                  55                  60

CGT CCG TGG TGC TAC GTT CAG GTT GGT CTG AAA CCG CTA GTT CAG GAA 240
Arg Pro Trp Cys Tyr Val Gln Val Gly Leu Lys Pro Leu Val Gln Glu
65                  70                  75                  80

TGC ATG GTT CAC GAC TGC GCT GAC GGT AAA AAA CCG TCT TCT CCG CCG 288
Cys Met Val His Asp Cys Ala Asp Gly Lys Lys Pro Ser Ser Pro Pro
                85                  90                  95

GAA GAG CTC AAA TTC CAG TGC GGT CAA AAA ACC CTA CGT CCG CGT TTT 336
Glu Glu Leu Lys Phe Gln Cys Gly Gln Lys Thr Leu Arg Pro Arg Phe
            100                 105                 110

AAA ATC ATC GGT GGT GAG TTC ACC ACC ATC GAA AAC CAG CCG TGG TTC 384
Lys Ile Ile Gly Gly Glu Phe Thr Thr Ile Glu Asn Gln Pro Trp Phe
        115                 120                 125

GCT GCT ATC TAC CGT CGT CAC CGT GGT GGT TCT GTT ACC TAC GTT TGC 432
Ala Ala Ile Tyr Arg Arg His Arg Gly Gly Ser Val Thr Tyr Val Cys
    130                 135                 140

GGT GGT TCT CTG ATC TCT CCG TGC TGG GTT ATC TCT GCT ACC CAC TGC 480
Gly Gly Ser Leu Ile Ser Pro Cys Trp Val Ile Ser Ala Thr His Cys
145                 150                 155                 160

TTC ATC GAC TAC CCG AAA AAA GAA GAC TAC ATC GTT TAC CTC GGC CGT 528
Phe Ile Asp Tyr Pro Lys Lys Glu Asp Tyr Ile Val Tyr Leu Gly Arg
                165                 170                 175

TCT CGT TTA AAC TCT AAC ACC CAG GGT GAA ATG AAA TTC GAA GTT GAA 576
Ser Arg Leu Asn Ser Asn Thr Gln Gly Glu Met Lys Phe Glu Val Glu
            180                 185                 190

AAC CTG ATC CTG CAC AAA GAC TAC TCT GCT GAC ACC CTG GCT CAC CAC 624
Asn Leu Ile Leu His Lys Asp Tyr Ser Ala Asp Thr Leu Ala His His
        195                 200                 205

AAC GAC ATC GCT CTG CTA AAA ATC CGT TCT AAA GAA GGT CGT TGC GCT 672
Asn Asp Ile Ala Leu Leu Lys Ile Arg Ser Lys Glu Gly Arg Cys Ala
    210                 215                 220

CAG CCG TCT CGT ACC ATC CAG ACC ATC TGC CTG CCG TCT ATG TAC AAC 720
Gln Pro Ser Arg Thr Ile Gln Thr Ile Cys Leu Pro Ser Met Tyr Asn
225                 230                 235                 240

GAC CCG CAG TTC GGT ACC TCT TGC GAA ATC ACC GGT TTC GGT AAA GAA 768
Asp Pro Gln Phe Gly Thr Ser Cys Glu Ile Thr Gly Phe Gly Lys Glu
                245                 250                 255

AAC TCT ACC GAC TAC CTG TAC CCG GAA CAG CTG AAA ATG ACC GTT GTT 816
Asn Ser Thr Asp Tyr Leu Tyr Pro Glu Gln Leu Lys Met Thr Val Val
            260                 265                 270

AAA CTG ATC TCT CAC CGT GAA TGC CAG CAG CCG CAC TAC TAC GGT TCT 864
Lys Leu Ile Ser His Arg Glu Cys Gln Gln Pro His Tyr Tyr Gly Ser
        275                 280                 285

GAA GTT ACC ACC AAA ATG CTG TGC GCT GCT GAC CCG CAG TGG AAA ACC 912
Glu Val Thr Thr Lys Met Leu Cys Ala Ala Asp Pro Gln Trp Lys Thr
    290                 295                 300

GAC TCT TGC CAA GGT GAC TCT GGT GGT CCA CTA GTT TGC TCT CTC CAG 960
Asp Ser Cys Gln Gly Asp Ser Gly Gly Pro Leu Val Cys Ser Leu Gln
305                 310                 315                 320

GGT CGT ATG ACC CTG ACC GGT ATT GTT TCT TGG GGT CGT GGT TGC GCT 1008
Gly Arg Met Thr Leu Thr Gly Ile Val Ser Trp Gly Arg Gly Cys Ala
                325                 330                 335

CTG AAA GAC AAA CCG GGT GTT TAC ACC CGT GTT TCT CAC TTC CTG CCG 1056
Leu Lys Asp Lys Pro Gly Val Tyr Thr Arg Val Ser His Phe Leu Pro
            340                 345                 350

TGG ATC CGT TCT CAC ACC AAA GAA GAA AAC GGT CTG GCT CTG AGC CCG 1104
Trp Ile Arg Ser His Thr Lys Glu Glu Asn Gly Leu Ala Leu Ser Pro
        355                 360                 365

GTT AAA GCT TAC CCG CGG CCG CTG GGT GGT GGT GGT AAC GGT GAC TTC 1152
Val Lys Ala Tyr Pro Arg Pro Leu Gly Gly Gly Gly Asn Gly Asp Phe
    370                 375                 380

GAA GAA ATC CCG GAA GAA TAC CTG CAA TAA 1182
Glu Glu Ile Pro Glu Glu Tyr Leu Gln *
385                 390

(2) ANGABEN ZU SEQ ID NO: 46:

(i) SEQUENZKENNZEICHEN:
(A) LÄNGE: 394 Aminosäuren
(B) ART: Aminosäure
(D) TOPOLOGIE: linear

(ii) ART DES MOLEKÜLS: Protein
(xi) SEQUENZBESCHREIBUNG: SEQ ID NO: 46:

Met Ser Lys Thr Cys Tyr Glu Gly Asn Gly His Phe Tyr Arg Gly Lys
1               5                   10                  15

Ala Ser Thr Asp Thr Met Gly Arg Pro Cys Leu Pro Trp Asn Ser Ala
            20                  25                  30

Thr Val Leu Gln Gln Thr Tyr His Ala His Arg Ser Asp Ala Leu Gln
            35                  40                  45

Leu Gly Leu Gly Lys His Asn Tyr Cys Arg Asn Pro Asp Asn Arg Arg
        50                  55                  60

Arg Pro Trp Cys Tyr Val Gln Val Gly Leu Lys Pro Leu Val Gln Glu
65                  70                  75                  80

Cys Met Val His Asp Cys Ala Asp Gly Lys Lys Pro Ser Ser Pro Pro
                85                  90                  95

Glu Glu Leu Lys Phe Gln Cys Gly Gln Lys Thr Leu Arg Pro Arg Phe
            100                 105                 110

Lys Ile Ile Gly Gly Glu Phe Thr Thr Ile Glu Asn Gln Pro Trp Phe
        115                 120                 125

Ala Ala Ile Tyr Arg Arg His Arg Gly Gly Ser Val Thr Tyr Val Cys
    130                 135                 140

Gly Gly Ser Leu Ile Ser Pro Cys Trp Val Ile Ser Ala Thr His Cys
145                 150                 155                 160

Phe Ile Asp Tyr Pro Lys Lys Glu Asp Tyr Ile Val Tyr Leu Gly Arg
                165                 170                 175

Ser Arg Leu Asn Ser Asn Thr Gln Gly Glu Met Lys Phe Glu Val Glu
            180                 185                 190

Asn Leu Ile Leu His Lys Asp Tyr Ser Ala Asp Thr Leu Ala His His
        195                 200                 205

Asn Asp Ile Ala Leu Leu Lys Ile Arg Ser Lys Glu Gly Arg Cys Ala
    210                 215                 220

Gln Pro Ser Arg Thr Ile Gln Thr Ile Cys Leu Pro Ser Met Tyr Asn
225                 230                 235                 240

Asp Pro Gln Phe Gly Thr Ser Cys Glu Ile Thr Gly Phe Gly Lys Glu
                245                 250                 255

Asn Ser Thr Asp Tyr Leu Tyr Pro Glu Gln Leu Lys Met Thr Val Val
            260                 265                 270

Lys Leu Ile Ser His Arg Glu Cys Gln Gln Pro His Tyr Tyr Gly Ser
        275                 280                 285

Glu Val Thr Thr Lys Met Leu Cys Ala Ala Asp Pro Gln Trp Lys Thr
    290                 295                 300

Asp Ser Cys Gln Gly Asp Ser Gly Gly Pro Leu Val Cys Ser Leu Gln
305                 310                 315                 320

Gly Arg Met Thr Leu Thr Gly Ile Val Ser Trp Gly Arg Gly Cys Ala
                325                 330                 335

Leu Lys Asp Lys Pro Gly Val Tyr Thr Arg Val Ser His Phe Leu Pro
            340                 345                 350

Trp Ile Arg Ser His Thr Lys Glu Glu Asn Gly Leu Ala Leu Ser Pro
        355                 360                 365

Val Lys Ala Tyr Pro Arg Pro Leu Gly Gly Gly Gly Asn Gly Asp Phe
    370                 375                 380

Glu Glu Ile Pro Glu Glu Tyr Leu Gln  *
385                 390

(2) ANGABEN ZU SEQ ID NO: 47:

     (i) SEQUENZKENNZEICHEN:
          (A) LÄNGE: 1182 Basenpaare
          (B) ART: Nucleotid
          (C) STRANGFORM: Einzelstrang
          (D) TOPOLOGIE: linear

    (ii) ART DES MOLEKÜLS: Sonstige Nucleinsäure

   (iii) HYPOTHETISCH: NEIN

    (iv) ANTISENSE: NEIN


    (ix) MERKMAL:
          (A) NAME/SCHLÜSSEL: CDS
          (B) LAGE:1..1182
          (D) SONSTIGE ANGABEN:/function= "N-term. Methionin wird
               in E.coli proteolytisch abgespalten"
               /product= "Synthetisches Gen fuer das Protein M23
               (pSJ95)"


    (xi) SEQUENZBESCHREIBUNG: SEQ ID NO: 47:

ATG AGC AAA ACT TGC TAC GAA GGT AAC GGT CAC TTC TAC CGT GGT AAG        48
Met Ser Lys Thr Cys Tyr Glu Gly Asn Gly His Phe Tyr Arg Gly Lys
  1               5                  10                  15

GCT TCT ACC GAC ACC ATG GGT CGT CCG TGC CTG CCG TGG AAC TCT GCT        96
Ala Ser Thr Asp Thr Met Gly Arg Pro Cys Leu Pro Trp Asn Ser Ala
             20                  25                  30

ACC GTT CTG CAG CAG ACC TAC CAC GCT CAC CGT TCT GAT GCA TTG CAG       144
Thr Val Leu Gln Gln Thr Tyr His Ala His Arg Ser Asp Ala Leu Gln
         35                  40                  45

CTG GGT CTG GGT AAA CAC AAC TAC TGC CGT AAC CCG GAC AAC CGT CGT       192
Leu Gly Leu Gly Lys His Asn Tyr Cys Arg Asn Pro Asp Asn Arg Arg
     50                  55                  60

CGT CCG TGG TGC TAC GTT CAG GTT GGT CTG AAA CCG CTA GTT CAG GAA       240
Arg Pro Trp Cys Tyr Val Gln Val Gly Leu Lys Pro Leu Val Gln Glu
 65                  70                  75                  80

TGC ATG GTT CAC GAC TGC GCT GAC GGT AAA AAA CCG TCT TCT CCG CCG       288
Cys Met Val His Asp Cys Ala Asp Gly Lys Lys Pro Ser Ser Pro Pro
                 85                  90                  95

GAA GAG CTC AAA TTC CAG TGC GGT CAA AAA ACC CTA CGT CCG CGT TTT       336
Glu Glu Leu Lys Phe Gln Cys Gly Gln Lys Thr Leu Arg Pro Arg Phe
            100                 105                 110

AAA ATC ATC GGT GGT GAG TTC ACC ACC ATC GAA AAC CAG CCG TGG TTC       384
Lys Ile Ile Gly Gly Glu Phe Thr Thr Ile Glu Asn Gln Pro Trp Phe
        115                 120                 125

GCT GCT ATC TAC CGT CGT CAC CGT GGT GGT TCT GTT ACC TAC GTT TGC 432
Ala Ala Ile Tyr Arg Arg His Arg Gly Gly Ser Val Thr Tyr Val Cys
    130                 135                 140

GGT GGT TCT CTG ATC TCT CCG TGC TGG GTT ATC TCT GCT ACC CAC TGC 480
Gly Gly Ser Leu Ile Ser Pro Cys Trp Val Ile Ser Ala Thr His Cys
145                 150                 155                 160

TTC ATC GAC TAC CCG AAA AAA GAA GAC TAC ATC GTT TAC CTC GGC CGT 528
Phe Ile Asp Tyr Pro Lys Lys Glu Asp Tyr Ile Val Tyr Leu Gly Arg
                165                 170                 175

TCT CGT TTA AAC TCT AAC ACC CAG GGT GAA ATG AAA TTC GAA GTT GAA 576
Ser Arg Leu Asn Ser Asn Thr Gln Gly Glu Met Lys Phe Glu Val Glu
            180                 185                 190

AAC CTG ATC CTG CAC AAA GAC TAC TCT GCT GAC ACC CTG GCT CAC CAC 624
Asn Leu Ile Leu His Lys Asp Tyr Ser Ala Asp Thr Leu Ala His His
        195                 200                 205

AAC GAC ATC GCT CTG CTA AAA ATC CGT TCT AAA GAA GGT CGT TGC GCT 672
Asn Asp Ile Ala Leu Leu Lys Ile Arg Ser Lys Glu Gly Arg Cys Ala
    210                 215                 220

CAG CCG TCT CGT ACC ATC CAG ACC ATC TGC CTG CCG TCT ATG TAC AAC 720
Gln Pro Ser Arg Thr Ile Gln Thr Ile Cys Leu Pro Ser Met Tyr Asn
225                 230                 235                 240

GAC CCG CAG TTC GGT ACC TCT TGC GAA ATC ACC GGT TTC GGT AAA GAA 768
Asp Pro Gln Phe Gly Thr Ser Cys Glu Ile Thr Gly Phe Gly Lys Glu
                245                 250                 255

AAC TCT ACC GAC TAC CTG TAC CCG GAA CAG CTG AAA ATG ACC GTT GTT 816
Asn Ser Thr Asp Tyr Leu Tyr Pro Glu Gln Leu Lys Met Thr Val Val
            260                 265                 270

AAA CTG ATC TCT CAC CGT GAA TGC CAG CAG CCG CAC TAC TAC GGT TCT 864
Lys Leu Ile Ser His Arg Glu Cys Gln Gln Pro His Tyr Tyr Gly Ser
        275                 280                 285

GAA GTT ACC ACC AAA ATG CTG TGC GCT GCT GAC CCG CAG TGG AAA ACC 912
Glu Val Thr Thr Lys Met Leu Cys Ala Ala Asp Pro Gln Trp Lys Thr
    290                 295                 300

GAC TCT TGC CAA GGT GAC TCT GGT GGT CCA CTA GTT TGC TCT CTC CAG 960
Asp Ser Cys Gln Gly Asp Ser Gly Gly Pro Leu Val Cys Ser Leu Gln
305                 310                 315                 320

GGT CGT ATG ACC CTG ACC GGT ATT GTT TCT TGG GGT CGT GGT TGC GCT 1008
Gly Arg Met Thr Leu Thr Gly Ile Val Ser Trp Gly Arg Gly Cys Ala
                325                 330                 335

CTG AAA GAC AAA CCG GGT GTT TAC ACC CGT GTT TCT CAC TTC CTG CCG 1056
Leu Lys Asp Lys Pro Gly Val Tyr Thr Arg Val Ser His Phe Leu Pro
            340                 345                 350

TGG ATC CGT TCT CAC ACC AAA GAA GAA AAC GGT CTG GCT CTG AGC CCG 1104
Trp Ile Arg Ser His Thr Lys Glu Glu Asn Gly Leu Ala Leu Ser Pro
        355                 360                 365

GTT GTT GCT TTC CCG CGG CCG CTG GGT GGT GGT GGT AAC GGT GAC TTC 1152
Val Val Ala Phe Pro Arg Pro Leu Gly Gly Gly Gly Asn Gly Asp Phe
    370                 375                 380

GAA GAA ATC CCG GAA GAA TAC CTG CAA TAA                                    1182
Glu Glu Ile Pro Glu Glu Tyr Leu Gln  *
385                 390

(2) ANGABEN ZU SEQ ID NO: 48:

(i) SEQUENZKENNZEICHEN:
(A) LÄNGE: 394 Aminosäuren
(B) ART: Aminosäure
(D) TOPOLOGIE: linear

(ii) ART DES MOLEKÜLS: Protein
(xi) SEQUENZBESCHREIBUNG: SEQ ID NO: 48:

Met Ser Lys Thr Cys Tyr Glu Gly Asn Gly His Phe Tyr Arg Gly Lys
  1               5                  10                  15

Ala Ser Thr Asp Thr Met Gly Arg Pro Cys Leu Pro Trp Asn Ser Ala
             20                  25                  30

Thr Val Leu Gln Gln Thr Tyr His Ala His Arg Ser Asp Ala Leu Gln
         35                  40                  45

Leu Gly Leu Gly Lys His Asn Tyr Cys Arg Asn Pro Asp Asn Arg Arg
     50                  55                  60

Arg Pro Trp Cys Tyr Val Gln Val Gly Leu Lys Pro Leu Val Gln Glu
 65                  70                  75                  80

Cys Met Val His Asp Cys Ala Asp Gly Lys Lys Pro Ser Ser Pro Pro
                 85                  90                  95

Glu Glu Leu Lys Phe Gln Cys Gly Gln Lys Thr Leu Arg Pro Arg Phe
            100                 105                 110

Lys Ile Ile Gly Gly Glu Phe Thr Thr Ile Glu Asn Gln Pro Trp Phe
        115                 120                 125

Ala Ala Ile Tyr Arg Arg His Arg Gly Gly Ser Val Thr Tyr Val Cys
    130                 135                 140

Gly Gly Ser Leu Ile Ser Pro Cys Trp Val Ile Ser Ala Thr His Cys
145                 150                 155                 160

Phe Ile Asp Tyr Pro Lys Lys Glu Asp Tyr Ile Val Tyr Leu Gly Arg
                165                 170                 175

Ser Arg Leu Asn Ser Asn Thr Gln Gly Glu Met Lys Phe Glu Val Glu
            180                 185                 190

Asn Leu Ile Leu His Lys Asp Tyr Ser Ala Asp Thr Leu Ala His His
        195                 200                 205

Asn Asp Ile Ala Leu Leu Lys Ile Arg Ser Lys Glu Gly Arg Cys Ala
    210                 215                 220

Gln Pro Ser Arg Thr Ile Gln Thr Ile Cys Leu Pro Ser Met Tyr Asn
225                 230                 235                 240

Asp Pro Gln Phe Gly Thr Ser Cys Glu Ile Thr Gly Phe Gly Lys Glu
                245                 250                 255

Asn Ser Thr Asp Tyr Leu Tyr Pro Glu Gln Leu Lys Met Thr Val Val
            260                 265                 270

Lys Leu Ile Ser His Arg Glu Cys Gln Gln Pro His Tyr Tyr Gly Ser
        275                 280                 285

Glu Val Thr Thr Lys Met Leu Cys Ala Ala Asp Pro Gln Trp Lys Thr
    290                 295                 300

Asp Ser Cys Gln Gly Asp Ser Gly Gly Pro Leu Val Cys Ser Leu Gln
305                 310                 315                 320

Gly Arg Met Thr Leu Thr Gly Ile Val Ser Trp Gly Arg Gly Cys Ala
                325                 330                 335

Leu Lys Asp Lys Pro Gly Val Tyr Thr Arg Val Ser His Phe Leu Pro
            340                 345                 350

Trp Ile Arg Ser His Thr Lys Glu Glu Asn Gly Leu Ala Leu Ser Pro
        355                 360                 365

Val Val Ala Phe Pro Arg Pro Leu Gly Gly Gly Gly Asn Gly Asp Phe
    370                 375                 380

Glu Glu Ile Pro Glu Glu Tyr Leu Gln  *
385                 390

(2) ANGABEN ZU SEQ ID NO: 49:

(i) SEQUENZKENNZEICHEN:
(A) LÄNGE: 63 Basenpaare
(B) ART: Nucleotid
(C) STRANGFORM: Einzelstrang
(D) TOPOLOGIE: linear

(ii) ART DES MOLEKÜLS: Sonstige Nucleinsäure
(A) BESCHREIBUNG: /desc = "synthetische DNA;
Nukleotidsequenz fuer Oligo O105"

(iii) HYPOTHETISCH: NEIN

(iv) ANTISENSE: NEIN

(xi) SEQUENZBESCHREIBUNG: SEQ ID NO: 49:

TATGAGCAAA ACTTGCTACG AAGGTAACGG TCACTTCTAC CGTGGTAAGG CTTCTACCGA     60

CAC                                                                   63

(2) ANGABEN ZU SEQ ID NO: 50:

(i) SEQUENZKENNZEICHEN:
(A) LÄNGE: 65 Basenpaare
(B) ART: Nucleotid
(C) STRANGFORM: Einzelstrang
(D) TOPOLOGIE: linear

(ii) ART DES MOLEKÜLS: Sonstige Nucleinsäure
(A) BESCHREIBUNG: /desc = "Synthetische DNA;
Nukleotidsequenz fuer Oligo O106"

(iii) HYPOTHETISCH: NEIN

(iv) ANTISENSE: NEIN

(xi) SEQUENZBESCHREIBUNG: SEQ ID NO: 50:

CATGGTGTCG GTAGAAGCCT TACCACGGTA GAAGTGACCG TTACCTTCGT AGCAAGTTTT 60

GCTCA 65

(2) ANGABEN ZU SEQ ID NO: 51:

(i) SEQUENZKENNZEICHEN:
(A) LÄNGE: 83 Basenpaare
(B) ART: Nucleotid
(C) STRANGFORM: Einzelstrang
(D) TOPOLOGIE: linear

(ii) ART DES MOLEKÜLS: Sonstige Nucleinsäure
(A) BESCHREIBUNG: /desc = "Synthetische DNA;
Nukleotidsequenz fuer Oligo O220"

(iii) HYPOTHETISCH: NEIN

(iv) ANTISENSE: NEIN

(xi) SEQUENZBESCHREIBUNG: SEQ ID NO: 51:

CGGTTAAGGC TTTCCCGAGG CCTGGTGGTG GTGGTAACGG TGACTTCGAA GAAATCCCGG 60

AAGAGTACCT GTGATAGGAT CAA 83

(2) ANGABEN ZU SEQ ID NO: 52:

(i) SEQUENZKENNZEICHEN:
(A) LÄNGE: 91 Basenpaare
(B) ART: Nucleotid
(C) STRANGFORM: Einzelstrang
(D) TOPOLOGIE: linear

(ii) ART DES MOLEKÜLS: Sonstige Nucleinsäure
(A) BESCHREIBUNG: /desc = "Synthetische DNA;
Nukleotidsequenz fuer Oligo O221"

(iii) HYPOTHETISCH: NEIN

(iv) ANTISENSE: NEIN

(xi) SEQUENZBESCHREIBUNG: SEQ ID NO: 52:

CTAGTTGATC CTATCACAGG TACTCTTCCG GGATTTCTTC GAAGTCACCG TTACCACCAC 60

CACCAGGCCT CGGGAAAGCC TTAACCGGGC T 91

(2) ANGABEN ZU SEQ ID NO: 53:

(i) SEQUENZKENNZEICHEN:
(A) LÄNGE: 101 Basenpaare
(B) ART: Nucleotid
(C) STRANGFORM: Einzelstrang
(D) TOPOLOGIE: linear

(ii) ART DES MOLEKÜLS: Sonstige Nucleinsäure
(A) BESCHREIBUNG: /desc = "Synthetische DNA,
Nukleotidsequenz fuer Oligo O222"

(iii) HYPOTHETISCH: NEIN

(iv) ANTISENSE: NEIN

(xi) SEQUENZBESCHREIBUNG: SEQ ID NO: 53:

CGCCGAGCCC GCCGAGCCCG CCGGGTGGTT TCCCGAGGCC TGGTGGTGGT GGTAACGGTG 60

ACTTCGAAGA AATCCCGGAA GAGTACCTGT GATAGGATCA A 101

(2) ANGABEN ZU SEQ ID NO: 54:

(i) SEQUENZKENNZEICHEN:
(A) LÄNGE: 109 Basenpaare
(B) ART: Nucleotid
(C) STRANGFORM: Einzelstrang
(D) TOPOLOGIE: linear

(ii) ART DES MOLEKÜLS: Sonstige Nucleinsäure
(A) BESCHREIBUNG: /desc = "Synthetische DNA;
Nukleotidsequenz fuer Oligo O223"

(iii) HYPOTHETISCH: NEIN

(iv) ANTISENSE: NEIN

(xi) SEQUENZBESCHREIBUNG: SEQ ID NO: 54:

CTAGTTGATC CTATCACAGG TACTCTTCCG GGATTTCTTC GAAGTCACCG TTACCACCAC 60

CACCAGGCCT CGGGAAACCA CCCGGCGGGC TCGGCGGGCT CGGCGGGCT 109

(2) ANGABEN ZU SEQ ID NO: 55:

(i) SEQUENZKENNZEICHEN:
(A) LÄNGE: 83 Basenpaare
(B) ART: Nucleotid
(C) STRANGFORM: Einzelstrang
(D) TOPOLOGIE: linear

(ii) ART DES MOLEKÜLS: Sonstige Nucleinsäure
(A) BESCHREIBUNG: /desc = "Synthetische DNA;
Nukleotidsequenz fuer Oligo O224"

(iii) HYPOTHETISCH: NEIN

(iv) ANTISENSE: NEIN

(xi) SEQUENZBESCHREIBUNG: SEQ ID NO: 55:

CGCCGGGTGG TTTCCCGAGG CCTGGTGGTG GTGGTAACGG TGACTTCGAA GAAATCCCGG 60

AAGAGTACCT GTGATAGGAT CAA 83

(2) ANGABEN ZU SEQ ID NO: 56:

(i) SEQUENZKENNZEICHEN:
(A) LÄNGE: 91 Basenpaare
(B) ART: Nucleotid
(C) STRANGFORM: Einzelstrang
(D) TOPOLOGIE: linear

(ii) ART DES MOLEKÜLS: Sonstige Nucleinsäure
(A) BESCHREIBUNG: /desc = "Synthetische DNA;
Nukleotidsequenz fuer Oligo O225"

(iii) HYPOTHETISCH: NEIN

(iv) ANTISENSE: NEIN

(xi) SEQUENZBESCHREIBUNG: SEQ ID NO: 56:

CTAGTTGATC CTATCACAGG TACTCTTCCG GGATTTCTTC GAAGTCACCG TTACCACCAC 60

CACCAGGCCT CGGGAAACCA CCCGGCGGGC T 91

(2) ANGABEN ZU SEQ ID NO: 57:

(i) SEQUENZKENNZEICHEN:
(A) LÄNGE: 104 Basenpaare
(B) ART: Nucleotid
(C) STRANGFORM: Einzelstrang
(D) TOPOLOGIE: linear

(ii) ART DES MOLEKÜLS: Sonstige Nucleinsäure
(A) BESCHREIBUNG: /desc = "Synthetische DNA;
Nukleotidsequenz fuer Oligo O226"

(iii) HYPOTHETISCH: NEIN

(iv) ANTISENSE: NEIN

(xi) SEQUENZBESCHREIBUNG: SEQ ID NO: 57:

CGCCGAGCCC GCCGAGCCCG CCGGGTGGTT TCGGTCCGAG GCCTGGTGGT GGTGGTAACG 60

GTGACTTCGA AGAAATCCCG GAAGAGTACC TGTGATAGGA TCAA 104

(2) ANGABEN ZU SEQ ID NO: 58:

(i) SEQUENZKENNZEICHEN:
(A) LÄNGE: 112 Basenpaare
(B) ART: Nucleotid
(C) STRANGFORM: Einzelstrang
(D) TOPOLOGIE: linear

(ii) ART DES MOLEKÜLS: Sonstige Nucleinsäure
(A) BESCHREIBUNG: /desc = "Synthetische DNA,
Nukleotidsequenz fuer Oligo 0227"

(iii) HYPOTHETISCH: NEIN

(iv) ANTISENSE: NEIN

(xi) SEQUENZBESCHREIBUNG: SEQ ID NO: 58:

CTAGTTGATC CTATCACAGG TACTCTTCCG GGATTTCTTC GAAGTCACCG TTACCACCAC 60

CACCAGGCCT CGGACCGAAA CCACCCGGCG GGCTCGGCGG GCTCGGCGGG CT 112

(2) ANGABEN ZU SEQ ID NO: 59:

(i) SEQUENZKENNZEICHEN:
(A) LÄNGE: 58 Basenpaare
(B) ART: Nucleotid
(C) STRANGFORM: Einzelstrang
(D) TOPOLOGIE: linear

(ii) ART DES MOLEKÜLS: Sonstige Nucleinsäure
(A) BESCHREIBUNG: /desc = "Synthetische DNA;
Nukleotidsequenz fuer Oligo 0265"

(iii) HYPOTHETISCH: NEIN

(iv) ANTISENSE: NEIN

(xi) SEQUENZBESCHREIBUNG: SEQ ID NO: 59:

CACCCGGCGG AGACGGCGGG CTCAGAGCCA GACCGTTTTC TTCTTTGGTG TGAGAACG 58

(2) ANGABEN ZU SEQ ID NO: 60:

(i) SEQUENZKENNZEICHEN:
(A) LÄNGE: 58 Basenpaare
(B) ART: Nucleotid
(C) STRANGFORM: Einzelstrang
(D) TOPOLOGIE: linear

(ii) ART DES MOLEKÜLS: Sonstige Nucleinsäure
(A) BESCHREIBUNG: /desc = "Synthetische DNA;
Nukleotidsequenz fuer Oligo 0281"

(iii) HYPOTHETISCH: NEIN

(iv) ANTISENSE: NEIN

(xi) SEQUENZBESCHREIBUNG: SEQ ID NO: 60:

CGTCCGGGTG GTGGTGGTAA CGGTGACTTC GAAGAAATCC CGGAAGAATA CCTGTAAG 58

(2) ANGABEN ZU SEQ ID NO: 61:

(i) SEQUENZKENNZEICHEN:
(A) LÄNGE: 70 Basenpaare
(B) ART: Nucleotid
(C) STRANGFORM: Einzelstrang
(D) TOPOLOGIE: linear

(ii) ART DES MOLEKÜLS: Sonstige Nucleinsäure
(A) BESCHREIBUNG: /desc = "Synthetische DNA;
Nukleotidsequenz fuer Oligo O282"

(iii) HYPOTHETISCH: NEIN

(iv) ANTISENSE: NEIN

(xi) SEQUENZBESCHREIBUNG: SEQ ID NO: 61:

GATCCGTTCT CACACCAAAG AAGAAAACGG TCTGGCTCTG AGCCCGCCGT CTCCGCCGGG 60

TGGTTTCCCG 70

(2) ANGABEN ZU SEQ ID NO: 62:

(i) SEQUENZKENNZEICHEN:
(A) LÄNGE: 70 Basenpaare
(B) ART: Nucleotid
(C) STRANGFORM: Einzelstrang
(D) TOPOLOGIE: linear

(ii) ART DES MOLEKÜLS: Sonstige Nucleinsäure
(A) BESCHREIBUNG: /desc = "Synthethische DNA;
Nukleotidsequenz fuer Oligo O283"

(iii) HYPOTHETISCH: NEIN

(iv) ANTISENSE: NEIN

(xi) SEQUENZBESCHREIBUNG: SEQ ID NO: 62:

CTAGCTTACA GGTATTCTTC CGGGATTTCT TCGAAGTCAC CGTTACCACC ACCACCCGGA 60

CGCGGGAAAC 70

(2) ANGABEN ZU SEQ ID NO: 63:

(i) SEQUENZKENNZEICHEN:
(A) LÄNGE: 30 Basenpaare
(B) ART: Nucleotid
(C) STRANGFORM: Einzelstrang
(D) TOPOLOGIE: linear

(ii) ART DES MOLEKÜLS: Sonstige Nucleinsäure
(A) BESCHREIBUNG: /desc = "Synthetische DNA;
Nukleotidsequenz fuer Oligo O329"

(iii) HYPOTHETISCH: NEIN

(iv) ANTISENSE: NEIN

(xi) SEQUENZBESCHREIBUNG: SEQ ID NO: 63:

AAGAAATCCC GGAAGAATAC CTGCAATAAG 30

(2) ANGABEN ZU SEQ ID NO: 64:

(i) SEQUENZKENNZEICHEN:
(A) LÄNGE: 54 Basenpaare
(B) ART: Nucleotid
(C) STRANGFORM: Einzelstrang
(D) TOPOLOGIE: linear

(ii) ART DES MOLEKÜLS: Sonstige Nucleinsäure
(A) BESCHREIBUNG: /desc = "Synthetische DNA;
Nukleotidsequenz fuer Oligo O330"

(iii) HYPOTHETISCH: NEIN

(iv) ANTISENSE: NEIN

(xi) SEQUENZBESCHREIBUNG: SEQ ID NO: 64:

CGGTTAAGGC TTGGGGACCG CGGCCGCTGG GTGGTGGTGG TAACGGTGAC TTCG 54

(2) ANGABEN ZU SEQ ID NO: 65:

(i) SEQUENZKENNZEICHEN:
(A) LÄNGE: 42 Basenpaare
(B) ART: Nucleotid
(C) STRANGFORM: Einzelstrang
(D) TOPOLOGIE: linear

(ii) ART DES MOLEKÜLS: Sonstige Nucleinsäure
(A) BESCHREIBUNG: /desc = "Synthetische DNA;
Nukleotidsequenz fuer Oligo O331"

(iii) HYPOTHETISCH: NEIN

(iv) ANTISENSE: NEIN

(xi) SEQUENZBESCHREIBUNG: SEQ ID NO: 65:

ACCACCACCC AGCGGCCGCG GTCCCCAAGC CTTAACCGGG CT 42

(2) ANGABEN ZU SEQ ID NO: 66:

(i) SEQUENZKENNZEICHEN:
(A) LÄNGE: 50 Basenpaare
(B) ART: Nucleotid
(C) STRANGFORM: Einzelstrang
(D) TOPOLOGIE: linear

(ii) ART DES MOLEKÜLS: Sonstige Nucleinsäure
(A) BESCHREIBUNG: /desc = "Synthetische DNA;
Nukleotidsequenz fuer Oligo O332"

(iii) HYPOTHETISCH: NEIN

(iv) ANTISENSE: NEIN

(xi) SEQUENZBESCHREIBUNG: SEQ ID NO: 66:

CTAGCTTATT GCAGGTATTC TTCCGGGATT TCTTCGAAGT CACCGTTACC 50

(2) ANGABEN ZU SEQ ID NO: 67:

(i) SEQUENZKENNZEICHEN:
(A) LÄNGE: 21 Basenpaare
(B) ART: Nucleotid
(C) STRANGFORM: Einzelstrang
(D) TOPOLOGIE: linear

(ii) ART DES MOLEKÜLS: Sonstige Nucleinsäure
(A) BESCHREIBUNG: /desc = "Synthetische DNA;
Nukleotidsequenz fuer Oligo O333"

(iii) HYPOTHETISCH: NEIN

(iv) ANTISENSE: NEIN

(xi) SEQUENZBESCHREIBUNG: SEQ ID NO: 67:

CGGTTAAGGC TTTCGGACCG C 21

(2) ANGABEN ZU SEQ ID NO: 68:

(i) SEQUENZKENNZEICHEN:
(A) LÄNGE: 29 Basenpaare
(B) ART: Nucleotid
(C) STRANGFORM: Einzelstrang
(D) TOPOLOGIE: linear

(ii) ART DES MOLEKÜLS: Sonstige Nucleinsäure
(A) BESCHREIBUNG: /desc = "Synthetische DNA;
Nukleotidsequenz fuer Oligo O334"

(iii) HYPOTHETISCH: NEIN

(iv) ANTISENSE: NEIN

(xi) SEQUENZBESCHREIBUNG: SEQ ID NO: 68:

GGCCGCGGTC CGAAAGCCTT AACCGGGCT 29

(2) ANGABEN ZU SEQ ID NO: 69:

(i) SEQUENZKENNZEICHEN:
(A) LÄNGE: 21 Basenpaare
(B) ART: Nucleotid
(C) STRANGFORM: Einzelstrang
(D) TOPOLOGIE: linear

(ii) ART DES MOLEKÜLS: Sonstige Nucleinsäure
(A) BESCHREIBUNG: /desc = "Synthetische DNA,
Nukleotidsequenz fuer Oligo O335"

(iii) HYPOTHETISCH: NEIN

(iv) ANTISENSE: NEIN

(xi) SEQUENZBESCHREIBUNG: SEQ ID NO: 69:

CGGTTCGGGC TTTCGGTCCG C 21

(2) ANGABEN ZU SEQ ID NO: 70:

(i) SEQUENZKENNZEICHEN:
(A) LÄNGE: 29 Basenpaare
(B) ART: Nucleotid
(C) STRANGFORM: Einzelstrang
(D) TOPOLOGIE: linear

(ii) ART DES MOLEKÜLS: Sonstige Nucleinsäure
(A) BESCHREIBUNG: /desc = "Synthetische DNA;
Nukleotidsequenz fuer Oligo O336"

(iii) HYPOTHETISCH: NEIN

(iv) ANTISENSE: NEIN

(xi) SEQUENZBESCHREIBUNG: SEQ ID NO: 70:

GGCCGCGGAC CGAAAGCCCG AACCGGGCT 29

(2) ANGABEN ZU SEQ ID NO: 71:

(i) SEQUENZKENNZEICHEN:
(A) LÄNGE: 21 Basenpaare
(B) ART: Nucleotid
(C) STRANGFORM: Einzelstrang
(D) TOPOLOGIE: linear

(ii) ART DES MOLEKÜLS: Sonstige Nucleinsäure
(A) BESCHREIBUNG: /desc = "Synthetische DNA;
Nukleotidsequenz fuer Oligo O337"

(iii) HYPOTHETISCH: NEIN

(iv) ANTISENSE: NEIN

(xi) SEQUENZBESCHREIBUNG: SEQ ID NO: 71:

CGGTTAAGGC TTACGGACCG C 21

(2) ANGABEN ZU SEQ ID NO: 72:

(i) SEQUENZKENNZEICHEN:
(A) LÄNGE: 29 Basenpaare
(B) ART: Nucleotid
(C) STRANGFORM: Einzelstrang
(D) TOPOLOGIE: linear

(ii) ART DES MOLEKÜLS: Sonstige Nucleinsäure
(A) BESCHREIBUNG: /desc = "Synthetische DNA;
Nukleotidsequenz fuer Oligo O338"

(iii) HYPOTHETISCH: NEIN

(iv) ANTISENSE: NEIN

(xi) SEQUENZBESCHREIBUNG: SEQ ID NO: 72:

GGCCGCGGTC CGTAAGCCTT AACCGGGCT 29

(2) ANGABEN ZU SEQ ID NO: 73:

(i) SEQUENZKENNZEICHEN:
(A) LÄNGE: 21 Basenpaare
(B) ART: Nucleotid
(C) STRANGFORM: Einzelstrang
(D) TOPOLOGIE: linear

(ii) ART DES MOLEKÜLS: Sonstige Nucleinsäure
(A) BESCHREIBUNG: /desc = "Synthetische DNA;
Nukleotidsequenz fuer Oligo O339"

(iii) HYPOTHETISCH: NEIN

(iv) ANTISENSE: NEIN

(xi) SEQUENZBESCHREIBUNG: SEQ ID NO: 73:

CGGTTGTTGC TTTCGGTCCG C 21

(2) ANGABEN ZU SEQ ID NO: 74:

(i) SEQUENZKENNZEICHEN:
(A) LÄNGE: 29 Basenpaare
(B) ART: Nucleotid
(C) STRANGFORM: Einzelstrang
(D) TOPOLOGIE: linear

(ii) ART DES MOLEKÜLS: Sonstige Nucleinsäure
(A) BESCHREIBUNG: /desc = "Synthetische DNA;
Nukleotidsequenz fuer Oligo O340"

(iii) HYPOTHETISCH: NEIN

(iv) ANTISENSE: NEIN

(xi) SEQUENZBESCHREIBUNG: SEQ ID NO: 74:

GGCCGCGGAC CGAAAGCAAC AACCGGGCT 29

(2) ANGABEN ZU SEQ ID NO: 75:

(i) SEQUENZKENNZEICHEN:
(A) LÄNGE: 18 Basenpaare
(B) ART: Nucleotid
(C) STRANGFORM: Einzelstrang
(D) TOPOLOGIE: linear

(ii) ART DES MOLEKÜLS: Sonstige Nucleinsäure
(A) BESCHREIBUNG: /desc = "Synthetische DNA;
Nukleotidsequenz fuer Oligo O341"

(iii) HYPOTHETISCH: NEIN

(iv) ANTISENSE: NEIN

(xi) SEQUENZBESCHREIBUNG: SEQ ID NO: 75:

CGGTTCGGGC TTTCCCGC 18

(2) ANGABEN ZU SEQ ID NO: 76:

(i) SEQUENZKENNZEICHEN:
(A) LÄNGE: 26 Basenpaare
(B) ART: Nucleotid
(C) STRANGFORM: Einzelstrang
(D) TOPOLOGIE: linear

(ii) ART DES MOLEKÜLS: Sonstige Nucleinsäure
(A) BESCHREIBUNG: /desc = "Synthetische DNA;
Nukleotidsequenz fuer Oligo O342"

(iii) HYPOTHETISCH: NEIN

(iv) ANTISENSE: NEIN

(xi) SEQUENZBESCHREIBUNG: SEQ ID NO: 76:

GGCCGCGGGA AAGCCCGAAC CGGGCT 26

(2) ANGABEN ZU SEQ ID NO: 77:

(i) SEQUENZKENNZEICHEN:
(A) LÄNGE: 18 Basenpaare
(B) ART: Nucleotid
(C) STRANGFORM: Einzelstrang
(D) TOPOLOGIE: linear

(ii) ART DES MOLEKÜLS: Sonstige Nucleinsäure
(A) BESCHREIBUNG: /desc = "Synthetische DNA;
Nukleotidsequenz fuer Oligo O343"

(iii) HYPOTHETISCH: NEIN

(iv) ANTISENSE: NEIN

(xi) SEQUENZBESCHREIBUNG: SEQ ID NO: 77:

CGGTTAAGGC TTACCCGC 18

(2) ANGABEN ZU SEQ ID NO: 78:

(i) SEQUENZKENNZEICHEN:
(A) LÄNGE: 26 Basenpaare
(B) ART: Nucleotid
(C) STRANGFORM: Einzelstrang
(D) TOPOLOGIE: linear

(ii) ART DES MOLEKÜLS: Sonstige Nucleinsäure
(A) BESCHREIBUNG: /desc = "Synthetische DNA;
Nukleotidsequenz fuer Oligo O344"

(iii) HYPOTHETISCH: NEIN

(iv) ANTISENSE: NEIN

(xi) SEQUENZBESCHREIBUNG: SEQ ID NO: 78:

GGCCGCGGGT AAGCCTTAAC CGGGCT 26

(2) ANGABEN ZU SEQ ID NO: 79:

(i) SEQUENZKENNZEICHEN:
(A) LÄNGE: 18 Basenpaare
(B) ART: Nucleotid
(C) STRANGFORM: Einzelstrang
(D) TOPOLOGIE: linear

(ii) ART DES MOLEKÜLS: Sonstige Nucleinsäure
(A) BESCHREIBUNG: /desc = "Synthetische DNA;
Nukleotidsequenz fuer Oligo O347"

(iii) HYPOTHETISCH: NEIN

(iv) ANTISENSE: NEIN

(xi) SEQUENZBESCHREIBUNG: SEQ ID NO: 79:

CGGTTGTTGC TTTCCCGC 18

(2) ANGABEN ZU SEQ ID NO: 80:

(i) SEQUENZKENNZEICHEN:
(A) LÄNGE: 26 Basenpaare
(B) ART: Nucleotid
(C) STRANGFORM: Einzelstrang
(D) TOPOLOGIE: linear

(ii) ART DES MOLEKÜLS: Sonstige Nucleinsäure
(A) BESCHREIBUNG: /desc = "Synthetische DNA;
Nukleotidsequenz fuer Oligo O348"

(iii) HYPOTHETISCH: NEIN

(iv) ANTISENSE: NEIN

(xi) SEQUENZBESCHREIBUNG: SEQ ID NO: 80:

GGCCGCGGGA AAGCAACAAC CGGGCT 26

(2) ANGABEN ZU SEQ ID NO: 81:

(i) SEQUENZKENNZEICHEN:
(A) LÄNGE: 18 Basenpaare
(B) ART: Nucleotid
(C) STRANGFORM: Einzelstrang
(D) TOPOLOGIE: linear

(ii) ART DES MOLEKÜLS: Sonstige Nucleinsäure
(A) BESCHREIBUNG: /desc = "Synthetische DNA;
Nukleotidsequenz fuer Oligo O381"

(iii) HYPOTHETISCH: NEIN

(iv) ANTISENSE: NEIN

(xi) SEQUENZBESCHREIBUNG: SEQ ID NO: 81:

CGGTTAAGGC TTGGCCGC 18

(2) ANGABEN ZU SEQ ID NO: 82:

(i) SEQUENZKENNZEICHEN:
(A) LÄNGE: 26 Basenpaare
(B) ART: Nucleotid
(C) STRANGFORM: Einzelstrang
(D) TOPOLOGIE: linear

(ii) ART DES MOLEKÜLS: Sonstige Nucleinsäure
(A) BESCHREIBUNG: /desc = "Synthetische DNA;
Nukleotidsequenz fuer Oligo O383"

(iii) HYPOTHETISCH: NEIN

(iv) ANTISENSE: NEIN

(xi) SEQUENZBESCHREIBUNG: SEQ ID NO: 82:

GGCCGCGGCC AAGCCTTAAC CGGGCT 26

(2) ANGABEN ZU SEQ ID NO: 83:

(i) SEQUENZKENNZEICHEN:
(A) LÄNGE: 18 Basenpaare
(B) ART: Nucleotid
(C) STRANGFORM: Einzelstrang
(D) TOPOLOGIE: linear

(ii) ART DES MOLEKÜLS: Sonstige Nucleinsäure
(A) BESCHREIBUNG: /desc = "Synthetische DNA;
Nukleotidsequenz fuer Oligo O384"

(iii) HYPOTHETISCH: NEIN

(iv) ANTISENSE: NEIN

(xi) SEQUENZBESCHREIBUNG: SEQ ID NO: 83:

CGGTTAAGGC TTTCCCGC 18

(2) ANGABEN ZU SEQ ID NO: 84:

(i) SEQUENZKENNZEICHEN:
(A) LÄNGE: 26 Basenpaare
(B) ART: Nucleotid
(C) STRANGFORM: Einzelstrang
(D) TOPOLOGIE: linear

(ii) ART DES MOLEKÜLS: Sonstige Nucleinsäure
(A) BESCHREIBUNG: /desc = "Synthetische DNA;
Nukleotidsequenz fuer Oligo O385"

(iii) HYPOTHETISCH: NEIN

(iv) ANTISENSE: NEIN

(xi) SEQUENZBESCHREIBUNG: SEQ ID NO: 84:

GGCCGCGGGA AAGCCTTAAC CGGGCT 26

(2) ANGABEN ZU SEQ ID NO: 85:

(i) SEQUENZKENNZEICHEN:
(A) LÄNGE: 18 Basenpaare
(B) ART: Nucleotid
(C) STRANGFORM: Einzelstrang
(D) TOPOLOGIE: linear

(ii) ART DES MOLEKÜLS: Sonstige Nucleinsäure
(A) BESCHREIBUNG: /desc = "Synthetische DNA;
Nukleotidsequenz fuer Oligo O386"

(iii) HYPOTHETISCH: NEIN

(iv) ANTISENSE: NEIN

(xi) SEQUENZBESCHREIBUNG: SEQ ID NO: 85:

CGGTTGTAGT TTTCCCGC 18

(2) ANGABEN ZU SEQ ID NO: 86:

(i) SEQUENZKENNZEICHEN:
(A) LÄNGE: 18 Basenpaare
(B) ART: Nucleotid
(C) STRANGFORM: Einzelstrang
(D) TOPOLOGIE: linear

(ii) ART DES MOLEKÜLS: Sonstige Nucleinsäure
(A) BESCHREIBUNG: /desc = "Synthetische DNA;
Nukleotidsequenz fuer Oligo O387"

(iii) HYPOTHETISCH: NEIN

(iv) ANTISENSE: NEIN

(xi) SEQUENZBESCHREIBUNG: SEQ ID NO: 86:

CGGTTGAAGT TTTCCCGC 18

(2) ANGABEN ZU SEQ ID NO: 87:

(i) SEQUENZKENNZEICHEN:
(A) LÄNGE: 26 Basenpaare
(B) ART: Nucleotid
(C) STRANGFORM: Einzelstrang
(D) TOPOLOGIE: linear

(ii) ART DES MOLEKÜLS: Sonstige Nucleinsäure
(A) BESCHREIBUNG: /desc = "Synthetische DNA;
Nukleotidsequenz fuer Oligo O388"

(iii) HYPOTHETISCH: NEIN

(iv) ANTISENSE: NEIN

(xi) SEQUENZBESCHREIBUNG: SEQ ID NO: 87:

GGCCGCACTA CAACTACAAC CGGGCT 26

(2) ANGABEN ZU SEQ ID NO: 88:

(i) SEQUENZKENNZEICHEN:
(A) LÄNGE: 18 Basenpaare
(B) ART: Nucleotid
(C) STRANGFORM: Einzelstrang
(D) TOPOLOGIE: linear

(ii) ART DES MOLEKÜLS: Sonstige Nucleinsäure
(A) BESCHREIBUNG: /desc = "Synthetische DNA;
Nukleotidsequenz fuer Oligo O389"

(iii) HYPOTHETISCH: NEIN

(iv) ANTISENSE: NEIN

(xi) SEQUENZBESCHREIBUNG: SEQ ID NO: 88:

CGGTTGTAGT TGTAGTGC 18

(2) ANGABEN ZU SEQ ID NO: 89:

(i) SEQUENZKENNZEICHEN:
(A) LÄNGE: 26 Basenpaare
(B) ART: Nucleotid
(C) STRANGFORM: Einzelstrang
(D) TOPOLOGIE: linear

(ii) ART DES MOLEKÜLS: Sonstige Nucleinsäure
(A) BESCHREIBUNG: /desc = "Synthetische DNA;
Nukleotidsequenz fuer Oligo O390"

(iii) HYPOTHETISCH: NEIN

(iv) ANTISENSE: NEIN

(xi) SEQUENZBESCHREIBUNG: SEQ ID NO: 89:

GGCCGCGGGA AAACTTCAAC CGGGCT 26

(2) ANGABEN ZU SEQ ID NO: 90:

(i) SEQUENZKENNZEICHEN:
(A) LÄNGE: 26 Basenpaare
(B) ART: Nucleotid
(C) STRANGFORM: Einzelstrang
(D) TOPOLOGIE: linear

(ii) ART DES MOLEKÜLS: Sonstige Nucleinsäure
(A) BESCHREIBUNG: /desc = "Synthetische DNA;
Nukleotidsequenz fuer Oligo O391"

(iii) HYPOTHETISCH: NEIN

(iv) ANTISENSE: NEIN

(xi) SEQUENZBESCHREIBUNG: SEQ ID NO: 90:

GGCCGCGGGA AAACTACAAC CGGGCT 26

(2) ANGABEN ZU SEQ ID NO: 91:

(i) SEQUENZKENNZEICHEN:
(A) LÄNGE: 72 Basenpaare
(B) ART: Nucleotid
(C) STRANGFORM: Einzelstrang
(D) TOPOLOGIE: linear

(ii) ART DES MOLEKÜLS: Sonstige Nucleinsäure
(A) BESCHREIBUNG: /desc = "Synthetische DNA;
Nukleotidsequenz fuer Oligo O392"

(iii) HYPOTHETISCH: NEIN

(iv) ANTISENSE: NEIN

(xi) SEQUENZBESCHREIBUNG: SEQ ID NO: 91:

CTAGCTTATT CGTTTTTTTC TTCGTCTTCC CAGAACGGTT CGTATTTGTC GTTCGGGTTC 60

CGCAGCAGGA AC 72

(2) ANGABEN ZU SEQ ID NO: 92:

(i) SEQUENZKENNZEICHEN:
(A) LÄNGE: 72 Basenpaare
(B) ART: Nucleotid
(C) STRANGFORM: Einzelstrang
(D) TOPOLOGIE: linear

(ii) ART DES MOLEKÜLS: Sonstige Nucleinsäure
(A) BESCHREIBUNG: /desc = "Synthetische DNA;
Nukleotidsequenz fuer Oligo O393"

(iii) HYPOTHETISCH: NEIN

(iv) ANTISENSE: NEIN

(xi) SEQUENZBESCHREIBUNG: SEQ ID NO: 92:

GGCCGTTCCT GCTGCGGAAC CCGAACGACA AATACGAACC GTTCTGGGAA GACGAAGAAA 60

AAAACGAATA AG 72

(2) ANGABEN ZU SEQ ID NO: 93:

(i) SEQUENZKENNZEICHEN:
(A) LÄNGE: 18 Basenpaare
(B) ART: Nucleotid
(C) STRANGFORM: Einzelstrang
(D) TOPOLOGIE: linear

(ii) ART DES MOLEKÜLS: Sonstige Nucleinsäure
(A) BESCHREIBUNG: /desc = "Synthetische DNA;
Nukleotidsequenz fuer Oligo O453"

(iii) HYPOTHETISCH: NEIN

(iv) ANTISENSE: NEIN

(xi) SEQUENZBESCHREIBUNG: SEQ ID NO: 93:

TGGTTAAAGC TTTCCCGC 18

(2) ANGABEN ZU SEQ ID NO: 94:

(i) SEQUENZKENNZEICHEN:
(A) LÄNGE: 26 Basenpaare
(B) ART: Nucleotid
(C) STRANGFORM: Einzelstrang
(D) TOPOLOGIE: linear

(ii) ART DES MOLEKÜLS: Sonstige Nucleinsäure
(A) BESCHREIBUNG: /desc = "Synthetische DNA;
Nukleotidsequenz fuer Oligo O454"

(iii) HYPOTHETISCH: NEIN

(iv) ANTISENSE: NEIN

(xi) SEQUENZBESCHREIBUNG: SEQ ID NO: 94:

GGCCGCGGGA AAGCTTTAAC CAGGCT 26

(2) ANGABEN ZU SEQ ID NO: 95:

(i) SEQUENZKENNZEICHEN:
(A) LÄNGE: 18 Basenpaare
(B) ART: Nucleotid
(C) STRANGFORM: Einzelstrang
(D) TOPOLOGIE: linear

(ii) ART DES MOLEKÜLS: Sonstige Nucleinsäure
(A) BESCHREIBUNG: /desc = "Synthetische DNA;
Nukleotidsequenz fuer Oligo O455"

(iii) HYPOTHETISCH: NEIN

(iv) ANTISENSE: NEIN

(xi) SEQUENZBESCHREIBUNG: SEQ ID NO: 95:

TGGTTGTTGC TTTCCCGC 18

(2) ANGABEN ZU SEQ ID NO: 96:

(i) SEQUENZKENNZEICHEN:
(A) LÄNGE: 26 Basenpaare
(B) ART: Nucleotid
(C) STRANGFORM: Einzelstrang
(D) TOPOLOGIE: linear

(ii) ART DES MOLEKÜLS: Sonstige Nucleinsäure
(A) BESCHREIBUNG: /desc = "Synthetische DNA;
Nukleotidsequenz fuer Oligo O456"

(iii) HYPOTHETISCH: NEIN

(iv) ANTISENSE: NEIN

(xi) SEQUENZBESCHREIBUNG: SEQ ID NO: 96:

GGCCGCGGGA AAGCAACAAC CAGGCT 26

(2) ANGABEN ZU SEQ ID NO: 97:

(i) SEQUENZKENNZEICHEN:
(A) LÄNGE: 47 Basenpaare
(B) ART: Nucleotid
(C) STRANGFORM: Einzelstrang
(D) TOPOLOGIE: linear

(ii) ART DES MOLEKÜLS: Sonstige Nucleinsäure
(A) BESCHREIBUNG: /desc = "Synthetische DNA;
Nukleotidsequenz fuer Oligo O465"

(iii) HYPOTHETISCH: NEIN

(iv) ANTISENSE: NEIN

(xi) SEQUENZBESCHREIBUNG: SEQ ID NO: 97:

GGCCGCGGGA ACAGAGCCAG ACCGTTTTCT TCTTTGGTGT GAGAACG 47

(2) ANGABEN ZU SEQ ID NO: 98:

(i) SEQUENZKENNZEICHEN:
(A) LÄNGE: 47 Basenpaare
(B) ART: Nucleotid
(C) STRANGFORM: Einzelstrang
(D) TOPOLOGIE: linear

(ii) ART DES MOLEKÜLS: Sonstige Nucleinsäure
(A) BESCHREIBUNG: /desc = "Synthetische DNA;
Nukleotidsequenz fuer Oligo O466"

(iii) HYPOTHETISCH: NEIN

(iv) ANTISENSE: NEIN

(xi) SEQUENZBESCHREIBUNG: SEQ ID NO: 98:

GATCCGTTCT CACACCAAAG AAGAAAACGG TCTGGCTCTG TTCCCGC 47

(2) ANGABEN ZU SEQ ID NO: 99:

(i) SEQUENZKENNZEICHEN:
(A) LÄNGE: 38 Basenpaare
(B) ART: Nucleotid
(C) STRANGFORM: Einzelstrang
(D) TOPOLOGIE: linear

(ii) ART DES MOLEKÜLS: Sonstige Nucleinsäure
(A) BESCHREIBUNG: /desc = "Synthetische DNA;
Nukleotidsequenz fuer Oligo O467"

(iii) HYPOTHETISCH: NEIN

(iv) ANTISENSE: NEIN

(xi) SEQUENZBESCHREIBUNG: SEQ ID NO: 99:

CGGTTAAGGC TTTCCCGCGG CCGTTCCTGC TGCGGAAC 38

(2) ANGABEN ZU SEQ ID NO: 100:

(i) SEQUENZKENNZEICHEN:
(A) LÄNGE: 54 Basenpaare
(B) ART: Nucleotid
(C) STRANGFORM: Einzelstrang
(D) TOPOLOGIE: linear

(ii) ART DES MOLEKÜLS: Sonstige Nucleinsäure
(A) BESCHREIBUNG: /desc = "Synthetische DNA;
Nukleotidsequenz fuer Oligo O468"

(iii) HYPOTHETISCH: NEIN

(iv) ANTISENSE: NEIN

(xi) SEQUENZBESCHREIBUNG: SEQ ID NO: 100:

TTTGTCGTTC GGGTTCCGCA GCAGGAACGG CCGCGGGAAA GCCTTAACCG GGCT 54

(2) ANGABEN ZU SEQ ID NO: 101:

(i) SEQUENZKENNZEICHEN:
(A) LÄNGE: 44 Basenpaare
(B) ART: Nucleotid
(C) STRANGFORM: Einzelstrang
(D) TOPOLOGIE: linear

(ii) ART DES MOLEKÜLS: Sonstige Nucleinsäure
(A) BESCHREIBUNG: /desc = "Synthetische DNA;
Nukleotidsequenz fuer Oligo O469"

(iii) HYPOTHETISCH: NEIN

(iv) ANTISENSE: NEIN

(xi) SEQUENZBESCHREIBUNG: SEQ ID NO: 101:

CTAGCTTATT CGTTTTTTTC TTCGTCTTCC CAGAACGGTT CGTA 44

(2) ANGABEN ZU SEQ ID NO: 102:

(i) SEQUENZKENNZEICHEN:
(A) LÄNGE: 52 Basenpaare
(B) ART: Nucleotid
(C) STRANGFORM: Einzelstrang
(D) TOPOLOGIE: linear

(ii) ART DES MOLEKÜLS: Sonstige Nucleinsäure
(A) BESCHREIBUNG: /desc = "Synthetische DNA;
Nukleotidsequenz fuer Oligo O470"

(iii) HYPOTHETISCH: NEIN

(iv) ANTISENSE: NEIN

(xi) SEQUENZBESCHREIBUNG: SEQ ID NO: 102:

CCGAACGACA AATACGAACC GTTCTGGGAA GACGAAGAAA AAAACGAATA AG 52

## Beschreibung

Die Erfindung betrifft bifunktionelle Urokinasevarianten mit Verbesserten fibrinolytischen Eigenschaften und thrombinhemmender Wirkung, Plasmide zur Verwendung bei der Gewinnung dieser Polypeptide sowie Thrombolytika, die als Wirkstoff eine bifunktionelle Urokinasevariante enthalten.

Eine wichtige Eigenschaft des menschlichen Blutes ist die Fähigkeit, Verletzungen der Blutbahn durch Bildung von Thromben zu Verschließen. Die Blutgerinnung wird durch eine Reihe von im Blut enthaltenen Enzymen hervorgerufen, die in der sogenannten Gerinnungskaskade letztendlich dazu führen, daß das Enzym Thrombin das Vorläuferprotein Fibrinogen zu Fibrin proteolytisch umsetzt. Fibrin polymerisiert unter Einschluß von Thrombozyten, Erythrozyten und anderen Blutbestandteilen an der Stelle der Läsion unter Bildung eines Thrombus.

Darüber hinaus enthält das Blut auch eine Reihe von Enzymen, die der Gerinnung entgegenwirken und den Blutfluß nach der Regeneration der Gefäßwände sicherstellen. Das wichtigste Enzym für die Thrombolyse ist das Plasmin, das das Fibringespinnst proteolytisch angreift und dadurch die Auflösung der Thromben bewirkt. Plasmin wird durch proteolytische Spaltung des inaktiven Vorläuferproteins Plasminogen gebildet. Die Aktivierung wird von Plasminogenaktivatoren durch proteolytische Spaltung des Plasminogens bewirkt. Zwei endogene menschliche Plasminogenaktivatoren sind bekannt: die Urokinase, ein im Urin vorkommender Plasminogenaktivator, und der Gewebeplasminogenaktivator.

Herzinfarkt und Hirninfarkt sind eng mit der pathologischen Bildung von Thromben verknüpft. Bei beiden Infarktformen entstehen - meist infolge arteriosklerotischer Veränderungen der Arterien - unter bestimmten Bedingungen Thromben an den Gefäßwänden. Diese Thromben stören den Blutfluß in den Arterien, so daß das Gewebe nicht mehr ausreichend mit Sauerstoff versorgt werden kann. Dies führt beim Herzinfarkt zum partiellen oder vollständigen Absterben des Herzmuskels. Entsprechend führt die Blockade zerebraler Arterien zu schweren Schädigungen des Hirngewebes.

Zur Therapie von Infarktpatienten werden Plasminogenaktivatoren als Thrombolytika eingesetzt, die den Abbau der Thromben durch Plasmin starten. Zur Zeit stehen zur Therapie Streptokinase, APSAC (Anisolated Plasminogen Streptokinase Activator Complex), zweikettige Urokinase (UK), rekombinante einkettige Urokinase (rekombinante Prourokinase) und Gewebeplasminogenaktivator (tPA) zur Verfügung (Collen und Lijnen, Blood 78, 3114 - 3124 (1991)). Bei Streptokinase handelt es sich um ein Protein hämolytischer Streptokokken. Streptokinase aktiviert Plasminogen, indem es einen Komplex mit Plasminogen bildet und dadurch das Plasminogen in eine aktive Konformation überführt. Dieser Komplex selbst setzt freies Plasminogen zu Plasmin um, welches dann wiederum das Streptokinase-gebundene Plasminogen spaltet. Eine Weiterentwicklung der Streptokinase ist APSAC, eine in vitro hergestellte Verbindung aus Streptokinase und menschlichem Plasminogen. APSAC besitzt aufgrund einer chemischen Modifikation des aktiven Zentrums des Plasminogens eine gegenüber Streptokinase erhöhte biologische Halbwertszeit.

Urokinase ist ein menschliches Protein, das in zwei Formen als proteolytisch aktives Protein aus Urin gewonnen werden kann: der hochmolekularen Urokinase (HUK) und der niedermolekularen Urokinase (LUK) (Stump et al., J. Biol. Chem. 261, 1267 - 1273 (1986)). HUK und LUK sind Zweikettenmoleküle. Die Urokinase wird als einkettige Urokinase (Prourokinase) in verschiedenen Geweben gebildet und kann als Proenzym in geringen Mengen im menschlichen Blut nachgewiesen werden (Wun et al., J. Biol. Chem. 257, 3276 - 3283 (1982)). Die aktivierte Form der Prourokinase hat als HUK ein Molekulargewicht von 54 Kilodalton und besteht aus 3 Domänen: der amino-terminalen Growth-Factor-Domäne, dem Kringel und der Serin-Protease-Domäne (Günzler et al., Hoppe-Seyler's Z. Physiol. Chem. 363, 1155 - 1165 (1982); Steffens et al., Hoppe-Seyler's Z. Physiol. Chem. 363, 1043 - 1058 (1982)). Obwohl Prourokinase und Plasminogen als Proenzyme vorliegen, ist die Prourokinase aufgrund einer intrinsischen Aktivität in der Lage, Plasminogen in aktives Plasmin umzuwandeln. Die volle Aktivität erhält dieser Plasminogenaktivator aber erst, nachdem das gebildete Plasmin seinerseits die Prourokinase zwischen ¹⁵⁸Lysin und ¹⁵⁹Isoleucin gespalten hat (Lijnen et al., J. Biol. Chem. 261, 1253 - 1258 (1986)). Die gentechnische Gewinnung von Urokinase in Escherichia coli wurde erstmals von Heyneker beschrieben (Proceedings of the IVth International Symposium on Genetics of Industrial Microorganisms 1982). Unglycosilierte Prourokinase (Saruplase) wird unter Verwendung eines synthetischen Gens hergestellt (Brigelius-Flohé et al., Appl. Microbiol. Biotech. 36, 640 - 649 (1992)).

Der Gewebeplasminogenaktivator ist ein im Blut und im Gewebe vorkommendes Protein mit einem Molekulargewicht von 72 Kilodalton. Dieser Plasminogenaktivator besteht aus 5 Domänen: der amino-terminalen Finger-Domäne, der Growth-Factor-Domäne, dem Kringel 1, dem Kringel 2 und der Serin-Protease-Domäne. Im Gegensatz zur Prourokinase wird tPA erst durch Anbindung an Fibrin in die Lage versetzt, Plasminogen zu spalten. Wie Prourokinase wird tPA durch eine plasminkatalysierte Spaltung zwischen Kringel 2 und der Serin-Protease-Domäne in die aktive Form überführt. Hierbei bindet der Gewebeplasminogenaktivator an Fibrin, nicht aber an Fibrinogen, wodurch Plasminogen thrombusspezifisch aktiviert wird. Im Gegensatz zur zweikettigen Urokinase wird eine generelle Plasminogenaktivierung weitgehend vermieden (Collen und Lijnen, Blood 78, 3114 - 3124 (1991)).

Seit Anfang der 80er Jahre hat sich die aktive Behandlung des Myokardinfarktes mit Thrombolytika als wirksam und effizient erwiesen. In einer Reihe von Studien wurde gezeigt, daß die Behandlung von Herzinfarktpatienten mit Streptokinase, APSAC, UK, rekombinanter Prourokinase oder tPA zu einer deutlich verringerten Mortalität gegenüber nicht behandelten Patienten führt. Um die Wirksamkeit dieser Substanzen in der Therapie zu verbessern, sind unter Anwendung gentechnischer Methoden eine Reihe von Derivaten des Gewebeplasminogenaktivators und der Prourokinase hergestellt worden. Neben einer erhöhten fibrinolytischen Aktivität und der Verringerung von Nebenwirkungen steht die Entwicklung von Formen, die für eine Bolusapplikation geeignet sind, im Zentrum des Interesses. Eine Übersicht über die Ansätze zur Verbesserung der Plasminogenaktivatoren findet sich in Thrombosis and Haemostasis 66, 88 - 110 (1991) sowie in Trends in Biotech. 9, 86 - 90 (1991).

Um die Wirksamkeit von Plasminogenaktivatoren in der Lysetherapie zu verbessern und insbesondere ihre biologische Halbwertszeit zu erhöhen, wurden Deletions- und Substitutionsvarianten des Gewebeplasminogenaktivators hergestellt, bei denen beispielsweise die Finger- und die Growth-Factor-Domäne entfernt oder die Serin-Protease-Domäne gegen die Serin-Protease-Domäne der Urokinase ausgetauscht wurden (Collen et al., Thromb. Haemostasis 65, 174 - 180 (1991); Fromage et al., Fibrinolysis 5, 187 - 190 (1991); Lu et al., Blood 78, 125 - 131 (1991)). Tatsächlich zeigte sich, daß die Deletion der Finger- und Growth-Factor-Domäne die biologische Halbwertszeit der tPA-Varianten erhöhte (Lijnen und Collen, Thromb. Haemostasis 66, 94 - 95 (1991)). Eine Deletions- und Substitutionsvariante, die aus den beiden Kringel-Domänen des tPAs und der Serin-Protease-Domäne der UK besteht, war aufgrund einer deutlich längeren Halbwertszeit den ursprünglichen, das heißt nicht veränderten Plasminogenaktivatoren in der Thrombolyse überlegen. Diese Plasminogenaktivatorvarianten besaßen jedoch nur eine geringe Fibrinspezifität (Lu et al., Blood 78, 125 - 131 (1991)).

Verschiedene Versuche wurden unternommen, Plasminogenaktivatoren mit erhöhter Fibrinspezifität herzustellen. Um die Gefahr von Blutungen zu reduzieren, sollten derartige Wirkstoffe möglichst ausschließlich Plasminogen in der Nähe des Thrombus aktivieren, nicht jedoch eine systemische Plasminogenaktivierung hervorrufen. So ist beispielsweise eine Variante des tPAs bekannt, bei der Kringel 1 durch Kringel 2 des Ursprungsmoleküls ausgetauscht wurde. Diese Variante besitzt zwar eine erhöhte Affinität zu N-terminalen Lysin-Resten, nicht aber zu Fibrin. Im Tiermodell war diese Variante im Hinblick auf die Thrombolyse nicht wirksamer als der ursprüngliche Gewebeplasminogenaktivator (Collen et al., Thromb. Haemostasis 65, 174 - 180 (1991)). Andere bekannte Varianten, die aus Fusionen zwischen Thrombus-spezifischen Antikörpern und Plasminogenaktivatoren bestehen, sind im Tiermodell wirksamer als die ursprünglichen Plasminogenaktivatoren (Lijnen und Collen, Thromb. Haemostasis 66, 88 - 110 (1991)). Eine sehr hohe Fibrinspezifität besitzt ein aus der Fledermaus Desmodus retundus isolierter Plasminogenaktivator (Gardell et al., J. Biol. Chem. 264, 17947 bis 17952 (1989)). Dieser Plasminogenaktivator zeigt im Tierversuch eine gegenüber tPA verbesserte Thrombolyse bei erhöhter Halbwertszeit und verminderter systemischer Plasminogenaktivierung (Gardell et al., Circulation 84, 244 bis 253 (1991); Mellott et al., Arterioscl. Thromb. 12, 212 bis 221 (1992)).

Der Erfolg einer Behandlung von Infarktpatienten mit Plasminogenaktivatoren hängt jedoch nicht nur von der Thrombolyse ab, sondern auch davon, inwieweit der Wiederverschluß eröffneter Blutgefäße verhindert werden kann. Verschiedene Befunde weisen darauf hin, daß in den Thromben gebundenes Thronbin bei der Thrombolyse wieder als aktives Enzym freigesetzt wird und den Wiederverschluß von Gefäßen verursachen kann (Szczeklik et al., Arterioscl. Thromb. 12, 548 - 553 (1992); Eisenberg, Circulation 84, 2601 - 2603 (1991)). Tatsächlich wird die Wirkung von Thrombolytika durch gleichzeitige oder vorgeschaltete Gabe des Thrombininhibitors Heparin verbessert. Auch durch Gabe von Argatroban, Hirugen oder Protein C kann das Auftreten von Wiederverschlüssen in der Lysetherapie verringert werden (Schneider, Thromb. Res. 64, 677 - 689 (1990); Yao et al., Am. Physiol. 262 (Heart Circ. Physiol. 31, H 374 - H 379 (1992); Gruber et al., Circulation 84, 2454 - 2462 (1991). Darüber hinaus ist bekannt, daß die Mortalität von Herzinfarktpatienten durch vorherige Gabe von Heparin und nachfolgende Applikation von Prourokinase gegenüber einer Kontrollgruppe (Applikation von Prourokinase ohne vorherige Heparingabe) signifikant reduziert wird (Tebbe et al., Z. Kardiol. 80, Suppl. 3, 32 (1991)).

Einer der potentesten Thrombininhibitoren ist das aus dem Blutegel Hirudo medicinales isolierte Hirudin, das mit seiner carboxy-terminalen Hälfte spezifisch an die sogenannte Anionen-Bindungsstelle des Thrombins bindet. Bestimmte Aminosäuren der amino-terminalen Hälfte des Hirudinmoleküls verdecken den Zugang zur Substratbindungstasche des Thrombins (Rydel et al., Science 249, 277 - 280 (1990)). Darüber hinaus ist bekannt, daß Thrombin auch durch kleinere Derivate des Hirudins gehemmt werden kann, wobei insbesondere die von Maraganore et al. in Biochemistry 29, 7095 - 7101 (1990) beschriebenen Hirulog-Moleküle hervorzuheben sind (Krstenansky et al., J. Med. Chem. 30, 1688 - 1691 (1987); Yue et al., Protein Engineering 5, 77 - 85 (1992)).

Die Verwendung von Hirudin in Verbindung mit einem Plasminogenaktivator zur Behandlung thrombotisch bedingter Gefäßerkrankungen wird in den europäischen Patentanmeldungen EP 328 957 und EP 365 468 beschrieben. Die therapeutische Verwendung von Hirudin-Abkömmlingen in Kombination mit einem Thrombolytikum ist aus der internationalen Patentanmeldung WO 91/01142 bekannt.

In der internationalen Patentanmeldung WO 92/18139 werden chimäre Moleküle mit einem plasminogenaktivierenden Anteil, der nicht an Fibrin bindet, und einem Anteil mit einer Affinität gegenüber Nicht-Fibrin-Komponenten aus arteriosklerotischen Plaques, beispielsweise einem thrombininhibierenden Anteil, beschrieben. Die beiden Anteile können über ein 12-mer verbunden sein, das aus Alanin besteht oder überwiegend Alamin enthält. Innerhalb eines alaninreichen 12-mer's können 2 oder 3 Alaninreste durch Lysin oder Arginin ersetzt sein.

Aus der internationalen Patentanmeldung WO 91/09125 sind Fusionsproteine mit einer Spaltstelle zwischen erster und zweiter Sequenz, die eine fibrinolytische und/oder antithrombotische Wirkung besitzen, bekannt. Die vollständige fibrinolytische und/oder antithrombotische wirkung wird erst durch die Spaltung erzielt, wobei diese insbesondere durch Thrombin hervorgerufen werden kann. Dies hat jedoch zur Folge, daß der durch Spaltung freigesetzte Thrombininhibitor Thrombin hemmt und hierdurch eine weitere Spaltung durch Thrombin verhindert wird.

Thrombin kann auch durch ein Peptid gehemmt werden, das sich aus der amino-terminalen Sequenz des humanen Thrombinrezeptors ableitet (Vu et al., Nature 253, 674 - 677 (1991)). Der Thrombinrezeptor enthält in der amino-terminalen Region eine thrombinbindende Sequenz mit benachbarter Spaltstelle für Thrombin. Die thrombinbindende Region des Rezeptors ist in der Struktur dem carboxy-terminalen Bereich des Hirudins sehr ähnlich. Der Rezeptor wird durch Thrombin aktiviert, indem die Rezeptorsequenz gespalten wird. Aufgrund der Affinität zwischen Rezeptor und Thrombin wirkt ein Fragment des Rezeptors mit Bindungsregion und modifizierter Spaltstelle als Thrombininhibitor.

Ebenso kann Thrombin durch ein Peptid gehemmt werden, das von den Aminosäuren 41 bis 57 des Hämadins abgeleitet ist (Strube et al., J. Biol. Chem. 268, 8590 - 8595 (1993).

Die der Erfindung zugrundeliegende Aufgabe bestand in der Entwicklung von Wirkstoffen zur Behandlung thrombotisch bedingter Gefäßverschlüsse, die innerhalb sehr kurzer Zeit eine vollständige Thrombolyse bewirken und gleichzeitig den Wiederverschluß der Gefäße nach zunächst erfolgreicher Thrombolyse verhindern. Ferner soll mit diesen Wirkstoffen eine systemische Plasminogenaktivierung vermieden werden.

Es wurde nun gefunden, daß die an solche Wirkstoffe gestellten hohen Anforderungen von bestimmten bifunktionellen Urokinasevarianten erfüllt werden.

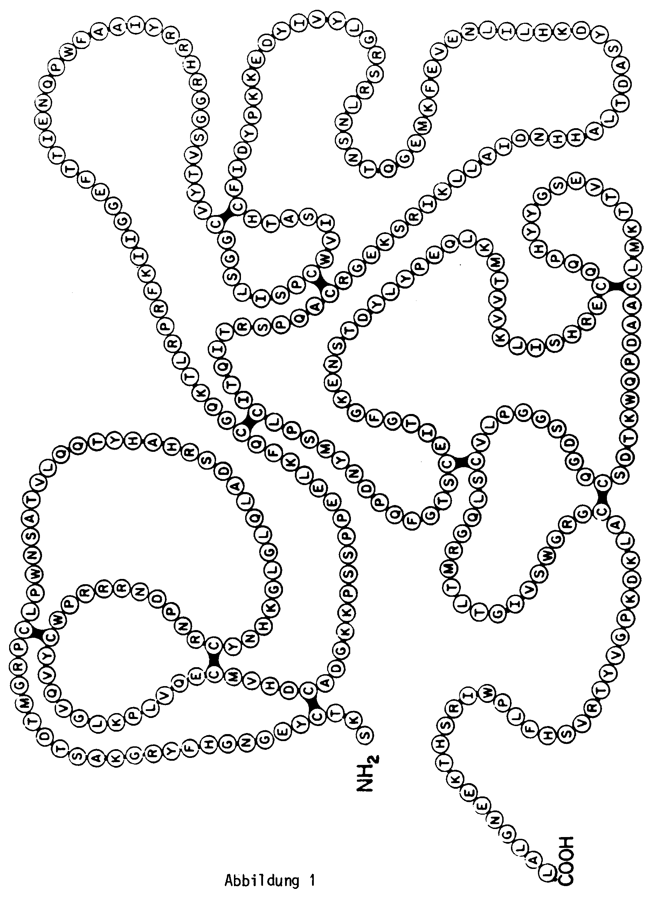

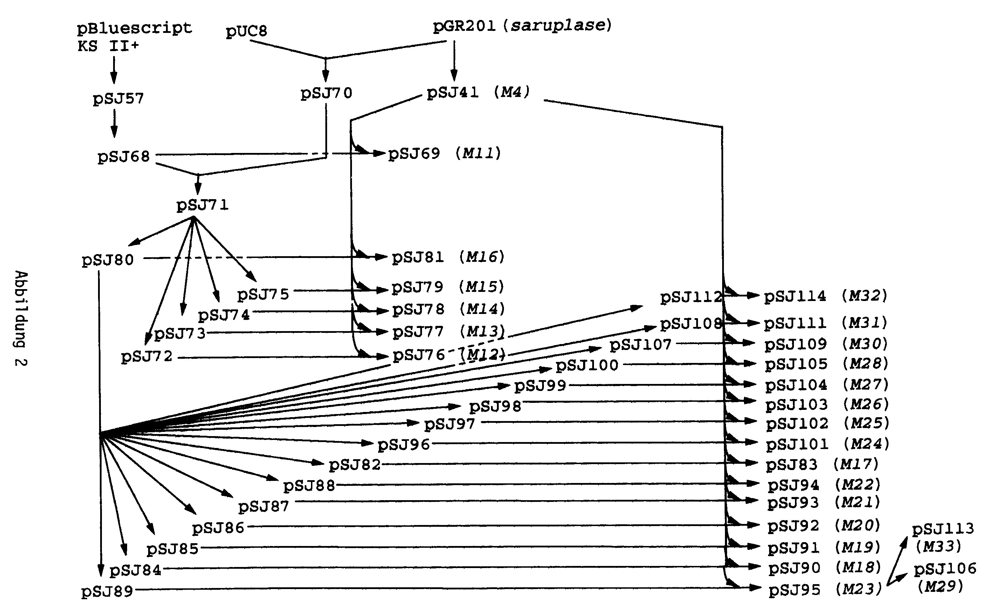

Gegenstand der Erfindung sind dementsprechend bifunktionelle Urokinasevarianten der allgemeinen Formel I
M4-X₁-Y₁
worin
- M4: die Aminosäuresequenz ⁴⁷Ser bis ⁴¹¹Leu der unglycosilierten Prourokinase gemäß Abbildung 1 (SEQ ID NO: 21 und 22) bedeutet,
- X₁: eine direkte Bindung zwischen M4 und Y₁ ist oder
ein Peptid der Sequenz
   Ser-Pro-Pro-Ser-Pro-Pro-Gly-Gly-Phe
   oder
   Ser-Pro-Pro-Ser-Pro-Pro-Ser-Pro-Pro-Gly-Gly-Phe
   oder
   Ser-Pro-Pro-Ser-Pro-Pro-Ser-Pro-Pro-Gly-Gly-Phe-Gly
   oder
eine Peptidsequenz der allgemeinen Formel II
   Ser-X₂-X₃-X₄-X₅-X₆-X₇
   darstellt, wobei X₂ Pro oder Leu, X₃ Val oder Pro, X₄ Lys, Val, Arg, Gly oder Glu, X₅ Ala, Val, Gly, Leu oder Ile, X₆ Phe, Trp, Tyr oder Val und X₇ Gly oder eine direkte Bindung zwischen X₆ und Y₁ bedeuten,
und
- Y₁: ein Peptid der Sequenz
Y₂-Arg-Pro-Y₃-Gly-Gly-Gly-Gly-Asn-Gly-Asp-Phe-Glu-Glu-Ile-Pro-Glu-Glu-Tyr-Leu-Y₄
oder
Y₂-Arg-Pro-Phe-Leu-Leu-Arg-Asn-Pro-Asn-Asp-Lys-Tyr-Glu-Pro-Phe-Trp-Glu-Asp-Glu-Glu-Lys-Asn-Glu
oder
Y₂-Arg-Pro-Ser-Ser-Glu-Phe-Glu-Glu-Phe-Glu-Ile-Asp-Glu-Glu-Glu-Lys
darstellt mit Y₂ Pro oder Val, Y₃ Leu oder eine direkte Bindung zwischen Pro und Gly und Y₄ Gln oder eine Hydroxylgruppe.

In bifunktionellen Urokinasevarianten der allgemeinen Formel I, in der Y₁ ein Peptid der Sequenz
Y₂-Arg-Pro-Y₃-Gly-Gly-Gly-Gly-Asn-Gly-Asp-Phe-Glu-Glu-Ile-Pro-Glu-Glu-Tyr-Leu-Y₄
darstellt, worin Y₂ Pro oder Val, Y₃ Leu oder eine direkte Bindung zwischen Pro und Gly und Y₄ Gln oder eine Hydroxylgruppe bedeuten, ist X₁ vorzugsweise eine Peptidsequenz der allgemeinen Formel II
Ser-X₂-X₃-X₄-X₅-X₆-X₇
worin X₂ Pro oder Leu, X₃ Val, X₄ Lys, Val oder Arg, X₅ Ala, Val oder Gly, X₆ Phe, Trp, Tyr oder Val und X₇ Gly oder eine direkte Bindung zwischen X₆ und Y₁ bedeuten. Besonders bevorzugt werden in diesen bifunktionellen Urokinasevarianten Peptidsequenzen der allgemeinen Formel II, in denen X₂ Pro oder Leu, X₃ Val, X₄ Lys oder Val, X₅ Ala oder Val, X₆ Phe, Trp oder Tyr und X₇, Gly oder eine direkte Bindung zwischen X₆ und Y₁ und insbesondere solche, in denen X₇ eine direkte Bindung zwischen X₆ und Y₁ bedeuten.

In bifunktionellen Urokinasevarianten der allgemeinen Formel I, in denen Y₁ ein Peptid der Sequenz
Y₂-Arg-Pro-Phe-Leu-Leu-Arg-Asn-Pro-Asn-Asp-Lys-Tyr-Glu-Pro-Phe-Trp-Glu-Asp-Glu-Glu-Lys-Asn-Glu
mit Y₂ Pro oder Val darstellt, ist X₁ vorzugsweise eine Peptidsequenz der allgemeinen Formel II
Ser-X₂-X₃-X₄-X₅-X₆-X₇
worin X₂ Pro oder Leu, X₃ Val, X₄ Lys oder Val, X₅ Ala oder Val, X₆ Phe oder Trp und X₇ eine direkte Bindung zwischen X₆ und Y₁ bedeuten.

Im Vergleich zu bekannten Plasminogenaktivatoren und bekannten Mischungen aus einem Plasminogenaktivator und einem Thrombininhibitor zeichnen sich die erfindungsgemäßen bifunktionellen Urokinasevarianten durch eine stärkere fibrinolytische Wirkung, verbunden mit nicht vorhersehbar guten thrombininhibierenden Eigenschaften aus. Darüber hinaus wird von den erfindungsgemäßen Polypeptiden Plasmafibrinogen überraschenderweise in deutlich geringeren Mengen verbraucht. Die hieraus resultierende, signifikant höhere Fibrinspezifität, insbesondere auch im Vergleich zu den bekannten Mischungen eines Plasminogenaktivators und eines Thrombininhibitors, bewirkt, daß die Gerinnungsfähigkeit des Blutes nur wenig beeinflußt wird und die Gefahr von unkontrollierten Blutungen als mögliche Komplikation eines systemischen Fibrinogenabbaus minimiert ist. Die hohe Fibrinspezifität der erfindungsgemäßen Urokinasevarianten ermöglicht somit Bolusapplikationen mit deutlich herabgesetztem Blutungsrisiko im Vergleich zu Bolusapplikationen bekannter Thrombolytika.

Bifunktionelle Urokinasevarianten der allgemeinen Formel I sind toxikologisch unbedenklich, so daß sie als solche in geeigneten pharmazeutischen Zubereitungen an Patienten mit thrombotisch bedingten Gefäßverschlüssen verabreicht werden können.

Weiterer Erfindungsgegenstand sind dementsprechend Thrombolytika, die als Wirkstoff eine bifunktionelle Urokinasevariante der allgemeinen Formel I enthalten.

Zur Behandlung thrombotisch bedingter Gefäßverschlüsse, beispielsweise Herzinfarkt, Hirninfakt, peripherer, akuter Arterienverschluß, Lungenembolie und tiefe Bein- und Beckenvenenthrombose, werden 0,1 bis 1 mg/kg eines erfindungsgemäßen Polypeptids benötigt. Die bifunktionellen Urokinasevarianten können intravenös und insbesondere durch Bolusinjektion verabreicht werden.

Die erfindungsgemäßen Thrombolytika enthalten neben mindestens einer bifunktionellen Urokinasevariante Hilfsstoffe, beispielsweise Trägermaterialien, Lösungsmittel, Verdünnungsmittel, Farbstoffe und Bindemittel. Die Auswahl der Hilfsstoffe sowie die einzusetzenden Mengen derselben hängt davon ab, wie das Arzneimittel appliziert werden soll und bereitet dem Fachmann keinerlei Probleme.

Die Herstellung der bifunktionellen Urokinasevarianten erfolgt mittels gentechnischer Verfahren. Erfindungsgegenstand sind dementsprechend auch Plasmide zur Verwendung bei der Gewinnung bifunktioneller Urokinasevarianten der allgemeinen Formel I, deren Operon einen regulierbaren Promotor, eine als Ribosomenbindestelle wirksame Shine-Dalgarno-Sequenz, ein Startcodon, ein synthetisches Strukturgen für eine bifunktionelle Urokinasevariante der allgemeinen Formel I und stromabwärts vom Strukturgen ein oder zwei Terminatoren aufweist.

Als regulierbarer Promotor eignet sich insbesondere der trp-Promotor oder der tac-Promotor. Als Terminator wird vorzugsweise der trp A Terminator und/oder der tet A/orf L Terminator aus Tn 10 eingesetzt.

In der Kontrollregion der erfindungsgemäßen Plasmide beträgt der Abstand zwischen der Shine-Dalgarno-Sequenz und dem Startcodon 6 - 12, vorzugsweise 8 - 10 Nukleotide.

Die Expression der erfindungsgemäßen Plasmide wird in Escherichia coli-Stämmen, insbesondere in Escherichia coli-Stämmen der Gruppe K 12, beispielsweise E.coli K 12 JM 101 (ATCC 33876), E.coli K 12 JM 103 (ATCC 39403), E.coli K 12 JM 105 (DSM 4162) und E.coli K 12 DH 1 (ATCC 33849) durchgeführt. In der bakteriellen Zelle fallen die erfindungsgemäßen bifunktionellen Urokinasevarianten der allgemeinen Formel I in hoher Ausbeute in Einschlußkörpern an, in denen das Protein in denaturierter Form vorliegt. Nach Isolierung der Einschlußkörper wird das denaturierte Protein proteinchemisch unter Einwirkung eines Redoxsystems in die gewünschte Tertiärstruktur zurückgefaltet.

### Beispiele

### 1) Darstellung, Isolierung und Reinigung erfindungsgemäßer bifunktioneller Urokinasevarianten

### a) Klonierungsarbeiten

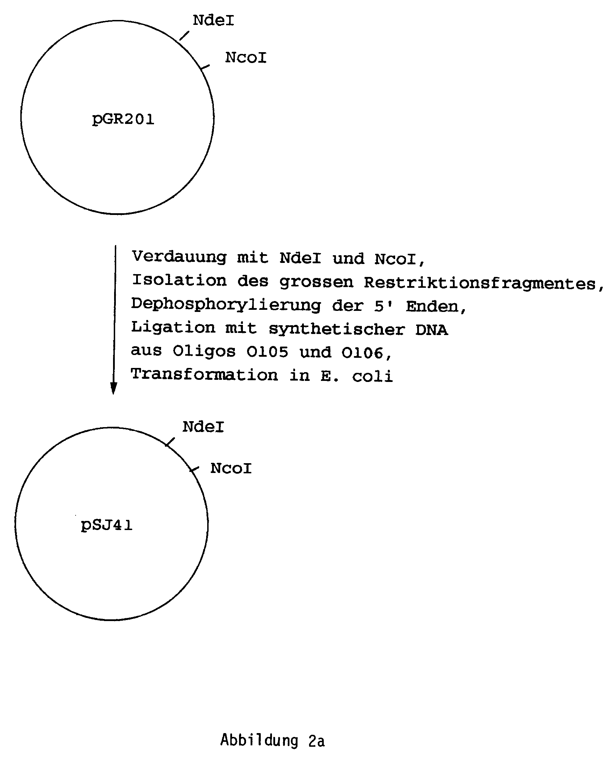

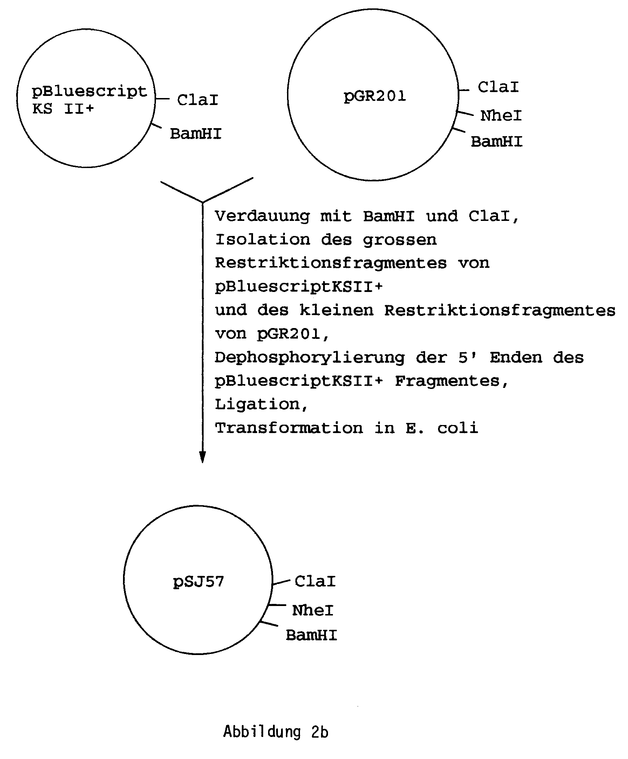

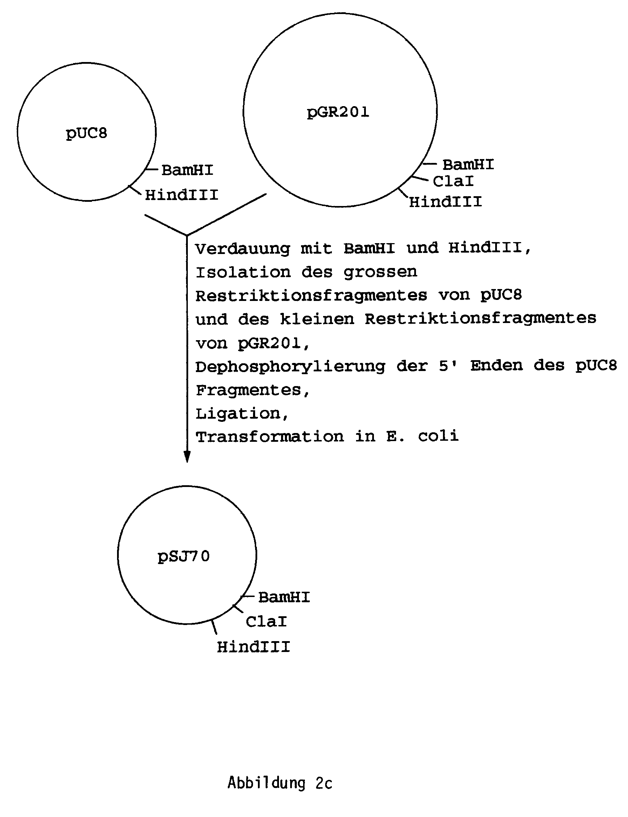

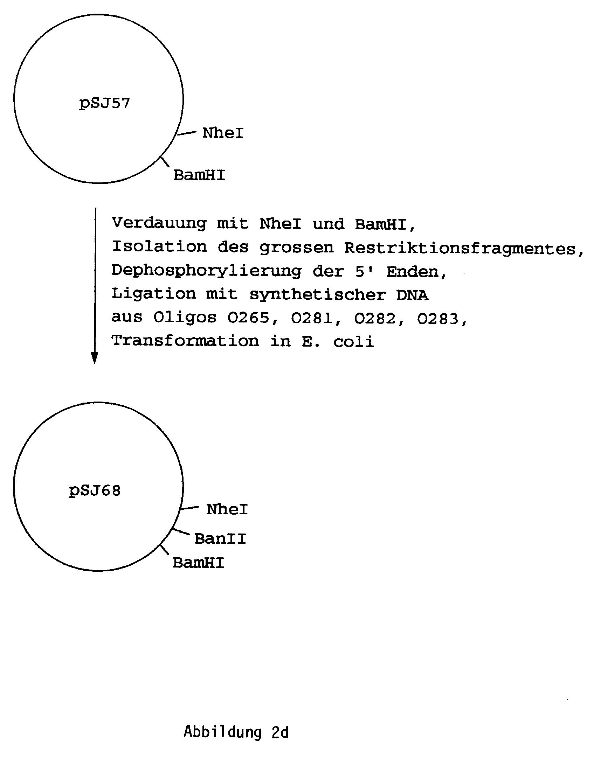

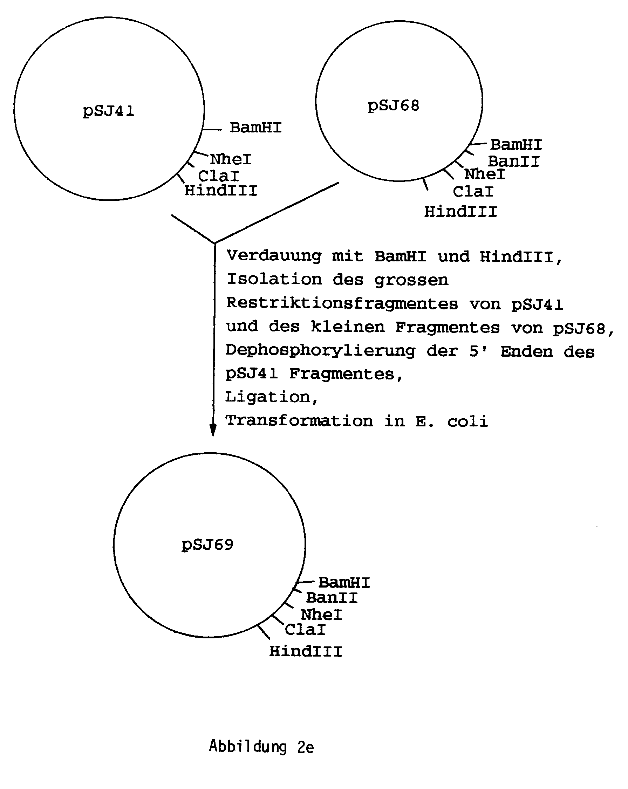

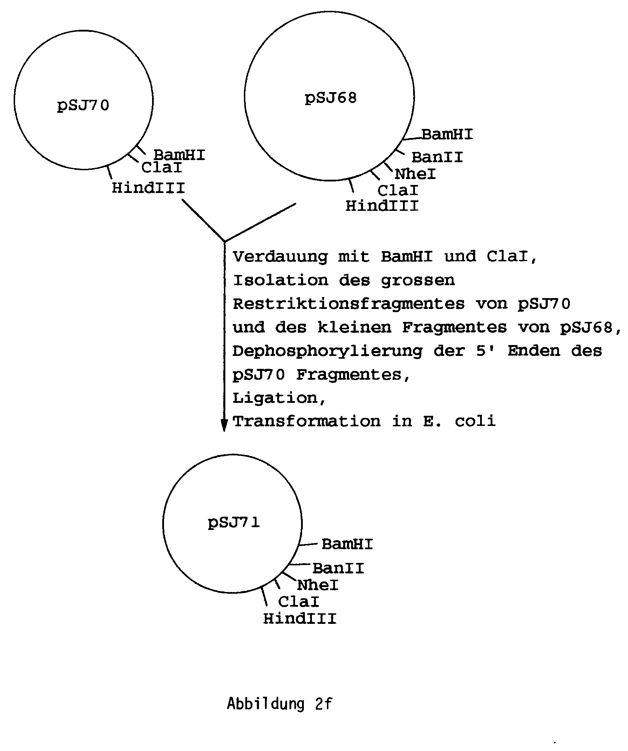

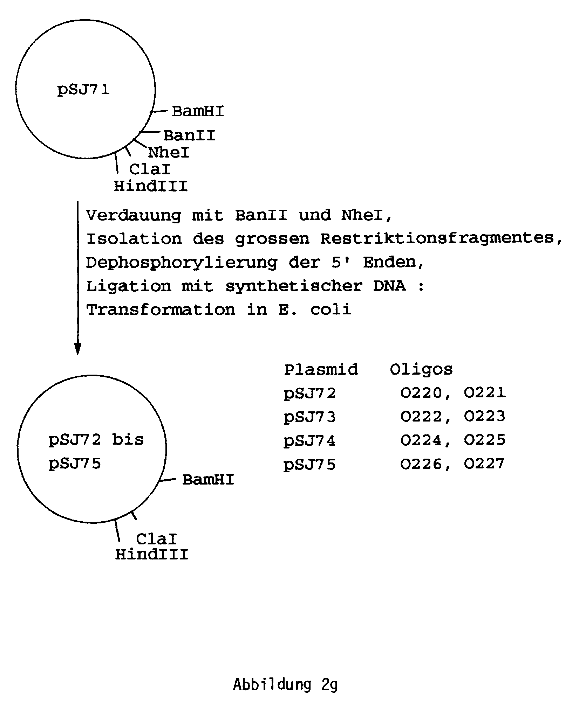

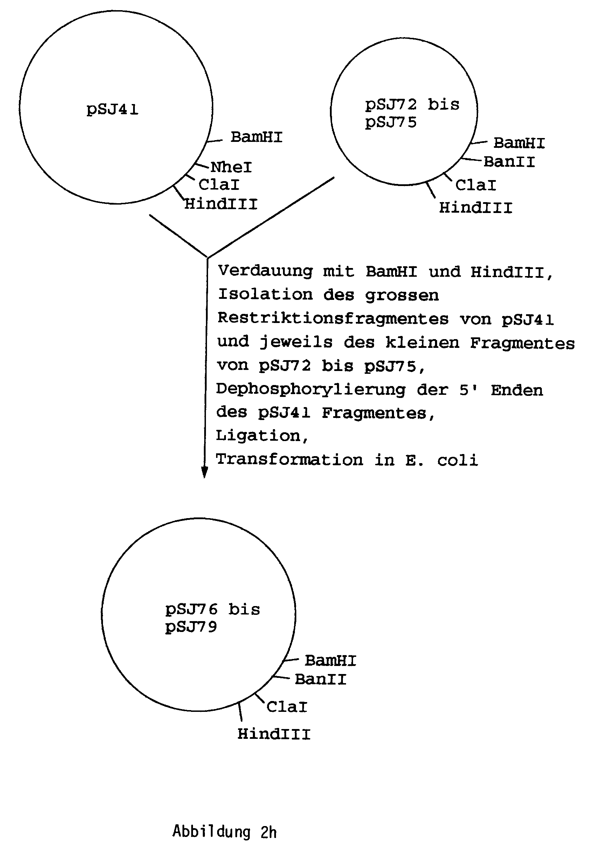

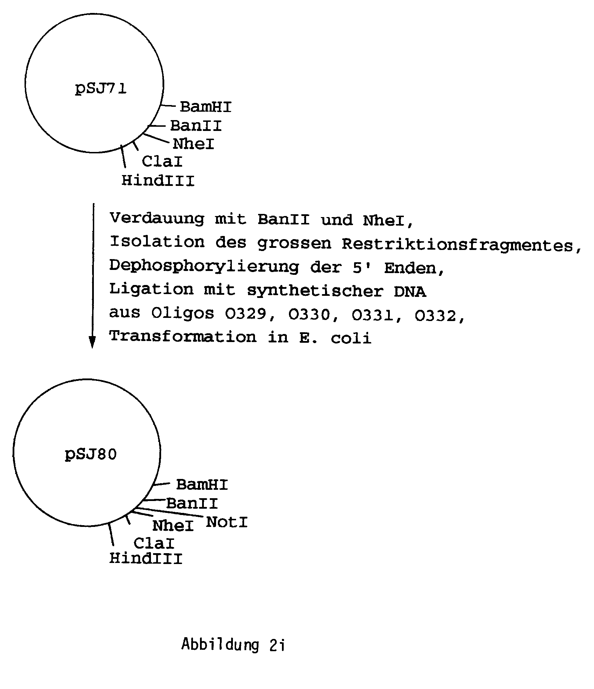

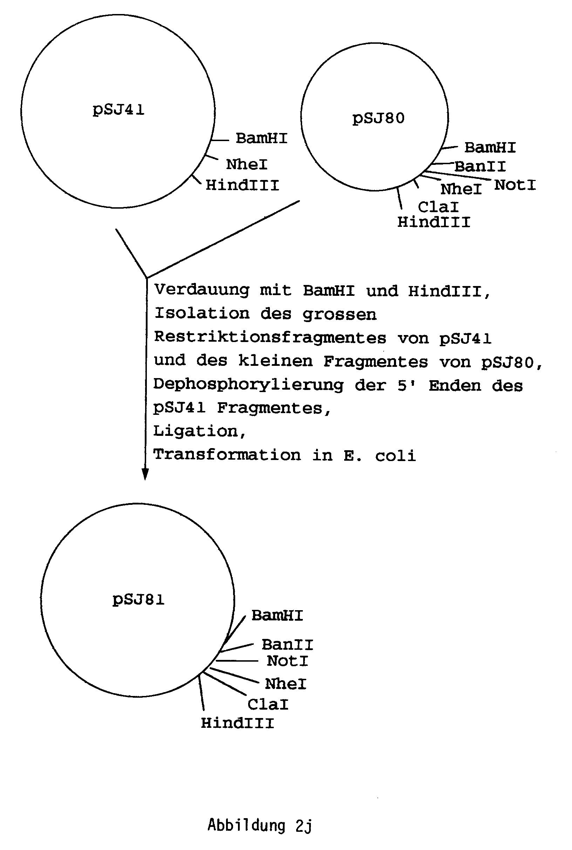

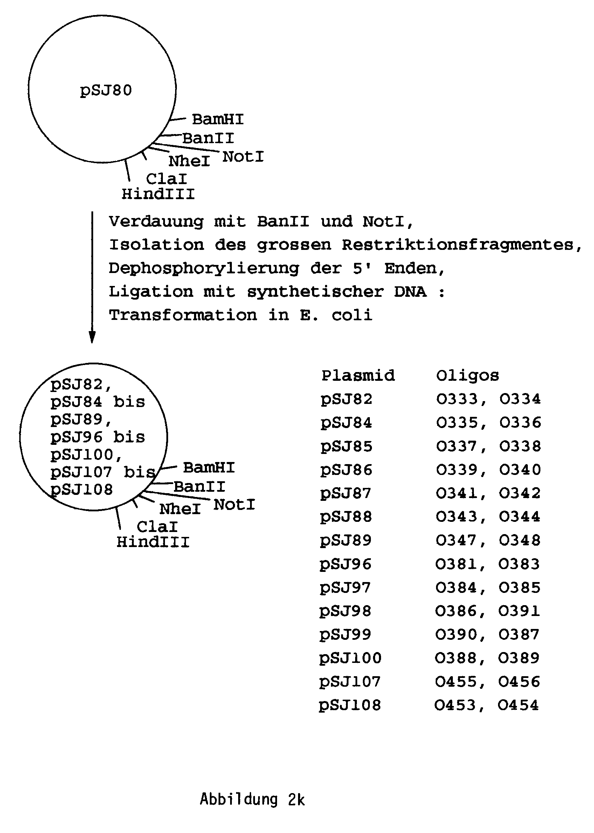

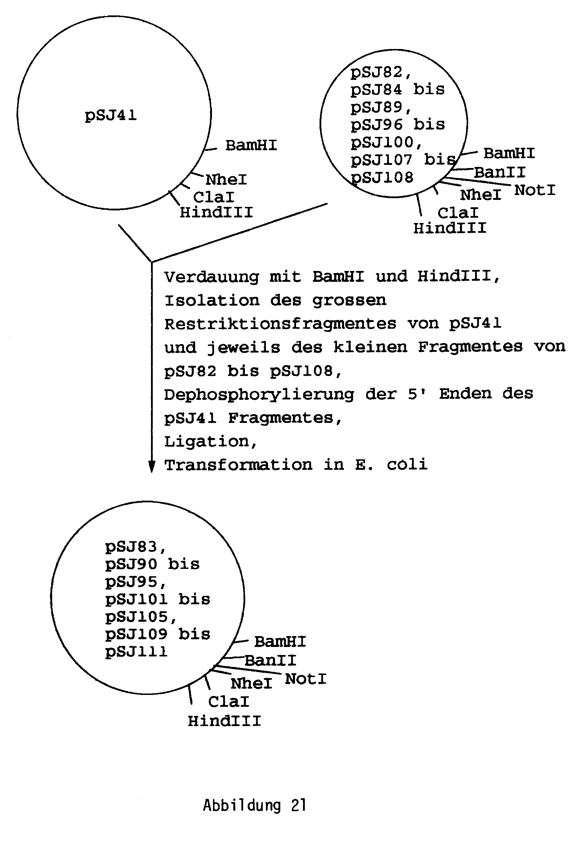

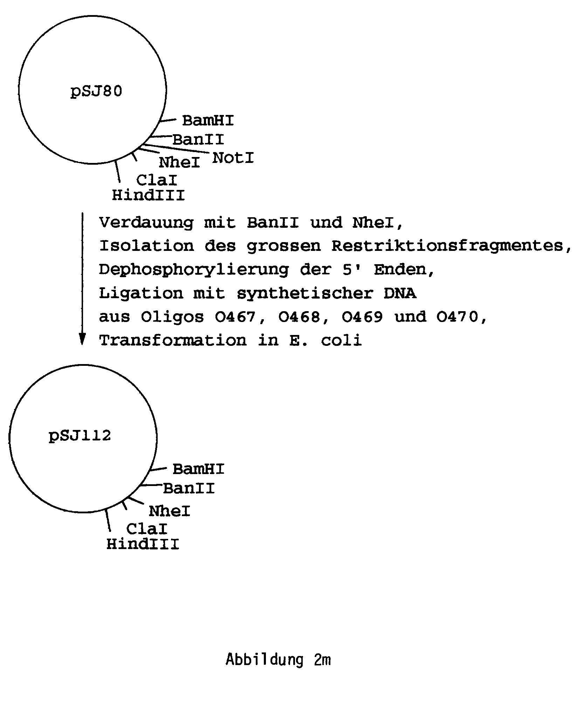

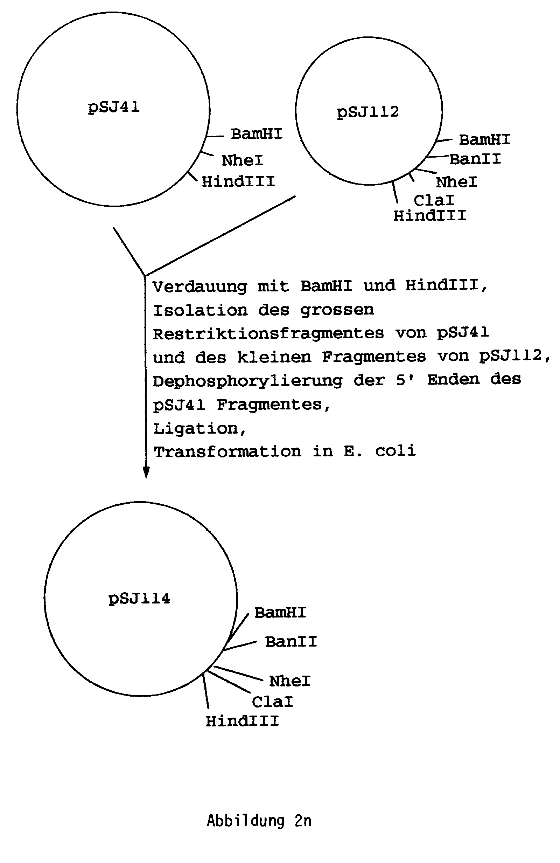

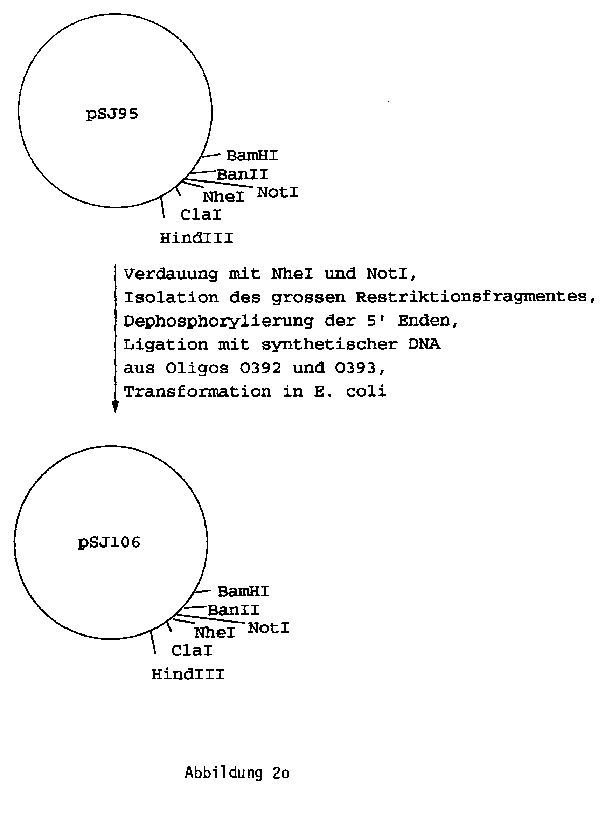

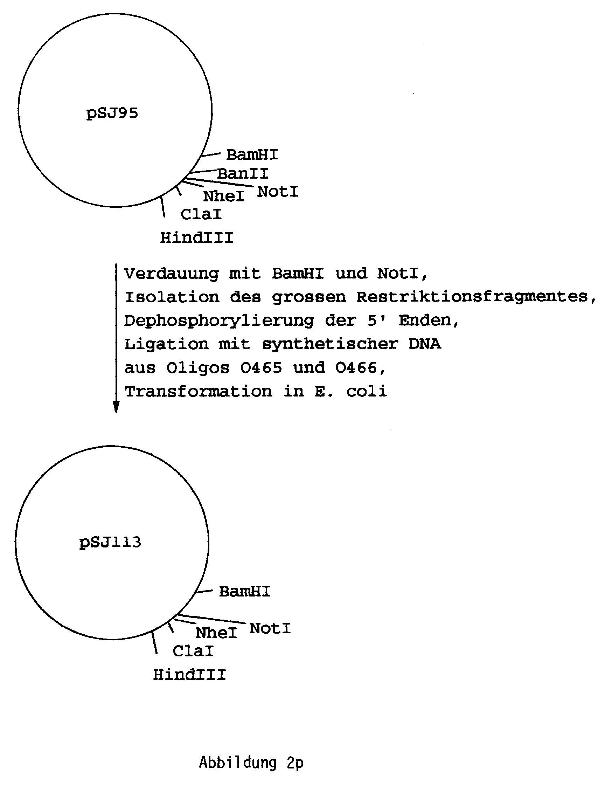

Die Expressionsplasmide für die gentechnische Herstellung der erfindungsgemäßen Polypeptide in Escherichia coli wurden in an sich bekannter Weise hergestellt. Die Abfolge der einzelnen Herstellungsschritte ist in den Abbildungen 2 und 2a bis 2p dargestellt. Ausgangsprodukte der Plasmidherstellung waren die Plasmide pBlueskript KS II + (Firma Stratagene, Heidelberg), pUC8 (Fa. Pharmacia, Freiburg) und pGR201. pGR201 ist identisch mit dem in EP 408 945 und Appl. Microbiol. Biotechn. 36, 640 - 649 (1992) beschriebenen Plasmid pBF160. Die Restriktionsendonukleasen BanII, BamHI, ClaI, HindIII, NcoI, NdeI, NheI und NotI sowie die DNA-modifizierenden Enzyme, wie die alkalische Phosphatase, T4-Ligase, T4-Kinase und T7-Polymerase, wurden von den Firmen Pharmacia, Stratagene, Boehringer (Mannheim) und Gibco (Eggenstein) bezogen. Die Veränderungen der Plasmide während ihrer Herstellung wurden durch Restriktionsanalyse und DNA-Sequenzierung überprüft. Die DNA-Sequenzierung wurde nach den Vorschriften des Herstellers mit einer Reagenziensammlung der Firma Pharmacia durchgeführt. Bei der Herstellung der Plasmide wurden verschiedene Oligodesoxyribonukleotide (Oligos) eingesetzt, deren Sequenzen zusammen mit den zugehörigen Bezeichnungen in der nachfolgenden Tabelle 1 angegeben sind.

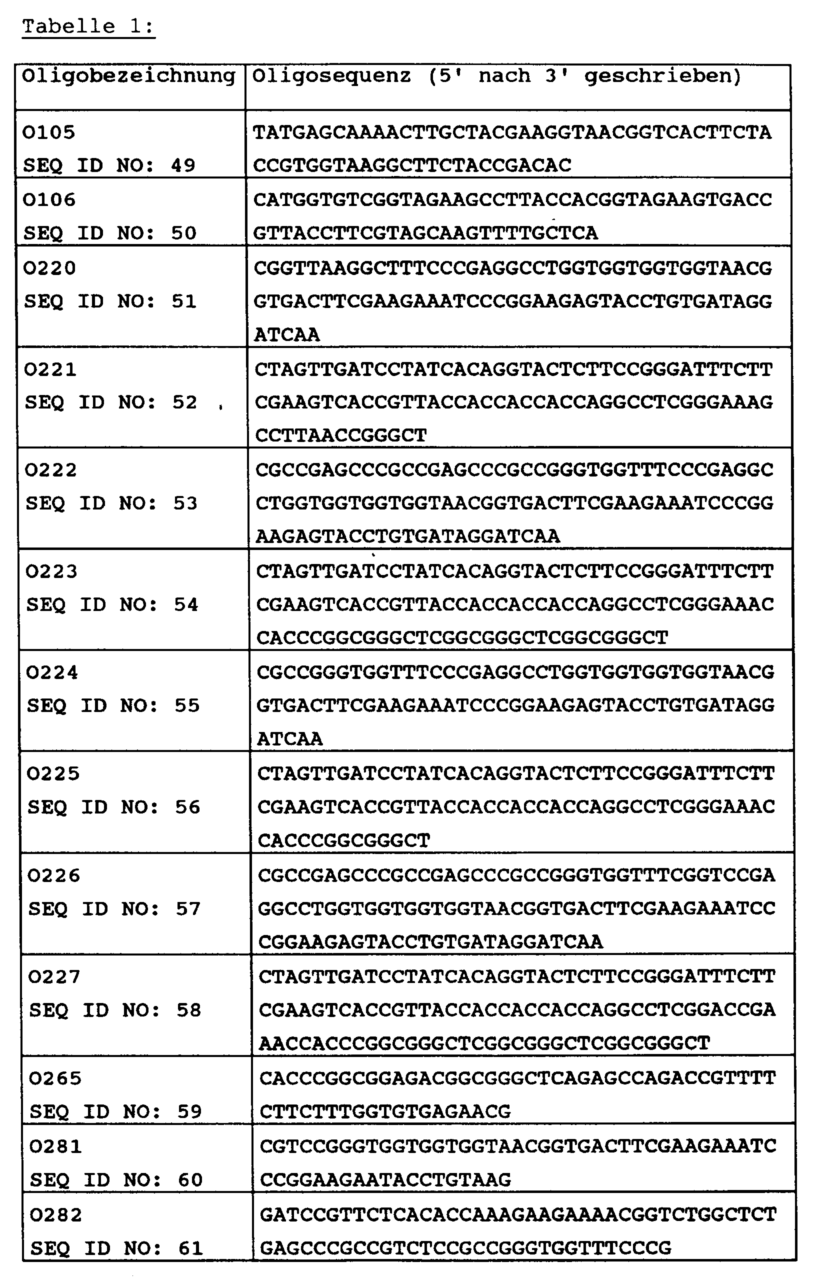

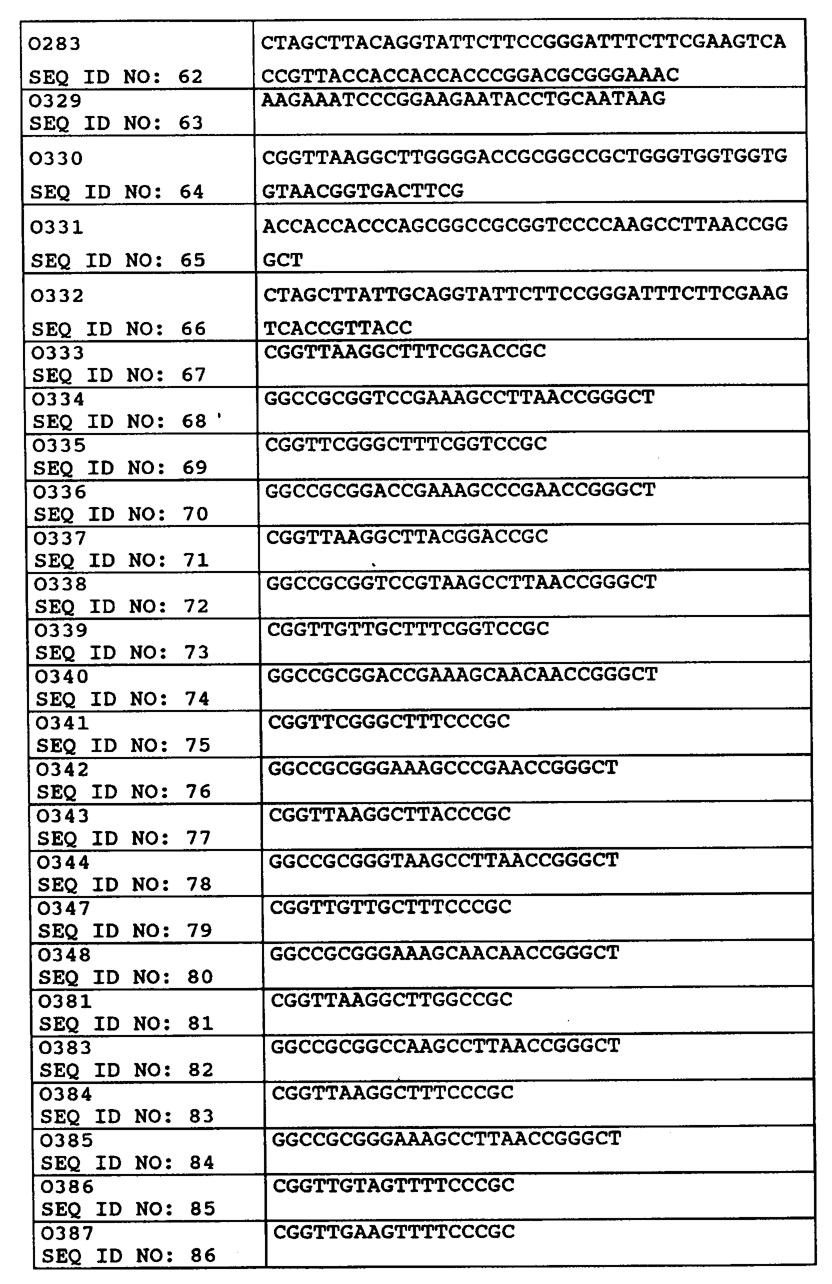

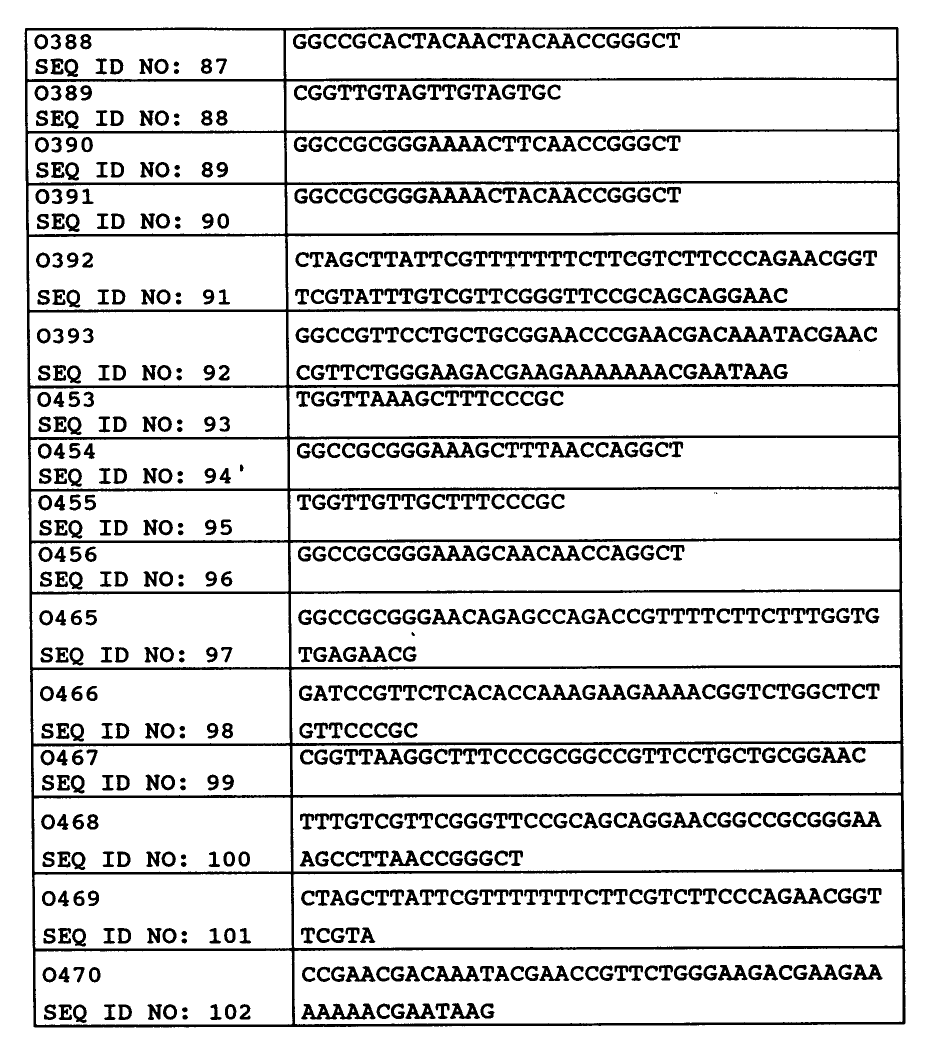

Die Oligodesoxyribonukleotide wurden in detritylierter Form im 0,1 µMol-Maßstab mit einem Synthesizer (Modell 391) der Firma Applied Biosystems (Weiterstadt) nach den Angaben des Herstellers unter Verwendung von b-cyanoethyl-geschützten Diisopropylaminophosphoamiditen gefertigt. Je 100 pmol Oligodesoxyribonukleotid wurden in 50 mM Tri(hydroxymethyl)aminomethan/HCl (Tris/HCl), 10 mM Magnesiumchlorid und 5 mM Dithiothreitol bei einem pH-Wert von 7,5 mit einer Enzymeinheit T4-Kinase in Anwesenheit von 10 mM Adenosintriphosphat phosphoryliert und anschließend im gleichen Puffer zu doppelsträngigen DNA-Molekülen umgeformt. Die erhaltenen synthetischen, doppelsträngigen DNA-Moleküle wurden durch Gelelektrophorese auf einem Polyacrylamidgel (5 % Polyacrylamid) gereinigt und anschließend in die Ligation mit den entsprechend vorbereiteten Plasmiden eingesetzt. Die Vorbereitung der Plasmide durch Verdauung mit Restriktionsenzymen, Isolierung der entsprechenden Restriktionsfragmente und Dephosphorylierung der 5'-Enden, die nachfolgende Ligation und die Transformation in E.coli K12 JM103 sowie alle weiteren gentechnischen Arbeiten wurden in an sich bekannter Weise durchgeführt und sind in Sambrook et al. "Molecular Cloning: A Laboratory Manual", 2. Auflage, Cold Spring Harbor Laboratory Press, Cold Spring Harbour, USA, 1989 angegeben.

### b) Herstellung von Dauerkulturen und Fermentation

Die rekombinanten Expressionsplasmide pSJ69, pSJ76, pSJ77, pSJ78, pSJ79, pSJ81, pSJ83, pSJ90, pSJ91, pSJ92, pSJ93, pSJ94, pSJ95, pSJ101, pSJ102, pSJ103, pSJ104, pSJ105, psJ106, pSJ109, pSJ111, pSJ114 und pSJ113 wurden in E.coli K12 JM103 (ATCC39403) eingebracht und auf Standard-I-Agar (150 mg/l Ampicillin) ausgestrichen (Sambrook et al. "Molecular Cloning: A Laboratory Manual"). Jeweils eine einzelne Kolonie einer jeden Transformation wurde in Standard-I-Medium (pH 7,0; 150 mg/l Ampicillin) bei 20° C bis zur optischen Dichte (OD) von 1 bei 578 nm kultiviert, in 5 Portionen von 2 ml als Dauerkultur unter Zusatz von Dimethylsulfoxid (DMSO) (7,5 % Endkonzentration) in flüssigem Stickstoff tiefgefroren und bei -70° C aufbewahrt. Zur Gewinnung der bifunktionellen Urokinasevarianten wurde jeweils 1 ml einer jeden Dauerkultur in 20 ml Standard-I-Medium (pH 7,0; 150 mg/l Ampicillin) suspendiert und bei 37° C bis zur OD von 1 bei 578 nm kultiviert.

Anschließend wurde die gesamte Menge der erhaltenen Kultur in 1 l Standard-I-Medium (pH 7,0; 150 mg/l Ampicillin) suspendiert und in Schüttelkolben bei 37° C fermentiert. Die Induktion erfolgte durch Zusatz von 2 ml Indolacrylsäurelösung (60 mg in 2 ml Ethanol) bei einer OD von 0,5 bis 1 bei 578 nm.

### c) Expressionstestung

Zur Testung der Expressionsrate (Ploug-Einheiten pro OD pro ml) wurden unmittelbar vor der Induktion und jede Stunde nach der Induktion (insgesamt 6 Stunden) Zellen entsprechend 1 ml einer Zellsuspension mit einer OD von 1 bei 578 nm abzentrifugiert. Die abzentrifugierten Zellen wurden mit Lysozym (1 mg Lysozym pro ml in 50 mM Tris/HCl-Puffer, pH 8,0, 50 mM Ethylendiamintetraessigsäure (EDTA) und 15 Vol-% Saccharose) aufgeschlossen. Die lysierten Zellen wurde in 4 - 5 M Guanidiniumhydrochlorid-lösung solubilisiert und nach Verdünnen auf 1,2 M Guanidiniumhydrochlorid unter Zusatz eines Reduktionsmittels (Glutathion oder Cystein) 2 - 5 Stunden der Rückfaltungsreaktion unterworfen (Winkler et al., Biochemistry 25 4041 bis 4045 (1986)). Die erhaltenen einkettigen bifunktionellen Urokinasevarianten wurden durch Zugabe von Plasmin in die entsprechenden zweikettigen Urokinasevarianten umgewandelt, deren Aktivität mit dem chromogenen Substrat pyro-Glu-Gly-Arg-p-nitroanilid, das nur von zweikettigen aktiven Urokinasen gespalten wird, bestimmt wurde. Die Aktivierung der erfindungsgemäßen bifunktionellen Urokinasevarianten mit Plasmin erfolgte in 50 mM Tris/HCl-Puffer, 12 mM Natriumchlorid, 0,02 % Tween 80 bei pH 7,4 und 37° C. Das Verhältnis bifunktionelle Urokinasevariante zu Plasmin lag bei etwa 100 - 1500 zu 1, bezogen auf Molarität, oder bei etwa 8.000 - 36.000 zu 1, bezogen auf Enzymeinheiten. Die Testinkubation erfolgte in 50 mM Tris/HCl-Puffer und 38 mM Natriumchlorid bei pH 8,8 in Gegenwart von 0,36 µM Aprotinin (zur Hemmung des Plasmins) und 0,27 mM Substrat pyro-Glu-Gly-Arg-p-nitroanilid bei 37° C. In Abhängigkeit von der Konzentration an bibunktioneller Urokinasevariante wurde die Reaktion nach 5 bis 60-minütiger Inkubation durch Zusatz von 50 %-iger Essigsäure gestoppt und die Extinktion bei 405 nm gemessen. Gemäß den Angaben des Herstellers des Substrats (Kabi Vitrum, Schweden) entspricht bei dieser Vorgehensweise eine Extinktionsänderung von 0,05 pro Minute bei 405 nm einer Urokinase-Aktivität von 25 Ploug-Einheiten pro ml Testlösung. Die erfindungsgemäßen bifunktionellen Urokinasevarianten wiesen spezifische Aktivitäten zwischen 120.000 und 155.000 Ploug-Einheiten pro mg gereinigtem Protein auf. Der Proteingehalt der Lösungen wurde mit dem BCA-Assay der Firma Pierce bestimmt.

### d) Isolierung und Reinigung

5 bis 6 Stunden nach der Induktion wurde die unter den in 1b) beschriebenen Bedingungen durchgeführte Fermentation beendet (Dichte 5 - 6 OD bei 578 nm). Die Zellen wurden abzentrifugiert. Der Zellniederschlag wurde in 200 ml Wasser resuspendiert und im Hochdruckhomogenisator aufgeschlossen. Nach erneuter Zentrifugation wurde der Niederschlag, der die gesamte Menge an einkettiger bifunktioneller Urokinasevariante enthielt, in 500 ml 5 M Guanidiniumhydrochlorid, 40 mM Cystein, 1 mM EDTA bei einem pH-Wert von 8,0 gelöst und mit 2000 ml 25 mM Tris/HCl mit einem pH-Wert von 9,0 verdünnt. Die Rückfaltungsreaktion war nach ca. 12 Stunden abgeschlossen.

Die erhaltenen bifunktionellen Urokinasevarianten wurden nach Zusatz von 8 g Kieselgel durch 2-stündiges Rühren vollständig an Kieselgel gebunden. Das beladene Kieselgel wurde abgetrennt und mit Acetat-Puffer (pH 4,0) gewaschen. Die Urokinasevarianten wurden mit 0,5 M Tetramethylammoniumchlorid (TMAC) in 0,1 M Acetat-Puffer (pH 4) eluiert. Nach zwei chromatographischen Trennungen (Kupfer-Chelat-Säule und Kationenaustauscher) wurden die Urokinasevarianten in reiner Form erhalten. Durch N-terminale Sequenzanalyse wurde zum einen die Einkettigkeit und zum anderen die gewünschte amino-terminale Sequenz nachgewiesen. Die proteinchemische Charakterisierung des veränderten carboxy-terminalen Bereiches der einzelnen Varianten wurde nach modifizierter CNBr-Spaltung des Proteins (gelöst in 1 ml 90 % Ameisensäure und 1 ml Heptafluorbuttersäure) durch Spaltung der Peptidkette hinter Tryptophan-Resten erreicht. Das carboxy-terminale Peptid wurde vor der Sequenzanalyse über HPLC (High Pressure Liquid Chromatography) abgetrennt und gereinigt.

Alle isolierten und in Tabelle 2 aufgeführten bifunktionellen Urokinasevarianten zeigten in einem direkten Aktivitätstest mit dem chromogenen Substrat für Urokinase keine oder nur sehr geringe Aktivität (unter 1200 Ploug-Einheiten pro mg gereinigtem Protein). Erst nach Spaltung mit Plasmin (Bedingungen sind in Abschnitt 1c) angegeben) wurde eine Enzymaktivität zwischen 120.000 und 155.000 Ploug-Einheiten pro mg gereinigtem Protein erhalten. Alle Urokinasevarianten wurden demnach in E.coli K12 JM103 als einkettige Proteine exprimiert.

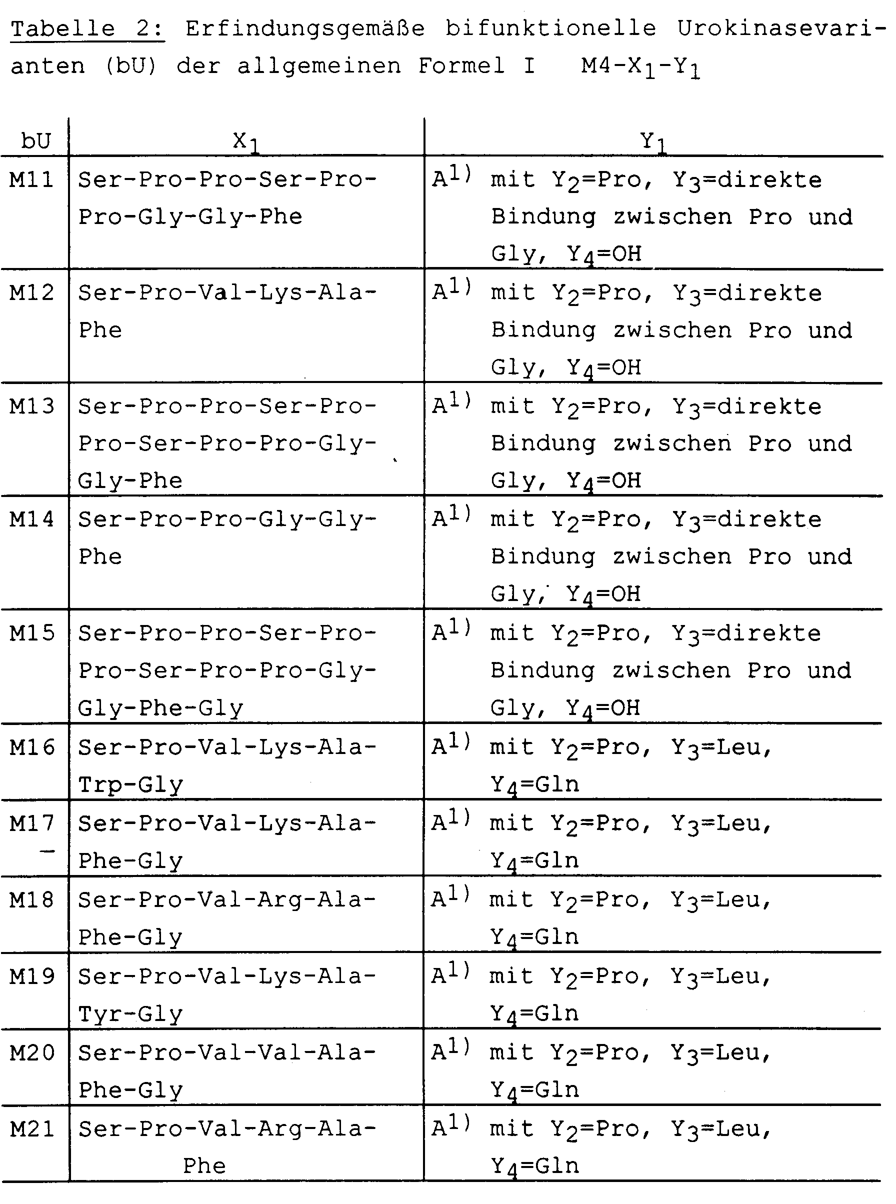

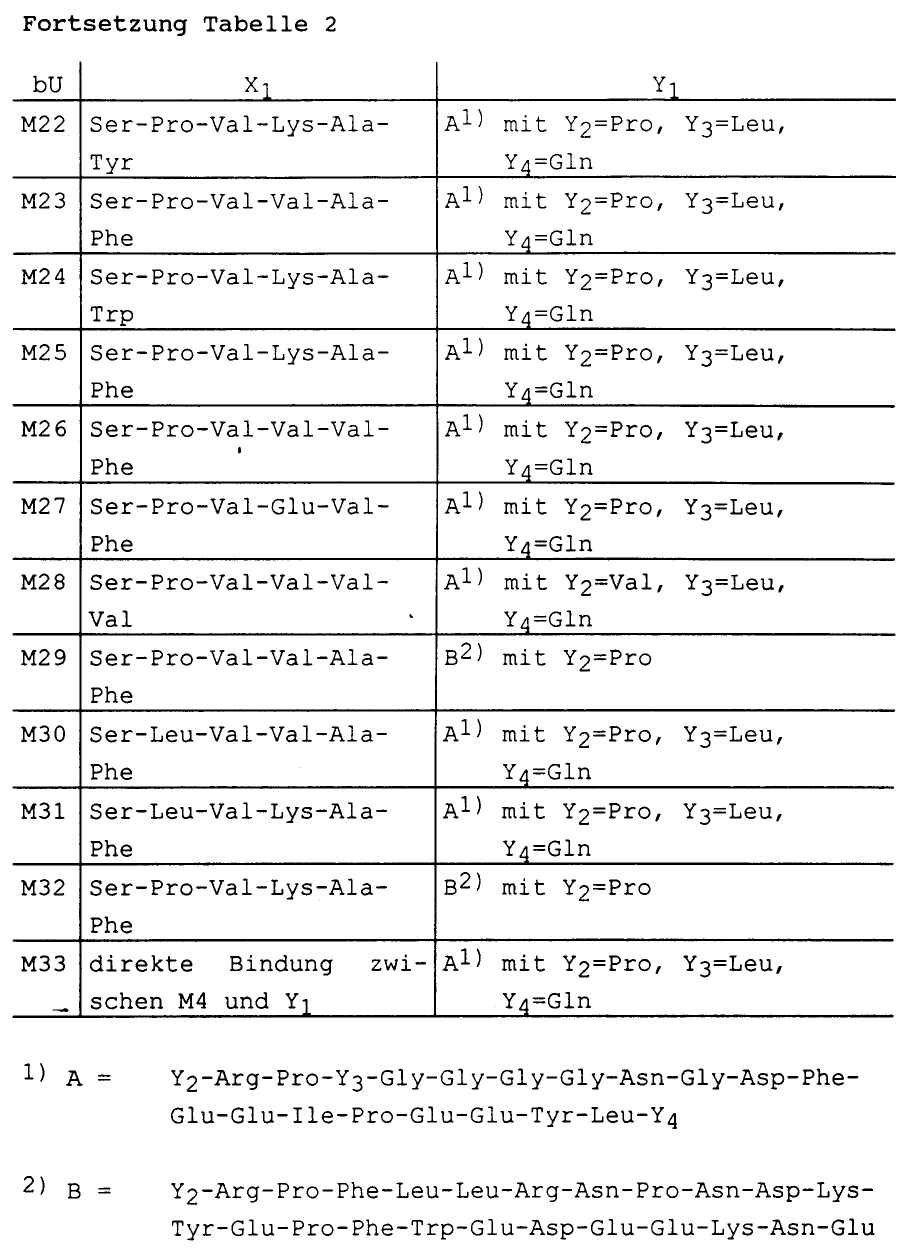

### 2) Pharmakologische Untersuchungen

### Bestimmung der thrombinhemmenden Wirkung

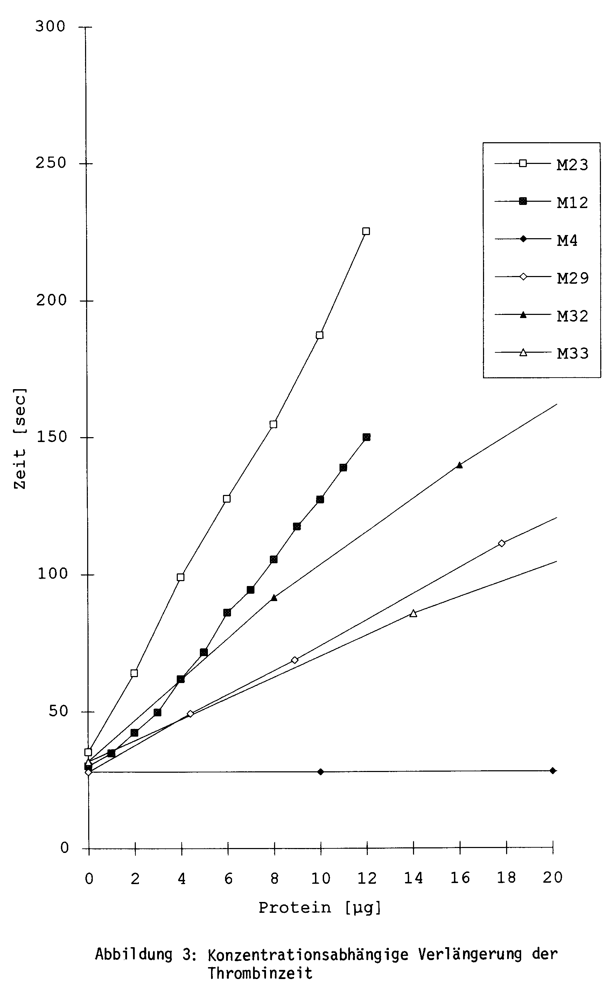

Die Inhibitoraktivität der erfindungsgemäßen bifunktionellen Urokinasevarianten wurde durch Messung der Thrombinzeit bestimmt, indem 200 µl einer 1 : 10 Verdünnung an humanem Citratplasma in Veronalpuffer mit 50 µl Thrombinlösung (0,2 Einheiten) und 50 µl einer wäßrigen Lösung enthaltend 0,5 - 50 µg einer bifunktionellen Urokinasevariante gemischt wurden. Dann wurde die Zeit bis zur Bildung eines Fibringespinnst gemessen. In Tabelle 3 sind die gemessenen Hemmfaktoren aufgeführt, die die Verlängerung der Thrombinzeit in Anwesenheit von jeweils 10 µg einer erfindungsgemäßen bifunktionellen Urokinasevariante angeben. Die konzentrationsabhängige Verlängerung der Thrombinzeit wurde ebenfalls bestimmt und ist für die bifunktionellen Urokinasevarianten M12, M23, M29, M32, M33 und als Vergleich für M4 in Abbildung 3 graphisch dargestellt. Im Gegensatz zu den erfindungsgemäßen bifunktionellen Urokinasevarianten wurde die Zeit bis zur Gerinnung weder durch M4, das heißt für die Aminosäuresequenz ⁴⁷Ser bis ⁴¹¹Leu der unglycosilierten Prourokinase gemäß Abbildung 1, noch durch unglycosilierte Prourokinase (Saruplase), noch durch LUK selbst in einer Dosierung von 1 mg verlängert.

**Tabelle 3**

| Verlängerung der Thrombinzeit durch erfindungsgemäße bifunktionelle Urokinasevarianten der allgemeinen Formel I M4-X₁-Y₁ | |
|---|---|
| bifunktionelle Urokinase-variante | Hemmfaktor¹) |
| M11 (SEQ ID NO: 23 und 24) | 1,8 |
| M12 (SEQ ID NO: 25 und 26) | 4,6 |
| M13 (SEQ ID NO: 27 und 28) | 1,7 |
| M14 (SEQ ID NO: 29 und 30) | 1,8 |
| M15 (SEQ ID NO: 31 und 32) | 2,5 |
| M16 (SEQ ID NO: 33 und 34) | 3,2 |
| M17 (SEQ ID NO: 35 und 36) | 3,1 |
| M18 (SEQ ID NO: 37 und 38) | 2,9 |
| M19 (SEQ ID NO: 39 und 40) | 2,0 |
| M20 (SEQ ID NO: 41 und 42) | 2,2 |
| M21 (SEQ ID NO: 43 und 44) | 2,3 |
| M22 (SEQ ID NO: 45 und 46) | 3,7 |
| M23 (SEQ ID NO: 47 und 48) | 5,3 |
| M24 (SEQ ID NO: 1 und 2) | 6,2 |
| M25 (SEQ ID NO: 3 und 4) | 2,9 |
| M26 (SEQ ID NO: 5 und 6) | 3,2 |
| M27 (SEQ ID NO: 7 und 8) | 2,0 |
| M28 (SEQ ID NO: 9 und 10) | 2,1 |
| M29 (SEQ ID NO: 11 und 12) | 2,6 |
| M30 (SEQ ID NO: 13 und 14) | 3,4 |
| M31 (SEQ ID NO: 15 und 16) | 2,0 |
| M32 (SEQ ID NO: 19 und 20) | 3,0 |
| M33 (SEQ ID NO: 17 und 18) | 2,0 |

| | |
|---|---|
| ¹⁾ bezogen auf die Wirkung von 10 µg Protein Hemmfaktor = Quotient aus der Thrombinzeit in Anwesenheit eines Inhibitors und der Thrombinzeit in Abwesenheit eines Inhibitors | |

### Pharmakologische Eigenschaften der bifunktionellen Urokinasevarianten M12 und M23 im Tierexperiment

In einem pharmakologischen in-vivo-Modell wurde die Wirkung der bifunktionellen Urokinasevarianten M12 und M23 auf die Thrombolyse arterieller Gefäßverschlüsse vergleichend zu Saruplase (unglycosilierte Prourokinase) getestet. Dazu wurden an narkotisierten Kaninchen in ein temporär isoliertes ca. 1 cm langes Segment der Femoralarterie lokal über einen Seitenast Thrombin und mit ¹²⁵J-markiertes Humanfibrinogen injiziert. Hierdurch bildete sich ein Thrombus, der zu einem kompletten Gefäßverschluß führte. Die Größe des gebildeten Thrombus wurde durch die inkorporierte Radioaktivität des Humanfibrins über einen extrakorporalen gamma-Detektor bestimmt. Die elektromagnetische Messung der Blutdurchströmung und die Erfassung der Thrombusradioaktivität erfolgten kontinuierlich über den gesamten Versuchszeitraum. Die fibrinolytische Wirkung wurde somit sowohl als Reperfusion des thrombotisch verschlossenen Gefäßes als auch über den Abbau des radioaktiv markierten inkorporierten Fibrins des Thrombus quantifiziert. Vor Applikation der erfindungsgemäßen bifunktionellen Urokinasevarianten sowie 30, 60 und 90 Minuten nach Applikation der Urokinasevarianten wurden Blutproben entnommen, in denen die Plasmakonzentrationen an Fibrinogen bestimmt wurden. Je 6 mg/kg M12, M23 und Saruplase wurden als intravenöse Bolusinjektionen appliziert. Da M12 und M23 im Gegensatz zu Saruplase eine zusätzliche antikoagulatorische Wirkung besitzen, wurde in einer 4. Versuchsgruppe Saruplase mit dem Antikoagulans Heparin (150 U/kg i.v.-Bolus) kombiniert. Die Gruppengröße betrug jeweils 6 Tiere.

Während der 90-minütigen Versuchsdauer betrug die Thrombolyse des markierten Thrombusfibrins 46 ± 11 % für M12, 43 ± 12 % für M23, 22 ± 5 % für Saruplase und 39 ± 15 % für die Saruplase-Heparin-Kombination. Bolusapplikationen von M12 und M23 führte bei allen 6 Tieren zur Eröffnung des thrombotisch verschlossenen Gefäßes; durch die Applikation von Saruplase konnte bei 5 von 6 Tieren das Gefäß und durch die Applikation von Saruplase und Heparin bei 4 von 6 Tieren das Gefäß reperfundiert werden. Die maximale Höhe des Reperfusionsflusses (in % des Ausgangswertes) betrug 95 ± 10 % für M12 und 82 ± 9 % für M23 und unterschied sich signifikant von der maximalen Höhe des Reperfusionsflusses von 43 ± 12 % für Saruplase. Die maximale Höhe des Reperfusionsflusses von 58 ± 8 % bei der Behandlung mit Saruplase und Heparin lag zwischen den Ergebnissen von M12 und M23 einerseits und Saruplase andererseits und ist nach beiden Seiten nicht signifikant verschieden. Die gesamte fibrinolytische Wirkung wurde als Fläche des Reperfusionsflusses (als % des Ausgangsflusses) über die 90-minütige Versuchsdauer ermittelt. Dieser Gesamteffekt betrug 4.502 ± 1.127 % · min für M12 und 4.270 ± 885 % · min für M23 und war für beide erfindungsgemäßen Urokinasevarianten signifikant größer als der Wert von 1.519 ± 643 % · min für Saruplase. Mit der kombinierten Saruplase-Heparin-Behandlung wurde ein Gesamteffekt von 2.217 ± 761 % · min gemessen, der nicht signifikant besser war als bei alleiniger Saruplasebehandlung und deutlich unter den Ergebnissen von M12 und M23 lag. Die Ergebnisse sind in Tabelle 4 zusammengefaßt.

**Tabelle 4**

| Thrombolytische Wirkung nach i.v.-Bolusapplikation; Femoralarterien-Thrombose, narkotisiertes Kaninchen. | | | | |
|---|---|---|---|---|
| Polypeptid | Dosis | % ¹²⁵J-Fibrinolyse | Maximaler Reperfusionsfluß (% des Vorwertes) | Kumulativer Reperfusionsfluß (% · min) |
| M12 | 6 mg/kg | 46 ± 11 | 95 ± 10* | 4502 ± 1127 |
| M23 | 6 mg/kg | 43 ± 12 | 82 ± 9* | 4270 ± 885* |
| Saruplase | 6 mg/kg | 22 ± 5 | 43 ± 12 | 1519 ± 643 |
| Saruplase + Heparin | 6 mg/kg 150 U/kg | 39 ± 15 | 58 ± 8 | 2217 ± 761 |

| | | | | |
|---|---|---|---|---|
| * p <0.05 vs Saruplase | | | | |

Überraschenderweise wurde gefunden, daß sowohl nach der Bolusapplikation von M12 als auch nach der Bolusapplikation von M23 die Plasmakonzentrationen an Fibrinogen signifikant weniger abfielen als nach Bolusapplikation von Saruplase. Die Ergebnisse sind in Tabelle 5 zusammengefaßt.

**Tabelle 5**

| Wirkung der Bolusapplikationen von M12 und M23 im Vergleich zu Saruplase, ohne und mit Heparin, auf die Abnahme der Plasma-Fibrinogenkonzentration; narkotisierte Kaninchen | | | | |
|---|---|---|---|---|
| Polypeptid | Dosis | Abnahme an Plasma-Fibrinogen (%-Änderung gegenüber Ausgangswert) | | |
| | | Zeit nach Applikation | | |
| | | 30 min | 60 min | 90 min |
| M12 | 6 mg/kg | -19 ± 9 | -20 ± 9* | -19 ± 9* |
| M23 | 6 mg/kg | -20 ± 11* | -21 ± 11* | -20 ± 11* |
| Saruplase | 6 mg/kg | -64 ± 7 | -66 ± 6 | -67 ± 6 |
| Saruplase + Heparin | 6 mg/kg + 150 U/kg | n.b.¹⁾ | -46 ± 8 | -45 ± 9 |

| | | | | |
|---|---|---|---|---|
| * p<0.05 vs Saruplase | | | | |
| ¹⁾ n.b. = nicht bestimmt | | | | |

Die Ergebnisse zeigen, daß die erfindungsgemäßen bifunktionellen Urokinasevarianten M12 und M23 arterielle Thromben, die einen kompletten Gefäßverschluß bewirken, auflösen und die Durchblutung der thrombosierten Gefäße wieder herstellen. Diese Wirkung wurde durch einmalige Bolusapplikation von M12 bzw. M23 an nicht-heparinisierten Tieren erzielt. Überraschenderweise war diese im Vergleich zu Saruplase stärkere fibrinolytische Wirkung von M12 und M23 mit einem geringeren Verbrauch an Plasmafibrinogen verbunden. Das bedeutet, daß M12 und M23 eine signifikant höhere Fibrinspezifität im Vergleich zu Saruplase aufweisen.

Die bessere Schonung von Plasmafibrinogen durch M12 und M23 im Vergleich zu Saruplase bedeutet, daß die Gerinnungsfähigkeit des Blutes besser erhalten bleibt und damit die Gefahr von unkontrollierten Blutungen als mögliche Komplikation eines systemischen Fibrinogenabbaus herabgesetzt ist. M12 und M23 sind in Bezug auf das Risiko hämostaseologischer Nebenwirkungen somit als sicherer einzustufen als Saruplase.

## Patentansprüche

1. Bifunktionelle Urokinasevarianten der allgemeinen Formel I
M4-X₁-Y₁
worin
M4 die Aminosäuresequenz ⁴⁷Ser bis ⁴¹¹Leu der unglycosilierten Prourokinase gemäß Abbildung 1 (SEQ ID NO: 21 und 22) bedeutet,
X₁ eine direkte Bindung zwischen M4 und Y₁ ist oder
ein Peptid der Sequenz
Ser-Pro-Pro-Ser-Pro-Pro-Gly-Gly-Phe
oder
Ser-Pro-Pro-Ser-Pro-Pro-Ser-Pro-Pro-Gly-Gly-Phe
oder
Ser-Pro-Pro-Ser-Pro-Pro-Ser-Pro-Pro-Gly-Gly-Phe-Gly
oder
eine Peptidsequenz der allgemeinen Formel II
Ser-X₂-X₃-X₄-X₅-X₆-X₇
darstellt, wobei X₂ Pro oder Leu, X₃ Val oder Pro, X₄ Lys, Val, Arg, Gly oder Glu, X₅ Ala, Val, Gly, Leu oder Ile, X₆ Phe, Trp, Tyr oder Val und X₇ Gly oder eine direkte Bindung zwischen X₆ und Y₁ bedeuten,
und
Y₁ ein Peptid der Sequenz
Y₂-Arg-Pro-Y₃-Gly-Gly-Gly-Gly-Asn-Gly-Asp-Phe-Glu-Glu-Ile-Pro-Glu-Glu-Tyr-Leu-Y₄
oder
Y₂-Arg-Pro-Phe-Leu-Leu-Arg-Asn-Pro-Asn-Asp-Lys-Tyr-Glu-Pro-Phe-Trp-Glu-Asp-Glu-Glu-Lys-Asn-Glu
oder
Y₂-Arg-Pro-Ser-Ser-Glu-Phe-Glu-Glu-Phe-Glu-Ile-Asp-Glu-Glu-Glu-Lys
darstellt mit Y₂ Pro oder Val, Y₃ Leu oder eine direkte Bindung zwischen Pro und Gly und Y₄ Gln oder eine Hydroxylgruppe.

2. Urokinasevarianten nach Anspruch 1, dadurch gekennzeichnet, daß Y₁ ein Peptid der Sequenz
Y₂-Arg-Pro-Y₃-Gly-Gly-Gly-Gly-Asn-Gly-Asp-Phe-Glu-Glu-Ile-Pro-Glu-Glu-Tyr-Leu-Y₄
bedeutet.

3. Urokinasevarianten nach Anspruch 1, dadurch gekennzeichnet, daß Y₁ ein Peptid der Sequenz
Y₂-Arg-Pro-Phe-Leu-Leu-Arg-Asn-Pro-Asn-Asp-Lys-Tyr-Glu-Pro-Phe-Trp-Glu-Asp-Glu-Glu-Lys-Asn-Glu
bedeutet.

4. Urokinasevarianten nach Anspruch 1 und/oder Anspruch 2, dadurch gekennzeichnet, daß X₁ eine Peptidsequenz der allgemeinen Formel II darstellt, in der X₂ Pro oder Leu, X₃ Val, X₄ Lys, Val oder Arg, X₅ Ala, Val oder Gly, X₆ Phe, Trp, Tyr oder Val und X₇ Gly oder eine direkte Bindung zwischen X₆ und Y₁ bedeuten.

5. Urokinasevarianten nach Anspruch 4, dadurch gekennzeichnet, daß X₄ Lys oder Val, X₅ Ala oder Val, X₆ Phe Trp oder Tyr und X₇ Gly oder eine direkte Bindung zwischen X₆ und Y₁ bedeuten.

6. Urokinasevarianten nach Anspruch 4 und/oder Anspruch 5, dadurch gekennzeichnet, daß X₇ eine direkte Bindung zwischen X₆ und Y₁ ist.

7. Urokinasevarianten nach Anspruch 1 und/oder Anspruch 3, dadurch gekennzeichnet, daß X₁ eine Peptidsequenz der allgemeinen Formel II darstellt, in der X₂ Pro oder Leu, X₃ Val, X₄ Lys oder Val, X₅ Ala oder Val, X₆ Phe oder Trp und X₇ eine direkte Bindung zwischen X₆ und Y₁ bedeuten.

8. Plasmide zur Verwendung bei der Gewinnung einer bifunktionellen Urokinasevariante gemäß den Ansprüchen 1 bis 7, dadurch gekennzeichnet, daß das Operon einen regulierbaren Promotor, eine als Ribosomenbindestelle wirksame Shine-Dalgarno-Sequenz, ein Startcodon, ein synthetisches Strukturgen für eine bifunktionelle Urokinasevariante der allgemeinen Formel I gemäß den Ansprüchen 1 bis 7 und stromabwärts vom Strukturgen 1 oder 2 Terminatoren aufweist und daß die Plasmide zur Expression der bifunktionellen Urokinasevariante in Stämmen von Escherichia coli geeignet sind.

9. Plasmide gemäß Anspruch 8, dadurch gekennzeichnet, daß der Abstand zwischen der Shine-Dalgarno-Sequenz und dem Startcodon 6 bis 12, vorzugsweise 8 bis 10 Nukleotide beträgt.

10. Plasmide nach Anspruch 8 und/oder Anspruch 9, ausgewählt aus der Gruppe pSJ 69, pSJ 76, pSJ 77, pSJ 78, pSJ 79, pSJ 81, pSJ 83, pSJ 90, pSJ 91, pSJ 92, pSJ 93, pSJ 94, pSJ 95, pSJ 101, pSJ 102, pSJ 103, pSJ 104, pSJ 105, pSJ 106, pSJ 109, pSJ 111, pSJ114 und pSJ113.

11. Plasmide nach Anspruch 10, ausgewählt aus der Gruppe pSJ 76, pSJ 81, pSJ 83, pSJ 90, pSJ 91, pSJ 92, pSJ 93, pSJ 94, pSJ 95, pSJ 101, pSJ 102, pSJ 103, pSJ 105, pSJ 106, pSJ 109, pSJ 111 und pSJ 114.

12. Plasmide nach Anspruch 10 und/oder Anspruch 11, ausgewählt aus der Gruppe pSJ 76, pSJ 81, pSJ 83, pSJ 91, pSJ 92, pSJ 94, pSJ 95, pSJ 101, pSJ 102, pSJ 103, pSJ 106, pSJ 109, pSJ 111 und pSJ 114.

13. Plasmide nach einem oder mehreren der Ansprüche 10 bis 12, ausgewählt aus der Gruppe pSJ 76, pSJ 94, pSJ 95, pSJ 101, pSJ 102, pSJ 103, pSJ 106, pSJ 109, pSJ 111 und pSJ 114.

14. Verfahren zur Herstellung von Plasmiden gemäß den Ansprüchen 8 bis 13, dadurch gekennzeichnet, daß man diese aus den Plasmiden pBlueskript KS II+, pUC 8 und pGR 201 gemäß Abbildungen 2 und 2a bis 2p gewinnt.

15. Verwendung eines Plasmids nach den Ansprüchen 8 bis 13 bei der Gewinnung einer bifunktionellen Urokinasevariante der allgemeinen Formel I gemäß den Ansprüchen 1 bis 7, dadurch gekennzeichnet, daß man mit einem Plasmid einen Escherichia coli-Stamm in an sich bekannter Weise transformiert, die Expression des Strukturgens induziert, das gebildete Vorstufenprotein der bifunktionellen Urokinasevariante der allgemeinen Formel I vom Medium und den lysierten Bakterienzellen abtrennt, das Vorstufenprotein solubilisiert und dann durch Einwirkung eines Redoxsystems zum Polypeptid der allgemeinen Formel I rückfaltet.

16. Thrombolytikum, das als Wirkstoff eine bifunktionelle Urokinasevariante der allgemeinen Formel I gemäß den Ansprüchen 1 bis 7 enthält.

17. Thrombolytikum nach Anspruch 16, dadurch gekennzeichnet, daß es zur Bolusapplikation geeignet ist.

## Claims

1. Bifunctional urokinase variants of the general formula I
M4-X₁-Y₁
in which
M4 means the amino acid sequence ⁴⁷Ser to ⁴¹¹Leu of the nonglycosylated prourokinase according to Figure 1 (SEQ ID No.: 21 and 22),
X₁ is a direct bond between M4 and Y₁ or represents
a peptide having the sequence
Ser-Pro-Pro-Ser-Pro-Pro-Gly-Gly-Phe
or
Ser-Pro-Pro-Ser-Pro-Pro-Ser-Pro-Pro-Gly-Gly-Phe
or
Ser-Pro-Pro-Ser-Pro-Pro-Ser-Pro-Pro-Gly-Gly-Phe-Gly
or
a peptide sequence of the general formula II
Ser-X₂-X₃-X₄-X₅-X₆-X₇
wherein X₂ means Pro or Leu, X₃ means Val or Pro, X₄ means Lys, Val, Arg, Gly or Glu, X₅ means Ala, Val, Gly, Leu or Lle, X₆ means Phe, Trp, Tyr or Val and X₇ means Gly or a direct bond between X₆ and Y₁
and
Y₁ represents a peptide having the sequence
Y₂-Arg-Pro-Y₃-Gly-Gly-Gly-Gly-Asn-Gly-Asp-Phe-Glu-Glu-Ile-Pro-Glu-Glu-Tyr-Leu-Y₄
or
Y₂-Arg-Pro-Phe-Leu-Leu-Arg-Asn-Pro-Asn-Asp-Lys-Tyr-Glu-Pro-Phe-Trp-Glu-Asp-Glu-Glu-Lys-Asn-Glu
or
Y₂-Arg-Pro-Ser-Ser-Glu-Phe-Glu-Glu-Phe-Glu-Ile-Asp-Glu-Glu-Glu-Lys
with Y₂ being Pro or Val, Y₃ Leu or a direct bond between Pro and Gly and Y₄ Gln or a hydroxyl group.

2. Urokinase variants according to claim 1, characterised in that Y₁ means a peptide having the sequence
Y₂-Arg-Pro-Y₃-Gly-Gly-Gly-Gly-Asn-Gly-Asp-Phe-Glu-Glu-Ile-Pro-Glu-Glu-Tyr-Leu-Y₄.

3. Urokinase variants according to claim 1, characterised in that Y₁ means a peptide having the sequence
Y₂-Arg-Pro-Phe-Leu-Leu-Arg-Asn-Pro-Asn-Asp-Lys-Tyr-Glu-Pro-Phe-Trp-Glu-Asp-Glu-Glu-Lys-Asn-Glu.

4. Urokinase variants according to claim 1 and/or claim 2, characterised in that X₁ represents a peptide sequence of the general formula II, in which X₂ means Pro or Leu, X₃ means Val, X₄ means Lys, Val or Arg, X₅ means Ala, Val or Gly, X₆ means Phe, Trp, Tyr or Val and X₇ means Gly or a direct bond between X₆ and Y₁.

5. Urokinase variants according to claim 4, characterised in that X₄ means Lys or Val, X₅ means Ala or Val, X₆ means Phe, Trp or Tyr and X₇ means Gly or a direct bond between X₆ and Y₁.

6. Urokinase variants according to claim 4 and/or claim 5, characterised in that X₇ is a direct bond between X₆ and Y₁.

7. Urokinase variants according to claim 1 and/or claim 3, characterised in that X₁ represents a peptide sequence of the general formula II, in which X₂ means Pro or Leu, X₃ means Val, X₄ means Lys or Val, X₅ means Ala or Val, X₆ means Phe or Trp and X₇ means a direct bond between X₆ and Y₁.

8. Plasmids for use in obtaining a bifunctional urokinase variant according to claims 1 to 7 characterised in that the operon has a controllable promoter, a Shine-Dalgarno sequence effective as a ribosome binding site, a start codon, a synthetic structural gene for a bifunctional urokinase variant of the general formula I according to claims 1 to 7 and, downstream from the structural gene, one or two terminators and that the plasmids are suitable for expressing the bifunctional urokinase variant in strains of *Escherichia coli*.

9. Plasmids according to claim 8, characterised in that the distance between the Shine-Dalgarno sequence and the start codon is 6-12, preferably 8-10, nucleotides.

10. Plasmids according to claim 8 and/or claim 9, selected from the group pSJ 69, pSJ 76, PSJ 77, pSJ 78, pSJ 79, pSJ 81, PSJ 83, pSJ 90, pSJ 91, pSJ 92, pSJ 93, pSJ 94, pSJ 95, pSJ 101, pSJ 102, pSJ 103, PSJ 104, pSJ 105, pSJ 106, pSJ 109, pSJ 111, pSJ 114 and pSJ 113.

11. Plasmids according to claim 10, selected from the group pSJ 76, pSJ 81, pSJ 83, pSJ 90, pSJ 91, pSJ 92, pSJ 93, pSJ 94, pSJ 95, pSJ 101, pSJ 102, pSJ 103, pSJ 105, pSJ 106, pSJ 109, pSJ 111 and pSJ 114.

12. Plasmids according to claim 10 and/or claim 11, selected from the group pSJ 76, pSJ 81, pSJ 83, pSJ 91, pSJ 92, pSJ 94, pSJ 95, pSJ 101, pSJ 102, pSJ 103, pSJ 106, pSJ 109, pSJ 111 and pSJ 114.

13. Plasmids according to one or more of claims 10 to 12, selected from the group pSJ 76, pSJ 94, pSJ 95, pSJ 101, pSJ 102, pSJ 103, pSJ 106, pSJ 109, pSJ 111 and pSJ 114.

14. Process for the production of plasmids according to claims 8 to 13, characterised in that they are obtained from the plasmids pBlueskript KS II+, pUC 8 and pGR 201 according to Figures 2 and 2a to 2p.

15. Use of a plasmid according to claims 8 to 13 in obtaining a bifunctional urokinase variant of the general formula I according to claims 1 to 7, characterised in that an *Escherichia coli* strain is transformed with a plasmid in a manner known *per se*, expression of the structural gene is induced, the resultant precursor protein of the bifunctional urokinase variant of the general formula I is separated from the medium and the lysed bacterial cells, the precursor protein is solubilised and then refolded under the action of a redox system to yield the polypeptide of the general formula I.

16. Thrombolytic which contains a bifunctional urokinase variant of the general formula I according to claims 1 to 7 as the active substance.

17. Thrombolytic according to claim 16, characterised in that it is suitable for bolus administration.

## Revendications

1. Variantes bifonctionnelles de l'urokinase, de formule générale I :
M4-X₁-Y₁
dans laquelle
M4 représente la séquence d'amino-acide de ⁴⁷Ser à ⁴¹¹Leu de pro-urokinase non glycosylée selon la figure 1 (SEQ ID NO : 21 et 22),
X₁ est une liaison directe entre M4 et Y₁, ou représente
un peptide de séquence
Ser-Pro-Pro-Ser-Pro-Pro-Gly-Gly-Phe
ou
Ser-Pro-Pro-Ser-Pro-Pro-Ser-Pro-Pro-Gly-Gly-phe
ou
Ser-Pro-Pro-Ser-Pro-Pro-Ser-Pro-Pro-Gly-Gly-Phe-Gly
ou
représente une séquence peptidique de formule générale II
Ser-X₂-X₃-X₄-X₅-X₆-X₇
dans laquelle X₂ représente Pro ou Leu, X₃ représente Val ou Pro, X₄ représente Lys, Val, Arg, Gly ou Glu, X₅ représente Ala, Val, Gly, Leu ou Ile, X₆ représente Phe, Trp, Tyr ou Val et X₇ représente Gly ou une liaison directe entre X₆ et Y₁,
et
Y₁ représente un peptide de séquence
Y₂-Arg-Pro-Y₃-Gly-Gly-Gly-Gly-Asn-Gly-Asp-Phe-Glu-Glu-Ile-Pro-Glu-Glu-Tyr-Leu-Y₄
ou
Y₂-Arg-Pro-Phe-Leu-Leu-Arg-Asn-Pro-Asn-Asp-Lys-Tyr-Glu-Pro-Phe-Trp-Glu-Asp-Glu-Glu-Lys-Asn-Glu
ou
Y₂-Arg-Pro-Ser-Ser-Glu-Phe-Glu-Glu-Phe-Glu-Ile-Asp-Glu-Glu-Glu-Lys
où Y₂ représente Pro ou Val, Y₃ représente Leu ou une liaison directe entre Pro et Gly, et Y₄ représente Gln ou un groupe hydroxyle.

2. Variantes d'urokinase suivant la revendication 1, caractérisées en ce que Y₁ désigne un peptide de séquence :
Y₂-Arg-Pro-Y₃-Gly-Gly-Gly-Gly-Asn-Gly-Asp-Phe-Glu-Glu-Ile-Pro-Glu-Glu-Tyr-Leu-Y₄.

3. Variantes d'urokinase suivant la revendication 1, caractérisées en ce que Y₁ désigne un peptide de séquence :
Y₂-Arg-Pro-Phe-Leu-Leu-Arg-Asn-Pro-Asn-Asp-Lys-Tyr-Glu-Pro-Phe-Trp-Glu-Asp-Glu-Glu-Lys-Asn-Glu.

4. Variantes d'urokinase suivant la revendication 1 et/ou la revendication 2, caractérisées en ce que X₁ représente une séquence peptidique de formule générale II dans laquelle X₂ représente Pro ou Leu, X₃ représente Val, X₄ représente Lys, Val ou Arg, X₅ représente Ala, Val ou Gly, X₆ représente Phe, Trp, Tyr ou Val et X₇ représente Gly ou une liaison directe entre X₆ et Y₁.

5. Variantes d'urokinase suivant la revendication 4, caractérisées en ce que X₄ représente Lys ou Val, X₅ représente Ala ou Val, X₆ représente Phe, Trp ou Tyr, et X₇ représente Gly ou une liaison directe entre X₆ et Y₁.

6. Variantes d'urokinase suivant la revendication 4 et/ou la revendication 5, caractérisées en ce que X₇ est une liaison directe entre X₆ et Y₁.

7. Variantes d'urokinase suivant la revendication 1 et/ou la revendication 3, caractérisées en ce que X₁ est une séquence peptidique de formule générale II dans laquelle X₂ représente Pro ou Leu, X₃ représente Val, X₄ représente Lys ou Val, X₅ représente Ala ou Val, X₆ représente Phe ou Trp, et X₇ représente une liaison directe entre X₆ et Y₁.

8. Plasmides destinés à être utilisés pour l'obtention d'une variante bifonctionnelle d'urokinase suivant les revendications 1 à 7, caractérisés en ce que l'opéron présente un promoteur susceptible de régulation, une séquence de Shine-Dalgarno agissant comme site de liaison de ribosomes, un codon initiateur, un gène de structure synthétique pour une variante bifonctionnelle d'urokinase de formule générale I suivant les revendications 1 à 7, et en aval du gène de structure 1, un ou deux terminateurs, et en ce que les plasmides conviennent pour l'expression de la variante bifonctionnelle d'urokinase dans des souches d'*Escherichia coli*.

9. Plasmides suivant la revendication 8, caractérisés en ce que la distance entre la séquence de Shine-Dalgarno et le codon initiateur s'élève à 6-12, de préférence à 8-10 nucléotides.

10. Plasmides suivant la revendication 8 et/ou la revendication 9, choisis dans le groupe consistant en pSJ69, pSJ76, pSJ77, pSJ78, pSJ79, pSJ81, pSJ83, pSJ90, pSJ91, pSJ92, pSJ93, pSJ94, pSJ95, pSJ101, pSJ102, pSJ103, pSJ104, pSJ105, pSJ106, pSJ109, pSJ111, pSJ114 et pSJ113.

11. Plasmides suivant la revendication 10, choisis dans le groupe consistant en pSJ76, pSJ81, pSJ83, pSJ90, pSJ91, pSJ92, pSJ93, pSJ94, pSJ95, pSJ101, pSJ102, pSJ103, pSJ105, pSJ106, pSJ109, pSJ111 et pSJ114.

12. Plasmides suivant la revendication 10 et/ou la revendication 11, choisis dans le groupe consistant en pSJ76, pSJ81, pSJ83, pSJ91, pSJ92, pSJ94, pSJ95, pSJ101, pSJ102, pSJ103, pSJ106, pSJ109, pSJ111 et pSJ114.

13. Plasmides suivant une ou plusieurs des revendications 10 à 12, choisis dans le groupe consistant en pSJ76, pSJ94, pSJ95, pSJ101, pSJ102, pSJ103, pSJ106, pSJ109, pSJ111 et pSJ114.

14. Procédé de préparation de plasmides suivant les revendications 8 à 13, caractérisé en ce que l'on obtient ces plasmides à partir des plasmides pBlueskript KS II+, pUC 8 et pGR 201 suivant les figures 2 et 2a à 2p.

15. Utilisation d'un plasmide suivant les revendications 8 à 13 pour l'obtention d'une variante bifonctionnelle d'urokinase de formule générale I suivant les revendications 1 à 7, caractérisée en ce que l'on transforme avec un plasmide une souche d*'Escherichia coli* d'une manière connue, on induit l'expression du gène de structure, on sépare la protéine formée précurseur de la variante bifonctionnelle d'urokinase de formule générale I du milieu et des cellules bactériennes lysées, on solubilise la protéine précurseur, puis on la replie par l'action d'un système d'oxydo-réduction en le polypeptide de formule générale I.

16. Thrombolytique qui contient comme substance active une variante bifonctionnelle d'urokinase de formule générale I suivant les revendications 1 à 7.

17. Thrombolytique suivant la revendication 16, caractérisé en ce qu'il convient pour l'administration d'un bol.
